# EUROPEAN PATENT APPLICATION

(11) **EP 4 635 944 A1**
(43) Date of publication of application: **22.10.2025**
(21) Application number: 23903586.8
(22) Date of filing: 15.12.2023
(51) Int. Cl.: C07D 221/22, A61K 31/439, A61K 31/444, A61P 25/00, A61P 43/00, C07D 401/06, C07D 401/10, C07D 405/10, C07D 409/10, C07D 413/06

(54) **2-AZABICYCLO[3.1.1]HEPTANE COMPOUND**

(30) Priority: 16.12.2022 JP 2022201488
(71) Applicant: Daiichi Sankyo Company, Limited, Tokyo 103-8426 (JP)
(72) Inventor: YAMAGUCHI, Akitake, Tokyo 103-8426 (JP); TAKEMOTO, Naohiro, Tokyo 103-8426 (JP); MATSUSHITA, Kaoru, Tokyo 103-8426 (JP); KOBAYASHI, Jun, Tokyo 103-8426 (JP); INUI, Masaharu, Tokyo 103-8426 (JP); INOUE, Masahiro, Tokyo 103-8426 (JP); MAEHARA, Shunsuke, Tokyo 103-8426 (JP); ABE, Koji, Tokyo 103-8426 (JP)
(74) Representative: Marks & Clerk LLP
(86) International application number: PCT/JP2023/044987
(87) International publication number: WO 2024/128305

(57) **Abstract**

An object of the present invention is to provide a novel compound that acts as an agonist for orexin type 2 receptors and can be used in treatment of diseases related to orexin type 2 receptors, in particular, narcolepsy. A compound represented by a general formula (I) or pharmaceutically acceptable salt thereof. (Here, in the formula (I), R¹, R², R³, R⁴, R⁵, R⁶, and Z each have the same meaning as defined in the specification)

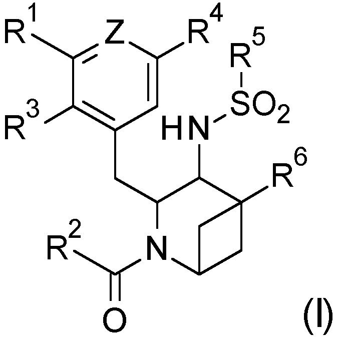

## Description

### Technical Field

The present invention relates to a compound having a specific chemical structure that can be used as an orexin type 2 receptor agonist, or pharmaceutically acceptable salt thereof.

### Background Art

Orexin is a neuropeptide specifically produced in certain nerve cells localized in the lateral hypothalamic area of the brain and is an endogenous ligand for orexin receptors, which are G protein-coupled receptors mainly present in the brain.

It has been reported that orexin-deficient mice exhibit symptoms very similar to those of human narcolepsy (Non Patent Literature 1), and that intracerebroventricular administration of orexin peptide shows symptomatic improvement, including suppression of cataplexy and increased arousal (Non Patent Literature 2). It has also been reported that the orexin concentration of the cerebrospinal fluid of narcolepsy patients is markedly decreased (Non Patent Literature 3), suggesting that the cause of narcolepsy is due to a deficiency of orexin.

There are two subtypes of orexin receptors, type 1 (OX1R) and type 2 (OX2R) (Non Patent Literature 4). Mutations in OX2R have been reported as a cause of hereditary narcolepsy in dogs (Non Patent Literature 5). It has also been revealed that OX2R-deficient mice exhibit narcolepsy-like symptoms (Non Patent Literature 6), suggesting that OX2R is strongly involved in the arousal-maintaining action. Such a background has suggested that OX2R agonists may be therapeutic drugs for narcolepsy and diseases presenting with hypersomnia symptoms (Non Patent Literature 7). Up until now, the compounds described in, for example, Patent Literatures 1 to 9 have been reported as OX2R agonists, all of which are structurally different from the compounds according to the present invention.

Idiosyncratic liver toxicity (IDT, idiosyncratic drug toxicity), which is caused by taking drugs, is severe toxicity that is observed in a proportion of about 1 in 100,000 people, and is very difficult to predict from animal studies (Non Patent Literature 8). One of the known pathogenic mechanisms is covalent bonding between electrophilic reactive metabolites (ERMs), which are produced during drug metabolism, and biopolymers (Non Patent Literature 9). The coexistence of glutathione (GSH) as a nucleophile in an *in vitro* metabolic reaction system using human liver microsomes, whereby the risk of IDT is reduced using GSH conjugate generation as an indicator, is useful.

### Citation List

### Patent Literature

Patent Literature 1: International Publication No. WO 2017/135306 (U.S. Patent Application Publication No. 2016/075240)
Patent Literature 2: International Publication No. WO 2019/027058 (U.S. Patent Application Publication No. 2016/633960)
Patent Literature 3: International Publication No. WO 2020/158958 (U.S. Patent Application Publication No. 2017/427490)
Patent Literature 4: International Publication No. WO 2021/108628
Patent Literature 5: International Publication No. WO 2020/167701
Patent Literature 6: International Publication No. WO 2022/051596
Patent Literature 7: International Publication No. WO 2021/107023 (U.S. Patent Application Publication No. 2017/610291)
Patent Literature 8: International Publication No. WO 2022/014680
Patent Literature 9: International Publication No. WO 2015/088000 (U.S. Patent Application Publication No. 2015/103085)

### Non Patent Literature

Non Patent Literature 1: Cell, 98, 1999, 437-451
Non Patent Literature 2: Proc. Natl. Acad. Sci. USA, 101, 2004, 4649-4654
Non Patent Literature 3: Lancet, 355, 2000, 39-40
Non Patent Literature 4: Cell, 92, 1998, 573-585
Non Patent Literature 5: Cell, 98, 1999, 365-376
Non Patent Literature 6: Neuron, 38, 2003, 715-730
Non Patent Literature 7: CNS Drugs, 27, 2013, 83-90
Non Patent Literature 8: Chem. Res. Toxicol. 17, 2004, 3-16
Non Patent Literature 9: Curr. Opin. Drug Discovery Dev. 4, 2001, 55-59

### Summary of Invention

### Technical Problem

An object of the present invention is to provide a novel compound that acts as an agonist for orexin type 2 receptors and can be used in treatment of diseases related to orexin type 2 receptors, in particular, narcolepsy.

### Solution to Problem

The present invention relates to the following (1) to (14).
(1) A compound represented by the following general formula (I) or pharmaceutically acceptable salt thereof: 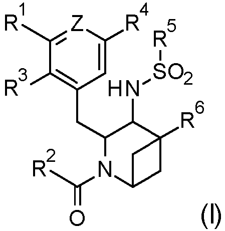 wherein
   R¹ represents a phenyl group or pyridyl group optionally having 1 or 2 substituents independently selected from group A below, a phenyl group substituted with 5 deuterium atoms, a 2,2-difluoro-1,3-benzodioxolyl group, or a 5-membered heteroaryl group containing 1 or 2 atoms independently selected from the group consisting of a nitrogen atom and a sulfur atom in a ring;
   the heteroaryl group optionally has 1 or 2 substituents independently selected from group B below;
   R² represents a C₁-C₆ alkyl group optionally having 1 to 5 substituents independently selected from group C below, a C₃-C₆ cycloalkyl group optionally having 1 or 2 substituents selected from group D below, a 4- or 5-membered saturated heterocyclic group containing 1 atom selected from the group consisting of a nitrogen atom and an oxygen atom in a ring, a 5-membered heteroaryl group containing 1 or 2 atoms independently selected from the group consisting of a nitrogen atom and an oxygen atom in a ring, a C₁-C₆ alkoxy group, a C₁-C₆ alkylamino group, a di-C₁-C₆ alkylamino group, or a bicyclopentyl group;
   R³ represents a hydrogen atom or a halogen atom;
   R⁴ represents a hydrogen atom or a halogen atom;
   R⁵ represents a C₁-C₆ alkyl group optionally substituted with 1 to 3 halogen atoms, a C₃-C₆ cycloalkyl group, a di-C₁-C₆ alkylamino group, or a vinyl group;
   R⁶ represents a hydrogen atom or a halogen atom; and
   Z represents CH or a nitrogen atom.
   Group A: a halogen atom, a C₁-C₆ alkyl group optionally substituted with 1 to 3 halogen atoms, a C₁-C₆ alkoxy group optionally substituted with 1 to 3 halogen atoms, a C₃-C₆ cycloalkyl group, and a cyano group,
   Group B: a halogen atom and a C₁-C₆ alkyl group
   Group C: a hydroxy group, a halogen atom, a deuterium atom, a C₁-C₆ alkoxy group, a C₃-C₆ cycloalkyl group, and a pyrazolyl group
   Group D: a halogen atom, a C₁-C₆ alkyl group optionally substituted with 1 to 3 hydroxy groups, a cyano group, and a hydroxy group
(2) The compound or pharmaceutically acceptable salt thereof according to (1),
   wherein, in the above formula (I),
   R¹ represents a phenyl group or pyridyl group optionally having 1 or 2 substituents independently selected from the group consisting of a fluorine atom, a chlorine atom, a difluoromethyl group, a trifluoromethyl group, and a cyclopropyl group.
(3) The compound or pharmaceutically acceptable salt thereof according to (1) or (2),
   wherein, in the above formula (I),
   R² represents a C₁-C₆ alkyl group optionally having 1 to 5 substituents independently selected from the group consisting of a hydroxy group, a fluorine atom, and a deuterium atom, a cyclopropyl group optionally substituted with 1 or 2 fluorine atoms, or an isoxazolyl group.
(4) The compound or pharmaceutically acceptable salt thereof according to any one of (1) to (3),
   wherein, in the above formula (I),
   R³ represents a fluorine atom;
   R⁴ represents a hydrogen atom or a fluorine atom; and
   Z represents CH or a nitrogen atom.
(5) The compound or pharmaceutically acceptable salt thereof according to any one of (1) to (4),
   wherein, in the above formula (I),
   R⁵ represents a methyl group, an ethyl group, a monofluoromethyl group, or a cyclopropyl group; and
   R⁶ represents a hydrogen atom or a fluorine atom.
(6) The compound or pharmaceutically acceptable salt thereof according to any one of (1) to (5),
   wherein, in the above formula (I),
   R¹ represents the following formula (II): 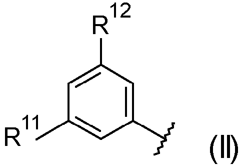 wherein
   R¹¹ and R¹² each independently represent a hydrogen atom, a fluorine atom, or a chlorine atom;
   R² is any one of the following R^{2a} to R^{2e}: 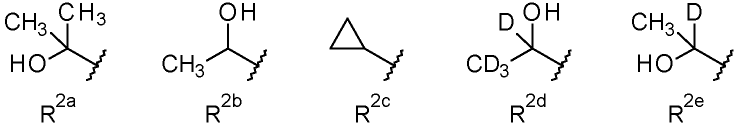 R³, R⁴, and R⁶ each independently represent a hydrogen atom or a fluorine atom;
   R⁵ represents a methyl group or an ethyl group; and
   Z represents CH or a nitrogen atom.
(7) A compound or pharmaceutically acceptable salt thereof, which is any one selected from the following group:
   N-{(3S,4S)-3-[(2-fluoro[biphenyl]-3-yl)methyl]-2-[(2R)-2-hydroxy(²H₄)propanoyl]-2-azabicyclo[3.1.1]heptan-4-yl}methanesulfonamide;
   N-{(3S,4R)-3-[(2,5-difluoro[biphenyl]-3-yl)methyl]-5-fluoro-2-[(2R)-2-hydroxy(²H₄)propanoyl]-2-azabicyclo[3.1.1]heptan-4-yl}methanesulfonamide;
   N-{(3S,4S)-3-[(2,5-difluoro[biphenyl]-3-yl)methyl]-2-[(2R)-2-hydroxy(²H₄)propanoyl]-2-azabicyclo[3.1.1]heptan-4-yl}methanesulfonamide;
   N-{(3S,4R)-5-fluoro-3-[(2-fluoro[biphenyl]-3-yl)methyl]-2-[(2R)-2-hydroxy(²H₄)propanoyl]-2-azabicyclo[3.1.1]heptan-4-yl}methanesulfonamide;
   N-{(3S,4S)-3-[(2-fluoro[biphenyl]-3-yl)methyl]-2-[(2R)-2-hydroxy(2-²H)propanoyl]-2-azabicyclo[3.1.1]heptan-4-yl}methanesulfonamide;
   N-{(3S,4S)-3-[(2,5-difluoro[biphenyl]-3-yl)methyl]-2-[(2R)-2-hydroxy(2-²H)propanoyl]-2-azabicyclo[3.1.1]heptan-4-yl}methanesulfonamide;
   N-{(3S,4R)-5-fluoro-3-[(2-fluoro[biphenyl]-3-yl)methyl]-2-[(2R)-2-hydroxy(2-²H)propanoyl]-2-azabicyclo[3.1.1]heptan-4-yl}methanesulfonamide;
      and
   N-{(3S,4R)-3-[(2,5-difluoro[biphenyl]-3-yl)methyl]-5-fluoro-2-[(2R)-2-hydroxy(2-²H)propanoyl]-2-azabicyclo[3.1.1]heptan-4-yl}methanesulfonamide.
(8) A compound or pharmaceutically acceptable salt thereof, which is any one selected from the following group:
   N-{(3S,4S)-3-[(2-fluoro[biphenyl]-3-yl)methyl]-2-[(2R)-2-hydroxy(2-²H)propanoyl]-2-azabicyclo[3.1.1]heptan-4-yl}methanesulfonamide;
      and
   N-{(3S,4S)-3-[(2,5-difluoro[biphenyl]-3-yl)methyl]-2-[(2R)-2-hydroxy(2-²H)propanoyl]-2-azabicyclo[3.1.1]heptan-4-yl}methanesulfonamide.
(9) A pharmaceutical composition containing the compound or pharmaceutically acceptable salt thereof according to any one of (1) to (8).
(10) An orexin type 2 receptor agonist containing the compound or pharmaceutically acceptable salt thereof according to any one of (1) to (8).
(11) A prophylactic or therapeutic agent for narcolepsy, containing the compound or pharmaceutically acceptable salt thereof according to any one of (1) to (8).
(12) A method for preventing or treating narcolepsy, including administering the compound or pharmaceutically acceptable salt thereof according to any one of (1) to (8) in an effective amount.
(13) The compound or pharmaceutically acceptable salt thereof according to any one of (1) to (8), for use in prevention or treatment of narcolepsy.
(14) Use of the compound or pharmaceutically acceptable salt thereof according to any one of (1) to (8), in production of a medicament for prevention or treatment of narcolepsy.

### Advantageous Effects of Invention

The compounds of the present invention or pharmaceutically acceptable salts thereof act as excellent orexin type 2 receptor agonists. Furthermore, the specific compounds of the present invention or pharmaceutically acceptable salts thereof can provide effects in which excellent metabolic stability is provided and/or IDT risk is reduced.

### Description of Embodiments

As used herein, the term "halogen atom" refers to, for example, a fluorine atom, a chlorine atom, a bromine atom, or an iodine atom.

As used herein, the term "C₁-C₆ alkyl group" refers to a linear or branched alkyl group having 1 to 6 carbon atoms, and examples thereof include a methyl group, an ethyl group, a propyl group, an isopropyl group, a butyl group, an isobutyl group, a s-butyl group, a t-butyl group, a pentyl group, an isopentyl group, a 2-methylbutyl group, a neopentyl group, a 1-ethylpropyl group, a hexyl group, an isohexyl group, and a 4-methylpentyl group.

As used herein, the term "C₁-C₆ alkoxy group" refers to an alkoxy group formed from the above-described C₁-C₆ alkyl group, and examples thereof include a methoxy group, an ethoxy group, a propoxy group, an isopropoxy group, a butoxy group, an isobutoxy group, a s-butoxy group, a t-butoxy group, a pentoxy group, an isopentoxy group, a 2-methylbutoxy group, a neopentoxy group, a 1-ethylpropoxy group, a hexoxy group, an isohexoxy group, and a 4-methylpentoxy group.

As used herein, the term "C₃-C₆ cycloalkyl group" refers to, for example, a cyclopropyl group, a cyclobutyl group, a cyclopentyl group, or a cyclohexyl group.

As used herein, the term "C₁-C₆ alkylamino group" refers to a group in which one C₁-C₆ alkyl group described above is bonded to an amino group, and examples thereof include a methylamino group, an ethylamino group, a propylamino group, an isopropylamino group, a butylamino group, an isobutylamino group, a s-butylamino group, a t-butylamino group, a pentylamino group, an isopentylamino group, a 2-methylbutylamino group, a neopentylamino group, a 1-ethylpropylamino group, a hexylamino group, an isohexylamino group, and a 4-methylpentylamino group.

As used herein, the term "di-C₁-C₆ alkyl amino group" refers to a group in which two C₁-C₆ alkyl groups described above that are the same or different are bonded to an amino group, and examples thereof include a dimethylamino group, a methylethylamino group, and a diethylamino group.

As used herein, the term "C₁-C₆ alkyl group optionally substituted with 1 to 3 halogen atoms" refers to the above-described C₁-C₆ alkyl group and a group in which the above-described C₁-C₆ alkyl group is substituted with halogen atoms that are the same or different. Examples of the group in which the above-described C₁-C₆ alkyl group is substituted with halogen atoms that are the same or different include a fluoromethyl group, a difluoromethyl group, a trifluoromethyl group, a chloromethyl group, a dichloromethyl group, and a trichloromethyl group.

As used herein, the term "C₁-C₆ alkyl group optionally substituted with 1 to 3 hydroxy groups" refers to the above-described C₁-C₆ alkyl group and a group in which the above-described C₁-C₆ alkyl group is substituted with hydroxy groups. Examples of the group in which the above-described C₁-C₆ alkyl group is substituted with hydroxy groups include a hydroxymethyl group, a 1-hydroxyethyl group, a 2-hydroxyethyl group, a 1-hydroxy-1-methylethyl group, and a 2-hydroxy-1-methylethyl group.

As used herein, the term "C₁-C₆ alkoxy group optionally substituted with 1 to 3 halogen atoms" refers to the above-described C₁-C₆ alkoxy group and a group in which the above-described C₁-C₆ alkoxy group is substituted with halogen atoms that are the same or different. Examples of the group in which the above-described C₁-C₆ alkoxy group is substituted with halogen atoms that are the same or different include a fluoromethoxy group, a difluoromethoxy group, a trifluoromethoxy group, a chloromethoxy group, a dichloromethoxy group, and a trichloromethoxy group.

As used herein, the term "saturated heterocycle" refers to a saturated ring containing a heteroatom in the ring.

As used herein, the term "heteroaryl group" refers to a substituent derived from an aromatic group containing a heteroatom in the ring.

As used herein, the term "biaryl group" refers to a substituent having a structure in which two aromatic rings are linked by a single bond, and one or both of the aromatic rings constituting the biaryl group may be a heteroaryl group.

As used herein, the term "5-membered heteroaryl group containing 1 or 2 atoms independently selected from the group consisting of a nitrogen atom and a sulfur atom in the ring" refers to, for example, a pyrrolyl group, a thienyl group, a pyrazolyl group, an imidazolyl group, a thiazolyl group, or an isothiazolyl group.

As used herein, the term "5-membered heteroaryl group containing 1 or 2 atoms independently selected from the group consisting of a nitrogen atom, an oxygen atom, and a sulfur atom in the ring" refers to, for example, a furyl group, a pyrrolyl group, a thienyl group, a pyrazolyl group, an imidazolyl group, an isoxazolyl group, or an oxazolyl group.

As used herein, the term "4- or 5-membered saturated heterocyclic group containing 1 atom selected from the group consisting of a nitrogen atom and an oxygen atom in the ring" refers to, for example, an azetidinyl group, an oxetanyl group, a pyrrolidinyl group, or a tetrahydrofuranyl group.

Next, suitable substituents in the general formula (I) will be described.

R¹ is suitably a phenyl group, a 3,5-difluorophenyl group, or a 3-chlorophenyl group, and more suitably a phenyl group.

R² is suitably any one of the following R^{2a} to R^{2e}, and more suitably R^{2d} or R^{2e}.

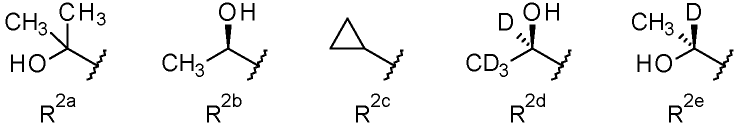

A suitable combination of R³, R⁴, and Z is any one of the following R^{3a} to R^{3c}, and more suitably R^{3a} or R^{3c}. 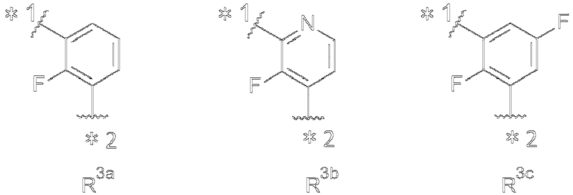 In the formula, *1 is bonded to a phenyl group and *2 is bonded to a methylene group.

R⁵ is suitably a methyl group or an ethyl group, and more suitably a methyl group.

R⁶ is suitably a hydrogen atom or a fluorine atom.

A suitable combination of substituents in the general formula (I) in another aspect is that R¹ is a phenyl group, R² is R^{2d} or R^{2e}, the combination of R³, R⁴, and Z is R^{3a} or R^{3c}, R⁵ is a methyl group, and R⁶ is a hydrogen atom or a fluorine atom.

Another aspect of the present invention is represented by the following general formula (III).

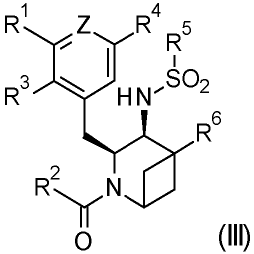

The definitions of R¹, R², R³, R⁴, R⁵, R⁶, and Z in the formula (III), suitable combinations, and other details are the same as the definitions in the general formula (I) in the present specification.

The compound represented by the general formula (I) of the present invention can be made into a pharmaceutically acceptable salt, if desired. The pharmaceutically acceptable salt refers to a salt that can be administered into the body of an animal (including human) to be used as a medicament. In the case where the compound represented by the general formula (I) of the present invention has an acidic group or a basic group, it can be made into a salt by allowing it to react with a base or an acid.

Examples of the salt based on the acidic group include an alkali metal salt such as sodium salt, potassium salt, and lithium salt; an alkaline earth metal salt such as magnesium salt and calcium salt; and an organic base salt such as N-methylmorpholine salt, triethylamine salt, tributylamine salt, diisopropylethylamine salt, dicyclohexylamine salt, N-methylpiperidine salt, pyridine salt, 4-pyrrolidinopyridine salt, and picoline salt, and an amino acid salt such as glycine salt, lysine salt, arginine salt, ornithine salt, glutamic acid salt, and aspartic acid salt.

Examples of the salt based on the basic group include an inorganic acid salt such as a hydrogen halide acid salt like hydrofluoric acid salt, hydrochloric acid salt, hydrobromic acid salt, and hydriodic acid salt, nitric acid salt, perchloric acid salt, sulfuric acid salt, and phosphoric acid salt; an organic acid salt such as a lower alkane sulfonic acid salt like methanesulfonic acid salt, trifluoromethanesulfonic acid salt, and ethanesulfonic acid salt, an arylsulfonic acid salt like benzenesulfonic acid salt and p-toluenesulfonic acid salt, acetic acid salt, malic acid salt, fumaric acid salt, succinic acid salt, citric acid salt, ascorbic acid salt, tartaric acid salt, oxalic acid salt, and maleic acid salt; and an amino acid salt such as glycine salt, lysine salt, arginine salt, ornithine salt, glutamic acid salt, and aspartic acid salt.

When leaving the compound represented by general formula (I) of the present invention or pharmaceutically acceptable salt thereof in the atmosphere or recrystallizing it, it may absorb water and adsorbed water may adhere thereto, or it may become a hydrate, and the present invention also encompasses compounds of such various hydrates, solvates, and crystalline polymorphs.

In the compound represented by the general formula (I) of the present invention, pharmaceutically acceptable salt thereof, or solvate thereof, there may be a variety of isomers such as geometric isomers including cis and trans isomers, tautomers, rotational isomers, or optical isomers (including enantiomers and diastereomers) such as d and I isomers, depending on the type and combination of substituents. The compounds of the present invention also encompass all of their isomers, stereoisomers, and mixtures of these isomers and stereoisomers in any ratio, unless otherwise limited. The mixtures of these isomers can be separated by a known separation means. When the compounds according to the present invention are mixtures of isomers, the ratio of certain diastereomers depends on the substrate, solvent and base, but is not limited. For example, in mixtures of isomers of the compounds according to the present invention, the ratio of a specific diastereomer (this may be a racemate): stereoisomers other than the specific diastereomer may be 1:1 or more, or they may be single diastereomers. Here, as used herein, the term "single diastereomer" may encompass a mixture of isomers of the compounds according to the invention in which the ratio of a specific diastereomer (this may be a racemate): stereoisomers other than the specific diastereomer is 20:5 (20/5) or more, may encompass a mixture with a ratio of 20:3 (20/3) or more, or may encompass a mixture with a ratio of 20:1 (20/1) or more. Also, in the case where the compounds according to the present invention are mixtures of enantiomers, the enantiomeric excess is not particularly limited and may be, for example, 0% ee or more (including a racemate), may be 20% ee or more, may be 40% ee or more, may be 60% ee or more, may be 80% ee or more, may be 85% ee or more, or may be 90% ee or more. In addition, in the compounds according to the present invention, the term "single enantiomer" may encompass a mixture of the enantiomers according to the present invention with an enantiomeric excess of, for example, 92% ee or more, may encompass a mixture of 94% ee or more, may encompass a mixture of 96% ee or more, or may encompass a mixture of 99% ee or more.

The compound represented by the general formula (I) of the present invention or pharmaceutically acceptable salt thereof also includes labeled forms, that is, compounds in which one or two or more atoms therein have been replaced with isotopes (for example, ²H, ³H, ¹³C, ¹⁴C, ³⁵S, and the like).

The compound represented by the general formula (I) of the present invention or pharmaceutically acceptable salt thereof is normally named in accordance with the nomenclature of the International Union of Pure and Applied Chemistry (IUPAC).

In the names of the compounds of the present invention, if the structures of the compounds have an atom that serves as a chiral center, its absolute configuration may be indicated by R and S (described together with the position number).

The relative configuration may be indicated by, when the configuration of a chiral center described first is designated as R or S, adding asterisks to the configuration indication (R* and S*), or by placing the prefix (symbol) rel- (meaning "relative") in front of the name.

As for racemic mixtures, their absolute configuration is normally indicated without using R and S in particular, but may be indicated by using the symbols RS and SR instead of R* and S* or by placing the prefix (symbol) rac- (meaning "racemic") in front of the name.

The present invention also encompasses so-called prodrugs. Prodrugs are compounds having a group that can be converted to an amino group, a hydroxy group, a carboxy group, or the like of the compounds by hydrolysis or under physiological conditions, and groups that form such prodrugs are the groups described in Prog. Med., vol. 5, pp. 2157-2161, 1985, and others. More specifically, examples of such prodrugs may include:
(1) in the case where the compounds have an amino group, compounds in which such an amino group has been acylated, alkylated, or phosphorylated (for example, compounds in which such an amino group has been eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated);
(2) in the case where the compounds have a hydroxy group, compounds in which such a hydroxy group has been acylated, alkylated, phosphorylated, or borated (for example, compounds in which such a hydroxy group has been acetylated, palmitoylated, propanoylated, pivaloylated, succinylated, fumarylated, alanylated, or dimethylaminomethylcarbonylated); and
(3) in the case where the compounds have a carboxy group, compounds in which such a carboxy group has been esterified or amidated (for example, compounds in which such a carboxy group has been ethyl esterified, phenyl esterified, carboxymethyl esterified, dimethylaminomethyl esterified, pivaloyloxymethyl esterified, ethoxycarbonyloxyethyl esterified, amidated, or methylamidated).

### (Applicable diseases)

The compounds according to the present invention can be used as a medicament for treatment and/or prevention of narcolepsy. In addition, the compounds according to the present invention can also be used as a medicament for treatment, remission and/or prevention of, besides narcolepsy, idiopathic hypersomnia, sleep apnea syndrome, other hypersomnias (for example, hypersomnias associated with neurodegenerative diseases such as Parkinson's disease and Lewy body dementia, and psychiatric diseases such as major depression, schizophrenia, and bipolar disorder), circadian rhythm disorder, and hypersomnia symptoms associated with obesity, diabetes, and even analgesic drugs including morphine. The possibility of using the compounds according to the present invention for treatment or prevention of the above diseases is supported by the fact that the compounds according to the present invention can be used as an orexin type 2 receptor agonist and orexin is considered to be involved not only in the action on sleep and arousal, but also in the action on feeding, emotion, cognitive function, and others. The compounds of the present invention can also be used as a medicament for prevention of the occurrence of diseases as described above, in order to improve the quality of daytime life by preventing excessive daytime sleepiness, cataplexy, and others.
In addition, the compounds of the present invention may be used not only to activate the activity of individuals with decreased orexin, but also as an orexin type 2 receptor agonist for general individuals with no orexin decrease. Furthermore, the compounds of the present invention can be used in a manner where the arousal state can be maintained during activity time (for example, about 12 to 16 hours) among 24 hours and the drug efficacy wears off before entering sleep time and is less likely to suppress sleep.

### (Combination therapy)

The compounds according to the present invention may be used in combination with existing narcolepsy therapeutic drugs such as modafinil, sodium oxybate, pitolisant, and methylphenidate, as well as antidepressive drugs. The compounds according to the present invention may also be used in combination with, when used for treatment of pharmaceutical agent-induced hypersomnia symptoms, the pharmaceutical agent that causes the symptoms.

### (Dosage form, dose, and method of use of pharmaceutical composition)

The pharmaceutical composition of the present invention contains the compound of the present invention or pharmaceutically acceptable salt thereof, and may contain a pharmaceutically acceptable carrier. It can be administered as various injections for intravenous injection, intramuscular injection, subcutaneous injection, and the like, or by various methods such as oral administration or transdermal administration. The pharmaceutically acceptable carrier means a pharmaceutically acceptable material involved in transporting the compound of the present invention or a composition containing the compound of the present invention from one organ or viscera to another organ or viscera (for example, an excipient, a diluent, an additive, a solvent, or the like).

A formulation containing the compound of the present invention or pharmaceutically acceptable salt thereof as an active ingredient is prepared using an additive such as a carrier or an excipient used in normal formulations. Administration of the compounds of the present invention may be performed in any form, including oral administration with a tablet, a pill, a capsule, a granule, a powder, a liquid, and the like, or parenteral administration with an injection for intraarticular, intravenous, intramuscular, and other uses, a suppository, an eye drop, an eye ointment, a transdermal liquid, an ointment, a transdermal patch, a transmucosal liquid, a transmucosal patch, an inhalant, and the like.

As the solid composition for oral administration, a tablet, a powder, a granule, or the like is used. Such a solid composition is composed of one or two or more active ingredients and at least one inert excipient, such as lactose, mannitol, dextrose, hydroxypropyl cellulose, microcrystalline cellulose, starch, polyvinylpyrrolidone, and/or magnesium aluminometasilicate. Such a solid composition may contain an inert additive, such as a lubricant like magnesium stearate, a disintegrant like sodium carboxymethyl starch, a stabilizer, or a solubilization aid, in accordance with a conventional method. The tablet or pill may be coated with a sugar coating or a film of a gastrosoluble or enterosoluble substance, if necessary.

When used as a tablet, for example, the following can be used as a carrier: an excipient such as lactose, saccharose, sodium chloride, glucose, urea, starch, calcium carbonate, kaolin, crystalline cellulose, or silicic acid; a binding agent such as water, ethanol, propanol, simple syrup, glucose solution, starch solution, gelatin solution, carboxymethyl cellulose, shellac, methyl cellulose, potassium phosphate, or polyvinylpyrrolidone; a disintegrant such as dried starch, sodium alginate, agar powder, laminaran powder, sodium bicarbonate, calcium carbonate, polyoxyethylene sorbitan fatty acid esters, sodium lauryl sulfate, glycerol monostearate, starch, or lactose; a disintegration inhibitor such as saccharose, stearin, cocoa butter, or hydrogenated oil; an absorption promoter such as quaternary ammonium salts or sodium lauryl sulfate; a moisturizer such as glycerol or starch; an adsorbent such as starch, lactose, kaolin, bentonite, or colloidal silicic acid; and a lubricant such as purified talc, stearate, borax powder, or polyethylene glycol. Also, if necessary, it can be made into a tablet on which an ordinary coating has been applied, such as a sugar-coated tablet, a gelatin-coated tablet, an enteric-coated tablet, a film-coated tablet, a double-coated tablet, or a multilayered tablet.

When used as a pill, for example, the following can be used as a carrier: an excipient such as glucose, lactose, cocoa butter, starch, hydrogenated vegetable oils, kaolin, or talc; a binding agent such as gum arabic powder, powdered tragacanth, gelatin, or ethanol; and a disintegrant such as laminaran or agar.

As the liquid composition for oral administration, a pharmaceutically acceptable emulsion, solution, suspension, syrup, elixir, or the like is used. To such a liquid composition, it is possible to add a commonly used inert diluent, such as purified water or ethanol. The liquid composition may contain, in addition to the inert diluent, a solubilizing agent, an auxiliary agent such as wetting agent, a sweetening agent, a flavoring agent, an aromatic agent, and a preservative.

As the injection for parenteral administration, a sterile aqueous or non-aqueous solution, suspension, emulsion, or the like is used. Aqueous solvents include, for example, distilled water for injection or saline solution. Non-aqueous solvents include, for example, propylene glycol, polyethylene glycol, vegetable oils such as olive oil, alcohols such as ethanol, or Polysorbate 80. Such an injection composition may further contain an isotonizing agent, a preservative, a wetting agent, an emulsifier, a dispersing agent, a stabilizer, or a solubilization aid. These injection compositions can be made sterile by, for example, filtration through a bacteria withholding filter, by blending with a disinfectant, or by irradiation. Alternatively, these injection compositions can be used by producing a sterile solid composition and dissolving or suspending it in sterile water or a sterile injectable solvent prior to use.

When used as an injection, it can be used as a liquid, an emulsion, or a suspension. The liquid, emulsion, or suspension is preferably made sterile and isotonic to the blood. There is no particular limitation on the solvent to be used in production of the liquid, emulsion, or suspension, as long as it can be used as a diluent for medical purposes, and examples thereof include water, ethanol, propylene glycol, ethoxylated isostearyl alcohol, polyoxylated isostearyl alcohol, and polyoxyethylene sorbitan fatty acid esters. Note that, in this case, the formulation may contain salt, glucose, or glycerol in an amount sufficient to prepare an isotonic solution, and it may also contain an ordinary solubilization aid, buffering agent, analgesic agent, or the like.

The above formulation may also contain a coloring agent, a preserving agent, a flavor, a flavoring agent, a sweetening agent, and the like, if necessary, and may further contain other pharmaceuticals.
The amount of the compound contained in the above formulation is not particularly limited and can be widely selected as appropriate, but it is normally contained in an amount of 0.5 to 70% by weight, preferably 1 to 30% by weight, of the entire composition.

The dosage and frequency of administration of the compounds of the present invention are determined as appropriate, taking into consideration the symptoms, age, and other factors of the patient (warm-blooded animal, especially human). For normal oral administration, a daily dosage of about 0.001 to 100 mg/kg of body weight, preferably 0.1 to 30 mg/kg, and still more preferably 0.1 to 10 mg/kg, is appropriate, which is administered at one time, or divided into two or more doses. For intravenous administration, a daily dosage of about 0.0001 to 10 mg/kg of body weight is appropriate, which is administered once a day or in multiple divided doses.

For example, the compounds of the present invention may be administered in a dose that does not suppress sleep by orally administering it once a day at the time of awakening from sleep (for example, in the morning) so that the drug efficacy wears off when the activity time of the day (for example, about 12 to 16 hours) is over and before the next sleep time.

### (Production method)

Hereinafter, representative methods for producing a compound represented by a general formula (1) or pharmaceutically acceptable salt thereof will be described. The compounds of the present invention can be produced by various production methods. The production methods shown below are only examples, and the present invention should not be construed as being limited thereto.

The compound represented by the general formula (1) or pharmaceutically acceptable salt thereof can be produced by applying various known production methods, utilizing the characteristics based on the basic skeleton or the type of substituent. Examples of the known methods include "ORGANIC FUNCTIONAL GROUP PREPARATIONS", 2nd edition, ACADEMIC PRESS, INC., 1989, "Comprehensive Organic Transformations", VCH Publishers Inc., 1989, and others.

At that time, depending on the type of functional group present in the compound, it may be effective in terms of production technology to protect such a functional group with an appropriate protecting group at the raw material or intermediate stage, or to replace it with a group that can be easily converted to such a functional group.

Examples of such a functional group include an amino group, a hydroxy group, and a carboxy group, and examples of protecting groups for them are the protecting groups described in, for example, "Greene's Protective Groups in Organic Synthesis (4th edition, John Wiley & Sons, Inc., 2006)" authored by T. W. Greene and P. G. Wuts.

The protecting group or the group that can be easily converted to such a functional group may be selected and used as appropriate depending on the respective reaction conditions of the production methods for producing the compound.

According to such methods, the desired compound can be obtained by introducing the group, carrying out the reaction, and then removing the protecting group or converting it to the desired group, if necessary.

Hereinafter, representative methods for producing the compound represented by the general formula (1) of the present invention or pharmaceutically acceptable salt thereof will be exemplified. Note that the production method of the present invention is not limited to the examples shown below.

### (Method A)

Method A is a method for producing the compound represented by the general formula (1) from a compound represented by a general formula (2).

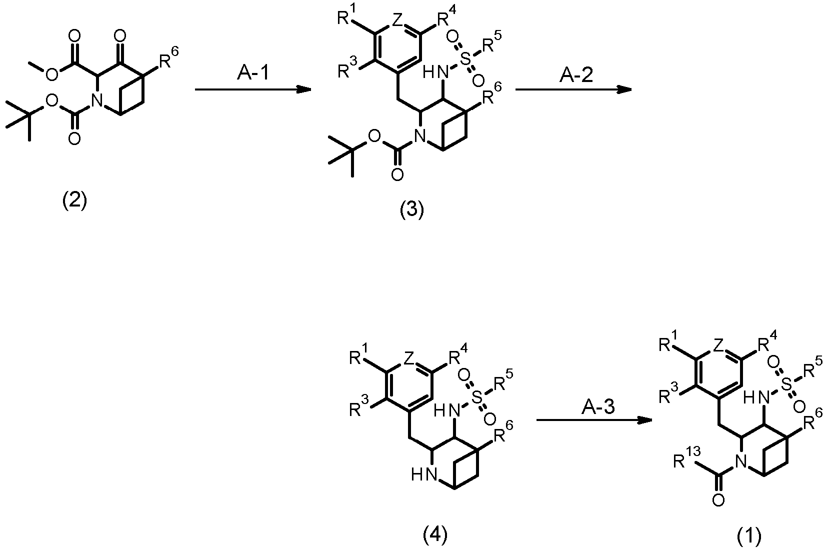

(In the formula, R¹ to R⁶ and Z are as defined above, and R¹³ represents R² or a precursor thereof.)

### (Step A-1)

Step A-1 includes Step A-1a, Step A-1b, Step A-1c, and Step A-1d as the essential reactions, and may include Step A-1e and Step A-1f, if necessary. As for Step A-1b to Step A-1f, any of the steps may be carried out first, and a person skilled in the art can easily select their order.
Step A-1a: a reaction of introducing a methylenebiaryl group or a methylenearyl group by a substitution reaction between a β-ketoester and an alkyl bromide
Step A-1b: a reaction of decarboxylating the β-ketoester
Step A-1c: a reaction of converting the ketone to an amine
Step A-1d: a reaction of sulfonylating or sulfamoylating the amine
Step A-1e: a reaction of converting the bromine atom on the aromatic ring of the methylenearyl group to an aryl group or a heteroaryl group
Step A-1f: a reaction of reducing the alkene

### (Step A-1a)

The present step is a step of introducing a methylenebiaryl group or a methylenearyl group into a β-ketoester using an appropriate base and an alkyl bromide in an inert solvent.

There is no particular limitation on the solvent that can be used, as long as it does not inhibit the reaction and dissolves the starting materials to some extent, and for example it is selected from the following solvent group. The solvent group consists of an aromatic hydrocarbon such as benzene, toluene, and xylene; a halogenated hydrocarbon such as dichloromethane and chloroform; an ether such as diethyl ether, tetrahydrofuran, 1,4-dioxane, and 1,2-dimethoxyethane; an alcohol such as methanol, deuterated methanol, ethanol, deuterated ethanol, and tert-butanol; an ester such as ethyl acetate and propyl acetate; a nitrile such as acetonitrile; an amide such as formamide and N,N-dimethylformamide; a sulfoxide such as dimethyl sulfoxide; an organic acid such as acetic acid; water; and a mixture thereof.

There is no particular limitation on the base to be used, as long as it is used as a base in a normal reaction, but suitable examples thereof may include an organic base such as triethylamine, N,N-diisopropylethylamine, N-methylmorpholine, lutidine, and pyridine; an alkali metal carbonate such as sodium carbonate, potassium carbonate, and cesium carbonate; an alkaline earth metal carbonate such as magnesium carbonate; an alkali metal bicarbonate such as potassium bicarbonate; an alkaline earth metal bicarbonate such as calcium bicarbonate; an alkali metal hydroxide such as sodium hydroxide; an alkaline earth metal hydroxide such as magnesium hydroxide; and an alkali metal phosphate such as tripotassium phosphate.

The reaction temperature varies depending on the raw material compounds, reagents, and other factors, but is normally - 20°C to 120°C, and suitably 0°C to 80°C.
The reaction time varies depending on the raw material compounds, reagents, and other factors, but is normally 5 minutes to 48 hours, and suitably 10 minutes to 24 hours.
After termination of the reaction, the target compound of the present reaction can be obtained by, for example, concentrating the reaction mixture, adding an organic solvent such as ethyl acetate, washing with water, then separating the organic layer containing the target compound, drying over anhydrous sodium sulfate or the like, and then distilling off the solvent.
The obtained compound can be further purified, if necessary, by a conventional method such as recrystallization, reprecipitation, or silica gel column chromatography.

### (Step A-1b)

Step A-1b is a step of decarboxylating the β-ketoester compound.

The essential reaction includes Step A-1b1: a decarboxylation reaction. If necessary, Step A-1b2: a tert-butoxycarbonylation reaction can also be added.

### (Step A-1b1)

The present step is a step of decarboxylating the β-ketoester using an acid or a metal salt.

As for the solvent to be used, the same one used in the above-described Step A-1a can be used.
There is no particular limitation on the acid to be used, as long as it is used as an acid in a normal reaction, but suitable examples thereof may include an inorganic acid such as hydrochloric acid, sulfuric acid, and nitric acid.

There is no particular limitation on the metal salt to be used, but examples thereof include lithium chloride and lithium sulfate. The reaction temperature varies depending on the raw material compounds, reagents, and other factors, but is normally 0°C to 160°C, and suitably 80°C to 140°C.
The reaction time varies depending on the raw material compounds, reagents, and other factors, but is normally 5 minutes to 96 hours, and suitably 1 hour to 48 hours.
After termination of the reaction, the target compound of the present reaction can be obtained through, for example, steps such as concentration, separation, and drying in the same manner as in the above-described Step A-1a, and if necessary, may be further purified by a conventional method in the same manner as described above.

### (Step A-1b2)

The present step is a step of tert-butoxycarbonylation in the presence or absence of an appropriate base.

As for the solvent to be used, the same one used in the above-described Step A-1a can be used.
There is no particular limitation on the tert-butoxycarbonylating agent to be used, as long as it is used as a tert-butoxycarbonylating agent in a normal reaction, but suitable examples thereof may include di-tert-butyl dicarbonate and tert-butyl phthalimide carbonate.
As for the base to be used, the same one used in the above-described Step A-1a can be used.
The reaction temperature varies depending on the raw material compounds, reagents, and other factors, but is normally -40°C to 100°C, and suitably 0°C to 60°C.
The reaction time varies depending on the raw material compounds, reagents, and other factors, but is normally 5 minutes to 48 hours, and suitably 1 hour to 24 hours.
After termination of the reaction, the target compound of the present reaction can be obtained through, for example, steps such as concentration, separation, and drying in the same manner as in the above-described Step A-1a, and if necessary, may be further purified by a conventional method in the same manner as described above.

### (Step A-1c)

Step A-1c is carried out by converting the ketone to an oxime and then reducing it to an amine.

The essential reactions include Step A-1c1: a condensation reaction between the ketone and hydroxylamine hydrochloride, and Step A-1c2: a reaction of reducing the oxime to an amine.

### (Step A-1c1)

The present step is a step of converting the ketone to an oxime using an appropriate base and hydroxylamine hydrochloride in an inert solvent.

As for the solvent to be used, the same one used in the above-described Step A-1a can be used.
As for the base to be used, the same one used in the above-described Step A-1a can be used.
The reaction temperature varies depending on the raw material compounds, reagents, and other factors, but is normally -20°C to 120°C, and suitably 0°C to 80°C.
The reaction time varies depending on the raw material compounds, reagents, and other factors, but is normally 5 minutes to 48 hours, and suitably 30 minutes to 24 hours.
After termination of the reaction, the target compound of the present reaction can be obtained through, for example, steps such as concentration, separation, and drying in the same manner as in the above-described Step A-1a, and if necessary, may be further purified by a conventional method.

### (Step A-1c2)

The present step is a step of converting the oxime to an amine using an appropriate reducing agent in an inert solvent, in the presence or absence of an additive.

As for the solvent to be used, the same one used in the above-described Step A-1a can be used.
There is no particular limitation on the reducing agent to be used, but examples thereof include sodium borohydride, lithium borohydride, lithium aluminum hydride, sodium cyanoborohydride, sodium triacetoxyborohydride, and zinc powder.
There is no particular limitation on the additive to be used, as long as it is used as a known method, but suitable examples thereof may include a metal oxide such as molybdenum(VI) oxide; and a metal salt such as nickel(II) chloride.
The reaction temperature varies depending on the raw material compounds, reagents, and other factors, but is normally -20°C to 120°C, and suitably 0°C to 80°C.
The reaction time varies depending on the raw material compounds, reagents, and other factors, but is normally 5 minutes to 96 hours, and suitably 30 minutes to 48 hours.
After termination of the reaction, the target compound of the present reaction can be obtained through, for example, steps such as concentration, separation, and drying in the same manner as in the above-described Step A-1a, and if necessary, may be further purified by a conventional method.
Column chromatography using a chiral column can provide an optically active compound.

### (Step A-1d)

The present step is a step of sulfonylating or sulfamoylating the amine using an appropriate base and sulfonyl chloride or sulfamoyl chloride in an inert solvent, in the presence or absence of an additive. As for the solvent to be used, the same one used in the above-described Step A-1a can be used.
As for the base to be used, the same one used in the above-described Step A-1a can be used.
There is no particular limitation on the additive to be used, as long as it is used as a known method, but suitable examples thereof may include N,N-dimethylaminopyridine.
The reaction temperature varies depending on the raw material compounds, reagents, and other factors, but is normally -20°C to 120°C, and suitably 0°C to 80°C.
After termination of the reaction, the target compound of the present reaction can be obtained through, for example, steps such as concentration, separation, and drying in the same manner as in the above-described Step A-1a, and if necessary, may be further purified by a conventional method.
Column chromatography using a chiral column can provide an optically active compound.

### (Step A-1e)

The present step is a step of converting the bromine atom on the aryl ring or heteroaryl ring introduced in Step A-1a to an aryl group or a heteroaryl group. Here, description will be given for a method (Step A-1e1) in which a boronic acid or ester thereof is used in the presence of an appropriate base and in the presence of an appropriate metal catalyst, and a method (Step A-1e2) in which a boron compound is used in the presence of an appropriate base and in the presence of an appropriate metal catalyst to convert the bromine atom to a boronic acid ester, followed by the use of an aryl halide or a heteroaryl halide in the presence of an appropriate base and in the presence of an appropriate metal catalyst, but they are not limited to these methods.

### (Step A-1e1)

Step A-1e1 is a step of converting the bromine atom on the aryl ring or heteroaryl ring to an aryl group or a heteroaryl group using a boronic acid or ester thereof in an inert solvent, in the presence of an appropriate base and in the presence of an appropriate metal catalyst.

As for the solvent to be used, the same one used in the above-described Step A-1a can be used.
As for the base to be used, the same one used in the above-described Step A-1a can be used, or a metal alkoxide such as sodium tert-butoxide or potassium tert-butoxide may be used.
There is no particular limitation on the metal catalyst to be used, as long as it is used as a known method, but suitable examples thereof may include a palladium catalyst such as tetrakis(triphenylphosphine)palladium, bis(tri-tert-butylphosphine)palladium, [1,1'-bis(diphenylphosphino)ferrocene]palladium(II) dichloride, bis(dibenzylideneacetone)palladium, tris(dibenzylideneacetone)dipalladium, bis[1,2-bis(diphenylphosphino)ethane]palladium, (2-dicyclohexylphosphino-2'4'6'-triisopropyl-1,1'-biphenyl)[2-(2'-amino-1,1'-biphenyl)]palladium(II) methanesulfonate, chloro(2-dicyclohexylphosphino-2',4',6'-triisopropyl-1,1'-biphenyl)[2-(2'-amino-1,1'-biphenyl)]palladium(II), and palladium-activated carbon. There is no particular limitation on the boronic acid to be used, but examples thereof may include phenylboronic acid, 3-chlorophenylboronic acid, and 3,5-difluorophenylboronic acid. There is no particular limitation on the boronic acid ester to be used, but examples thereof may include 3-cyclopropylphenylboronic acid pinacol ester.
The reaction temperature varies depending on the raw material compounds, reagents, and other factors, but is normally 0°C to 150°C, and suitably 20°C to 120°C.
The reaction time varies depending on the raw material compounds, reagents, and other factors, but is normally 5 minutes to 24 hours, and suitably 10 minutes to 12 hours.
After termination of the reaction, the target compound of the present reaction can be obtained through, for example, steps such as concentration, separation, and drying in the same manner as in the above-described Step A-1a, and if necessary, may be further purified by a conventional method.

### (Step A-1e2)

Step A-1e2 is carried out by converting the bromine atom on the aryl ring or heteroaryl ring to a boronic acid ester, followed by reaction with an aryl halide or heteroaryl halide.

The essential reactions include Step A-1e2a: a reaction of converting the bromine atom on the aryl ring or heteroaryl ring to a boronic acid ester, and Step A-1e2b: a reaction of converting from the boronic acid ester and an aryl halide or a heteroaryl halide to a biaryl compound.

### (Step A-1e2a)

Step A-1e2a is a step of converting the bromine atom on the aryl ring or heteroaryl ring to a boronic acid ester using a boron compound in an inert solvent, in the presence of an appropriate base and in the presence of an appropriate metal catalyst.

As for the solvent to be used, the same one used in the above-described Step A-1a can be used.
As for the base to be used, the same one used in the above-described Step A-1a can be used, or a metal alkoxide such as sodium tert-butoxide or potassium tert-butoxide may be used.
As for the metal catalyst to be used, the same one used in the above-described Step A-1e1 can be used.
Examples of the boron compound to be used may include bis(pinacolato)diboron and pinacolborane.
The reaction temperature varies depending on the raw material compounds, reagents, and other factors, but is normally 0°C to 150°C, and suitably 20°C to 120°C.
The reaction time varies depending on the raw material compounds, reagents, and other factors, but is normally 5 minutes to 24 hours, and suitably 10 minutes to 12 hours.
After termination of the reaction, the target compound of the present reaction can be obtained through, for example, steps such as concentration, separation, and drying in the same manner as in the above-described Step A-1a, and if necessary, may be further purified by a conventional method.

### (Step A-1e2b)

Step A-1e2b is a step of converting the boronic acid ester to an aryl group or a heteroaryl group using an aryl halide or a heteroaryl halide in an inert solvent, in the presence of an appropriate base and in the presence of an appropriate metal catalyst.

As for the solvent to be used, the same one used in the above-described Step A-1a can be used.
As for the base to be used, the same one used in the above-described Step A-1a can be used, or a metal alkoxide such as sodium tert-butoxide or potassium tert-butoxide may be used.
As for the metal catalyst to be used, the same one used in the above-described Step A-1e1 can be used.
Examples of the aryl halide to be used include 1-bromo-3-(difluoromethyl)-5-fluorobenzene.
Examples of the heteroaryl halide to be used include 2,6-dichloropyridine and 2-bromo-6-cyclopropylpyridine.
The reaction temperature varies depending on the raw material compounds, reagents, and other factors, but is normally 0°C to 150°C, and suitably 20°C to 120°C.
The reaction time varies depending on the raw material compounds, reagents, and other factors, but is normally 5 minutes to 24 hours, and suitably 10 minutes to 12 hours.
After termination of the reaction, the target compound of the present reaction can be obtained by, for example, concentrating the reaction mixture, adding an organic solvent such as ethyl acetate, washing with water, then separating the organic layer containing the target compound, drying over anhydrous sodium sulfate or the like, and then distilling off the solvent.
The obtained compound can be further purified, if necessary, by a conventional method such as recrystallization, reprecipitation, or silica gel column chromatography.

### (Step A-1f)

The present step is a step of reducing the alkene using hydrogen in an inert solvent, in the presence of an appropriate metal catalyst.

As for the solvent to be used, the same one used in the above-described Step A-1a can be used.
There is no particular limitation on the metal catalyst to be used, but suitable examples thereof may include a metal catalyst such as palladium-activated carbon, platinum-activated carbon, nickel, and osmium-activated carbon.
Examples of the hydrogen source to be used may include hydrogen gas, ammonium formate, and hydrazine.
The reaction temperature varies depending on the raw material compounds, reagents, and other factors, but is normally -10°C to 150°C, and suitably 0°C to 80°C.
The reaction time varies depending on the raw material compounds, reagents, and other factors, but is normally 5 minutes to 24 hours, and suitably 10 minutes to 12 hours.
After termination of the reaction, the target compound of the present reaction can be obtained through, for example, steps such as concentration, separation, and drying in the same manner as in the above-described Step A-1a, and if necessary, may be further purified by a conventional method.
Column chromatography using a chiral column can provide an optically active compound.

### (Step A-2)

Step A-2 is a step of producing a compound represented by a general formula (4) from a compound represented by a general formula (3). Here, description will be given for a step of removing the tert-butoxycarbonyl group in the compound represented by the general formula (3) using an appropriate acid in an inert solvent, but it is not limited to this step.

As for the solvent to be used, the same one used in the above-described Step A-1a can be used.
There is no particular limitation on the acid to be used, as long as it is used as an acid in a normal reaction, but suitable examples thereof may include an inorganic acid such as hydrochloric acid, sulfuric acid, and nitric acid; and an organic acid such as trifluoroacetic acid. The reaction temperature varies depending on the raw material compounds, reagents, and other factors, but is normally -10°C to 100°C, and suitably 0°C to 50°C.

The reaction time varies depending on the raw material compounds, reagents, and other factors, but is normally 5 minutes to 24 hours, and suitably 10 minutes to 6 hours.

After termination of the reaction, the target compound of the present reaction can be obtained through, for example, steps such as concentration, separation, and drying in the same manner as in the above-described Step A-1a, and if necessary, may be further purified by a conventional method.

### (Step A-3)

Step A-3 is a step of producing the compound represented by the general formula (1) from the compound represented by the general formula (4).

The essential reaction includes Step A-3a: a reaction of converting the compound represented by the general formula (4) to an amide, or urea, or carbamate compound. In addition, if necessary, Step A-3b: a reaction of solvolyzing the ester or Step A-3c: a reaction of converting the pyruvic acid amide to a deuterated lactic acid amide can be added.

### (Step A-3a)

The present step is a step of converting the compound represented by the general formula (4) to an amide, or urea, or carbamate compound. Here, description will be given for a method (Step A-3a1) in which an acid chloride, or an isocyanic acid ester, or a chloroformic acid ester, or di-tert-butyl dicarbonate is used in the presence or absence of an appropriate base, for the compound represented by the general formula (4), and a method (Step A-3a2) in which the compound represented by the general formula (4) and a carboxylic acid are amidated using an appropriate condensing agent, but they are not limited to these methods.

### (Step A-3a1)

The present step is a step of converting the compound represented by the general formula (4) to an amide, or urea, or carbamate compound using an acid chloride, or an isocyanic acid ester, or a chloroformic acid ester in an inert solvent, in the presence or absence of an appropriate base.

As for the solvent to be used, the same one used in the above-described Step A-1a can be used.
As for the base to be used, the same one used in the above-described Step A-1a can be used.
There is no particular limitation on the acid chloride to be used, as long as it is used in a normal reaction, but examples thereof may include a carboxylic acid chloride such as acetyl chloride.
There is no particular limitation on the isocyanic acid ester to be used, as long as it is used in a normal reaction, but examples thereof include methyl isocyanate.
There is no particular limitation on the chloroformic acid ester to be used, as long as it is used in a normal reaction, but examples thereof include ethyl chloroformate.
The reaction temperature varies depending on the raw material compounds, reagents, and other factors, but is normally -20°C to 120°C, and suitably 0°C to 50°C.
The reaction time varies depending on the raw material compounds, reagents, and other factors, but is normally 5 minutes to 48 hours, and suitably 10 minutes to 24 hours.
After termination of the reaction, for example, the target compound of the present reaction can be obtained through, for example, steps such as concentration, separation, and drying in the same manner as in the above-described Step A-1a, and if necessary, may be further purified by a conventional method.

### (Step A-3a2)

The present step is a step of condensing the compound represented by the general formula (4) and a carboxylic acid, and is carried out using a condensing agent in an inert solvent, in the presence or absence of a base.

As for the solvent to be used, the same one used in the above-described Step A-1a can be used.
As for the base to be used, the same one used in the above-described Step A-1a can be used.
There is no particular limitation on the condensing agent to be used, as long as it is used as a condensing agent to form an amide bond (for example, the methods described in Shoichi Kusumoto et al., The Experimental Science IV; The Chemical Society of Japan; Maruzen, 1990, Nobuo Izumiya et al., Fundamentals and Experiments of Peptide Synthesis; Maruzen, 1985, and others), but suitable examples thereof may include O-(7-azabenzotriazol-1-yl)-N,N,N',N'-tetramethyluronium hexafluorophosphate (HATU) and O-(benzotriazol-1-yl)-N,N,N',N'-tetramethyluronium hexafluorophosphate (HBTU), as well as 2-(1H-benzotriazol-1-yl)-1,1,3,3-tetramethyluronium tetrafluoroborate (TBTU), 1-(3-dimethylaminopropyl)-3-ethylcarbodiimide hydrochloride (EDCI), 4-(2-{[(cyclohexylimino)methylene]amino}ethyl-4-methylmorpholi-4-nium paratoluenesulfonate (CMC), dicyclohexylcarbodiimide (DCC), 1,1'-carbonylbis(1H-imidazole) (CDI), (1H-benzotriazol-1-yloxy)(tripyrrolidin-1-yl)phosphonium hexafluorophosphate (PyBOP), bromo(tripyrrolidin-1-yl)phosphonium hexafluorophosphate (PyBrOP), 4-(4,6-dimethoxy-1,3,5-triazin-2-yl)-4-methylmorpholinium chloride (DMT-MM), and 2-chloro-4,6-dimethoxy-1,3,5-triazine (DMT). Furthermore, 1-hydroxybenzotriazole (HOBT), N,N-dimethylaminopyridine, or the like may be added as an additive.
The reaction temperature varies depending on the raw material compounds, reagents, and other factors, but is normally -10°C to 150°C, and suitably 0°C to 100°C.
The reaction time varies depending on the raw material compounds, reagents, and other factors, but is normally 5 minutes to 48 hours, and suitably 10 minutes to 24 hours.
After termination of the reaction, the target compound of the present reaction can be obtained through, for example, steps such as concentration, separation, and drying in the same manner as in the above-described Step A-1a, and if necessary, may be further purified by a conventional method.
Column chromatography using a chiral column can provide an optically active compound represented by the general formula (1).

### (Step A-3b)

The present step is a step of solvolyzing the carboxylic acid ester included in R¹³ in the compound represented by the general formula (1) in an inert solvent, in the presence of an appropriate base.

As for the solvent to be used, the same one used in the above-described Step A-1a can be used.
As for the base to be used, the same one used in the above-described Step A-1a can be used.
The reaction temperature varies depending on the raw material compounds, reagents, and other factors, but is normally -10°C to 100°C, and suitably 0°C to 60°C.
The reaction time varies depending on the raw material compounds, reagents, and other factors, but is normally 5 minutes to 48 hours, and suitably 10 minutes to 24 hours.
The target compound of the present reaction can be obtained through, for example, steps such as concentration, separation, and drying in the same manner as in the above-described Step A-1a, and if necessary, may be further purified by a conventional method. Column chromatography using a chiral column can provide an optically active compound represented by the general formula (1).

### (Step A-3c)

Step A-3c is a step of converting the pyruvic acid amide included in the compound represented by the general formula (1) to a deuterated lactic acid amide.

The essential reaction includes Step A-3c1: a reaction of converting the ketone to an alcohol by a deuterated reducing agent. In addition, if necessary, Step A-3c2: a reaction of deuterating the methyl group can be added before Step A-3c1.

### (Step A-3c1)

The present step is a step of converting the ketone to an alcohol in an inert solvent, in the presence of an appropriate deuterated reducing agent.

As for the solvent to be used, the same one used in the above-described Step A-1a can be used.
There is no particular limitation on the deuterated reducing agent to be used, as long as it is used in a normal reaction, but examples thereof include sodium borodeuteride, lithium aluminum deuteride, deuterium, and deuterated sodium formate.
The reaction temperature varies depending on the raw material compounds, reagents, and other factors, but is normally -20°C to 90°C, and suitably 0°C to 50°C.
The reaction time varies depending on the raw material compounds, reagents, and other factors, but is normally 5 minutes to 48 hours, and suitably 10 minutes to 24 hours.
After termination of the reaction, the target compound of the present reaction can be obtained through, for example, steps such as concentration, separation, and drying in the same manner as in the above-described Step A-1a, and if necessary, may be further purified by a conventional method.
Column chromatography using a chiral column can provide an optically active compound represented by the general formula (1).

### (Step A-3c2)

The present step is a step of deuterating the methyl group in the pyruvic acid amide in an inert solvent, in the presence of an appropriate base.
As for the solvent to be used, the same one used in the above-described Step A-1a can be used.
As for the base to be used, the same one used in the above-described Step A-1a can be used.
The reaction temperature varies depending on the raw material compounds, reagents, and other factors, but is normally -10°C to 100°C, and suitably 0°C to 60°C.
The reaction time varies depending on the raw material compounds, reagents, and other factors, but is normally 5 minutes to 72 hours, and suitably 10 minutes to 24 hours.
After termination of the reaction, the target compound of the present reaction can be subjected to the subsequent reaction without purification.

The compound represented by the general formula (1) of the present invention can also be obtained by the method described below as an alternative method.

### (Method B)

Method B is a method for producing the compound represented by the general formula (1) from the compound represented by the general formula (2). 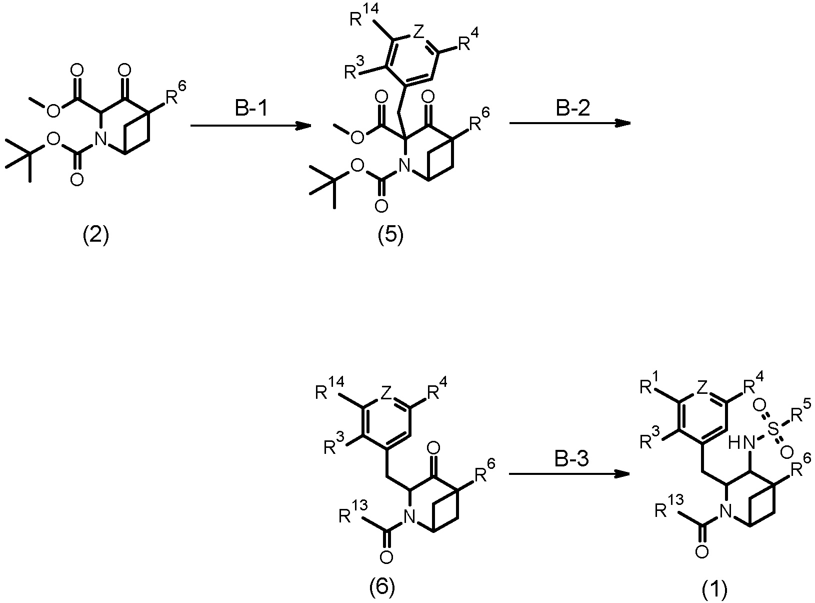 (In the formula, R¹ to R⁶ and Z indicate synonyms of the above, R¹³ represents R² or a precursor thereof, and R¹⁴ represents R¹ or a halogen atom.)

Hereinafter, each step will be described in detail.

### (Step B-1)

The present step is a step of condensing the compound represented by the general formula (2) and an alkyl bromide to produce a compound represented by a general formula (5), and is carried out in accordance with Step A-1a.

### (Step B-2)

Step B-2 is a step of producing a compound represented by a general formula (6) from the compound represented by the general formula (5).

The essential reactions include Step B-2a: a decarboxylation reaction involving elimination of the tert-butoxycarbonyl group, and Step B-2b: a reaction of converting to an amide or a carbamate.

### (Step B-2a)

The present step is a step of carrying out decarboxylation of the β-ketoester, involving elimination of the tert-butoxycarbonyl group, using an acid.
As for the solvent to be used, the same one used in the above-described Step A-1a can be used.
There is no particular limitation on the acid to be used, as long as it is used as an acid in a normal reaction, but suitable examples thereof may include an inorganic acid such as hydrochloric acid, sulfuric acid, and nitric acid.

The reaction temperature varies depending on the raw material compounds, reagents, and other factors, but is normally 0°C to 160°C, and suitably 80°C to 140°C.
The reaction time varies depending on the raw material compounds, reagents, and other factors, but is normally 5 minutes to 96 hours, and suitably 1 hour to 48 hours.
After termination of the reaction, the target compound of the present reaction can be obtained by, for example, distilling off the solvent in the reaction mixture.
The obtained compound can be further purified, if necessary, by a conventional method such as recrystallization, reprecipitation, or silica gel column chromatography.

### (Step B-2b)

The present step is a step of converting to the compound represented by the general formula (6), and is carried out in accordance with Step A-3a1.

### (Step B-3)

Step B-3 is a step of producing the compound represented by the general formula (1) from the compound represented by the general formula (6).

The essential reactions include Step B-3a: a reaction of converting the ketone to an amine, and Step B-3b: a reaction of sulfonylating or sulfamoylating the amine. In addition, if necessary, Step B-3c: a reaction of converting the bromine atom to an aryl group or a heteroaryl group, and Step B-3d: a reaction of solvolyzing the ester can be added.

### (Step B-3a)

The present step is a step of converting the ketone to an oxime and then reducing it to an amine, and is carried out in accordance with Step A-1c.

### (Step B-3b)

The present step is a step of sulfonylating or sulfamoylating the amine, and is carried out in accordance with Step A-1d.

### (Step B-3c)

The present step is a step of converting the bromine atom on the aryl ring or heteroaryl ring to an aryl group or a heteroaryl group, and is carried out in accordance with Step A-1e.

### (Step B-3d)

The present step is a step of solvolyzing the carboxylic acid ester included in R¹³ in the compound represented by the general formula (1), and is carried out in accordance with Step A-3b.

The compound represented by the general formula (1) of the present invention can also be obtained by the method described below as an alternative method.

### (Method C)

Method C is a method for producing the compound represented by the general formula (1) from the compound represented by the general formula (2).

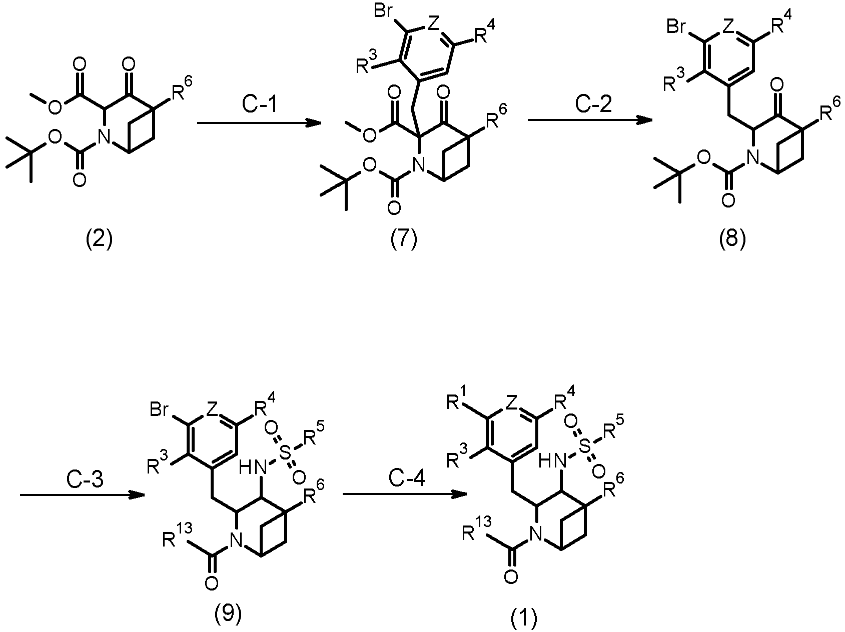

### (In the formula, R¹ to R⁶ and Z indicate synonyms of the above, and R¹³ represents R² or a precursor thereof.)

Hereinafter, each step will be described in detail.

### (Step C-1)

The present step is a step of producing a compound represented by a general formula (7) by a substitution reaction between the compound represented by the general formula (2) and an alkyl bromide, and is carried out in accordance with Step A-1a.

### (Step C-2)

Step C-2 is a step of producing a compound represented by a general formula (8) from the compound represented by the general formula (7) by a decarboxylation reaction, and is carried out in accordance with Step A-1b.

### (Step C-3)

Step C-3 is a step of producing a compound represented by a general formula (9) from the compound represented by the general formula (8).

The essential reactions include Step C-3a: an elimination reaction of the tert-butoxycarbonyl group, Step C-3b: a reaction of converting to an amide or a carbamate, Step C-3c: a reaction of converting the ketone to an amine, and Step C-3d: a reaction of sulfonylating or sulfamoylating the amine.

### (Step C-3a)

Step C-3a is a step of removing the tert-butoxycarbonyl group using an appropriate acid, and is carried out in accordance with Step A-2.

### (Step C-3b)

The present step is a step of converting to the compound represented by the general formula (9), and is carried out in accordance with Step A-3a1.

### (Step C-3c)

The present step is a step of converting the ketone to an oxime and then reducing it to an amine, and is carried out in accordance with Step A-1c.

### (Step C-3d)

The present step is a step of sulfonylating or sulfamoylating the amine, and is carried out in accordance with Step A-1d.

### (Step C-4)

Step C-4 is a step of producing the compound represented by the general formula (1) from the compound represented by the general formula (9).

The essential reaction includes Step C-4a: a reaction of converting the bromine atom on the aryl ring or heteroaryl ring to an aryl group or a heteroaryl group. In addition, if necessary, Step C-4b: a reaction of solvolyzing the ester can be added.

### (Step C-4a)

Step C-4a is a step of converting the bromine atom on the aryl ring or heteroaryl ring to an aryl group or a heteroaryl group, and is carried out in accordance with Step A-1e.

### (Step C-4b)

Step C-4b is a step of solvolyzing the carboxylic acid ester included in R¹³ in the compound represented by the general formula (1), and is carried out in accordance with Step A-3b.

### Examples

Hereinafter, the present invention will be specifically described with reference to Examples, but the present invention is not limited to them and they are not to be construed as limiting in any sense. Also, reagents, solvents, and starting materials not specifically described herein are readily available from commercial supply sources.

The abbreviations used in Examples have the following meanings.
mg: milligram, g: gram, mL: milliliter, mol: mole, MHz: megahertz, Boc: tert-butoxycarbonyl group, D: deuterium, MS (mass spectrometry), ESI (electrospray ionization), APCI (atmospheric pressure chemical ionization), DMSO (dimethyl sulfoxide)

In the following Examples, as for the nuclear magnetic resonance (hereinafter, ¹H NMR: 400 MHz) spectrum, chloroform-d, methanol-d4, and dimethyl sulfoxide-d6 were used as deuterated solvents, tetramethylsilane was used as the reference material, and the chemical shift values are described in terms of δ values (ppm). As for the splitting pattern, s stands for singlet, d for doublet, dd for double doublet, dt for double triplet, t for triplet, td for triple doublet, q for quartet, m for multiplet, and br for broad.

Also, only for the present Examples shown below, the term "single diastereomer" means that the ratio of a specific diastereomer (this may be a racemate):stereoisomers other than the specific diastereomer is 20:1 (20/1) or more, and the term "single enantiomer" means 96% ee (er = 98/2) or more.

### Example 1

### N-{(3S,4S)-2-(2-Hydroxy-2-methylpropanoyl)-3-[(2,3',5'-trifluoro[biphenyl]-3-yl)methyl]-2-azabicyclo[3.1.1]heptan-4-yl}methanesulfonamide

### (1A) 3-(Bromomethyl)-2,3',5'-trifluoro[biphenyl]

A mixture of 1,3-difluoro-5-(2-fluoro-3-methylphenyl)benzene (CAS Registration No.: 2409648-15-1, 50 g), N-bromosuccinimide (50 g), azobisisobutyronitrile (4.0 g), and acetonitrile (500 mL) was degassed and then stirred at 80°C for 6 hours in a nitrogen atmosphere. The solvent was distilled off under reduced pressure, and the residue was purified by reverse phase silica gel column chromatography [eluting solvent: 0.225% aqueous formic acid solution/acetonitrile = 40/60 to 20/80 (V/V)] to afford 55 g (yield: 81%) of the marked compound.

### (1B) 2-tert-Butyl 3-methyl 4-oxo-3-[(2,3',5'-trifluoro[biphenyl]-3-yl)methyl]-2-azabicyclo[3.1.1]heptane-2,3-dicarboxylate

To a mixture of O4-tert-butyl O3-methyl 2-oxo-4-azabicyclo[3.1.1]heptane-3,4-dicarboxylate (CAS Registration No.: 2241048-56-4, 0.600 g) and N,N-dimethylformamide (9.0 mL), the compound synthesized in Example 1A (0.671 g) and cesium carbonate (1.45 g) were added and the mixture was stirred at room temperature for 4 hours. A saturated aqueous ammonium chloride solution and water were added to the reaction mixture, and the reaction mixture was extracted with ethyl acetate. The organic layer was washed with water and a saturated saline solution, and dried over anhydrous sodium sulfate. After filtration, the residue obtained by distilling off the solvent under reduced pressure was purified by silica gel column chromatography [eluting solvent: n-hexane/ethyl acetate = 100/0 to 67/33 (V/V)] to afford 0.77 g (yield: 71%) of the marked compound.

### (1C) 2-Methyl-1-oxo-1-{4-oxo-3-[(2,3',5'-trifluoro[biphenyl]-3-yl)methyl]-2-azabicyclo[3.1.1]heptan-2-yl}propan-2-yl acetate

To a mixture of the compound synthesized in Example 1B (2.86 g) and acetic acid (5.72 mL), hydrochloric acid (12 M, 28.6 mL) was added and the mixture was stirred at room temperature for 30 minutes. Lithium chloride (0.248 g) was added to the reaction mixture, which was stirred at 100°C for 6 hours and cooled to room temperature. The solvent was distilled off under reduced pressure, toluene was added, and the mixture was azeotroped 3 times. To a mixture of the resulting residue and tetrahydrofuran (43 mL), triethylamine (4.05 mL) and 2-acetoxyisobutyryl chloride (1.27 mL) were added at 0°C and the mixture was stirred at room temperature for 2 hours. A saturated aqueous ammonium chloride solution and water were added to the reaction mixture, which was then extracted with ethyl acetate. The organic layer was washed with a saturated saline solution, and dried over anhydrous sodium sulfate. After filtration, the solvent was distilled off under reduced pressure, and the residue was purified by silica gel column chromatography [eluting solvent: n-hexane/ethyl acetate = 100/0 to 0/100 (V/V)] to afford 1.99 g (yield: 74%) of the marked compound.

### (1D) 1-{4-Amino-3-[(2,3',5'-trifluoro[biphenyl]-3-yl)methyl]-2-azabicyclo[3.1.1]heptan-2-yl2-methyl-1-oxopropan-2-yl acetate

To a mixture of the compound synthesized in Example 1C (0.620 g) and ethanol (6.2 mL), hydroxylamine hydrochloride (0.328 g) and sodium acetate (0.387 g) were added, and the mixture was stirred at 80°C for 2 hours and cooled to room temperature. The solvent was distilled off under reduced pressure, water and a saturated saline solution were added, and the mixture was extracted with ethyl acetate. The organic layer was dried over anhydrous sodium sulfate, and after filtration, the solvent was distilled off under reduced pressure. To a mixture of the resulting residue and methanol (6.2 mL), molybdenum(VI) oxide (0.583 g) was added at 0°C. Sodium borohydride (1.02 g) was slowly added little by little and the mixture was stirred at 0°C for 2 hours. After adding a saturated aqueous sodium bicarbonate solution and ethyl acetate to the reaction mixture, Celite filtration was carried out, followed by washing with ethyl acetate. The filtrate was concentrated under reduced pressure and extracted with ethyl acetate. The organic layer was washed with a saturated saline solution, and dried over anhydrous sodium sulfate. After filtration, the solvent was distilled off under reduced pressure to afford 0.622 g of the crude marked compound.

### (1E) 2-Methyl-1-{(3S*,4S*)-4-[(methylsulfonyl)amino]-3-[(2,3',5'-trifluoro[biphenyl]-3-yl)methyl]-2-azabicyclo[3.1.1]heptan-2-yl}-1-oxopropan-2-yl acetate

To a mixture of the compound synthesized in Example 1D (205 mg), triethylamine (0.371 mL), and tetrahydrofuran (4.1 mL), methanesulfonyl chloride (0.104 mL) was added under ice cooling and the mixture was stirred at room temperature for 2 hours. After adding a saturated aqueous sodium bicarbonate solution, the mixture was extracted with ethyl acetate, and the organic layer was dried over sodium sulfate. After filtration, the solvent was distilled off under reduced pressure, and the residue was purified by silica gel column chromatography [eluting solvent: n-hexane/ethyl acetate = 100/0 to 0/100 (V/V)] to afford 167 mg (yield: 70%) of the marked compound as a single diastereomer (racemate).

### (1F) N-{ (3S* ,4S*)-2-(2-Hydroxy-2-methylpropanoyl)-3-[(2,3',5'-trifluoro[biphenyl]-3-yl)methyl]-2-azabicyclo[3.1.1] heptan-4-yl}methanesulfonamide

To a mixture of the compound synthesized in Example 1E (167 mg), tetrahydrofuran (2.00 mL), and water (0.668 mL), lithium hydroxide monohydrate (0.26 g) was added at room temperature and the mixture was stirred at 50°C for 12 hours. After adding water, the mixture was extracted with ethyl acetate, and the organic layer was washed with a saturated saline solution and dried over sodium sulfate. After filtration, the solvent was distilled off under reduced pressure, and the residue was purified by NH silica gel column chromatography [eluting solvent: n-hexane/ethyl acetate = 50/50 to 0/100 (V/V)] to afford 0.154 g (yield: quantitative) of the marked compound as a single diastereomer (racemate).

### (1G) N-{(3S,4S)-2-(2-Hydroxy-2-methylpropanoyl)-3-[(2,3',5'-trifluoro[biphenyl]-3-yl)methyl]-2-azabicyclo[3.1.1] heptan-4-yl}methanesulfonamide

The compound synthesized in Example 1F (154 mg) was subjected to chiral HPLC [column: CHIRALPAK (R) IA (20 mm I.D. × 250 mm), mobile phase: n-hexane/ethanol = 70/30 (V/V), temperature: 40°C] to afford 41.3 mg (yield: 27%) of the marked compound, which was eluted first, as a single diastereomer and as a single enantiomer.

### Example 2

### N-{(3S,4S)-2-(2-Hydroxy-2-methylpropanoyl)-3-[(2,3',5'-trifluoro[biphenyl]-3-yl)methyl]-2-azabicyclo[3.1.1]heptan-4-yl}-N,N-dimethylsulfamide

### (2A) 1-{4-(Hydroxyimino)-3-[(2,3',5'-trifluoro[biphenyl]-3-yl)methyl]-2-azabicyclo[3.1.1]heptan-2-yl}-2-methyl-1-oxopropan-2-yl acetate

To a mixture of the compound synthesized in Example 1C (55 g) and ethanol (700 mL), hydroxylamine hydrochloride (30 g) and sodium acetate (44 g) were added at room temperature, and the mixture was then stirred at 80°C for 2 hours. The mixture was diluted with ethyl acetate, washed with water and a saturated saline solution, and then dried over sodium sulfate. After filtration, the solvent was distilled off under reduced pressure to afford 60 g of the crude marked compound.

### (2B) 1-{(3S*,4S*)-4-Amino-3-[(2,3',5'-trifluoro[biphenyl]-3-yl)methyl]-2-azabicyclo[3.1.1]heptan-2-yl}-2-methyl-1-oxopropan-2-yl acetate

To a mixture of the compound synthesized in Example 2A (60 g), molybdenum(VI) oxide (27 g), and methanol (1000 mL), sodium borohydride (50 g) was added at 0°C, and the mixture was then stirred at room temperature for 2 hours. The mixture was added to an aqueous ammonium chloride solution (500 mL), and the insoluble material was removed by filtration. The filtrate was extracted twice with ethyl acetate, and the organic layers were combined and washed with a saturated saline solution. After drying over sodium sulfate, the organic layer was filtered and the solvent was distilled off under reduced pressure. The residue was purified by reverse phase silica gel column chromatography [eluting solvent: 0.1% aqueous trifluoroacetic acid solution/acetonitrile = 72/28 to 52/48 (V/V)] to afford 30 g (yield: 52%) of the marked compound as a single diastereomer.

### (2C) 1-{(3S,4S)-4-Amino-3-[(2,3',5'-trifluoro[biphenyl]-3-yl)methyl]-2-azabicyclo[3.1.1]heptan-2-yl}-2-methyl-1-oxopropan-2-yl acetate

The compound synthesized in Example 2B (23 g) was subjected to chiral SFC [column: CHIRALPAK (R) AD (50 mm I.D. × 250 mm), mobile phase: carbon dioxide/2-propanol = 60/40 (V/V)] to afford 11 g (yield: 44%) of the marked compound, which was eluted first, as a single diastereomer and as a single enantiomer.

### (2D) 1-{(3S,4S)-4-[(Dimethylsulfamoyl)amino]-3-[(2,3',5'-trifluoro[biphenyl]-3-yl)methyl]-2-azabicyclo[3.1.1]heptan-2-yl}-2-methyl-1-oxopropan-2-yl acetate

To a mixture of the compound synthesized in Example 2C (100 mg), N,N-dimethylaminopyridine (30 mg), N,N-diisopropylethylamine (74 mg), and tetrahydrofuran (5.0 mL), dimethylsulfamoyl chloride (50 mg) was added at room temperature and the mixture was stirred at 40°C for 10 hours. The solvent was distilled off under reduced pressure, and the residue was purified by reverse phase silica gel column chromatography [eluting solvent: 10 mM aqueous ammonium carbonate solution/acetonitrile = 59/41 to 29/71 (V/V)] to afford 30 mg (yield: 24%) of the marked compound as a single diastereomer and as a single enantiomer.

### (2E) N'-{(3S,4S)-2-(2-Hydroxy-2-methylpropanoyl)-3-[(2,3',5'-trifluoro[biphenyl]-3-yl)methyl]-2-azabicyclo[3.1.1] heptan-4-yl}-N,N-dimethylsulfamide

To a mixture of the compound synthesized in Example 2D (30 mg), water (1.0 mL), and methanol (5.0 mL), lithium hydroxide monohydrate (6.0 mg) was added at room temperature and the mixture was stirred for 10 hours. The solvent was distilled off under reduced pressure, and the residue was purified by reverse phase silica gel column chromatography [eluting solvent: 0.225% aqueous formic acid solution/acetonitrile = 52/48 to 22/78 (V/V)] to afford 9.2 mg (yield: 33%) of the marked compound as a single diastereomer and as a single enantiomer.

### Example 3

### N-{(3S,4S)-2-(2-Hydroxy-2-methylpropanoyl)-3-[(2,3',5'-trifluoro[biphenyl]-3-yl)methyl]-2-azabicyclo[3.1.1]heptan-4-yl}ethanesulfonamide

### (3A) 1-{(3S,4S)-4-[(Ethylsulfonyl)amino]-3-[(2,3',5'-trifluoro[biphenyl]-3-yl)methyl]-2-azabicyclo[3.1.1]heptan-2-yl}-2-methyl-1-oxopropan-2-yl acetate

To a mixture of the compound synthesized in Example 2C (1.00 g), triethylamine (0.602 mL), and tetrahydrofuran (20 mL), ethanesulfonyl chloride (0.267 mL) was added under ice cooling and the mixture was stirred at room temperature for 6 hours. After adding a saturated aqueous sodium bicarbonate solution, the mixture was extracted with ethyl acetate, and after washing with a saturated saline solution, the organic layer was dried over sodium sulfate. After filtration, the solvent was distilled off under reduced pressure, and the residue was purified by silica gel column chromatography [eluting solvent: n-hexane/ethyl acetate = 100/0 to 0/100 (V/V)] to afford 1.20 g (yield: quantitative) of the marked compound as a single diastereomer and as a single enantiomer.

### (3B) N-{(3S,4S)-2-(2-Hydroxy-2-methylpropanoyl)-3-[(2,3',5'-trifluoro[biphenyl]-3-yl)methyl]-2-azabicyclo[3.1.1] heptan-4-yl}ethanesulfonamide

To a mixture of the compound synthesized in Example 3A (1.20 g), tetrahydrofuran (14 mL), and water (4.8 mL), lithium hydroxide monohydrate (1.82 g) was added at room temperature and the mixture was stirred at 50°C for 12 hours. After adding a saturated saline solution, the mixture was extracted with ethyl acetate, and the organic layer was washed with a saturated saline solution and dried over sodium sulfate. After filtration, the solvent was distilled off under reduced pressure, and the residue was purified by NH silica gel column chromatography [eluting solvent: n-hexane/ethyl acetate = 50/50 to 0/100 (V/V)] to afford 0.448 g (yield: 40%) of the marked compound as a single diastereomer and as a single enantiomer.

### Example 4

### N-{(3S,4S)-2-(2-Hydroxy-2-methylpropanoyl)-3-[(2,3',5'-trifluoro[biphenyl]-3-yl)methyl]-2-azabicyclo[3.1.1]heptan-4-yl}propane-2-sulfonamide

### (4A) 2-Methyl-1-oxo-1-{(3S,4S)-4-[(propan-2-ylsulfonyl)amino]-3-[(2,3',5'-trifluoro[biphenyl]-3-yl)methyl]-2-azabicyclo[3.1.1]heptan-2-yl}propan-2-yl acetate

To a mixture of the compound synthesized in Example 2C (50 mg) and tetrahydrofuran (1.0 mL), 1,8-diazabicyclo[5.4.0]-7-undecene (41 mg) and isopropylsulfonyl chloride (23 mg) were added at 0°C, and the mixture was then stirred at room temperature for 12 hours. The reaction solution was poured into water and extracted with ethyl acetate. The organic layer was washed with a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 45/55 to 25/75 (V/V)] to afford 15 mg (yield: 24%) of the marked compound as a single diastereomer and as a single enantiomer.

### (4B) N-{(3S,4S)-2-(2-Hydroxy-2-methylpropanoyl)-3-[(2,3',5'-trifluoro[biphenyl]-3-yl)methyl]-2-azabicyclo[3.1.1] heptan-4-yl}propane-2-sulfonamide

To a mixture of the compound synthesized in Example 4A (15 mg), water (0.3 mL), and methanol (1.5 mL), lithium hydroxide monohydrate (4.0 mg) was added at room temperature and the mixture was stirred for 12 hours. The solvent was distilled off under reduced pressure, and the residue was purified by reverse phase silica gel column chromatography [eluting solvent: 0.225% aqueous formic acid solution/acetonitrile = 52/48 to 32/68 (V/V)] to afford 10 mg (yield: 74%) of the marked compound as a single diastereomer and as a single enantiomer.

### Example 5

### N-{2-(Bicyclo[1.1.1]pentan-1-ylcarbonyl)-3-[(2,3',5'-trifluoro[biphenyl]-3-yl)methyl]-2-azabicyclo[3.1.1]heptan-4-yl}ethanesulfonamide

### (5A) 3-[(2,3',5'-Trifluoro[biphenyl]-3-yl)methyl]-2-azabicyclo[3.1.1]heptan-4-one hydrochloride

To a mixture of the compound synthesized in Example 1B (63 g), lithium chloride (5.4 g), and acetic acid (320 mL), hydrochloric acid (12 M, 320 mL) was added at room temperature and the mixture was stirred at 100°C for 12 hours. The solvent was distilled off under reduced pressure to afford 55 g of the crude marked compound.

### (5B) tert-Butyl 4-oxo-3-[(2,3',5'-trifluoro[biphenyl]-3-yl)methyl]-2-azabicyclo[3.1.1]heptane-2-carboxylate

To a mixture of the compound synthesized in Example 5A (23 g), triethylamine (33 g), and tetrahydrofuran (500 mL), di-tert-butyl dicarbonate (18 g) was added at room temperature and the mixture was stirred for 1 hour. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: petroleum ether/ethyl acetate = 10/1 to 5/1 (V/V)] to afford 11 g (yield: 41%) of the marked compound.

### (5C) tert-Butyl 4-(hydroxyimino)-3-[(2,3',5'-trifluoro[biphenyl]-3-yl)methyl]-2-azabicyclo[3.1.1]heptane-2-carboxylate

Using the compound synthesized in Example 5B (11 g), 12 g of the crude marked compound was obtained by the same method as in Example 2A.

### (5D) tert-Butyl 4-amino-3-[(2,3',5'-trifluoro[biphenyl]-3-yl)methyl]-2-azabicyclo[3.1.1]heptane-2-carboxylate

To a mixture of the compound synthesized in Example 5C (12 g), molybdenum(VI) oxide (5.8 g), and methanol (200 mL), sodium borohydride (10 g) was added at 0°C, and the mixture was then stirred at room temperature for 2 hours. The mixture was added to an aqueous ammonium chloride solution (500 mL), and the insoluble material was removed by filtration. The filtrate was extracted twice with ethyl acetate, and the organic layers were combined and washed with a saturated saline solution. After drying over sodium sulfate, the solvent was distilled off under reduced pressure. The residue was purified by reverse phase silica gel column chromatography [eluting solvent: 0.1% aqueous trifluoroacetic acid solution/acetonitrile = 70/30 to 45/55 (V/V)] to afford 7.0 g (yield: 58%) of the marked compound as a single diastereomer (racemate).

### (5E) tert-Butyl 4-amino-3-[(2,3',5'-trifluoro[biphenyl]-3-yl)methyl]-2-azabicyclo[3.1.1]heptane-2-carboxylate

The compound synthesized in Example 5D (7.0 g) was subjected to chiral SFC [column: CHIRALPAK (R) IG (50 mm I.D. × 250 mm), mobile phase: carbon dioxide/2-propanol = 55/45 (V/V)] to afford 2.3 g (yield: 33%) of the marked compound, which was eluted first, as a single diastereomer and as a single enantiomer.

### (5F) tert-Butyl 4-[(ethylsulfonyl)amino]-3-[(2,3',5'-trifluoro[biphenyl]-3-yl)methyl]-2-azabicyclo[3.1.1]heptane-2-carboxylate

To a mixture of the compound synthesized in Example 5E (1.4 g), N,N-diisopropylethylamine (1.0 g), and tetrahydrofuran (20 mL), ethanesulfonyl chloride (952 mg) was added at room temperature and the mixture was stirred for 10 hours. The solvent was distilled off under reduced pressure, and the residue was purified by reverse phase silica gel column chromatography [eluting solvent: 0.225% aqueous formic acid solution/acetonitrile = 50/50 to 20/80 (V/V)] to afford 0.90 g (yield: 53%) of the marked compound as a single diastereomer and as a single enantiomer.

### (5G) N-{3-[(2,3',5'-Trifluoro[biphenyl]-3-yl)methyl]-2-azabicyclo[3.1.1]heptan-4-yl}ethanesulfonamide hydrochloride

To a mixture of the compound synthesized in Example 5F (0.90 g) and ethyl acetate (10 mL), a solution of hydrogen chloride in ethyl acetate (4 M, 8.0 mL) was added at room temperature and the mixture was stirred for 10 hours. The solvent was distilled off under reduced pressure to afford 0.80 g of the crude marked compound as a single diastereomer and as a single enantiomer.

### (5H) N-{2-(Bicyclo[1.1.1]pentan-1-ylcarbonyl)-3-[(2,3',5'-trifluoro[biphenyl]-3-yl)methyl]-2-azabicyclo[3.1.1]heptan-4-yl}ethanesulfonamide

To a mixture of bicyclo[1.1.1]pentane-1-carboxylic acid (CAS Registration No.: 22287-28-1, 10 mg) and N,N-dimethylformamide (2.0 mL), O-(7-azabenzotriazol-1-yl)-N,N,N',N'-tetramethyluronium hexafluorophosphate (40 mg) was added at room temperature and the mixture was stirred at 35°C for 10 minutes. After adding the compound synthesized in Example 5G (30 mg) and triethylamine (35 mg), the mixture was stirred at 35°C for 2 hours. After removing the insoluble material by filtration, the filtrate was concentrated, and the residue was purified by reverse phase silica gel column chromatography [eluting solvent: 0.225% aqueous formic acid solution/acetonitrile = 43/57 to 23/77 (V/V)] to afford 14 mg (yield: 41%) of the marked compound as a single diastereomer and as a single enantiomer.

### Example 6

### 1-Fluoro-N-{2-(2-hydroxy-2-methylpropanoyl)-3-[(2,3',5'-trifluoro[biphenyl]-3-yl)methyl]-2-azabicyclo[3.1.1]heptan-4-yl}methanesulfonamide

### (6A) tert-Butyl 4-{[(fluoromethyl)sulfonyl]amino}-3-[(2,3',5'-trifluoro[biphenyl]-3-yl)methyl]-2-azabicyclo[3.1.1]heptane-2-carboxylate

Using the compound synthesized in Example 5E (50 mg) and fluoromethanesulfonyl chloride (20 mg), 40 mg (yield: 65%) of the marked compound was obtained as a single diastereomer and as a single enantiomer by the same method as in Example 5F.

### (6B) 1-Fluoro-N-{3-[(2,3',5'-trifluoro[biphenyl]-3-yl)methyl]-2-azabicyclo[3.1.1]heptan-4-yl}methanesulfonamide hydrochloride

To a mixture of the compound synthesized in Example 6A (40 mg) and ethyl acetate (2.0 mL), a solution of hydrogen chloride in ethyl acetate (4 M, 3.0 mL) was added at room temperature and the mixture was stirred for 10 hours. The solvent was distilled off under reduced pressure to afford 40 mg of the crude marked compound as a single diastereomer and as a single enantiomer.

### (6C) 1-(4-{[(Fluoromethyl)sulfonyl]amino}-3-[(2,3',5'-trifluoro[biphenyl]-3-yl)methyl]-2-azabicyclo[3.1.1]heptan-2-yl)-2-methyl-1-oxopropan-2-yl acetate

To a mixture of the compound synthesized in Example 6B (40 mg), triethylamine (28.3 mg), and tetrahydrofuran (5.0 mL), 2-acetoxyisobutyryl chloride (30 mg) was added at room temperature, and the mixture was then stirred for 10 hours. The mixture was diluted with ethyl acetate, washed with water and a saturated saline solution, and then dried over sodium sulfate. After filtration, the solvent was distilled off under reduced pressure to afford 50 mg of the crude marked compound as a single diastereomer and as a single enantiomer.

### (6D) 1-Fluoro-N-{2-(2-hydroxy-2-methylpropanoyl)-3-[(2,3',5'-trifluoro[biphenyl]-3-yl)methyl]-2-azabicyclo[3.1.1] heptan-4-yl}methanesulfonamide

To a mixture of the compound synthesized in Example 6C (50 mg), water (1.0 mL), and methanol (5.0 mL), lithium hydroxide monohydrate (18.9 mg) was added at room temperature and the mixture was stirred for 10 hours. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 55/45 to 35/65 (V/V)] to afford 11 mg (yield: 24%) of the marked compound as a single diastereomer and as a single enantiomer.

### Example 7

### 4-[(Ethylsulfonyl)amino]-N,N-dimethyl-3-[(2,3',5'-trifluoro[biphenyl]-3-yl)methyl]-2-azabicyclo[3.1.1]heptane-2-carboxamide

### (7A) 4-[(Ethylsulfonyl)amino]-N,N-dimethyl-3-[(2,3',5'-trifluoro[biphenyl]-3-yl)methyl]-2-azabicyclo[3.1.1]heptane-2-carboxamide

To a mixture of the compound synthesized in Example 5G (50 mg), triethylamine (33 mg), and tetrahydrofuran (5.0 mL), N,N-dimethylcarbamoyl chloride (17 mg) was added at room temperature and the mixture was stirred for 10 hours. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 53/47 to 23/77 (V/V)] to afford 15 mg (yield: 28%) of the marked compound as a single diastereomer and as a single enantiomer.

### Example 8

### N-{(3S,4S)-2-(2-Hydroxy-2-methylpropanoyl)-3-[(2,2',3'-trifluoro[biphenyl]-3-yl)methyl]-2-azabicyclo[3.1.1]heptan-4-yl}methanesulfonamide

### (8A) 2-tert-Butyl 3-methyl 3-(3-bromo-2-fluorobenzyl)-4-oxo-2-azabicyclo[3.1.1]heptane-2,3-dicarboxylate

To a mixture of O4-tert-butyl O3-methyl 2-oxo-4-azabicyclo[3.1.1]heptane-3,4-dicarboxylate (CAS Registration No.: 2241048-56-4, 5.0 g), 1-bromo-3-(bromomethyl)-2-fluorobenzene (CAS Registration No.: 149947-16-0, 6.0 g), and N,N-dimethylformamide (60 mL), cesium carbonate (9.0 g) was added at room temperature and the mixture was stirred for 3 hours. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: petroleum ether/ethyl acetate = 1/0 to 9/1 (V/V)] to afford 6.0 g (yield: 71%) of the marked compound.

### (8B) 3-(3-Bromo-2-fluorobenzyl)-2-azabicyclo[3.1.1]heptan-4-one hydrochloride

To a mixture of the compound synthesized in Example 8A (6.0 g), lithium chloride (557 mg), and acetic acid (30 mL), hydrochloric acid (12 M, 30 mL) was added and the mixture was stirred at 100°C for 12 hours. The solvent was distilled off under reduced pressure to afford 10 g of the crude marked compound.

### (8C) tert-Butyl 3-(3-bromo-2-fluorobenzyl)-4-oxo-2-azabicyclo[3.1.1]heptane-2-carboxylate

To a mixture of the compound synthesized in Example 8B (6.0 g) and tetrahydrofuran (60 mL), di-tert-butyl dicarbonate (6.0 g) and triethylamine (9.0 g) were added at 0°C and the mixture was stirred at 0°C for 2 hours. The mixture was diluted with ethyl acetate, washed with water and a saturated saline solution, and dried over sodium sulfate. After filtration, the solvent was distilled off under reduced pressure, and the residue was purified by silica gel column chromatography [eluting solvent: petroleum ether/ethyl acetate = 1/0 to 9/1 (V/V)] to afford 3.2 g (yield: 45%) of the marked compound.

### (8D) tert-Butyl 3-(3-bromo-2-fluorobenzyl)-4-(hydroxyimino)-2-azabicyclo[3.1.1]heptane-2-carboxylate

A mixture of the compound synthesized in Example 8C (3.2 g), hydroxylamine hydrochloride (2.5 g), sodium acetate (2.6 g), and ethanol (40 mL) was stirred at 80°C for 12 hours. The mixture was diluted with ethyl acetate, washed with water and a saturated saline solution, and then dried over sodium sulfate. After filtration, the solvent was distilled off under reduced pressure to afford 3.0 g of the crude marked compound.

### (8E) tert-Butyl (3S*,4S*)-4-amino-3-(3-bromo-2-fluorobenzyl)-2-azabicyclo[3.1.1]heptane-2-carboxylate

To a mixture of the compound synthesized in Example 8D (3.0 g), molybdenum(VI) oxide (1.6 g), and methanol (100 mL), sodium borohydride (6.0 g) was added at 0°C, and the mixture was then stirred at room temperature for 3 hours. The reaction solution was poured into an aqueous ammonium chloride solution and extracted with ethyl acetate. The organic layer was washed with a saturated saline solution, then dried over sodium sulfate, and filtered. The solvent was distilled off under reduced pressure, and purification by reverse phase silica gel column chromatography [eluting solvent: 0.225% aqueous formic acid solution/acetonitrile = 80/20 to 50/50 (V/V)] was performed to afford the marked compound as a single diastereomer (racemate).

### (8F) tert-Butyl (3S,4S)-4-amino-3-(3-bromo-2-fluorobenzyl)-2-azabicyclo[3.1.1]heptane-2-carboxylate

The entire amount of the compound synthesized in Example 8E was subjected to chiral SFC [column: CHIRALPAK (R) AD (30 mm I.D. × 250 mm), mobile phase: carbon dioxide/2-propanol = 70/30 (V/V)] to afford 800 mg (yield: 28%) of the marked compound, which was eluted first, as a single diastereomer and as a single enantiomer.

### (8G) tert-Butyl (3S,4S)-3-(3-bromo-2-fluorobenzyl)-4-[(methylsulfonyl)amino]-2-azabicyclo[3.1.1]heptane-2-carboxylate

To a mixture of the compound synthesized in Example 8F (1.0 g), N,N-diisopropylethylamine (0.97 g), and tetrahydrofuran (20 mL), methanesulfonyl chloride (0.57 g) was added at 0°C, and the mixture was then stirred at room temperature for 2 hours. The reaction solution was poured into an aqueous sodium bicarbonate solution and extracted with ethyl acetate. The organic layer was washed with a saturated saline solution, and then dried over sodium sulfate. After filtration, the solvent was distilled off under reduced pressure, and purification by reverse phase silica gel column chromatography [eluting solvent: 0.225% aqueous formic acid solution/acetonitrile = 57/43 to 27/73 (V/V)] was performed to afford 1.0 g (yield: 84%) of the marked compound as a single diastereomer and as a single enantiomer.

### (8H) N-[(3S,4S)-3-(3-Bromo-2-fluorobenzyl)-2-azabicyclo[3.1.1]heptan-4-yl]methanesulfonamide hydrochloride

To a mixture of the compound synthesized in Example 8G (1.0 g) and methanol (0.5 mL), a solution of hydrogen chloride in 1,4-dioxane (4 M, 20 mL) was added and the mixture was stirred at room temperature for 1 hour. The solvent was distilled off under reduced pressure to afford 0.80 g of the crude marked compound as a single diastereomer and as a single enantiomer.

### (8I) 1-{(3S,4S)-3-(3-Bromo-2-fluorobenzyl)-4-[(methylsulfonyl)amino]-2-azabicyclo[3.1.1]heptan-2-yl}-2-methyl-1-oxopropan-2-yl acetate

To a mixture of the compound synthesized in Example 8H (0.80 g), N,N-diisopropylethylamine (1.25 g), and tetrahydrofuran (10 mL), 2-acetoxyisobutyryl chloride (0.32 g) was added at room temperature, and the mixture was then stirred for 1 hour. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 58/42 to 38/62 (V/V)] to afford 700 mg (yield: 72%) of the marked compound as a single diastereomer and as a single enantiomer.

### (8J) 2-Methyl-1-{(3S,4S)-4-[(methylsulfonyl)amino]-3-[(2,2',3'-trifluoro[biphenyl]-3-yl)methyl]-2-azabicyclo[3.1.1]heptan-2-yl}-1-oxopropan-2-yl acetate

A mixture of the compound synthesized in Example 8I (50 mg), 2,3-difluorophenylboronic acid (19 mg), tripotassium phosphate (63 mg), (2-dicyclohexylphosphino-2',4',6'-triisopropyl-1,1'-biphenyl)[2-(2'-amino-1,1'-biphenyl)]palladium(II) methanesulfonate (4.0 mg), water (0.3 mL), and tetrahydrofuran (2.0 mL) was degassed and then stirred at 70°C for 2 hours in a nitrogen atmosphere. The mixture was diluted with ethyl acetate, washed with water and a saturated saline solution, and then dried over sodium sulfate. After filtration, the solvent was distilled off under reduced pressure, and the residue was purified by preparative thin layer silica gel chromatography [eluting solvent: petroleum ether/ethyl acetate = 1/1 (V/V)] to afford 30 mg (yield: 56%) of the marked compound as a single diastereomer and as a single enantiomer.

### (8K) N-{(3S,4S)-2-(2-Hydroxy-2-methylpropanoyl)-3-[(2,2',3'-trifluoro[biphenyl]-3-yl)methyl]-2-azabicyclo[3.1.1] heptan-4-yl}methanesulfonamide

To a mixture of the compound synthesized in Example 8J (30 mg), water (0.4 mL), and methanol (2.0 mL), lithium hydroxide (7.0 mg) was added at room temperature and the mixture was stirred for 1 hour. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 61/39 to 41/59 (V/V)] to afford 34 mg (yield: 61%) of the marked compound as a single diastereomer and as a single enantiomer.

### Example 9

### N-{2-(Azetidin-1-ylcarbonyl)-3-[(2,3',5'-trifluoro[biphenyl]-3-yl)methyl]-2-azabicyclo[3.1.1]heptan-4-yl}ethanesulfonamide

### (9A) N-{2-(Azetidin-1-ylcarbonyl)-3-[(2,3',5'-trifluoro[biphenyl]-3-yl)methyl]-2-azabicyclo[3.1.1] heptan-4-yl}ethanesulfonamide

To a mixture of the compound synthesized in Example 5G (100 mg) and dichloromethane (8.0 mL), triethylamine (66 mg) and 4-nitrophenyl chloroformate (55 mg) were added at 0°C, and the mixture was then stirred at room temperature for 12 hours. The solvent was distilled off under reduced pressure and the residue was added to a mixture of azetidine hydrochloride (89 mg), N,N-diisopropylethylamine (84 mg), and N,N-dimethylformamide (5.0 mL), which was stirred at 80°C for 12 hours. Water was added, the mixture was extracted twice with ethyl acetate, and the combined organic layers were washed with a saturated saline solution. After drying over sodium sulfate, the organic layer was filtered and the solvent was distilled off under reduced pressure. The residue was purified by reverse phase silica gel column chromatography [eluting solvent: 0.225% aqueous formic acid solution/acetonitrile = 50/50 to 30/70 (V/V)] to afford 13 mg (yield: 12%) of the marked compound as a single diastereomer and as a single enantiomer.

### Example 10

### N-[(3S,4S)-3-[(2-Fluoro[biphenyl]-3-yl)methyl]-2-(2-hydroxy-2-methylpropanoyl)-2-azabicyclo[3.1.1]heptan-4-yl]methanesulfonamide

### (10A) 1-{(3S,4S)-3-[(2-Fluoro[biphenyl]-3-yl)methyl]-4-[(methylsulfonyl)amino]-2-azabicyclo[3.1.1]heptan-2-yl}-2-methyl-1-oxopropan-2-yl acetate

Using the compound synthesized in Example 8I (50 mg) and phenylboronic acid (12 mg), 40 mg (yield: 78%) of the marked compound was obtained as a single diastereomer and as a single enantiomer by the same method as in Example 8J.

### (10B) N-[(3S,4S)-3-[(2-Fluoro[biphenyl]-3-yl)methyl]-2-(2-hydroxy-2-methylpropanoyl)-2-azabicyclo[3.1.1]heptan-4-yl]methanesulfonamide

To a mixture of the compound synthesized in Example 10A (40 mg), methanol (1.5 mL), and water (0.3 mL), lithium hydroxide (10 mg) was added at room temperature and the mixture was stirred for 12 hours. The solvent was distilled off under reduced pressure, and hydrochloric acid (2 M) was added to adjust the pH to 3. The mixture was extracted with ethyl acetate, and after concentrating the organic layer, the residue was purified by reverse phase silica gel column chromatography [eluting solvent: 0.225% aqueous formic acid solution/acetonitrile = 58/42 to 38/62 (V/V)] to afford 17 mg (yield: 46%) of the marked compound as a single diastereomer and as a single enantiomer.

### Example 11

### N-[(3S,4S)-3-[(2-Fluoro-3'-methyl[biphenyl]-3-yl)methyl]-2-(2-hydroxy-2-methylpropanoyl)-2-azabicyclo[3.1.1]heptan-4-yl]methanesulfonamide

### (11A) 1-{(3S,4S)-3-[(2-Fluoro-3'-methyl[biphenyl]-3-yl)methyl]-4-[(methylsulfonyl)amino]-2-azabicyclo[3.1.1]heptan-2-yl}-2-methyl-1-oxopropan-2-yl acetate

Using the compound synthesized in Example 8I (50 mg) and 3-methylphenylboronic acid (16 mg), 44 mg (yield: 86%) of the marked compound was obtained as a single diastereomer and as a single enantiomer by the same method as in Example 8J.

### (11B) N-[(35,45)-3-[(2-Fluoro-3'-methyl[biphenyl]-3-yl)methyl]-2-(2-hydroxy-2-methylpropanoyl)-2-azabicyclo[3.1.1]heptan-4-yl]methanesulfonamide

To a mixture of the compound synthesized in Example 11A (42 mg), methanol (3.0 mL), and water (0.5 mL), lithium hydroxide monohydrate (17 mg) was added at room temperature and the mixture was stirred for 1 hour. The solvent was distilled off under reduced pressure, and the residue was purified by reverse phase silica gel column chromatography [eluting solvent: 0.225% aqueous formic acid solution/acetonitrile = 62/38 to 32/68 (V/V)] to afford 21 mg (yield: 55%) of the marked compound as a single diastereomer and as a single enantiomer.

### Example 12

### N-{5-Fluoro-2-(2-hydroxy-2-methylpropanoyl)-3-[(2,3',5'-trifluoro[biphenyl]-3-yl)methyl]-2-azabicyclo[3.1.1]heptan-4-yl}methanesulfonamide

### (12A) Methyl 3-{trans-3-[(tert-butoxycarbonyl)amino]-1-fluorocyclobutyl}-3-oxopropanoate

To a mixture of trans-3-(tert-butoxycarbonylamino)-1-fluoro-cyclobutanecarboxylic acid (CAS Registration No.: 2408768-09-0, 20 g), 2,2-dimethyl-1,3-dioxane-4,6-dione (15 g), N,N-dimethylaminopyridine (16 g), and dichloromethane (200 mL), 1-(3-dimethylaminopropyl)-3-ethylcarbodiimide (20 g) was added at room temperature and the mixture was stirred for 12 hours. The mixture was diluted with dichloromethane, sequentially washed with an aqueous potassium hydrogen sulfate solution and a saturated saline solution, and dried over sodium sulfate. After filtration, the solvent was distilled off under reduced pressure, methanol (200 mL) was added to the residue, and the mixture was stirred at 90°C for 3 hours. The solvent was distilled off under reduced pressure, and the residue was purified by silica gel column chromatography [eluting solvent: petroleum ether/ethyl acetate = 1/0 to 5/1 (V/V)] to afford 19 g (yield: 77%) of the marked compound.

### (12B) Methyl 3-{trans-3-[(tert-butoxycarbonyl)amino]-1-fluorocyclobutyl}-2-diazo-3-oxopropanoate

To a mixture of the compound synthesized in Example 12A (35 g), 4-acetamidobenzenesulfonyl azide (31 g), and acetonitrile (500 mL), triethylamine (38 g) was added at 0°C, and the mixture was then stirred at room temperature for 2 hours. The insoluble material was removed by filtration, and the solvent was distilled off under reduced pressure. Dichloromethane was added, and the insoluble material was removed by filtration again. The solvent was distilled off under reduced pressure, and the residue was purified by silica gel column chromatography [eluting solvent: petroleum ether/ethyl acetate = 1/0 to 10/1 (V/V)] to afford 25 g (yield: 66%) of the marked compound.

### (12C) 2-tert-Butyl 3-methyl 5-fluoro-4-oxo-2-azabicyclo[3.1.1]heptane-2,3-dicarboxylate

To a mixture of the compound synthesized in Example 12B (1.04 g) and toluene (20.8 mL), rhodium(II) acetate dimer (0.0729 g) was added, and the mixture was stirred at 90°C for 10 minutes and cooled to room temperature. Water was added to the reaction mixture, which was extracted with ethyl acetate. The organic layer was washed with water and a saturated saline solution, and dried over sodium sulfate. After filtration, the residue obtained by distilling off the solvent under reduced pressure was purified by silica gel column chromatography [eluting solvent: n-hexane/ethyl acetate = 2/1 (V/V)] to afford 0.948 g (yield: quantitative) of the marked compound.

### (12D) 2-tert-Butyl 3-methyl 5-fluoro-4-oxo-3-[(2,3',5'-trifluoro[biphenyl]-3-yl)methyl]-2-azabicyclo[3.1.1]heptane-2,3-dicarboxylate

To a mixture of the compound synthesized in Example 12C (0.948 g) and N,N-dimethylformamide (14.2 mL), 3-(bromomethyl)-2,3',5'-trifluoro[biphenyl] (0.994 g) from Example 1A and cesium carbonate (1.61 g) were added and the mixture was stirred at room temperature for 2 hours. Water was added to the reaction mixture, which was extracted with ethyl acetate. The organic layer was washed with water and a saturated saline solution, and dried over sodium sulfate. After filtration, the residue obtained by distilling off the solvent under reduced pressure was purified by silica gel column chromatography [eluting solvent: n-hexane/ethyl acetate = 3/1 (V/V)] to afford 1.22 g (yield: 73%) of the marked compound.

### (12E) 1-{5-Fluoro-4-oxo-3-[(2,3',5'-trifluoro[biphenyl]-3-yl)methyl]-2-azabicyclo[3.1.1]heptan-2-yl}-2-methyl-1-oxopropan-2-yl acetate

To a mixture of the compound synthesized in Example 12D (1.22 g) and acetic acid (2.44 mL), hydrochloric acid (12 M, 12.2 mL) was added and the mixture was stirred at room temperature for 30 minutes. Lithium chloride (1.02 g) was added to the reaction mixture, which was stirred at 100°C for 8 hours and cooled to room temperature. The solvent was distilled off under reduced pressure, toluene was added, and the mixture was azeotroped 2 times. To a mixture of the resulting residue and tetrahydrofuran (18.3 mL), triethylamine (1.67 mL) and 2-acetoxyisobutyryl chloride (0.522 mL) were added at 0°C and the mixture was stirred at room temperature for 2 hours. A saturated aqueous ammonium chloride solution was added to the reaction mixture, which was then extracted with ethyl acetate. The organic layer was washed with a saturated saline solution, and dried over sodium sulfate. After filtration, the solvent was distilled off under reduced pressure and the residue was purified by silica gel column chromatography [eluting solvent: n-hexane/ethyl acetate = 1/2 (V/V)] to afford 160 mg (yield: 14%) of the marked compound.

### (12F) 1-{4-Amino-5-fluoro-3-[(2,3',5'-trifluoro[biphenyl]-3-yl)methyl]-2-azabicyclo[3.1.1]heptan-2-yl}-2-methyl-1-oxopropan-2-yl acetate

To a mixture of the compound synthesized in Example 12E (135 mg) and ethanol (13.5 mL), hydroxylamine hydrochloride (68.8 mg) and sodium acetate (81.2 mg) were added, and the mixture was stirred at 80°C for 12 hours and cooled to room temperature. A saturated saline solution and water were added, the mixture was extracted with ethyl acetate, and the organic layer was dried over sodium sulfate. After filtration, the solvent was distilled off under reduced pressure. To a mixture of the resulting residue and methanol (6.7 mL), molybdenum(VI) oxide (1.22 g) was added at 0°C. Sodium borohydride (1.60 g) was slowly added little by little and the mixture was stirred at 0°C for 2 hours. After adding a saturated aqueous sodium bicarbonate solution and ethyl acetate to the reaction mixture, Celite filtration was carried out, followed by washing with ethyl acetate. The filtrate was concentrated under reduced pressure and extracted with ethyl acetate. The organic layer was washed with a saturated saline solution, and dried over anhydrous sodium sulfate. After filtration, the solvent was distilled off under reduced pressure, and the residue was purified by NH silica gel column chromatography [eluting solvent: n-hexane/ethyl acetate = 100/0 to 50/50 (V/V)] to afford 94.5 mg (yield: 70%) of the marked compound as a single diastereomer (racemate).

### (12G) 1-{5-Fluoro-4-[(methylsulfonyl)amino]-3-[(2,3',5'-trifluoro[biphenyl]-3-yl)methyl]-2-azabicyclo[3.1.1]heptan-2-yl}-2-methyl-1-oxopropan-2-yl acetate

To a mixture of the compound synthesized in Example 12F (94.5 mg), triethylamine (0.127 mL), and tetrahydrofuran (1.89 mL), methanesulfonyl chloride (0.0429 mL) was added at room temperature and the mixture was stirred for 2 hours. After adding a saturated saline solution, the mixture was extracted with ethyl acetate, and the organic layer was dried over sodium sulfate. After filtration, the solvent was distilled off under reduced pressure, and the residue was purified by silica gel column chromatography [eluting solvent: n-hexane/ethyl acetate = 70/30 to 0/100 (V/V)] to afford 51.2 mg (yield: 50%) of the marked compound as a single diastereomer (racemate).

### (12H) N-{5-Fluoro-2-(2-hydroxy-2-methylpropanoyl)-3-[(2,3',5'-trifluoro[biphenyl]-3-yl)methyl]-2-azabicyclo[3.1.1]heptan-4-yl}methanesulfonamide

To a mixture of the compound synthesized in Example 12G (51.2 mg), tetrahydrofuran (0.51 mL), methanol (0.26 mL), and water (0.26 mL), lithium hydroxide monohydrate (71.8 mg) was added at room temperature and the mixture was stirred at 50°C for 6 hours. After adding a saturated aqueous sodium bicarbonate solution, the mixture was extracted with ethyl acetate, and the organic layer was dried over sodium sulfate. After filtration, the solvent was distilled off under reduced pressure, and the residue was purified by NH silica gel column chromatography [eluting solvent: n-hexane/ethyl acetate = 50/50 to 0/100 (V/V)] to afford 35.3 mg (yield: 80%) of the marked compound as a single diastereomer (racemate).

### (12I) N-{5-Fluoro-2-(2-hydroxy-2-methylpropanoyl)-3-[(2,3',5'-trifluoro[biphenyl]-3-yl)methyl]-2-azabicyclo[3.1.1]heptan-4-yl}methanesulfonamide

The compound synthesized in Example 12H (191 mg) was subjected to chiral HPLC [column: CHIRALPAK (R) IA (20 mm I.D. × 250 mm), mobile phase: n-hexane/ethanol = 70/30 (V/V), temperature: 40°C] to afford 82.3 mg (yield: 43%) of the marked compound, which was eluted first, as a single diastereomer and as a single enantiomer.

### Example 13

### N-{3-[(3',5'-Difluoro[biphenyl]-3-yl)methyl]-2-(2-hydroxy-2-methylpropanoyl)-2-azabicyclo[3.1.1]heptan-4-yl}methanesulfonamide

### (13A) 2-tert-Butyl 3-methyl 3-(3-bromobenzyl)-4-oxo-2-azabicyclo[3.1.1]heptane-2,3-dicarboxylate

Using O4-tert-butyl O3-methyl 2-oxo-4-azabicyclo[3.1.1]heptane-3,4-dicarboxylate (CAS Registration No.: 2241048-56-4, 200 mg) and 3-bromobenzyl bromide (0.223 g), 274 mg (yield: 84%) of the marked compound was obtained by the same method as in Example 1B.

### (13B) 1-[3-(3-Bromobenzyl)-4-oxo-2-azabicyclo[3.1.1]heptan-2-yl]-2-methyl-1-oxopropan-2-yl acetate

To a mixture of the compound synthesized in Example 13A (274 mg) and acetic acid (0.548 mL), hydrochloric acid (12 M, 2.74 mL) was added and the mixture was stirred at room temperature for 30 minutes. Lithium chloride (26.5 mg) was added to the reaction mixture, which was stirred at 100°C for 9 hours and cooled to room temperature. The solvent was distilled off under reduced pressure, toluene was added, and the mixture was azeotroped 2 times. To a mixture of the resulting residue and tetrahydrofuran (4.11 mL), triethylamine (0.433 mL) and 2-acetoxyisobutyryl chloride (0.136 mL) were added at 0°C and the mixture was stirred at room temperature for 2 hours. A saturated aqueous ammonium chloride solution and water were added to the reaction mixture, which was then extracted with ethyl acetate. The organic layer was washed with a saturated saline solution, and dried over anhydrous sodium sulfate. The solvent was distilled off under reduced pressure and the residue was purified by silica gel column chromatography [eluting solvent: n-hexane/ethyl acetate = 90/10 to 50/50 (V/V)] to afford 222 mg (yield: 87%) of the marked compound.

### (13C) 1-[4-Amino-3-(3-bromobenzyl)-2-azabicyclo[3.1.1]heptan-2-yl]-2-methyl-1-oxopropan-2-yl acetate

To a mixture of the compound synthesized in Example 13B (222 mg) and ethanol (2.22 mL), hydroxylamine hydrochloride (0.132 g) and sodium acetate (0.156 g) were added, and the mixture was stirred at 80°C for 2 hours and cooled to room temperature. The solvent was distilled off under reduced pressure, water was added, and the mixture was extracted with ethyl acetate. The organic layer was washed with a saturated saline solution, and dried over sodium sulfate. After filtration, the solvent was distilled off under reduced pressure. To a mixture of the resulting residue and methanol (6.66 mL), molybdenum(VI) oxide (0.392 g) was added at 0°C. Sodium borohydride (0.412 g) was added little by little and the mixture was stirred at 0°C for 2 hours. After adding a saturated aqueous sodium bicarbonate solution and ethyl acetate to the reaction mixture, Celite filtration was carried out, followed by washing with ethyl acetate. The filtrate was concentrated under reduced pressure and extracted with ethyl acetate. The organic layer was washed with a saturated saline solution, and dried over anhydrous sodium sulfate. After filtration, the solvent was distilled off under reduced pressure to afford 0.223 g of the crude marked compound.

### (13D) 1-{3-(3-Bromobenzyl)-4-[(methylsulfonyl)amino]-2-azabicyclo[3.1.1]heptan-2-yl}-2-methyl-1-oxopropan-2-yl acetate

Using the compound synthesized in Example 13C (239 mg) and methanesulfonyl chloride (0.127 mL), 111 mg (yield: 42%) of the marked compound was obtained as a single diastereomer (racemate) by the same method as in Example 1E.

### (13E) 1-{3-[(3',5'-Difluoro[biphenyl]-3-yl)methyl]-4-[(methylsulfonyl)amino]-2-azabicyclo[3.1.1]heptan-2-yl}-2-methyl-1-oxopropan-2-yl acetate

Using the compound synthesized in Example 13D (111 mg) and 3,5-difluorophenylboronic acid (53.9 mg), 118 mg (yield: 99%) of the marked compound was obtained as a single diastereomer (racemate) by the same method as in Example 8J.

### (13F) N-{3-[(3',5'-Difluoro[biphenyl]-3-yl)methyl]-2-(2-hydroxy-2-methylpropanoyl)-2-azabicyclo[3.1.1]heptan-4-yl}methanesulfonamide

Using the compound synthesized in Example 13E (118 mg), 76 mg (yield: 75%) of the marked compound was obtained as a single diastereomer (racemate) by the same method as in Example 12H.

### Example 14

### N-[(3S,4S)-3-[(2-Fluoro-3'-methoxy[biphenyl]-3-yl)methyl]-2-(2-hydroxy-2-methylpropanoyl)-2-azabicyclo[3.1.1]heptan-4-yl]methanesulfonamide

### (14A) 1-{(3S,4S)-3-[(2-Fluoro-3'-methoxy[biphenyl]-3-yl)methyl]-4-[(methylsulfonyl)amino]-2-azabicyclo[3.1.1]heptan-2-yl}-2-methyl-1-oxopropan-2-yl acetate

Using the compound synthesized in Example 8I (50 mg) and 3-methoxyphenylboronic acid (20 mg), 30 mg (yield: 57%) of the marked compound was obtained as a single diastereomer and as a single enantiomer by the same method as in Example 8J.

### (14B) N-[(3S,4S)-3-[(2-Fluoro-3'-methoxy[biphenyl]-3-yl)methyl]-2-(2-hydroxy-2-methylpropanoyl)-2-azabicyclo[3.1.1]heptan-4-yl]methanesulfonamide

To a mixture of the compound synthesized in Example 14A (30 mg), methanol (2.0 mL), and water (0.5 mL), lithium hydroxide (8 mg) was added at room temperature and the mixture was stirred for 12 hours. The solvent was distilled off under reduced pressure, and hydrochloric acid (2 M) was added to adjust the pH to 3. The mixture was extracted with ethyl acetate, and after concentrating the organic layer, the residue was purified by reverse phase silica gel column chromatography [eluting solvent: 0.225% aqueous formic acid solution/acetonitrile = 57/43 to 37/63 (V/V)] to afford 17 mg (yield: 60%) of the marked compound as a single diastereomer and as a single enantiomer.

### Example 15

### N-{(3S,4S)-2-[(2S)-2-Hydroxypropanoyl]-3-[(2,3',5'-trifluoro[biphenyl]-3-yl)methyl]-2-azabicyclo[3.1.1]heptan-4-yl}methanesulfonamide

### (15A) tert-Butyl (3S,4S)-4-[(methylsulfonyl)amino]-3-[(2,3',5'-trifluoro[biphenyl]-3-yl)methyl]-2-azabicyclo[3.1.1]heptane-2-carboxylate

Using the compound synthesized in Example 8G (96.0 mg) and 3,5-difluorophenylboronic acid (48 mg), 99 mg (yield: 96%) of the marked compound was obtained as a single diastereomer and as a single enantiomer by the same method as in Example 8J.

### (15B) N-{(3S,4S)-3-[(2,3',5'-Trifluoro[biphenyl]-3-yl)methyl]-2-azabicyclo[3.1.1]heptan-4-yl}methanesulfonamide hydrochloride

Using the compound synthesized in Example 15A (99 mg), 91 mg of the crude marked compound was obtained as a single diastereomer and as a single enantiomer by the same method as in Example 8H.

### (15C) N-{(3S,4S)-2-[(2S)-2-Hydroxypropanoyl]-3-[(2,3',5'-trifluoro[biphenyl]-3-yl)methyl]-2-azabicyclo[3.1.1]heptan-4-yl}methanesulfonamide

Using the compound synthesized in Example 15B (50 mg) and 2-hydroxypropanoic acid (12 mg), 6.7 mg (yield: 12%) of the marked compound was obtained as a single diastereomer and as a single enantiomer by the same method as in Example 5H.

### Example 16

### N-{(3S,4S)-2-[(2R)-2-Hydroxypropanoyl]-3-[(2,3',5'-trifluoro[biphenyl]-3-yl)methyl]-2-azabicyclo[3.1.1]heptan-4-yl}methanesulfonamide

### (16A) N-{(3S,4S)-2-[(2R)-2-Hydroxypropanoyl]-3-[(2,3',5'-trifluoro[biphenyl]-3-yl)methyl]-2-azabicyclo[3.1.1]heptan-4-yl}methanesulfonamide

To a mixture of (R)-2-hydroxypropanoic acid (8.0 mg) and N,N-dimethylformamide (1.0 mL), O-(7-azabenzotriazol-1-yl)-N,N,N',N'-tetramethyluronium hexafluorophosphate (40 mg) was added and the mixture was stirred at 35°C for 1.5 hours. N-{(3S,4S)-3-[(2,3',5'-Trifluoro[biphenyl]-3-yl)methyl]-2-azabicyclo[3.1.1]heptan-4-yl}methanesulfonamide monohydrochloride (30 mg) of Example 15B and triethylamine (35 mg) were added and the mixture was stirred at 35°C for 2.5 hours. The insoluble material was removed by filtration, and after concentrating the filtrate, purification by reverse phase silica gel column chromatography [eluting solvent: 0.225% aqueous formic acid solution/acetonitrile = 60/40 to 40/60 (V/V)] was performed to afford 14 mg (yield: 43%) of the marked compound as a single diastereomer and as a single enantiomer.

### Example 17

### N-{(3S,4S)-2-[(2R)-Tetrahydrofuran-2-ylcarbonyl]-3-[(2,3',5'-trifluoro[biphenyl]-3-yl)methyl]-2-azabicyclo[3.1.1]heptan-4-yl}methanesulfonamide

### (17A) N-{(3S,4S)-2-[(2R)-Tetrahydrofuran-2-ylcarbonyl]-3-[(2,3',5'-trifluoro[biphenyl]-3-yl)methyl]-2-azabicyclo[3.1.1] heptan-4-yl}methanesulfonamide

Using the compound synthesized in Example 15B (50 mg) and (R)-tetrahydrofuran-2-carboxylic acid (16 mg), 27 mg (yield: 47%) of the marked compound was obtained as a single diastereomer and as a single enantiomer by the same method as in Example 5H.

### Example 18

### N-[(3S,4S)-3-{[2-Fluoro-3'-(trifluoromethyl)[biphenyl]-3-yl]methyl}-2-(2-hydroxy-2-methylpropanoyl)-2-azabicyclo[3.1.1]heptan-4-yl]methanesulfonamide

### (18A) 1-{(3S,4S)-3-{[2-Fluoro-3'-(trifluoromethyl)[biphenyl]-3-yl]methyl}-4-[(methylsulfonyl)amino]-2-azabicyclo[3.1.1]heptan-2-yl}-2-methyl-1-oxopropan-2-yl acetate

Using the compound synthesized in Example 8I (50 mg) and 3-trifluoromethylphenylboronic acid (24 mg), 60 mg of the crude marked compound was obtained as a single diastereomer and as a single enantiomer by the same method as in Example 8J.

### (18B) N-[(3S,4S)-3-{[2-Fluoro-3'-(trifluoromethyl)[biphenyl]-3-yl]methyl}-2-(2-hydroxy-2-methylpropanoyl)-2-azabicyclo[3.1.1]heptan-4-yl]methanesulfonamide

To a mixture of the compound synthesized in Example 18A (60 mg), methanol (5.0 mL), and water (1.0 mL), lithium hydroxide monohydrate (22 mg) was added at room temperature and the mixture was stirred for 10 hours. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 53/47 to 33/67 (V/V)] to afford 16 mg (yield: 30%) of the marked compound as a single diastereomer and as a single enantiomer.

### Example 19

### N-{(3S,4S)-2-[(1-Fluorocyclopropyl)carbonyl]-3-[(2,3',5'-trifluoro[biphenyl]-3-yl)methyl]-2-azabicyclo[3.1.1]heptan-4-yl}methanesulfonamide

### (19A) N-{(35,45)-2-[(1-fluorocyclopropyl)carbonyl]-3-[(2,3',5'-trifluoro[biphenyl]-3-yl)methyl]-2-azabicyclo[3.1.1] heptan-4-yl}methanesulfonamide

Using the compound synthesized in Example 15B (30 mg) and 1-fluorocyclopropanecarboxylic acid (10 mg), 29 mg (yield: 88%) of the marked compound was obtained as a single diastereomer and as a single enantiomer by the same method as in Example 5H.

### Example 20

### N-{(3S,4S)-2-(Cyclopropylcarbonyl)-3-[(2,3',5'-trifluoro[biphenyl]-3-yl)methyl]-2-azabicyclo[3.1.1]heptan-4-yl}methanesulfonamide

### (20A) N-{(3S,4S)-2-(Cyclopropylcarbonyl)-3-[(2,3',5'-trifluoro[biphenyl]-3-yl)methyl]-2-azabicyclo[3.1.1]heptan-4-yl}methanesulfonamide

To a mixture of the compound synthesized in Example 15B (150 mg) and tetrahydrofuran (5.0 mL), N,N-diisopropylethylamine (236 mg) and cyclopropanecarbonyl chloride (46 mg) were added and the mixture was stirred at room temperature for 1.5 hours. The reaction solution was poured into water and extracted with ethyl acetate. The organic layer was washed with a saturated saline solution, and dried over sodium sulfate. After filtration, the solvent was distilled off under reduced pressure, and the residue was purified by reverse phase silica gel column chromatography [eluting solvent: 0.225% aqueous formic acid solution/acetonitrile = 55/45 to 25/75 (V/V)] to afford 96 mg (yield: 55%) of the marked compound as a single diastereomer and as a single enantiomer.

### Example 21

### N-[(3S,4S)-3-[3-(6-Chloropyridin-2-yl)-2-fluorobenzyl]-2-(2-hydroxy-2-methylpropanoyl)-2-azabicyclo[3.1.1]heptan-4-yl]methanesulfonamide

### (21A) tert-Butyl (3S,4S)-3-[2-fluoro-3-(4,4,5,5-tetramethyl-1,3,2-dioxaborolan-2-yl)benzyl]-4-[(methylsulfonyl)amino]-2-azabicyclo[3.1.1]heptane-2-carboxylate

A mixture of the compound synthesized in Example 8G (200 mg), bis(pinacolato)diboron (220 mg), potassium acetate (65 mg), [1,1'-bis(diphenylphosphino)ferrocene]palladium(II) dichloride dichloromethane adduct (35 mg), and 1,4-dioxane (5.0 mL) was degassed and then stirred at 100°C for 2 hours in a nitrogen atmosphere. After filtration, the solvent was distilled off under reduced pressure to afford 110 mg of the crude marked compound as a single diastereomer and as a single enantiomer.

### (21B) tert-Butyl (3S,4S)-3-[3-(6-chloropyridin-2-yl)-2-fluorobenzyl]-4-[(methylsulfonyl)amino]-2-azabicyclo[3.1.1]heptane-2-carboxylate

A mixture of the compound synthesized in Example 21A (110 mg), cesium carbonate (140 mg), 2,6-dichloropyridine (45 mg), (2-dicyclohexylphosphino-2',4',6'-triisopropyl-1,1'-biphenyl)[2-(2'-amino-1,1'-biphenyl)]palladium(II) methanesulfonate (10 mg), water (0.5 mL), and 1,4-dioxane (3.0 mL) was degassed and then stirred at 100°C for 2 hours in a nitrogen atmosphere. The reaction solution was poured into water and extracted with ethyl acetate, and the organic layer was washed with a saturated saline solution. The organic layer was dried over sodium sulfate and filtered, and the solvent was then distilled off under reduced pressure. The residue was purified by preparative thin layer silica gel chromatography [eluting solvent: petroleum ether/ethyl acetate = 1/1 (V/V)] to afford 90 mg (yield: 84%) of the marked compound as a single diastereomer and as a single enantiomer.

### (21C) N-{(3S,4S)-3-[3-(6-Chloropyridin-2-yl)-2-fluorobenzyl]-2-azabicyclo[3.1.1] heptan-4-yl}methanesulfonamide hydrochloride

Using the compound synthesized in Example 21B (90 mg), 80 mg of the crude marked compound was obtained as a single diastereomer and as a single enantiomer by the same method as in Example 8H.

### (21D) 1-{(3S,4S)-3-[3-(6-Chloropyridin-2-yl)-2-fluorobenzyl]-4-[(methylsulfonyl)amino]-2-azabicyclo[3.1.1]heptan-2-yl}-2-methyl-1-oxopropan-2-yl acetate

To a mixture of the compound synthesized in Example 21C (80 mg) and tetrahydrofuran (2.0 mL), N,N-diisopropylethylamine (118 mg) and 2-acetoxyisobutyryl chloride (45 mg) were added at room temperature, and the mixture was stirred at room temperature for 2 hours. The reaction solution was poured into water and extracted with ethyl acetate. The organic layer was washed with a saturated saline solution, and dried over sodium sulfate. After filtration, the solvent was distilled off under reduced pressure, and the residue was purified by preparative thin layer silica gel chromatography [eluting solvent: petroleum ether/ethyl acetate = 1/1 (V/V)] to afford 80 mg (yield: 96%) of the marked compound as a single diastereomer and as a single enantiomer.

### (21E) N-[(3S,4S)-3-[3-(6-Chloropyridin-2-yl)-2-fluorobenzyl]-2-(2-hydroxy-2-methylpropanoyl)-2-azabicyclo[3.1.1]heptan-4-yl]methanesulfonamide

To a mixture of the compound synthesized in Example 21D (80 mg), water (0.5 mL), and tetrahydrofuran (2.0 mL), lithium hydroxide (20 mg) was added at room temperature and the mixture was stirred for 12 hours. The reaction solution was poured into water and extracted with ethyl acetate. The organic layer was washed with a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 68/32 to 38/62 (V/V)] to afford 18 mg (yield: 23%) of the marked compound as a single diastereomer and as a single enantiomer.

### Example 22

### N-[2-(Cyclopropylcarbonyl)-3-{[2-(3,5-difluorophenyl)-3-fluoropyridin-4-yl]methyl}-2-azabicyclo[3.1.1]heptan-4-yl]methanesulfonamide

### (22A) 2-(3,5-Difluorophenyl)-3-fluoro-4-methylpyridine

To a mixture of 2-bromo-3-fluoro-4-methylpyridine (CAS Registration No.: 884494-37-5, 9.5 g), 3,5-difluorophenylboronic acid (10.5 g), water (5.0 mL), and 1,4-dioxane (100 mL), potassium carbonate (14 g), [1,1'-bis(diphenylphosphino)ferrocene]palladium(II) dichloride (2.0 g) were added and the mixture was stirred at 100°C for 12 hours in a nitrogen atmosphere. The solvent was distilled off under reduced pressure, and the residue was purified by silica gel column chromatography [eluting solvent: petroleum ether/ethyl acetate = 10/1 (V/V)] to afford 10.2 g (yield: 91%) of the marked compound.

### (22B) 4-(Bromomethyl)-2-(3,5-difluorophenyl)-3-fluoropyridine

To a mixture of the compound synthesized in Example 22A (8.0 g) and carbon tetrachloride (80 mL), N-bromosuccinimide (8.0 g) and benzoyl peroxide (1.3 g) were added and the mixture was stirred at 80°C for 12 hours. After washing with water, the solvent was distilled off under reduced pressure. The residue was purified by reverse phase silica gel column chromatography [eluting solvent: 0.225% aqueous formic acid solution/acetonitrile = 45/55 to 25/75 (V/V)] to afford 5.8 g (yield: 54%) of the marked compound.

### (22C) 2-tert-Butyl 3-methyl 3-{[2-(3,5-difluorophenyl)-3-fluoropyridin-4-yl]methyl}-4-oxo-2-azabicyclo[3.1.1]heptane-2,3-dicarboxylate

To a mixture of the compound synthesized in Example 22B (2.69 g), O4-tert-butyl O3-methyl 2-oxo-4-azabicyclo[3.1.1]heptane-3,4-dicarboxylate (CAS Registration No.: 2241048-56-4, 2.0 g), and N,N-dimethylformamide (30 mL), cesium carbonate (3.63 g) was added at room temperature and the mixture was stirred for 2 hours. Water was added, the mixture was extracted with ethyl acetate, and the organic layer was washed with a saturated saline solution and then dried over sodium sulfate. After filtration, the solvent was distilled off under reduced pressure, and the residue was purified by silica gel column chromatography [eluting solvent: petroleum ether/ethyl acetate = 10/1 to 4/1 (V/V)] to afford 2.95 g (yield: 81%) of the marked compound.

### (22D) tert-Butyl 3-{[2-(3,5-difluorophenyl)-3-fluoropyridin-4-yl]methyl}-4-oxo-2-azabicyclo[3.1.1]heptane-2-carboxylate

A mixture of the compound synthesized in Example 22C (1.1 g), lithium chloride (380 mg), and dimethyl sulfoxide (20 mL) was stirred at 130°C for 1 hour. The mixture was diluted with ethyl acetate, washed with water and a saturated saline solution, and then dried over sodium sulfate. After filtration, the solvent was distilled off under reduced pressure to afford 970 mg of the crude marked compound.

### (22E) 3-{[2-(3,5-Difluorophenyl)-3-fluoropyridin-4-yl]methyl}-2-azabicyclo[3.1.1]heptan-4-one hydrochloride

To a mixture of the compound synthesized in Example 22D (970 mg) and ethyl acetate (10 mL), a solution of hydrogen chloride in ethyl acetate (4 M, 10 mL) was added at room temperature and the mixture was stirred for 10 minutes. The solvent was distilled off under reduced pressure to afford 800 mg of the crude marked compound.

### (22F) 2-(Cyclopropylcarbonyl)-3-{[2-(3,5-difluorophenyl)-3-fluoropyridin-4-yl]methyl}-2-azabicyclo[3.1.1]heptan-4-one

To a mixture of the compound synthesized in Example 22E (800 mg), N,N-diisopropylethylamine (1.40 g), and dichloromethane (20 mL), cyclopropanecarbonyl chloride (453 mg) was added at room temperature and the mixture was stirred for 30 minutes. The mixture was diluted with dichloromethane, washed with water and a saturated saline solution, and then dried over sodium sulfate. After filtration, the solvent was distilled off under reduced pressure, and the residue was purified by silica gel column chromatography [eluting solvent: petroleum ether/ethyl acetate = 1/0 to 1/1 (V/V)] to afford 550 mg (yield: 63%) of the marked compound.

### (22G) Cyclopropyl[3-{[2-(3,5-difluorophenyl)-3-fluoropyridin-4-yl]methyl}-4-(hydroxyimino)-2-azabicyclo[3.1.1]heptan-2-yl]methanone

A mixture of the compound synthesized in Example 22F (550 mg), hydroxylamine hydrochloride (286 mg), sodium acetate (450 mg), and ethanol (10 mL) was stirred at 80°C for 2 hours. The mixture was diluted with ethyl acetate, washed with water and a saturated saline solution, and then dried over sodium sulfate. After filtration, the solvent was distilled off under reduced pressure to afford 500 mg of the crude marked compound.

### (22H) (4-Amino-3-{[2-(3,5-difluorophenyl)-3-fluoropyridin-4-yl]methyl}-2-azabicyclo[3.1.1]heptan-2-yl)(cyclopropyl)methanone

To a mixture of the compound synthesized in Example 22G (500 mg), molybdenum(VI) oxide (259 mg), and methanol (20 mL), sodium borohydride (634 mg) was added at 0°C, and the mixture was then stirred at room temperature for 2 hours. The reaction solution was poured into an aqueous ammonium chloride solution, and the insoluble material was removed by filtration. The mixture was extracted with ethyl acetate, and the organic layer was washed with a saturated saline solution and then dried over sodium sulfate. After filtration, the solvent was distilled off under reduced pressure, and purification by reverse phase silica gel column chromatography [eluting solvent: 0.225% aqueous formic acid solution/acetonitrile = 87/13 to 67/33 (V/V)] was performed to afford 60 mg (yield: 12%) of the marked compound as a single diastereomer (racemate).

### (22I) N-[2-(Cyclopropylcarbonyl)-3-{[2-(3,5-difluorophenyl)-3-fluoropyridin-4-yl]methyl}-2-azabicyclo[3.1.1]heptan-4-yl]methanesulfonamide

To a mixture of the compound synthesized in Example 22H (240 mg), N,N-diisopropylethylamine (309 mg), and tetrahydrofuran (10 mL), methanesulfonyl chloride (425 mg) was added at 0°C, and the mixture was then stirred at room temperature for 2 hours. The reaction solution was poured into an aqueous sodium bicarbonate solution and extracted with ethyl acetate. The organic layer was washed with a saturated saline solution, and then dried over sodium sulfate. After filtration, the solvent was distilled off under reduced pressure, and purification by reverse phase silica gel column chromatography [eluting solvent: 0.225% aqueous formic acid solution/acetonitrile = 64/36 to 34/66 (V/V)] was performed to afford 250 mg (yield: 87%) of the marked compound as a single diastereomer (racemate).

### (22J) N-[2-(Cyclopropylcarbonyl)-3-{[2-(3,5-difluorophenyl)-3-fluoropyridin-4-yl]methyl}-2-azabicyclo[3.1.1]heptan-4-yl]methanesulfonamide

The compound synthesized in Example 22I (250 mg) was subjected to chiral SFC [column: CHIRALPAK (R) IG (30 mm I.D. × 250 mm), mobile phase: carbon dioxide/methanol = 70/30 (V/V)] to afford 96 mg (yield: 39%) of the marked compound, which was eluted first, as a single diastereomer and as a single enantiomer.

### Example 23

### N-{(3S,4R)-5-Fluoro-2-[(2R)-2-hydroxypropanoyl]-3-[(2,3',5'-trifluoro[biphenyl]-3-yl)methyl]-2-azabicyclo[3.1.1]heptan-4-yl}methanesulfonamide or N-{(3R,4S)-5-fluoro-2-[(2R)-2-hydroxypropanoyl]-3-[(2,3',5'-trifluoro[biphenyl]-3-yl)methyl]-2-azabicyclo[3.1.1]heptan-4-yl}methanesulfonamide

### (23A) 2-tert-Butyl 3-methyl 3-(3-bromo-2-fluorobenzyl)-5-fluoro-4-oxo-2-azabicyclo[3.1.1]heptane-2,3-dicarboxylate

To a mixture of the compound synthesized in Example 12C (2.42 g), 3-bromo-2-fluorobenzyl bromide (2.71 g), and N,N-dimethylformamide (40 mL), cesium carbonate (4.12 g) was added at 0°C, and the mixture was stirred for 10 minutes and then stirred at room temperature for 4.5 hours. After adding water, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 95/5 to 75/25 (V/V)] to afford 2.14 g (yield: 54%) of the marked compound.

### (23B) tert-Butyl 3-(3-bromo-2-fluorobenzyl)-5-fluoro-4-oxo-2-azabicyclo[3.1.1]heptane-2-carboxylate

A mixture of the compound synthesized in Example 23A (1.77 g), lithium chloride (532 mg), and dimethyl sulfoxide (44 mL) was stirred at 130°C for 1 hour. After adding a saline solution, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 95/5 to 70/30 (V/V)] to afford 1.05 g (yield: 68%) of the marked compound.

### (23C) tert-Butyl 3-(3-bromo-2-fluorobenzyl)-5-fluoro-4-(hydroxyimino)-2-azabicyclo[3.1.1]heptane-2-carboxylate

A mixture of the compound synthesized in Example 23B (1.15 g), hydroxylamine hydrochloride (1.15 g), sodium acetate (1.36 g), and ethanol (25 mL) was stirred at 90°C for 5 hours. After adding a saline solution, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 90/10 to 40/60 (V/V)] to afford 833 mg (yield: 70%) of the marked compound.

### (23D) tert-Butyl 4-amino-3-(3-bromo-2-fluorobenzyl)-5-fluoro-2-azabicyclo[3.1.1]heptane-2-carboxylate

To a mixture of the compound synthesized in Example 23C (100 mg) and methanol (4 mL), molybdenum(VI) oxide (167 mg) was added at 0°C. Sodium borohydride (175 mg) was added little by little and the mixture was then stirred at 35°C for 4 hours. To this, molybdenum(VI) oxide (167 mg) was added at room temperature. Sodium borohydride (175 mg) was added little by little and the mixture was then stirred at 45°C for 2 hours. To this, molybdenum(VI) oxide (167 mg) was added at room temperature. Sodium borohydride (175 mg) was added little by little and the mixture was then stirred at 45°C for 4 hours. To a mixture of tert-butyl 3-(3-bromo-2-fluorobenzyl)-5-fluoro-4-(hydroxyimino)-2-azabicyclo[3.1.1]heptane-2-carboxylate (708 mg) of Example 23C prepared separately and methanol (25 mL), molybdenum(VI) oxide (1.18 g) was added at 0°C. Sodium borohydride (1.24 g) was added little by little and the mixture was then stirred at 45°C for 4 hours. To this, molybdenum(VI) oxide (1.18 g) was added at 0°C. Sodium borohydride (1.24 g) was added little by little and the mixture was then stirred at 45°C for 6 hours. To this, molybdenum(VI) oxide (1.18 g) was added at 0°C. Sodium borohydride (1.24 g) was added little by little and the mixture was then stirred at 45°C for 2 hours. To this, molybdenum(VI) oxide (1.18 g) was added at room temperature. Sodium borohydride (1.24 g) was added little by little and the mixture was then stirred at 45°C for 5 hours. The reaction mixtures were combined and added to a mixture of a saturated aqueous sodium bicarbonate solution and ethyl acetate at 0°C, and the mixture was then stirred at room temperature for 1 hour. After removing the insoluble material by filtration, the filtrate was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 85/15 to 20/80 (V/V)] to afford 449 mg (yield: 57%) of the marked compound.

### (23E) tert-Butyl (3S*,4R*)-3-(3-bromo-2-fluorobenzyl)-5-fluoro-4-[(methylsulfonyl)amino]-2-azabicyclo[3.1.1]heptane-2-carboxylate

To a mixture of the compound synthesized in Example 23D (449 mg), triethylamine (0.746 mL), and tetrahydrofuran (10 mL), a solution of methanesulfonyl chloride (370 mg) in tetrahydrofuran (2 mL) was added at 0°C, and the mixture was then stirred at room temperature overnight. After adding water, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 85/15 to 25/75 (V/V)] to afford 504 mg (yield: 95%) of the marked compound as a single diastereomer (racemate).

### (23F) tert-Butyl (3S*,4R*)-5-fluoro-4-[(methylsulfonyl)amino]-3-[(2,3',5'-trifluoro[biphenyl]-3-yl)methyl]-2-azabicyclo[3.1.1]heptane-2-carboxylate

A mixture of the compound synthesized in Example 23E (150 mg), 3,5-difluorophenylboronic acid (71.7 mg), (2-dicyclohexylphosphino-2',4',6'-triisopropyl-1,1'-biphenyl)[2-(2'-amino-1,1'-biphenyl)]palladium(II) methanesulfonate (25.6 mg), tripotassium phosphate (193 mg), tetrahydrofuran (3 mL), and water (1 mL) was stirred at 65°C for 2 hours. After adding water, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 90/10 to 40/60 (V/V)] to afford 189 mg (yield: quantitative) of the marked compound as a single diastereomer (racemate).

### (23G) N-{(3S*,4R*)-5-Fluoro-3-[(2,3',5'-trifluoro[biphenyl]-3-yl)methyl]-2-azabicyclo[3.1.1]heptan-4-yl}methanesulfonamide hydrochloride

To a mixture of the compound synthesized in Example 23F (189 mg) and 1,4-dioxane (2 mL), a solution of hydrogen chloride in 1,4-dioxane (4 M, 6 mL) was added at room temperature and the mixture was stirred for 4 hours. After diluting the reaction mixture with toluene, the solution was concentrated under reduced pressure to afford 141 mg of the crude marked compound as a single diastereomer (racemate).

### (23H) (2R)-1-{(3S*,4R*)-5-Fluoro-4-[(methylsulfonyl)amino]-3-[(2,3',5'-trifluoro[biphenyl]-3-yl)methyl]-2-azabicyclo[3.1.1]heptan-2-yl}-1-oxopropan-2-yl acetate

To a mixture of the compound synthesized in Example 23G (81.0 mg), (R)-2-acetoxypropanoic acid (46.0 mg), triethylamine (0.0966 mL), and N,N-dimethylformamide (3.5 mL), O-(7-azabenzotriazol-1-yl)-N,N,N',N'-tetramethyluronium hexafluorophosphate (132 mg) was added at room temperature, and the mixture was then stirred at 35°C for 4 hours. After adding water, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 70/30 to 10/90 (V/V)] to afford 101 mg (yield: quantitative) of the marked compound.

### (23I) N-{(3S*,4R*)-5-Fluoro-2-[(2R)-2-hydroxypropanoyl]-3-[(2,3',5'-trifluoro[biphenyl]-3-yl)methyl]-2-azabicyclo[3.1.1]heptan-4-yl}methanesulfonamide

To a mixture of the compound synthesized in Example 23H (101 mg), tetrahydrofuran (2.4 mL), methanol (0.8 mL), and water (0.8 mL), lithium hydroxide monohydrate (21.9 mg) was added at room temperature and the mixture was stirred for 5 hours. After adding a saline solution, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 70/30 to 0/100 (V/V)] to afford 80.6 mg (yield: 93%) of the marked compound.

### (23J) N-{(3S,4R)-5-Fluoro-2-[(2R)-2-hydroxypropanoyl]-3-[(2,3',5'-trifluoro[biphenyl]-3-yl)methyl]-2-azabicyclo[3.1.1]heptan-4-yl}methanesulfonamide or N-{(3R,4S)-5-fluoro-2-[(2R)-2-hydroxypropanoyl]-3-[(2,3',5'-trifluoro[biphenyl]-3-yl)methyl]-2-azabicyclo[3.1.1]heptan-4-yl}methanesulfonamide

The compound synthesized in Example 23I (80.1 mg) was purified by chiral HPLC [column: CHIRALPAK (R) IH (20 mm I.D. × 250 mm), mobile phase: n-hexane/ethanol = 50/50 (V/V), temperature: 40°C] to afford 32.3 mg (yield: 40%) of the marked compound, which was eluted first, as a single diastereomer and as a single enantiomer.

### Example 24

### N-{(3S,4S)-3-{[3'-(Difluoromethyl)-2-fluoro[biphenyl]-3-yl]methyl}-2-[(2R)-2-hydroxypropanoyl]-2-azabicyclo[3.1.1]heptan-4-yl}methanesulfonamide

### (24A) tert-Butyl (3S,4S)-3-{[3'-(difluoromethyl)-2-fluoro[biphenyl]-3-yl]methyl}-4-[(methylsulfonyl)amino]-2-azabicyclo[3.1.1]heptane-2-carboxylate

Using the compound synthesized in Example 8G (100 mg) and 3-(difluoromethyl)phenylboronic acid (54 mg), 200 mg of the marked compound mixed with byproducts was obtained as a single diastereomer and as a single enantiomer by the same method as in Example 8J.

### (24B) N-[(3S,4S)-3-{[3'-(Difluoromethyl)-2-fluoro[biphenyl]-3-yl]methyl}-2-azabicyclo[3.1.1]heptan-4-yl]methanesulfonamide

To a mixture of the compound synthesized in Example 24A (200 mg) and ethyl acetate (5.0 mL), a solution of hydrogen chloride in ethyl acetate (4 M, 2.0 mL) was added and the mixture was stirred at room temperature for 30 minutes. The solvent was distilled off under reduced pressure, and the residue was purified by reverse phase silica gel column chromatography [eluting solvent: 0.05% aqueous ammonia/acetonitrile = 67/33 to 37/63 (V/V)] to afford 110 mg (yield: 68%) of the marked compound as a single diastereomer and as a single enantiomer.

### (24C) N-{(3S,4S)-3-{[3'-(Difluoromethyl)-2-fluoro[biphenyl]-3-yl]methyl}-2-[(2R)-2-hydroxypropanoyl]-2-azabicyclo[3.1.1]heptan-4-yl}methanesulfonamide

Using the compound synthesized in Example 24B (50 mg) and (R)-2-hydroxypropanoic acid (21 mg), 15 mg (yield: 26%) of the marked compound was obtained as a single diastereomer and as a single enantiomer by the same method as in Example 5H.

### Example 25

### N-{(3S,4S)-3-[(2-Fluoro[biphenyl]-3-yl)methyl]-2-[(2R)-2-hydroxypropanoyl]-2-azabicyclo[3.1.1]heptan-4-yl}methanesulfonamide

### (25A) tert-Butyl (3S,4S)-3-[(2-fluoro[biphenyl]-3-yl)methyl]-4-[(methylsulfonyl)amino]-2-azabicyclo[3.1.1]heptane-2-carboxylate

To a mixture of the compound synthesized in Example 8G (500 mg) and tetrahydrofuran (5.0 mL), phenylboronic acid (153 mg), (2-dicyclohexylphosphino-2',4',6'-triisopropyl-1,1'-biphenyl)[2-(2'-amino-1,1'-biphenyl)]palladium(II) methanesulfonate (44 mg), and tripotassium phosphate (667 mg) were added, and after degassing, the mixture was stirred at 70°C for 1.5 hours in a nitrogen atmosphere. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: petroleum ether/ethyl acetate = 10/1 to 2/1 (V/V)] to afford 400 mg (yield: 80%) of the marked compound as a single diastereomer and as a single enantiomer.

### (25B) N-{(3S,4S)-3-[(2-Fluoro[biphenyl]-3-yl)methyl]-2-azabicyclo[3.1.1]heptan-4-yl}methanesulfonamide hydrochloride

To a mixture of the compound synthesized in Example 25A (400 mg) and ethyl acetate (5.0 mL), a solution of hydrogen chloride in ethyl acetate (4 M, 5.0 mL) was added and the mixture was stirred at room temperature for 30 minutes. The solvent was distilled off under reduced pressure to afford 280 mg of the crude marked compound as a single diastereomer and as a single enantiomer.

### (25C) N-{(3S,4S)-3-[(2-Fluoro[biphenyl]-3-yl)methyl]-2-[(2R)-2-hydroxypropanoyl]-2-azabicyclo[3.1.1]heptan-4-yl}methanesulfonamide

To a mixture of (R)-2-hydroxypropanoic acid (48 mg), O-(7-azabenzotriazol-1-yl)-N,N,N',N'-tetramethyluronium hexafluorophosphate (152 mg), and N,N-dimethylformamide (2.0 mL), the compound synthesized in Example 25B (100 mg) and triethylamine (135 mg) were added and the mixture was stirred at room temperature for 2 hours. After distilling off the solvent under reduced pressure, purification by reverse phase silica gel column chromatography [eluting solvent: 0.225% aqueous formic acid solution/acetonitrile = 70/30 to 40/60 (V/V)] was performed to afford 44 mg (yield: 37%) of the marked compound as a single diastereomer and as a single enantiomer.

### Example 26

### N-{(3S,4S)-2-[(2R)-2-Hydroxybutanoyl]-3-[(2,3',5'-trifluoro[biphenyl]-3-yl)methyl]-2-azabicyclo[3.1.1]heptan-4-yl}methanesulfonamide

### (26A) N-{(3S,4S)-2-[(2R)-2-Hydroxybutanoyl]-3-[(2,3',5'-trifluoro[biphenyl]-3-yl)methyl]-2-azabicyclo[3.1.1]heptan-4-yl}methanesulfonamide

Using the compound synthesized in Example 15B (100 mg) and (R)-2-hydroxybutanoic acid (35 mg), 25 mg (yield: 23%) of the marked compound was obtained as a single diastereomer and as a single enantiomer by the same method as in Example 5H.

### Example 27

### (3S,4S)-N-Methyl-4-[(methylsulfonyl)amino]-3-[(2,3',5'-trifluoro[biphenyl]-3-yl)methyl]-2-azabicyclo[3.1.1]heptane-2-carboxamide

### (27A) (3S,4S)-N-Methyl-4-[(methylsulfonyl)amino]-3-[(2,3',5'-trifluoro[biphenyl]-3-yl)methyl]-2-azabicyclo[3.1.1]heptane-2-carboxamide

To a mixture of the compound synthesized in Example 15B (50 mg), N,N-diisopropylethylamine (72 mg), and tetrahydrofuran (2.0 mL), N-methylcarbamoyl chloride (16 mg) was added and the mixture was stirred at room temperature for 30 minutes. The mixture was diluted with ethyl acetate, washed with water and a saturated saline solution, and dried over sodium sulfate. After filtration, the solvent was distilled off under reduced pressure, and the residue was purified by reverse phase silica gel column chromatography [eluting solvent: 0.225% aqueous formic acid solution/acetonitrile = 61/39 to 31/69 (V/V)] to afford 17 mg (yield: 34%) of the marked compound as a single diastereomer and as a single enantiomer.

### Example 28

### N-{(3S,4S)-2-(Methoxyacetyl)-3-[(2,3',5'-trifluoro[biphenyl]-3-yl)methyl]-2-azabicyclo[3.1.1]heptan-4-yl}methanesulfonamide

### (28A) N-{(3S,4S)-2-(Methoxyacetyl)-3-[(2,3',5'-trifluoro[biphenyl]-3-yl)methyl]-2-azabicyclo[3.1.1]heptan-4-yl}methanesulfonamide

To a mixture of the compound synthesized in Example 15B (50 mg) and tetrahydrofuran (1.0 mL), N,N-diisopropylethylamine (78 mg) and 2-methoxyacetyl chloride (15 mg) were added at room temperature and the mixture was stirred for 1 hour. The reaction solution was poured into water and extracted with ethyl acetate, and the organic layer was then washed with a saturated saline solution. The organic layer was dried over sodium sulfate and filtered, and the solvent was then distilled off under reduced pressure. The residue was purified by reverse phase silica gel column chromatography [eluting solvent: 0.225% aqueous formic acid solution/acetonitrile = 61/39 to 31/69 (V/V)] to afford 32 mg (yield: 55%) of the marked compound as a single diastereomer and as a single enantiomer.

### Example 29

### N-{(3S,45)-2-{[(1R,2S)-2-Fluorocyclopropyl]carbonyl}-3-[(2,3',5'-trifluoro[biphenyl]-3-yl)methyl]-2-azabicyclo[3.1.1]heptan-4-yl}methanesulfonamide

### (29A) N-{(3S,4S)-2-{[(1R,2S)-2-Fluorocyclopropyl]carbonyl}-3-[(2,3',5'-trifluoro[biphenyl]-3-yl)methyl]-2-azabicyclo[3.1.1]heptan-4-yl}methanesulfonamide

To a mixture of the compound synthesized in Example 15B (30.0 mg) and dichloromethane (2 mL), triethylamine (0.047 mL), O-(7-azabenzotriazol-1-yl)-N,N,N',N'-tetramethyluronium hexafluorophosphate (31 mg), and (1R,2S)-2-fluorocyclopropane-1-carboxylic acid (8 mg) were added and the mixture was stirred at room temperature for 1 hour. Water was added to the reaction mixture, which was extracted with ethyl acetate. The organic layer was washed with a saturated saline solution, and dried over anhydrous sodium sulfate. After filtration, the solvent was distilled off under reduced pressure, and the residue was purified by silica gel column chromatography [eluting solvent: n-hexane/ethyl acetate = 50/50 to 20/80 (V/V)] to afford 31 mg (yield: 93%) of the marked compound as a single diastereomer and as a single enantiomer.

### Example 30

### N-{(3S,45)-2-{[(1S,2R)-2-Fluorocyclopropyl]carbonyl}-3-[(2,3',5'-trifluoro[biphenyl]-3-yl)methyl]-2-azabicyclo[3.1.1]heptan-4-yl}methanesulfonamide

### (30A) N-{(3S,4S)-2-{[(1S,2R)-2-Fluorocyclopropyl]carbonyl}-3-[(2,3',5'-trifluoro[biphenyl]-3-yl)methyl]-2-azabicyclo[3.1.1]heptan-4-yl}methanesulfonamide

Using the compound synthesized in Example 15B (30.0 mg) and (1S,2R)-2-fluorocyclopropane-1-carboxylic acid (8.0 mg), 32 mg (yield: 96%) of the marked compound was obtained as a single diastereomer and as a single enantiomer by the same method as in Example 29A.

### Example 31

### N-{(3S,4S)-3-[(3'-Cyano-2,5'-difluoro[biphenyl]-3-yl)methyl]-2-[(2R)-2-hydroxypropanoyl]-2-azabicyclo[3.1.1]heptan-4-yl}methanesulfonamide

### (31A) tert-Butyl (3S,4S)-3-[(3'-cyano-2,5'-difluoro[biphenyl]-3-yl)methyl]-4-[(methylsulfonyl)amino]-2-azabicyclo[3.1.1]heptane-2-carboxylate

Using the compound synthesized in Example 8G (49.2 mg) and 3-cyano-5-fluorophenylboronic acid (25.5 mg), 62.4 mg (yield: 99%) of the marked compound was obtained as a single diastereomer and as a single enantiomer by the same method as in Example 8J.

### (31B) N-{(3S,4S)-3-[(3'-Cyano-2,5'-difluoro[biphenyl]-3-yl)methyl]-2-azabicyclo[3.1.1]heptan-4-yl}methanesulfonamide hydrochloride

To the compound synthesized in Example 31A (62.4 mg), a solution of hydrogen chloride in 1,4-dioxane (4 M, 1.0 mL) was added at room temperature and the mixture was stirred for 3 hours. The solvent was distilled off under reduced pressure, and washing with ethyl acetate to afford 37.0 mg (yield: 79%) of the marked compound as a single diastereomer and as a single enantiomer.

### (31C) (2R)-1-{(3S,4S)-3-[(3'-Cyano-2,5'-difluoro[biphenyl]-3-yl)methyl]-4-[(methylsulfonyl)amino]-2-azabicyclo[3.1.1]heptan-2-yl}-1-oxopropan-2-yl acetate

To a mixture of the compound synthesized in Example 31B (19.7 mg), (R)-2-acetoxypropanoic acid (0.0058 mL), and N,N-dimethylformamide (1.0 mL), N,N-diisopropylethylamine (0.0227 mL) and O-(7-azabenzotriazol-1-yl)-N,N,N',N'-tetramethyluronium hexafluorophosphate (33.0 mg) were added at room temperature and the mixture was stirred for 1 hour. After adding water, the mixture was extracted with ethyl acetate, and the organic layer was dried over sodium sulfate. After filtration, the solvent was distilled off under reduced pressure, and the residue was purified by silica gel column chromatography [eluting solvent: n-hexane/ethyl acetate = 70/30 to 20/80 (V/V)] to afford 27.3 mg (yield: quantitative) of the marked compound as a single diastereomer and as a single enantiomer.

### (31D) N-{(3S,4S)-3-[(3'-Cyano-2,5'-difluoro[biphenyl]-3-yl)methyl]-2-[(2R)-2-hydroxypropanoyl]-2-azabicyclo[3.1.1]heptan-4-yl}methanesulfonamide

To a mixture of the compound synthesized in Example 31C (27.3 mg) and methanol (1.0 mL), potassium carbonate (12.0 mg) was added at room temperature and the mixture was stirred for 1.5 hours. After adding an aqueous ammonium chloride solution, the mixture was extracted with ethyl acetate, and the organic layer was dried over sodium sulfate. After filtration, the solvent was distilled off under reduced pressure, and the residue was purified by silica gel column chromatography [eluting solvent: n-hexane/ethyl acetate = 1/1 to 0/1 (V/V)] to afford 11.4 mg (yield: 54%) of the marked compound as a single diastereomer and as a single enantiomer.

### Example 32

### N-{(3S,4S)-3-[2-Fluoro-3-(1-methyl-1H-pyrazol-3-yl)benzyl]-2-[(2R)-2-hydroxypropanoyl]-2-azabicyclo[3.1.1]heptan-4-yl}methanesulfonamide

### (32A) tert-Butyl (3S,4S)-3-[2-fluoro-3-(1-methyl-1H-pyrazol-3-yl)benzyl]-4-[(methylsulfonyl)amino]-2-azabicyclo[3.1.1]heptane-2-carboxylate

Using the compound synthesized in Example 8G (50.2 mg) and 1-methyl-3-(4,4,5,5-tetramethyl-1,3,2-dioxaborolan-2-yl)-1H-pyrazole (32.8 mg), 58.8 mg (yield: 99%) of the marked compound was obtained as a single diastereomer and as a single enantiomer by the same method as in Example 8J.

### (32B) N-{(3S,4S)-3-[2-Fluoro-3-(1-methyl-1H-pyrazol-3-yl)benzyl]-2-azabicyclo[3.1.1]heptan-4-yl}methanesulfonamide hydrochloride

To the compound synthesized in Example 32A (58.8 mg), a solution of hydrogen chloride in 1,4-dioxane (4 M, 1.0 mL) was added at room temperature and the mixture was stirred for 3 hours. The solvent was distilled off under reduced pressure, and washing with ethyl acetate to afford 38.8 mg (yield: 89%) of the marked compound as a single diastereomer and as a single enantiomer.

### (32C) (2R)-1-{(3S,4S)-3-[2-Fluoro-3-(1-methyl-1H-pyrazol-3-yl)benzyl]-4-[(methylsulfonyl)amino]-2-azabicyclo[3.1.1]heptan-2-yl}-1-oxopropan-2-yl acetate

Using the compound synthesized in Example 32B (19.8 mg) and (R)-2-acetoxypropionic acid (0.0064 mL), 8.2 mg (yield: 35%) of the marked compound was obtained as a single diastereomer and as a single enantiomer by the same method as in Example 31C.

### (32D) N-{(3S,4S)-3-[2-Fluoro-3-(1-methyl-1H-pyrazol-3-yl)benzyl]-2-[(2R)-2-hydroxypropanoyl]-2-azabicyclo[3.1.1]heptan-4-yl}methanesulfonamide

Using the compound synthesized in Example 32C (8.2 mg), 7.3 mg (yield: 97%) of the marked compound was obtained as a single diastereomer and as a single enantiomer by the same method as in Example 31D.

### Example 33

### N-{(3S,4S)-2-[(1-Cyanocyclopropyl)carbonyl]-3-[(2,3',5'-trifluoro[biphenyl]-3-yl)methyl]-2-azabicyclo[3.1.1]heptan-4-yl}methanesulfonamide

### (33A) N-{(3S,4S)-2-[(1-Cyanocyclopropyl)carbonyl]-3-[(2,3',5'-trifluoro[biphenyl]-3-yl)methyl]-2-azabicyclo[3.1.1]heptan-4-yl}methanesulfonamide

Using the compound synthesized in Example 15B (22.3 mg) and 1-cyano-1-cyclopropanecarboxylic acid (6.7 mg), 25.6 mg (yield: quantitative) of the marked compound was obtained as a single diastereomer and as a single enantiomer by the same method as in Example 31C.

### Example 34

### N-{(3S,4S)-2-(Hydroxyacetyl)-3-[(2,3',5'-trifluoro[biphenyl]-3-yl)methyl]-2-azabicyclo[3.1.1]heptan-4-yl}methanesulfonamide (34A) 2-{(3S,4S)-4-[(Methylsulfonyl)amino]-3-[(2,3',5'-trifluoro[biphenyl]-3-yl)methyl]-2-azabicyclo[3.1.1]heptan-2-yl}-2-oxoethyl acetate

To a mixture of the compound synthesized in Example 15B (50 mg), N,N-diisopropylethylamine (72 mg), and tetrahydrofuran (0.5 mL), 2-acetoxyacetyl chloride (18 mg) was added and the mixture was stirred at room temperature for 30 minutes. The mixture was diluted with ethyl acetate, washed with water and a saturated saline solution, and dried over sodium sulfate. After filtration, the solvent was distilled off under reduced pressure to afford 50 mg of the crude marked compound as a single diastereomer and as a single enantiomer.

### (34B) N-{(3S,4S)-2-(Hydroxyacetyl)-3-[(2,3',5'-trifluoro[biphenyl]-3-yl)methyl]-2-azabicyclo[3.1.1]heptan-4-yl}methanesulfonamide

To a mixture of the compound synthesized in Example 34A (50 mg), methanol (2.0 mL), and water (0.2 mL), lithium hydroxide (12 mg) was added and the mixture was stirred at room temperature for 1 hour. The mixture was diluted with ethyl acetate, washed with water and a saturated saline solution, and dried over sodium sulfate. After filtration, the solvent was distilled off under reduced pressure, and the residue was purified by reverse phase silica gel column chromatography [eluting solvent: 0.225% aqueous formic acid solution/acetonitrile = 59/41 to 39/61 (V/V)] to afford 33 mg (yield: 72%) of the marked compound as a single diastereomer and as a single enantiomer.

### Example 35

### (3S,4S)-4-[(Methylsulfonyl)amino]-N-(propan-2-yl)-3-[(2,3',5'-trifluoro[biphenyl]-3-yl)methyl]-2-azabicyclo[3.1.1]heptane-2-carboxamide

### (35A) (3S,4S)-4-[(Methylsulfonyl)amino]-N-(propan-2-yl)-3-[(2,3',5'-trifluoro[biphenyl]-3-yl)methyl]-2-azabicyclo[3.1.1] heptane-2-carboxamide

To a mixture of the compound synthesized in Example 15B (30 mg), N,N-diisopropylethylamine (43 mg), and tetrahydrofuran (0.5 mL), isopropyl isocyanate (7 mg) was added and the mixture was stirred at room temperature for 30 minutes. The mixture was diluted with ethyl acetate, washed with water and a saturated saline solution, and dried over sodium sulfate. After filtration, the solvent was distilled off under reduced pressure, and the residue was purified by reverse phase silica gel column chromatography [eluting solvent: 0.225% aqueous formic acid solution/acetonitrile = 53/47 to 23/77 (V/V)] to afford 23 mg (yield: 69%) of the marked compound as a single diastereomer and as a single enantiomer.

### Example 36

### N-{(3S,4R)-2-(Cyclopropylcarbonyl)-5-fluoro-3-[(2,3',5'-trifluoro[biphenyl]-3-yl)methyl]-2-azabicyclo[3.1.1]heptan-4-yl}methanesulfonamide or N-{(3R,4S)-2-(cyclopropylcarbonyl)-5-fluoro-3-[(2,3',5'-trifluoro[biphenyl]-3-yl)methyl]-2-azabicyclo[3.1.1]heptan-4-yl}methanesulfonamide

### (36A) N-{(3S*,4R*)-2-(Cyclopropylcarbonyl)-5-fluoro-3-[(2,3',5'-trifluoro[biphenyl]-3-yl)methyl]-2-azabicyclo[3.1.1]heptan-4-yl}methanesulfonamide

To a mixture of the compound synthesized in Example 23G (60.0 mg), N,N-diisopropylethylamine (0.0878 mL), and tetrahydrofuran (2.5 mL), cyclopropanecarbonyl chloride (0.0235 mL) was added at room temperature and the mixture was stirred for 3 hours. The mixture was diluted with ethyl acetate, sequentially washed with water and a saturated saline solution, and dried over sodium sulfate. After filtration, the filtrate was concentrated under reduced pressure, and the residue was purified by silica gel column chromatography [eluting solvent: n-hexane/ethyl acetate = 70/30 to 10/90 (V/V)] to afford 60.2 mg (yield: 94%) of the marked compound as a single diastereomer (racemate).

### (36B) N-{(3S,4R)-2-(Cyclopropylcarbonyl)-5-fluoro-3-[(2,3',5'-trifluoro[biphenyl]-3-yl)methyl]-2-azabicyclo[3.1.1]heptan-4-yl}methanesulfonamide or N-{(3R,4S)-2-(cyclopropylcarbonyl)-5-fluoro-3-[(2,3',5'-trifluoro[biphenyl]-3-yl)methyl]-2-azabicyclo[3.1.1]heptan-4-yl}methanesulfonamide

The compound synthesized in Example 36A (60.2 mg) was subjected to chiral HPLC [column: CHIRALPAK (R) IG (20 mm I.D. × 250 mm), mobile phase: n-hexane/ethanol = 60/40 (V/V), temperature: 40°C] to afford 27.5 mg (yield: 46%) of the marked compound, which was eluted first, as a single diastereomer and as a single enantiomer.

### Example 37

### (3S,4S)-N-Ethyl-3-[(2-fluoro[biphenyl]-3-yl)methyl]-4-[(methylsulfonyl)amino]-2-azabicyclo[3.1.1]heptane-2-carboxamide

### (37A) (3S,4S)-N-Ethyl-3-[(2-fluoro[biphenyl]-3-yl)methyl]-4-[(methylsulfonyl)amino]-2-azabicyclo[3.1.1]heptane-2-carboxamide

To a mixture of the compound synthesized in Example 25B (70 mg), N,N-diisopropylethylamine (66 mg), and tetrahydrofuran (1.0 mL), ethyl isocyanate (18 mg) was added at 0°C, and the mixture was then stirred at room temperature for 30 minutes. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 64/36 to 34/66 (V/V)] to afford 46 mg (yield: 60%) of the marked compound as a single diastereomer and as a single enantiomer.

### Example 38

### N-{(3S,4S)-3-[3-(2,2-Difluoro-1,3-benzodioxol-4-yl)-2-fluorobenzyl]-2-[(2R)-2-hydroxypropanoyl]-2-azabicyclo[3.1.1] heptan-4-yl}methanesulfonamide

### (38A) tert-Butyl (3S,4S)-3-[3-(2,2-difluoro-1,3-benzodioxol-4-yl)-2-fluorobenzyl]-4-[(methylsulfonyl)amino]-2-azabicyclo[3.1.1]heptane-2-carboxylate

Using the compound synthesized in Example 8G (100 mg) and (2,2-difluoro-1,3-benzodioxol-4-yl)boronic acid (50 mg), 100 mg (yield: 86%) of the marked compound was obtained as a single diastereomer and as a single enantiomer by the same method as in Example 8J.

### (38B) N-{(3S,4S)-3-[3-(2,2-Difluoro-1,3-benzodioxol-4-yl)-2-fluorobenzyl]-2-azabicyclo[3.1.1]heptan-4-yl}methanesulfonamide hydrochloride

Using the compound synthesized in Example 38A (100 mg), 75 mg of the crude marked compound was obtained as a single diastereomer and as a single enantiomer by the same method as in Example 5G.

### (38C) N-{(3S,4S)-3-[3-(2,2-Difluoro-1,3-benzodioxol-4-yl)-2-fluorobenzyl]-2-[(2R)-2-hydroxypropanoyl]-2-azabicyclo[3.1.1]heptan-4-yl}methanesulfonamide

Using the compound synthesized in Example 38B (65 mg) and (R)-2-hydroxypropanoic acid (26 mg), 10 mg (yield: 13%) of the marked compound was obtained as a single diastereomer and as a single enantiomer by the same method as in Example 5H.

### Example 39

### (3S,4S)-N-(Cyclopropylmethyl)-4-[(methylsulfonyl)amino]-3-[(2,3',5'-trifluoro[biphenyl]-3-yl)methyl]-2-azabicyclo[3.1.1]heptane-2-carboxamide

### (39A) (3S,4S)-N-(Cyclopropylmethyl)-4-[(methylsulfonyl)amino]-3-[(2,3',5'-trifluoro[biphenyl]-3-yl)methyl]-2-azabicyclo[3.1.1]heptane-2-carboxamide

To a mixture of cyclopropylmethylamine (16 mg), N,N-diisopropylethylamine (87 mg), and dichloromethane (2.0 mL), a solution of triphosgene (65 mg) in dichloromethane (1.0 mL) was added at 0°C and the mixture was stirred at 0°C for 3 hours. The compound synthesized in Example 15B (100 mg) was added and the mixture was stirred at room temperature for 1 hour. Water was added, the mixture was extracted with dichloromethane, and the organic layer was washed with a saturated saline solution and then dried over sodium sulfate. After filtration, the solvent was distilled off under reduced pressure, and the residue was purified by preparative thin layer silica gel chromatography [eluting solvent: petroleum ether/ethyl acetate = 1/1 (V/V)] to afford 24 mg (yield: 21%) of the marked compound as a single diastereomer and as a single enantiomer.

### Example 40

### N-{(3S,4S)-3-[(2,3'-Difluoro[biphenyl]-3-yl)methyl]-2-[(2R)-2-hydroxypropanoyl]-2-azabicyclo[3.1.1]heptan-4-yl}methanesulfonamide

### (40A) tert-Butyl (3S,4S)-3-[(2,3'-difluoro[biphenyl]-3-yl)methyl]-4-[(methylsulfonyl)amino]-2-azabicyclo[3.1.1]heptane-2-carboxylate

Using the compound synthesized in Example 8G (150 mg) and 3-fluorophenylboronic acid (66 mg), 130 mg (yield: 84%) of the marked compound was obtained as a single diastereomer and as a single enantiomer by the same method as in Example 8J.

### (40B) N-{(3S,4S)-3-[(2,3'-Difluoro[biphenyl]-3-yl)methyl]-2-azabicyclo[3.1.1]heptan-4-yl}methanesulfonamide hydrochloride

Using the compound synthesized in Example 40A (130 mg), 100 mg of the crude marked compound was obtained as a single diastereomer and as a single enantiomer by the same method as in Example 5G.

### (40C) (2R)-1-{(3S,4S)-3-[(2,3'-Difluoro[biphenyl]-3-yl)methyl]-4-[(methylsulfonyl)amino]-2-azabicyclo[3.1.1]heptan-2-yl}-1-oxopropan-2-yl acetate

Using the compound synthesized in Example 40B (30 mg) and (R)-2-acetoxypropanoic acid chloride (15 mg), 20 mg of the crude marked compound was obtained as a single diastereomer and as a single enantiomer by the same method as in Example 5H.

### (40D) N-{(3S,4S)-3-[(2,3'-Difluoro[biphenyl]-3-yl)methyl]-2-[(2R)-2-hydroxypropanoyl]-2-azabicyclo[3.1.1]heptan-4-yl}methanesulfonamide

To a mixture of the compound synthesized in Example 40C (20 mg) and methanol (3.0 mL), lithium hydroxide (5.0 mg) and water (0.4 mL) were added at room temperature and the mixture was stirred for 2 hours. The mixture was diluted with ethyl acetate, washed with water and a saturated saline solution, and dried over sodium sulfate. After filtration, the solvent was distilled off under reduced pressure, and purification by reverse phase silica gel column chromatography [eluting solvent: 0.225% aqueous formic acid solution/acetonitrile = 67/33 to 37/63 (V/V)] was performed to afford 5.9 mg (yield: 31%) of the marked compound as a single diastereomer and as a single enantiomer.

### Example 41

### N-{(3S,4S)-3-[(3'-Cyclopropyl-2-fluoro[biphenyl]-3-yl)methyl]-2-[(2R)-2-hydroxypropanoyl]-2-azabicyclo[3.1.1]heptan-4-yl}methanesulfonamide

### (41A) tert-Butyl (3S,4S)-3-[(3'-cyclopropyl-2-fluoro[biphenyl]-3-yl)methyl]-4-[(methylsulfonyl)amino]-2-azabicyclo[3.1.1]heptane-2-carboxylate

Using the compound synthesized in Example 8G (100 mg) and 2-(3-cyclopropylphenyl)-4,4,5,5-tetramethyl-1,3,2-dioxaborolane (70 mg), 60 mg (yield: 56%) of the marked compound was obtained as a single diastereomer and as a single enantiomer by the same method as in Example 8J.

### (41B) N-{(3S,4S)-3-[(3'-Cyclopropyl-2-fluoro[biphenyl]-3-yl)methyl]-2-azabicyclo[3.1.1]heptan-4-yl}methanesulfonamide hydrochloride

Using the compound synthesized in Example 41A (60 mg), 40 mg of the crude marked compound was obtained as a single diastereomer and as a single enantiomer by the same method as in Example 5G.

### (41C) (2R)-1-{(3S,4S)-3-[(3'-Cyclopropyl-2-fluoro[biphenyl]-3-yl)methyl]-4-[(methylsulfonyl)amino]-2-azabicyclo[3.1.1]heptan-2-yl}-1-oxopropan-2-yl acetate

Using the compound synthesized in Example 41B (40 mg) and (R)-2-acetoxypropanoic acid chloride (20 mg), 40 mg of the crude marked compound was obtained as a single diastereomer and as a single enantiomer by the same method as in Example 34A.

### (41D) N-{(3S,4S)-3-[(3'-Cyclopropyl-2-fluoro[biphenyl]-3-yl)methyl]-2-[(2R)-2-hydroxypropanoyl]-2-azabicyclo[3.1.1]heptan-4-yl}methanesulfonamide

To a mixture of the compound synthesized in Example 41C (40 mg), tetrahydrofuran (1.0 mL), and water (0.3 mL), lithium hydroxide (13 mg) was added at room temperature and the mixture was stirred for 2 hours. The reaction solution was poured into water, the mixture was extracted with ethyl acetate, and the organic layer was washed with a saturated saline solution and then dried over sodium sulfate. After filtration, the solvent was distilled off under reduced pressure, and the residue was purified by reverse phase silica gel column chromatography [eluting solvent: 0.225% aqueous formic acid solution/acetonitrile = 54/46 to 34/66 (V/V)] to afford 33 mg (yield: 90%) of the marked compound as a single diastereomer and as a single enantiomer.

### Example 42

### N-{(3S,4S)-3-{[2-Fluoro-3'-(trifluoromethoxy)[biphenyl]-3-yl]methyl}-2-[(2R)-2-hydroxypropanoyl]-2-azabicyclo[3.1.1]heptan-4-yl}methanesulfonamide

### (42A) tert-Butyl (3S,4S)-3-{[2-fluoro-3'-(trifluoromethoxy)[biphenyl]-3-yl]methyl}-4-[(methylsulfonyl)amino]-2-azabicyclo[3.1.1]heptane-2-carboxylate

To a mixture of the compound synthesized in Example 8G (100 mg), 1,4-dioxane (6.0 mL), and water (1.0 mL), 3-(trifluoromethoxy)phenylboronic acid (45 mg), [1,1'-bis(diphenylphosphino)ferrocene]palladium(II) dichloride (16 mg), and sodium carbonate (45 mg) were added and the mixture was stirred at 100°C for 3 hours. The insoluble material was removed by filtration, and the filtrate was washed with ethyl acetate and then dried over sodium sulfate. After filtration, the solvent was distilled off under reduced pressure, and the residue was purified by preparative thin layer silica gel chromatography [eluting solvent: petroleum ether/ethyl acetate = 3/1 (V/V)] to afford 80 mg of the marked compound mixed with byproducts as a single diastereomer and as a single enantiomer.

### (42B) N-[(3S,4S)-3-{[2-Fluoro-3'-(trifluoromethoxy)[biphenyl]-3-yl]methyl}-2-azabicyclo[3.1.1]heptan-4-yl]methanesulfonamide hydrochloride

Using the compound synthesized in Example 42A (80 mg), 50 mg of the crude marked compound was obtained as a single diastereomer and as a single enantiomer by the same method as in Example 5G.

### (42C) N-{(3S,4S)-3-{[2-Fluoro-3'-(trifluoromethoxy)[biphenyl]-3-yl]methyl}-2-[(2R)-2-hydroxypropanoyl]-2-azabicyclo[3.1.1]heptan-4-yl}methanesulfonamide

Using the compound synthesized in Example 42B (40 mg) and (R)-2-hydroxypropanoic acid (24 mg), 3.1 mg (yield: 6%) of the marked compound was obtained as a single diastereomer and as a single enantiomer by the same method as in Example 31C.

### Example 43

### N-{(3S,4S)-3-[3-(6-Cyclopropylpyridin-2-yl)-2-fluorobenzyl]-2-[(2R)-2-hydroxypropanoyl]-2-azabicyclo[3.1.1]heptan-4-yl}methanesulfonamide

### (43A) tert-Butyl (3S,4S)-3-[3-(6-cyclopropylpyridin-2-yl)-2-fluorobenzyl]-4-[(methylsulfonyl)amino]-2-azabicyclo[3.1.1]heptane-2-carboxylate

A mixture of the compound synthesized in Example 21A (60 mg), cesium carbonate (75 mg), 2-bromo-6-cyclopropylpyridine (CAS Registration No.: 1086381-26-1, 40 mg), (2-dicyclohexylphosphino-2',4',6'-triisopropyl-1,1'-biphenyl)[2-(2'-amino-1,1'-biphenyl)]palladium(II) methanesulfonate (6 mg), water (0.5 mL), and 1,4-dioxane (3.0 mL) was degassed and then stirred at 100°C for 2 hours in a nitrogen atmosphere. The reaction solution was poured into water and extracted with ethyl acetate, and the organic layer was washed with a saturated saline solution. The organic layer was dried over sodium sulfate and filtered, and the solvent was then distilled off under reduced pressure. The residue was purified by preparative thin layer silica gel chromatography [eluting solvent: petroleum ether/ethyl acetate = 1/1 (V/V)] to afford 50 mg (yield: 85%) of the marked compound as a single diastereomer and as a single enantiomer.

### (43B) N-{(3S,4S)-3-[3-(6-Cyclopropylpyridin-2-yl)-2-fluorobenzyl]-2-azabicyclo[3.1.1]heptan-4-yl}methanesulfonamide hydrochloride

Using the compound synthesized in Example 43A (50 mg), 40 mg of the crude marked compound was obtained as a single diastereomer and as a single enantiomer by the same method as in Example 5G.

### (43C) (2R)-1-{(3S,4S)-3-[3-(6-Cyclopropylpyridin-2-yl)-2-fluorobenzyl]-4-[(methylsulfonyl)amino]-2-azabicyclo[3.1.1]heptan-2-yl}-1-oxopropan-2-yl acetate

Using the compound synthesized in Example 43B (80 mg) and (R)-2-acetoxypropionic acid chloride (20 mg), 40 mg of the crude marked compound was obtained as a single diastereomer and as a single enantiomer by the same method as in Example 34A.

### (43D) N-{(3S,4S)-3-[3-(6-Cyclopropylpyridin-2-yl)-2-fluorobenzyl]-2-[(2R)-2-hydroxypropanoyl]-2-azabicyclo[3.1.1]heptan-4-yl}methanesulfonamide

To a mixture of the compound synthesized in Example 43C (40 mg), water (0.3 mL), and tetrahydrofuran (1.0 mL), lithium hydroxide (13 mg) was added at room temperature and the mixture was stirred for 2 hours. The reaction solution was poured into water and extracted with ethyl acetate. The organic layer was washed with a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 85/15 to 65/35 (V/V)] to afford 31 mg (yield: 81%) of the marked compound as a single diastereomer and as a single enantiomer.

### Example 44

### N-{(3S,4S)-3-[(2-Fluoro[biphenyl]-3-yl)methyl]-2-[(2R)-2-hydroxypropanoyl]-2-azabicyclo[3.1.1]heptan-4-yl}cyclopropanesulfonamide

### (44A) tert-Butyl (3S,4S)-3-(3-bromo-2-fluorobenzyl)-4-[(cyclopropylsulfonyl)amino]-2-azabicyclo[3.1.1]heptane-2-carboxylate

To a mixture of the compound synthesized in Example 8F (110 mg), triethylamine (0.0764 mL), and tetrahydrofuran (2.3 mL), cyclopropanesulfonyl chloride (0.0393 mL) was added under ice cooling, and the mixture was then stirred at room temperature for 1.5 hours. 4-Dimethylaminopyridine (33.7 mg), triethylamine (0.0764 mL), and cyclopropanesulfonyl chloride (0.0393 mL) were added and the mixture was stirred at 40°C for 3 hours. The mixture was diluted with ethyl acetate, sequentially washed with hydrochloric acid (1 M) and an aqueous sodium bicarbonate solution, and dried over sodium sulfate. After filtration, the solvent was distilled off under reduced pressure to afford 139 mg of the crude marked compound as a single diastereomer and as a single enantiomer.

### (44B) tert-Butyl (3S,4S)-4-[(cyclopropylsulfonyl)amino]-3-[(2-fluoro[biphenyl]-3-yl)methyl]-2-azabicyclo[3.1.1]heptane-2-carboxylate

To a mixture of the compound synthesized in Example 44A (139 mg), phenylboronic acid (47.1 mg), potassium carbonate (114 mg), and 1,4-dioxane (1.8 mL), (2-dicyclohexylphosphino-2',4',6'-triisopropyl-1,1'-biphenyl)[2-(2'-amino-1,1'-biphenyl)]palladium(II) methanesulfonate (11.7 mg) and water (0.46 mL) were added at room temperature and the mixture was stirred at 80°C for 1.5 hours in a nitrogen atmosphere. Phenylboronic acid (24 mg), potassium carbonate (50 mg), (2-dicyclohexylphosphino-2',4',6'-triisopropyl-1,1'-biphenyl)[2-(2'-amino-1,1'-biphenyl)]palladium(II) methanesulfonate (5.0 mg) were added and the mixture was stirred at 80°C for 3 hours. The mixture was diluted with ethyl acetate, sequentially washed with water and a saturated saline solution, and dried over sodium sulfate. After filtration, the solvent was distilled off under reduced pressure, and the residue was purified by silica gel column chromatography [eluting solvent: n-hexane/ethyl acetate = 9/1 to 3/2 (V/V)] to afford 127 mg (yield: 92%) of the marked compound as a single diastereomer and as a single enantiomer.

### (44C) N-{(3S,4S)-3-[(2-Fluoro[biphenyl]-3-yl)methyl]-2-azabicyclo[3.1.1]heptan-4-yl}cyclopropanesulfonamide hydrochloride

Using the compound synthesized in Example 44B (127 mg), 106 mg of the crude marked compound was obtained as a single diastereomer and as a single enantiomer by the same method as in Example 8H.

### (44D) (2R)-1-{(3S,45)-4-[(Cyclopropylsulfonyl)amino]-3-[(2-fluoro[biphenyl]-3-yl)methyl]-2-azabicyclo[3.1.1]heptan-2-yl}-1-oxopropan-2-yl acetate

To a mixture of the compound synthesized in Example 44C (31.0 mg), (R)-2-acetoxypropanoic acid (0.0119 mL), 1-hydroxy-7-azabenzotriazole (9.7 mg), and dichloromethane (0.70 mL), triethylamine (0.0295 mL) and 1-ethyl-3-(3-dimethylaminopropyl)carbodiimide hydrochloride (20.4 mg) were added at room temperature and the mixture stirred for 30 hours. The mixture was diluted with ethyl acetate, sequentially washed with hydrochloric acid (1 M), an aqueous sodium bicarbonate solution, and a saturated saline solution, and dried over sodium sulfate. After filtration, the solvent was distilled off under reduced pressure to afford 32.0 mg of the crude marked compound as a single diastereomer and as a single enantiomer.

### (44E) N-{(3S,4S)-3-[(2-Fluoro[biphenyl]-3-yl)methyl]-2-[(2R)-2-hydroxypropanoyl]-2-azabicyclo[3.1.1]heptan-4-yl}cyclopropanesulfonamide

Using the compound synthesized in Example 44D (32.0 mg), 20.8 mg (yield: 71%) of the marked compound was obtained as a single diastereomer and as a single enantiomer by the same method as in Example 12H.

### Example 45

### N-[(3S,4S)-3-[(2-Fluoro[biphenyl]-3-yl)methyl]-2-{[(1R,2R)-2-fluorocyclopropyl]carbonyl}-2-azabicyclo[3.1.1]heptan-4-yl]cyclopropanesulfonamide

### (45A) N-[(3S,4S)-3-[(2-Fluoro[biphenyl]-3-yl)methyl]-2-{[(1R,2R)-2-fluorocyclopropyl]carbonyl}-2-azabicyclo[3.1.1]heptan-4-yl]cyclopropanesulfonamide

Using the compound synthesized in Example 44C (25.0 mg) and (1R,2R)-2-fluorocyclopropane-1-carboxylic acid (8.9 mg), 26.6 mg (yield: 96%) of the marked compound was obtained as a single diastereomer and as a single enantiomer by the same method as in Example 5H.

### Example 46

### N-{(3S,4R)-2-Acetyl-3-[(3'-chloro-2-fluoro[biphenyl]-3-yl)methyl]-5-fluoro-2-azabicyclo[3.1.1]heptan-4-yl}methanesulfonamide

### (46A) tert-Butyl 4-amino-3-(3-bromo-2-fluorobenzyl)-5-fluoro-2-azabicyclo[3.1.1]heptane-2-carboxylate

To a mixture of the compound synthesized in Example 23C (1.5 g), molybdenum(VI) oxide (1.0 g), and methanol (150 mL), sodium borohydride (50 g) was added at 0°C, and the mixture was then stirred at room temperature for 23 hours. Sodium borohydride (20 g) was added and the mixture was stirred at room temperature for 18 hours. Sodium borohydride (20 g) was added and the mixture was stirred at room temperature for 4 hours. Sodium borohydride (10 g) was added and the mixture was stirred at room temperature for 1 hour. The reaction solution was poured into an aqueous ammonium chloride solution and extracted with ethyl acetate, and the organic layer was washed with a saturated saline solution. The organic layer was dried over sodium sulfate and filtered, and the solvent was then distilled off under reduced pressure. The residue was purified by reverse phase silica gel column chromatography [eluting solvent: 0.225% aqueous formic acid solution/acetonitrile = 88/12 to 58/42 (V/V)] to afford the marked compound.

### (46B) tert-Butyl (3S,4R)-4-amino-3-(3-bromo-2-fluorobenzyl)-5-fluoro-2-azabicyclo[3.1.1]heptane-2-carboxylate

The entire amount of the compound synthesized in Example 46A was subjected to chiral SFC [column: Phenomenex-Cellulose-2 (30 mm I.D. × 250 mm), mobile phase: carbon dioxide/methanol = 65/35 (V/V)] to afford 150 mg (yield: 10%) of the marked compound, which was eluted later, as a single diastereomer and as a single enantiomer.

### (46C) tert-Butyl (3S,4R)-3-(3-bromo-2-fluorobenzyl)-5-fluoro-4-[(methylsulfonyl)amino]-2-azabicyclo[3.1.1]heptane-2-carboxylate

To a mixture of the compound synthesized in Example 46B (150 mg), N,N-diisopropylethylamine (140 mg), and tetrahydrofuran (5.0 mL), methanesulfonyl chloride (500 mg) was added at 0°C, and the mixture was then stirred at room temperature for 1.5 hours. The reaction solution was poured into an aqueous sodium bicarbonate solution and extracted with ethyl acetate, and the organic layer was then washed with a saturated saline solution. The organic layer was dried over sodium sulfate and filtered, and the solvent was then distilled off under reduced pressure to afford 150 mg of the crude marked compound as a single diastereomer and as a single enantiomer.

### (46D) tert-Butyl (3S,4R)-3-[(3'-chloro-2-fluoro[biphenyl]-3-yl)methyl]-5-fluoro-4-[(methylsulfonyl)amino]-2-azabicyclo[3.1.1]heptane-2-carboxylate

A mixture of the compound synthesized in Example 46C (100 mg), 3-chlorophenylboronic acid (50 mg), cesium carbonate (130 mg), (2-dicyclohexylphosphino-2',4',6'-triisopropyl-1,1'-biphenyl)[2-(2'-amino-1,1'-biphenyl)]palladium(II) methanesulfonate (11 mg), and 1,4-dioxane (1.0 mL) was degassed and then stirred at 100°C for 2 hours in a nitrogen atmosphere. The reaction solution was poured into water and extracted with ethyl acetate, and the organic layer was then washed with a saturated saline solution. The organic layer was dried over sodium sulfate and filtered, and the solvent was then distilled off under reduced pressure. The residue was purified by reverse phase silica gel column chromatography [eluting solvent: 0.225% aqueous formic acid solution/acetonitrile = 88/12 to 58/42 (V/V)] to afford 10 mg (yield: 9%) of the marked compound as a single diastereomer and as a single enantiomer.

### (46E) N-{(3S,4R)-3-[(3'-Chloro-2-fluoro[biphenyl]-3-yl)methyl]-5-fluoro-2-azabicyclo[3.1.1]heptan-4-yl}methanesulfonamide trifluoroacetate

To a mixture of the compound synthesized in Example 46D (25 mg) and dichloromethane (1.5 mL), trifluoroacetic acid (4 g) was added and the mixture was stirred at room temperature for 1 hour. The solvent was distilled off under reduced pressure to afford 20 mg of the crude marked compound as a single diastereomer and as a single enantiomer.

### (46F) N-{(3S,4R)-2-Acetyl-3-[(3'-chloro-2-fluoro[biphenyl]-3-yl)methyl]-5-fluoro-2-azabicyclo[3.1.1]heptan-4-yl}methanesulfonamide

To a mixture of the compound synthesized in Example 46E (20 mg), acetic anhydride (10 mg), and N,N-dimethylformamide (1.0 mL), N,N-diisopropylethylamine (30 mg) was added and the mixture was stirred at room temperature for 2 hours. The insoluble material was removed by filtration, and the solvent was distilled off under reduced pressure. The residue was purified by reverse phase silica gel column chromatography [eluting solvent: 0.225% aqueous formic acid solution/acetonitrile = 56/44 to 36/64 (V/V)] to afford 23 mg (yield: quantitative) of the marked compound as a single diastereomer and as a single enantiomer.

### Example 47

### N-{(3S,4S)-3-{3-[6-(Difluoromethyl)pyridin-2-yl]-2-fluorobenzyl}-2-[(2R)-2-hydroxypropanoyl]-2-azabicyclo[3.1.1]heptan-4-yl}methanesulfonamide

### (47A) tert-Butyl (3S,4S)-3-{3-[6-(difluoromethyl)pyridin-2-yl]-2-fluorobenzyl}-4-[(methylsulfonyl)amino]-2-azabicyclo[3.1.1]heptane-2-carboxylate

A mixture of the compound synthesized in Example 21A (109 mg), cesium carbonate (135 mg), 2-bromo-6-(difluoromethyl)pyridine (CAS Registration No.: 872365-91-8, 43 mg), (2-dicyclohexylphosphino-2',4',6'-triisopropyl-1,1'-biphenyl)[2-(2'-amino-1,1'-biphenyl)]palladium(II) methanesulfonate (18 mg), water (0.8 mL), and 1,4-dioxane (4.0 mL) was degassed and then stirred at 100°C for 2 hours in a nitrogen atmosphere. The insoluble material was removed by filtration, and the filtrate was diluted with ethyl acetate. The organic layer was washed with water and a saturated saline solution, and then dried over sodium sulfate. After filtration, the solvent was distilled off under reduced pressure. The residue was purified by reverse phase silica gel column chromatography [eluting solvent: aqueous ammonium bicarbonate solution/acetonitrile = 75/25 to 45/55 (V/V)] to afford 56 mg (yield: 51%) of the marked compound as a single diastereomer and as a single enantiomer.

### (47B) N-[(3S,4S)-3-{3-[6-(Difluoromethyl)pyridin-2-yl]-2-fluorobenzyl}-2-azabicyclo[3.1.1]heptan-4-yl]methanesulfonamide hydrochloride

Using the compound synthesized in Example 47A (50 mg), 44 mg of the crude marked compound was obtained as a single diastereomer and as a single enantiomer by the same method as in Example 5G.

### (47C) (2R)-1-{(3S,4S)-3-{3-[6-(Difluoromethyl)pyridin-2-yl]-2-fluorobenzyl}-4-[(methylsulfonyl)amino]-2-azabicyclo[3.1.1]heptan-2-yl}-1-oxopropan-2-yl acetate

Using the compound synthesized in Example 47C (40 mg) and (R)-2-acetoxypropionic acid chloride (15 mg), 48 mg of the crude marked compound was obtained as a single diastereomer and as a single enantiomer by the same method as in Example 34A.

### (47D) N-{(3S,4S)-3-{3-[6-(Difluoromethyl)pyridin-2-yl]-2-fluorobenzyl}-2-[(2R)-2-hydroxypropanoyl]-2-azabicyclo[3.1.1]heptan-4-yl}methanesulfonamide

To a mixture of the compound synthesized in Example 47C (48 mg), water (0.5 mL), and tetrahydrofuran (2.0 mL), lithium hydroxide (10 mg) was added at room temperature and the mixture was stirred for 2 hours. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 72/28 to 42/58 (V/V)] to afford 18 mg (yield: 40%) of the marked compound as a single diastereomer and as a single enantiomer.

### Example 48

### N-{(3S,4S)-2-[(2R)-2-Methoxypropanoyl]-3-[(2,3',5'-trifluoro[biphenyl]-3-yl)methyl]-2-azabicyclo[3.1.1]heptan-4-yl}methanesulfonamide

### (48A) N-{(3S,4S)-2-[(2R)-2-Methoxypropanoyl]-3-[(2,3',5'-trifluoro[biphenyl]-3-yl)methyl]-2-azabicyclo[3.1.1]heptan-4-yl}methanesulfonamide

Using the compound synthesized in Example 15B (50 mg) and (R)-2-methoxypropanoic acid (17 mg), 42.5 mg (yield: 77%) of the marked compound was obtained as a single diastereomer and as a single enantiomer by the same method as in Example 31C.

### Example 49

### N-[(3S,4R)-3-{[3'-(Difluoromethyl)-2-fluoro[biphenyl]-3-yl]methyl}-5-fluoro-2-(2-hydroxy-2-methylpropanoyl)-2-azabicyclo[3.1.1]heptan-4-yl]methanesulfonamide

### (49A) tert-Butyl (3S,4R)-3-{[3'-(difluoromethyl)-2-fluoro[biphenyl]-3-yl]methyl}-5-fluoro-4-[(methylsulfonyl)amino]-2-azabicyclo[3.1.1]heptane-2-carboxylate

Using the compound synthesized in Example 46C (40 mg) and 3-(difluoromethyl)phenylboronic acid (20 mg), 20 mg (yield: 46%) of the marked compound was obtained as a single diastereomer and as a single enantiomer by the same method as in Example 8J.

### (49B) N-[(3S,4R)-3-{[3'-(Difluoromethyl)-2-fluoro[biphenyl]-3-yl]methyl}-5-fluoro-2-azabicyclo[3.1.1]heptan-4-yl]methanesulfonamide trifluoroacetate

To a mixture of the compound synthesized in Example 49A (20 mg) and dichloromethane (1.0 mL), trifluoroacetic acid (462 mg) was added and the mixture was stirred at room temperature for 1 hour. The solvent was distilled off under reduced pressure to afford 20 mg of the crude marked compound as a single diastereomer and as a single enantiomer.

### (49C) 1-{(3S,4R)-3-{[3'-(Difluoromethyl)-2-fluoro[biphenyl]-3-yl]methyl}-5-fluoro-4-[(methylsulfonyl)amino]-2-azabicyclo[3.1.1]heptan-2-yl}-2-methyl-1-oxopropan-2-yl acetate

Using the compound synthesized in Example 49B (20 mg) and 2-acetoxyisobutyryl chloride (12 mg), 20 mg of the crude marked compound was obtained as a single diastereomer and as a single enantiomer by the same method as in Example 34A.

### (49D) N-[(3S,4R)-3-{[3'-(Difluoromethyl)-2-fluoro[biphenyl]-3-yl]methyl}-5-fluoro-2-(2-hydroxy-2-methylpropanoyl)-2-azabicyclo[3.1.1]heptan-4-yl]methanesulfonamide

To a mixture of the compound synthesized in Example 49C (20 mg), water (0.3 mL), and tetrahydrofuran (1.0 mL), lithium hydroxide (10 mg) was added at room temperature, and the mixture was then stirred for 6 hours. The reaction solution was poured into water and extracted with ethyl acetate, and the organic layer was washed with a saturated saline solution. The organic layer was dried over sodium sulfate and filtered, and the solvent was then distilled off under reduced pressure. The residue was purified by reverse phase silica gel column chromatography [eluting solvent: 0.225% aqueous formic acid solution/acetonitrile = 55/45 to 35/65 (V/V)] to afford 11 mg (yield: 57%) of the marked compound as a single diastereomer and as a single enantiomer.

### Example 50

### N-[(3S,4R)-5-Fluoro-3-[(2-fluoro[biphenyl]-3-yl)methyl]-2-(2-hydroxy-2-methylpropanoyl)-2-azabicyclo[3.1.1]heptan-4-yl]methanesulfonamide

### (50A) tert-Butyl (3S,4R)-5-fluoro-3-[(2-fluoro[biphenyl]-3-yl)methyl]-4-[(methylsulfonyl)amino]-2-azabicyclo[3.1.1]heptane-2-carboxylate

Using the compound synthesized in Example 46C (120 mg) and phenylboronic acid (48 mg), 60 mg (yield: 50%) of the marked compound was obtained as a single diastereomer and as a single enantiomer by the same method as in Example 8J.

### (50B) N-{(3S,4R)-5-Fluoro-3-[(2-fluoro[biphenyl]-3-yl)methyl]-2-azabicyclo[3.1.1]heptan-4-yl}methanesulfonamide trifluoroacetate

To a mixture of the compound synthesized in Example 50A (60 mg) and dichloromethane (2.0 mL), trifluoroacetic acid (0.62 mL) was added and the mixture was stirred at room temperature for 1 hour. The solvent was distilled off under reduced pressure to afford 60 mg of the crude marked compound as a single diastereomer and as a single enantiomer.

### (50C) 1-{(3S,4R)-5-Fluoro-3-[(2-fluoro[biphenyl]-3-yl)methyl]-4-[(methylsulfonyl)amino]-2-azabicyclo[3.1.1]heptan-2-yl}-2-methyl-1-oxopropan-2-yl acetate

Using the compound synthesized in Example 50B (20 mg) and 2-acetoxyisobutyryl chloride (13 mg), 20 mg of the crude marked compound was obtained as a single diastereomer and as a single enantiomer by the same method as in Example 34A.

### (50D) N-[(3S,4R)-5-Fluoro-3-[(2-fluoro[biphenyl]-3-yl)methyl]-2-(2-hydroxy-2-methylpropanoyl)-2-azabicyclo[3.1.1]heptan-4-yl]methanesulfonamide

To a mixture of the compound synthesized in Example 50C (20 mg), water (0.3 mL), and tetrahydrofuran (1.0 mL), lithium hydroxide (10 mg) was added at room temperature, and the mixture was then stirred for 6 hours. The reaction solution was poured into water and extracted with ethyl acetate, and the organic layer was washed with a saturated saline solution. The organic layer was dried over sodium sulfate and filtered, and the solvent was then distilled off under reduced pressure. The residue was purified by reverse phase silica gel column chromatography [eluting solvent: 0.225% aqueous formic acid solution/acetonitrile = 58/42 to 38/62 (V/V)] to afford 11 mg (yield: 58%) of the marked compound as a single diastereomer and as a single enantiomer.

### Example 51

### N-[(3S,4S)-3-{[3'-(Difluoromethyl)-2,5'-difluoro[biphenyl]-3-yl]methyl}-2-(2-hydroxy-2-methylpropanoyl)-2-azabicyclo[3.1.1]heptan-4-yl]methanesulfonamide

### (51A) tert-Butyl (3S,4S)-3-{[3'-(difluoromethyl)-2,5'-difluoro[biphenyl]-3-yl]methyl}-4-[(methylsulfonyl)amino]-2-azabicyclo[3.1.1]heptane-2-carboxylate

A mixture of the compound synthesized in Example 21A (100 mg), cesium carbonate (124 mg), 1-bromo-3-(difluoromethyl)-5-fluorobenzene (51 mg), (2-dicyclohexylphosphino-2',4',6'-triisopropyl-1,1'-biphenyl)[2-(2'-amino-1,1'-biphenyl)]palladium(II) methanesulfonate (16 mg), water (0.5 mL), and tetrahydrofuran (5.0 mL) was degassed and then stirred at 100°C for 2 hours in a nitrogen atmosphere. The mixture was diluted with ethyl acetate, washed with water and a saturated saline solution, and then dried over sodium sulfate. After filtration, the solvent was distilled off under reduced pressure, and the residue was purified by preparative thin layer silica gel chromatography [eluting solvent: petroleum ether/ethyl acetate = 2/1 (V/V)] to afford 60 mg (yield: 58%) of the marked compound as a single diastereomer and as a single enantiomer.

### (51B) N-[(3S,4S)-3-{[3'-(Difluoromethyl)-2,5'-difluoro[biphenyl]-3-yl]methyl}-2-azabicyclo[3.1.1]heptan-4-yl]methanesulfonamide hydrochloride

Using the compound synthesized in Example 51A (50 mg), 60 mg of the crude marked compound was obtained as a single diastereomer and as a single enantiomer by the same method as in Example 5G.

### (51C) 1-{(3S,4S)-3-{[3'-(Difluoromethyl)-2,5'-difluoro[biphenyl]-3-yl]methyl}-4-[(methylsulfonyl)amino]-2-azabicyclo[3.1.1]heptan-2-yl}-2-methyl-1-oxopropan-2-yl acetate

Using the compound synthesized in Example 51B (60 mg) and 2-acetoxyisobutyryl chloride (31 mg), 50 mg of the crude marked compound was obtained as a single diastereomer and as a single enantiomer by the same method as in Example 34A.

### (51D) N-[(3S,4S)-3-{[3'-(Difluoromethyl)-2,5'-difluoro[biphenyl]-3-yl]methyl}-2-(2-hydroxy-2-methylpropanoyl)-2-azabicyclo[3.1.1]heptan-4-yl]methanesulfonamide

To a mixture of the compound synthesized in Example 51C (50 mg), water (0.3 mL), and methanol (3.0 mL), lithium hydroxide (10 mg) was added at room temperature and the mixture was stirred for 2 hours. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 60/40 to 30/70 (V/V)] to afford 35 mg (yield: 76%) of the marked compound as a single diastereomer and as a single enantiomer.

### Example 52

### N-[(3S,4S)-3-[(3'-Chloro-2,5'-difluoro[biphenyl]-3-yl)methyl]-2-(2-hydroxy-2-methylpropanoyl)-2-azabicyclo[3.1.1]heptan-4-yl]methanesulfonamide

### (52A) tert-Butyl (3S,4S)-3-[(3'-chloro-2,5'-difluoro[biphenyl]-3-yl)methyl]-4-[(methylsulfonyl)amino]-2-azabicyclo[3.1.1]heptane-2-carboxylate

Using the compound synthesized in Example 8G (100 mg) and 3-chloro-5-fluorophenylboronic acid (55 mg), 35 mg (yield: 32%) of the marked compound was obtained as a single diastereomer and as a single enantiomer by the same method as in Example 8J.

### (52B) N-{(3S,4S)-3-[(3'-Chloro-2,5'-difluoro[biphenyl]-3-yl)methyl]-2-azabicyclo[3.1.1] heptan-4-yl}methanesulfonamide hydrochloride

Using the compound synthesized in Example 52A (30 mg), 26 mg of the crude marked compound was obtained as a single diastereomer and as a single enantiomer by the same method as in Example 5G.

### (52C) 1-{(3S,4S)-3-[(3'-Chloro-2,5'-difluoro[biphenyl]-3-yl)methyl]-4-[(methylsulfonyl)amino]-2-azabicyclo[3.1.1]heptan-2-yl}-2-methyl-1-oxopropan-2-yl acetate

Using the compound synthesized in Example 52B (26 mg) and 2-acetoxyisobutyryl chloride (9.0 mg), 31 mg of the crude marked compound was obtained as a single diastereomer and as a single enantiomer by the same method as in Example 34A.

### (52D) N-[(3S,4S)-3-[(3'-Chloro-2,5'-difluoro[biphenyl]-3-yl)methyl]-2-(2-hydroxy-2-methylpropanoyl)-2-azabicyclo[3.1.1]heptan-4-yl]methanesulfonamide

To a mixture of the compound synthesized in Example 52C (28 mg), water (1.0 mL), and tetrahydrofuran (3.0 mL), lithium hydroxide monohydrate (11 mg) was added at room temperature and the mixture was stirred for 12 hours. The mixture was diluted with ethyl acetate and washed with water and a saturated saline solution. The mixture was dried over sodium sulfate and filtered. The solvent was distilled off under reduced pressure, and the residue was purified by reverse phase silica gel column chromatography [eluting solvent: 0.225% aqueous formic acid solution/acetonitrile = 50/50 to 30/70 (V/V)] to afford 20 mg (yield: 77%) of the marked compound as a single diastereomer and as a single enantiomer.

### Example 53

### N-{(3S,4R)-5-Fluoro-3-[(2-fluoro[biphenyl]-3-yl)methyl]-2-[(2R)-2-hydroxypropanoyl]-2-azabicyclo[3.1.1]heptan-4-yl}methanesulfonamide

### (53A) (2R)-1-{(3S,4R)-5-Fluoro-3-[(2-fluoro[biphenyl]-3-yl)methyl]-4-[(methylsulfonyl)amino]-2-azabicyclo[3.1.1]heptan-2-yl}-1-oxopropan-2-yl acetate

To a mixture of the compound synthesized in Example 50B (20 mg) and tetrahydrofuran (1.0 mL), N,N-diisopropylethylamine (60 mg) and (R)-2-acetoxypropionic acid chloride (12 mg) were added at room temperature and the mixture was stirred for 30 minutes. The mixture was diluted with ethyl acetate, washed with water and a saturated saline solution, and then dried over sodium sulfate. After filtration, the solvent was distilled off under reduced pressure to afford 20 mg of the crude marked compound as a single diastereomer and as a single enantiomer.

### (53B) N-{(3S,4R)-5-Fluoro-3-[(2-fluoro[biphenyl]-3-yl)methyl]-2-[(2R)-2-hydroxypropanoyl]-2-azabicyclo[3.1.1]heptan-4-yl}methanesulfonamide

To a mixture of the compound synthesized in Example 53A (20 mg), water (0.3 mL), and tetrahydrofuran (1.0 mL), lithium hydroxide (10 mg) was added at room temperature and the mixture was stirred for 6 hours. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 63/37 to 43/57 (V/V)] to afford 12 mg (yield: 68%) of the marked compound as a single diastereomer and as a single enantiomer.

### Example 54

### N-{(3S,4S)-3-{[2-Fluoro(2',3',4',5',6'-²H₅)[biphenyl]-3-yl]methyl}-2-[(2R)-2-hydroxypropanoyl]-2-azabicyclo[3.1.1]heptan-4-yl}methanesulfonamide

### (54A) tert-Butyl (3S,4S)-3-{[2-Fluoro(2',3',4',5',6'-²H₅)[biphenyl]-3-yl]methyl}-4-[(methylsulfonyl)amino]-2-azabicyclo[3.1.1]heptane-2-carboxylate

To a mixture of the compound synthesized in Example 8G (26.3 mg), phenyl-d5-boronic acid (10.5 mg), potassium carbonate (22.9 mg), and 1,4-dioxane (0.37 mL), [1,1'-bis(diphenylphosphino)ferrocene]palladium(II) dichloride dichloromethane adduct (3.2 mg) and water (0.09 mL) were added at room temperature and the mixture was stirred at 60°C for 2.5 hours in a nitrogen atmosphere. The mixture was diluted with ethyl acetate, sequentially washed with water and a saturated saline solution, and dried over sodium sulfate. After filtration, the solvent was distilled off under reduced pressure, and the residue was purified by silica gel column chromatography [eluting solvent: n-hexane/ethyl acetate = 9/1 to 1/1 (V/V)] to afford 12.4 mg (yield: 47%) of the marked compound as a single diastereomer and as a single enantiomer.

### (54B) N-[(3S,4S)-3-{[2-Fluoro(2',3',4',5',6'-²H₅)[biphenyl]-3-yl]methyl}-2-azabicyclo[3.1.1]heptan-4-yl]methanesulfonamide hydrochloride

Using the compound synthesized in Example 54A (12.4 mg), 9.7 mg of the crude marked compound was obtained as a single diastereomer and as a single enantiomer by the same method as in Example 8H.

### (54C) (2R)-1-{(3S,4S)-3-{[2-Fluoro(2',3',4',5',6'-²H₅)[biphenyl]-3-yl]methyl}-4-[(methylsulfonyl)amino]-2-azabicyclo[3.1.1]heptan-2-yl}-1-oxopropan-2-yl acetate

To a mixture of the compound synthesized in Example 54B (9.7 mg), (R)-2-acetoxypropanoic acid (0.0039 mL), and N,N-dimethylformamide (0.23 mL), triethylamine (0.0097 mL) and O-(7-azabenzotriazol-1-yl)-N,N,N',N'-tetramethyluronium hexafluorophosphate (13 mg) were added at room temperature and the mixture was stirred for 1 hour. (R)-2-Acetoxypropanoic acid (0.0028 mL), O-(7-azabenzotriazol-1-yl)-N,N,N',N'-tetramethyluronium hexafluorophosphate (5.5 mg), and triethylamine (0.0097 mL) were added and the mixture was stirred for 1 hour. The mixture was diluted with ethyl acetate, sequentially washed with an aqueous sodium bicarbonate solution and a saturated saline solution, and dried over sodium sulfate. After filtration, the solvent was distilled off under reduced pressure to afford 12.0 mg of the crude marked compound as a single diastereomer and as a single enantiomer.

### (54D) N-{(3S,4S)-3-{[2-Fluoro(2',3',4',5',6'-²H₅)[biphenyl]-3-yl]methyl}-2-[(2R)-2-hydroxypropanoyl]-2-azabicyclo[3.1.1]heptan-4-yl}methanesulfonamide

Using the compound synthesized in Example 54C (12.0 mg), 8.5 mg (yield: 77%) of the marked compound was obtained as a single diastereomer and as a single enantiomer by the same method as in Example 12H.

### Example 55

### N-[(3S,4R)-5-Fluoro-3-[(2-fluoro[biphenyl]-3-yl)methyl]-2-(1,3-oxazol-4-ylcarbonyl)-2-azabicyclo[3.1.1]heptan-4-yl]methanesulfonamide

### (55A) N-[(3S,4R)-5-Fluoro-3-[(2-fluoro[biphenyl]-3-yl)methyl]-2-(1,3-oxazol-4-ylcarbonyl)-2-azabicyclo[3.1.1] heptan-4-yl]methanesulfonamide

Using the compound synthesized in Example 50B (12 mg) and oxazole-4-carboxylic acid (5.0 mg), 13 mg (yield: 89%) of the marked compound was obtained as a single diastereomer and as a single enantiomer by the same method as in Example 5H.

### Example 56

### N-{(3S,4S)-3-[(2-Fluoro[biphenyl]-3-yl)methyl]-2-propanoyl-2-azabicyclo[3.1.1]heptan-4-yl}methanesulfonamide

### (56A) N-{(3S,4S)-3-[(2-Fluoro[biphenyl]-3-yl)methyl]-2-propanoyl-2-azabicyclo[3.1.1]heptan-4-yl}methanesulfonamide

To a mixture of the compound synthesized in Example 25B (40 mg), N,N-diisopropylethylamine (38 mg), and tetrahydrofuran (1.0 mL), propionyl chloride (11 mg) was added at room temperature and the mixture was stirred for 30 minutes. The reaction solution was purified by reverse phase silica gel column chromatography [eluting solvent: 0.225% aqueous formic acid solution/acetonitrile = 58/42 to 38/62 (V/V)] to afford 19 mg (yield: 46%) of the marked compound as a single diastereomer and as a single enantiomer.

### Example 57

### N-[(3S,4S)-3-[(2-Fluoro[biphenyl]-3-yl)methyl]-2-(2-methylpropanoyl)-2-azabicyclo[3.1.1]heptan-4-yl]methanesulfonamide

### (57A) N-[(3S,4S)-3-[(2-Fluoro[biphenyl]-3-yl)methyl]-2-(2-methylpropanoyl)-2-azabicyclo[3.1.1]heptan-4-yl]methanesulfonamide

Using the compound synthesized in Example 25B (40 mg) and isobutyryl chloride (12 mg), 16 mg (yield: 38%) of the marked compound was obtained as a single diastereomer and as a single enantiomer by the same method as in Example 56A.

### Example 58

### N-{(3S,4S)-3-[(2,5-Difluoro[biphenyl]-3-yl)methyl]-2-[(2R)-2-hydroxypropanoyl]-2-azabicyclo[3.1.1]heptan-4-yl}methanesulfonamide

### (58A) 2-tert-Butyl 3-methyl 3-(3-bromo-2,5-difluorobenzyl)-4-oxo-2-azabicyclo[3.1.1]heptane-2,3-dicarboxylate

To a mixture of O4-tert-butyl O3-methyl 2-oxo-4-azabicyclo[3.1.1]heptane-3,4-dicarboxylate (CAS Registration No.: 2241048-56-4, 902 mg), 1-bromo-3-(bromomethyl)-2,5-difluorobenzene (CAS Registration No.: 1805523-99-2, 1.15 g), and N,N-dimethylformamide (11 mL), cesium carbonate (1.64 g) was added at room temperature and the mixture was stirred for 1 hour. Water was added, the mixture was extracted with ethyl acetate, and the organic layer was washed with a saturated saline solution and then dried over sodium sulfate. After filtration, the solvent was distilled off under reduced pressure, and purification by silica gel column chromatography [eluting solvent: n-hexane/ethyl acetate = 90/10 to 75/25 (V/V)] was performed to afford 1.52 g (yield: 96%) of the marked compound.

### (58B) 3-(3-Bromo-2,5-difluorobenzyl)-2-azabicyclo[3.1.1]heptan-4-one hydrochloride

A mixture of the compound synthesized in Example 58A (1.52 g), lithium chloride (136 mg), hydrochloric acid (12 M, 16 mL), and acetic acid (3.2 mL) was stirred at 100°C for 7 hours and then stirred at 120°C for 10 hours. The solvent was distilled off under reduced pressure to afford 1.13 g of the crude marked compound.

### (58C) tert-Butyl 3-(3-bromo-2,5-difluorobenzyl)-4-oxo-2-azabicyclo[3.1.1]heptane-2-carboxylate

To a mixture of the compound synthesized in Example 58B (1.13 g) and dichloromethane (11 mL), a solution of di-tert-butyl dicarbonate (1.40 g) in dichloromethane (11 mL) and triethylamine (1.33 mL) were added at room temperature and the mixture was stirred at room temperature for 17 hours. After adding an aqueous sodium bicarbonate solution, the mixture was extracted with dichloromethane, and the organic layer was washed with a saturated saline solution and then dried over sodium sulfate. After filtration, the solvent was distilled off under reduced pressure, and the residue was purified by silica gel column chromatography [eluting solvent: n-hexane/ethyl acetate = 95/5 to 75/25 (V/V)] to afford 698 mg (yield: 52%) of the marked compound.

### (58D) tert-Butyl 3-(3-bromo-2,5-difluorobenzyl)-4-(hydroxyimino)-2-azabicyclo[3.1.1]heptane-2-carboxylate

To a mixture of the compound synthesized in Example 58C (698 mg) and ethanol (5.6 mL), hydroxylamine hydrochloride (699 mg) and sodium acetate (825 mg) were added, and the mixture was stirred at room temperature for 1.5 hours and then stirred at 50°C for 1 hour. The solvent was distilled off under reduced pressure, and water and ethyl acetate were added. The organic layer was washed with a saturated saline solution, and then dried over sodium sulfate. After filtration, the solvent was distilled off under reduced pressure to afford 744 mg of the crude marked compound.

### (58E) tert-Butyl 4-amino-3-(3-bromo-2,5-difluorobenzyl)-2-azabicyclo[3.1.1]heptane-2-carboxylate

To a mixture of the compound synthesized in Example 58D (744 mg) and methanol (8.4 mL), molybdenum(VI) oxide (1.21 g) and sodium borohydride (1.27 g) were added at 0°C, and the mixture was then stirred at room temperature for 18 hours. After adding an aqueous sodium bicarbonate solution, the insoluble material was removed by Celite filtration, followed by washing with ethyl acetate. The organic layer was washed with a saturated saline solution, and then dried over sodium sulfate. After filtration, the solvent was distilled off under reduced pressure to afford 637 mg of the crude marked compound.

### (58F) tert-Butyl (3S*,4S*)-3-(3-bromo-2,5-difluorobenzyl)-4-[(methylsulfonyl)amino]-2-azabicyclo[3.1.1]heptane-2-carboxylate

To a mixture of the compound synthesized in Example 58E (320 mg) and tetrahydrofuran (10 mL), triethylamine (0.532 mL) and methanesulfonyl chloride (0.179 mL) were added under ice cooling and the mixture was stirred at room temperature for 3 hours. Water was added to the reaction mixture, and the reaction mixture was extracted with ethyl acetate. The organic layer was washed with a saturated saline solution, and dried over anhydrous sodium sulfate. After filtration, the solvent was distilled off under reduced pressure, and the residue was purified by silica gel column chromatography [eluting solvent: n-hexane/ethyl acetate = 100/0 to 50/50 (V/V)] to afford 344 mg (yield: 90%) of the marked compound as a single diastereomer (racemate).

### (58G) tert-Butyl (3S*,4S*)-3-[(2,5-difluoro[biphenyl]-3-yl)methyl]-4-[(methylsulfonyl)amino]-2-azabicyclo[3.1.1]heptane-2-carboxylate

To a mixture of the compound synthesized in Example 58F (344 mg), phenylboronic acid (127 mg), tripotassium phosphate (442 mg), and tetrahydrofuran (5.16 mL), (2-dicyclohexylphosphino-2',4',6'-triisopropyl-1,1'-biphenyl)[2-(2'-amino-1,1'-biphenyl)]palladium(II) methanesulfonate (29.4 mg) and water (1.72 mL) were added at room temperature and the mixture was stirred at 60°C for 2 hours in a nitrogen atmosphere. After cooling to room temperature, the mixture was diluted with ethyl acetate, sequentially washed with water and a saturated saline solution, and dried over sodium sulfate. After filtration, the solvent was distilled off under reduced pressure, and the residue was purified by silica gel column chromatography [eluting solvent: n-hexane/ethyl acetate = 50/50 to 0/100 (V/V)] to afford 358 mg (yield: quantitative) of the marked compound as a single diastereomer (racemate).

### (58H) N-{(3S*,4S*)-3-[(2,5-Difluoro[biphenyl]-3-yl)methyl]-2-azabicyclo[3.1.1]heptan-4-yl}methanesulfonamide hydrochloride

To a mixture of the compound synthesized in Example 58G (357 mg) and methanol (1.79 mL), a solution of hydrogen chloride in 1,4-dioxane (4 M, 1.79 mL) was added at room temperature and the mixture was stirred for 3 hours. The solvent was distilled off under reduced pressure, ethyl acetate was added, and the mixture was stirred for 1 hour. The precipitated solid was separated by filtration to afford 230 mg (yield: 74%) of the marked compound as a single diastereomer (racemate).

### (58I) (2R)-1-{(3S,4S)-3-[(2,5-Difluoro[biphenyl]-3-yl)methyl]-4-[(methylsulfonyl)amino]-2-azabicyclo[3.1.1]heptan-2-yl}-1-oxopropan-2-yl acetate

To a mixture of the compound synthesized in Example 58H (50.0 mg), (R)-2-acetoxypropanoic acid (0.0261 mL), and N,N-dimethylformamide (2.33 mL), triethylamine (0.0646 mL) and O-(7-azabenzotriazol-1-yl)-N,N,N',N'-tetramethyluronium hexafluorophosphate (88.6 mg) were added at room temperature and the mixture was stirred for 3 hours. After adding a saturated saline solution, the mixture was extracted with ethyl acetate, and the organic layer was dried over sodium sulfate. After filtration, the solvent was distilled off under reduced pressure, and the residue was purified by silica gel column chromatography [eluting solvent: n-hexane/ethyl acetate = 70/30 to 0/100 (V/V)] to afford 38.6 mg (yield: 65%) of the marked compound as a single diastereomer and as a single enantiomer.

### (58J) N-{(3S,4S)-3-[(2,5-Difluoro[biphenyl]-3-yl)methyl]-2-[(2R)-2-hydroxypropanoyl]-2-azabicyclo[3.1.1]heptan-4-yl}methanesulfonamide

To a mixture of the compound synthesized in Example 58I (38.4 mg), tetrahydrofuran (2.00 mL), methanol (1.00 mL), and water (1.00 mL), lithium hydroxide monohydrate (63.6 mg) was added at room temperature and the mixture was stirred at 50°C for 6 hours. After adding a saturated aqueous sodium bicarbonate solution, the mixture was extracted with ethyl acetate, and the organic layer was washed with a saturated saline solution and dried over sodium sulfate. After filtration, the solvent was distilled off under reduced pressure, and the residue was purified by silica gel column chromatography [eluting solvent: n-hexane/ethyl acetate = 50/50 to 0/100 (V/V)] to afford 18.4 mg (yield: 52%) of the marked compound as a single diastereomer and as a single enantiomer.

### Example 59

### N-[(3S,4S)-3-[(2,5-Difluoro[biphenyl]-3-yl)methyl]-2-(2-hydroxy-2-methylpropanoyl)-2-azabicyclo[3.1.1]heptan-4-yl]methanesulfonamide or N-[(3R,4R)-3-[(2,5-difluoro[biphenyl]-3-yl)methyl]-2-(2-hydroxy-2-methylpropanoyl)-2-azabicyclo[3.1.1]heptan-4-yl]methanesulfonamide

### (59A) 1-{(3S*,4S*)-3-[(2,5-Difluoro[biphenyl]-3-yl)methyl]-4-[(methylsulfonyl)amino]-2-azabicyclo[3.1.1]heptan-2-yl}-2-methyl-1-oxopropan-2-yl acetate

To a mixture of the compound synthesized in Example 58H (75.1 mg), triethylamine (0.121 mL), and tetrahydrofuran (1.13 mL), 2-acetoxyisobutyryl chloride (0.0761 mL) was added at room temperature and the mixture was stirred for 2 hours. After adding a saturated saline solution, the mixture was extracted with ethyl acetate, and the organic layer was dried over sodium sulfate. After filtration, the solvent was distilled off under reduced pressure, and the residue was purified by silica gel column chromatography [eluting solvent: n-hexane/ethyl acetate = 70/30 to 0/100 (V/V)] to afford 90.0 mg (yield: 99%) of the marked compound as a single diastereomer (racemate).

### (59B) N-[(3S*,4S*)-3-[(2,5-Difluoro[biphenyl]-3-yl)methyl]-2-(2-hydroxy-2-methylpropanoyl)-2-azabicyclo[3.1.1]heptan-4-yl]methanesulfonamide

Using the compound synthesized in Example 59A (90.0 mg), 70.1 mg (yield: 85%) of the marked compound was obtained as a single diastereomer (racemate) by the same method as in Example 12H.

### (59C) N-[(3S,4S)-3-[(2,5-Difluoro[biphenyl]-3-yl)methyl]-2-(2-hydroxy-2-methylpropanoyl)-2-azabicyclo[3.1.1]heptan-4-yl]methanesulfonamide or N-[(3R,4R)-3-[(2,5-difluoro[biphenyl]-3-yl)methyl]-2-(2-hydroxy-2-methylpropanoyl)-2-azabicyclo[3.1.1]heptan-4-yl]methanesulfonamide

The compound synthesized in Example 59B (70.1 mg) was subjected to chiral HPLC [column: CHIRALPAK (R) IG (20 mm I.D. × 250 mm), mobile phase: n-hexane/2-propanol = 70/30 (V/V), temperature: 40°C] to afford 32.4 mg (yield: 46%) of the marked compound, which was eluted first, as a single diastereomer and as a single enantiomer.

### Example 60

### N-[3-{[2-(3-Chlorophenyl)-3-fluoropyridin-4-yl]methyl}-2-(cyclopropylcarbonyl)-2-azabicyclo[3.1.1]heptan-4-yl]methanesulfonamide

### (60A) 2-tert-Butyl 3-methyl 3-[(2-bromo-3-fluoropyridin-4-yl)methyl]-4-oxo-2-azabicyclo[3.1.1]heptane-2,3-dicarboxylate

Using 2-bromo-4-(bromomethyl)-3-fluoropyridine (CAS Registration No.: 1227585-59-2, 1.00 g) and O4-tert-butyl O3-methyl 2-oxo-4-azabicyclo[3.1.1]heptane-3,4-dicarboxylate (CAS Registration No.: 2241048-56-4, 1.00 g), 1.46 g (yield: 84%) of the marked compound was obtained by the same method as in Example 1B.

### (60B) tert-Butyl 3-[(2-bromo-3-fluoropyridin-4-yl)methyl]-4-oxo-2-azabicyclo[3.1.1]heptane-2-carboxylate

To a mixture of the compound synthesized in Example 60A (1.48 g) and dimethyl sulfoxide (10 mL), lithium chloride (528 mg) was added, and the mixture was stirred at 130°C for 3 hours and cooled to room temperature. Water was added to the reaction mixture, and the reaction mixture was extracted with ethyl acetate. The organic layer was washed with a saturated saline solution, and dried over sodium sulfate. After filtration, the residue obtained by distilling off the solvent under reduced pressure was purified by silica gel column chromatography [eluting solvent: n-hexane/ethyl acetate = 100/0 to 60/40 (V/V)] to afford 815 mg (yield: 66%) of the marked compound.

### (60C) tert-Butyl 3-[(2-bromo-3-fluoropyridin-4-yl)methyl]-4-(hydroxyimino)-2-azabicyclo[3.1.1]heptane-2-carboxylate

Using the compound synthesized in Example 60B (815 mg), the crude marked compound was obtained by the same method as in Example 2A.

### (60D) tert-Butyl 4-amino-3-[(2-bromo-3-fluoropyridin-4-yl)methyl]-2-azabicyclo[3.1.1]heptane-2-carboxylate

To a mixture of the compound synthesized in Example 60C (846 mg) and methanol (10 mL), molybdenum(VI) oxide (441 mg) and sodium borohydride (541 mg) were added at 0°C and the mixture was stirred at 0°C for 1 hour. The temperature was raised to room temperature and the mixture was stirred for 1 hour. Sodium borohydride (541 mg) and molybdenum(VI) oxide (441 mg) were added at 0°C and the mixture was stirred at 0°C for 1 hour. Sodium borohydride (541 mg) and molybdenum(VI) oxide (441 mg) were added and the mixture was further stirred at 0°C for 1 hour. A saturated aqueous sodium bicarbonate solution was added and the mixture was stirred at room temperature for 16 hours. Celite filtration was performed, followed by washing with ethyl acetate. The filtrate was extracted with ethyl acetate, and the organic layer was washed with a saturated saline solution and then dried over anhydrous sodium sulfate. After filtration, the solvent was distilled off under reduced pressure to afford the crude marked compound.

### (60E) tert-Butyl 3-[(2-bromo-3-fluoropyridin-4-yl)methyl]-4-[(methylsulfonyl)amino]-2-azabicyclo[3.1.1]heptane-2-carboxylate

Using the compound synthesized in Example 60D (817 mg) and methanesulfonyl chloride (468 mg), 518 mg (yield: 53% in 3 steps) of the marked compound was obtained as a single diastereomer (racemate) by the same method as in Example 5F.

### (60F) tert-Butyl 3-{[2-(3-chlorophenyl)-3-fluoropyridin-4-yl]methyl}-4-[(methylsulfonyl)amino]-2-azabicyclo[3.1.1]heptane-2-carboxylate

To a mixture of the compound synthesized in Example 60E (518 mg), 1,2-dimethoxyethane (8 mL), and water (4 mL), 3-chlorophenylboronic acid (254 mg), sodium carbonate (861 mg), and tetrakis(triphenylphosphine)palladium(0) (63 mg) were added and the mixture was stirred at 120°C for 90 minutes. Water was added to the reaction mixture, and the reaction mixture was extracted with ethyl acetate. The organic layer was washed with a saturated saline solution, and dried over anhydrous sodium sulfate. After filtration, the residue obtained by distilling off the solvent under reduced pressure was purified by silica gel column chromatography [eluting solvent: n-hexane/ethyl acetate = 70/30 to 20/80 (V/V)] to afford 491 mg (yield: 89%) of the marked compound as a single diastereomer (racemate).

### (60G) tert-Butyl 3-{[2-(3-chlorophenyl)-3-fluoropyridin-4-yl]methyl}-4-[(methylsulfonyl)amino]-2-azabicyclo[3.1.1]heptane-2-carboxylate

The compound synthesized in Example 60F (550 mg) was subjected to chiral HPLC [column: CHIRALPAK (R) IA (20 mm I.D. × 250 mm), mobile phase: n-hexane/tetrahydrofuran = 70/30 (V/V), temperature: 40°C] to afford 217 mg (yield: 39%) of the marked compound, which was eluted first, as a single diastereomer and as a single enantiomer.

### (60H) N-[3-{[2-(3-Chlorophenyl)-3-fluoropyridin-4-yl]methyl}-2-azabicyclo[3.1.1]heptan-4-yl]methanesulfonamide hydrochloride

Using the compound synthesized in Example 60G (217 mg), 227 mg of the crude marked compound was obtained as a single diastereomer and as a single enantiomer by the same method as in Example 8H.

### (60I) N-[3-{[2-(3-Chlorophenyl)-3-fluoropyridin-4-yl]methyl}-2-(cyclopropylcarbonyl)-2-azabicyclo[3.1.1]heptan-4-yl]methanesulfonamide

To a mixture of the compound synthesized in Example 60H (35.0 mg) and tetrahydrofuran (2 mL), N,N-diisopropylethylamine (0.062 mL) and cyclopropanecarbonyl chloride (0.013 mL) were added and the mixture was stirred at room temperature for 2 hours. Water was added to the reaction mixture, and the reaction mixture was extracted with ethyl acetate. The organic layer was washed with a saturated saline solution, and dried over anhydrous sodium sulfate. After filtration, the residue obtained by distilling off the solvent under reduced pressure was purified by silica gel column chromatography [eluting solvent: n-hexane/ethyl acetate = 40/60 to 0/100 (V/V)]. Through the addition of diethyl ether, solidification by ultrasonication, and separation by filtration, 34 mg (yield: 98%) of the marked compound was obtained as a single diastereomer and as a single enantiomer.

### Example 61

### N-[(3S,4R)-3-[(3'-Chloro-2-fluoro[biphenyl]-3-yl)methyl]-5-fluoro-2-(2-hydroxy-2-methylpropanoyl)-2-azabicyclo[3.1.1]heptan-4-yl]methanesulfonamide or N-[(3R,4S)-3-[(3'-chloro-2-fluoro[biphenyl]-3-yl)methyl]-5-fluoro-2-(2-hydroxy-2-methylpropanoyl)-2-azabicyclo[3.1.1]heptan-4-yl]methanesulfonamide

### (61A) tert-Butyl (3S*,4R*)-3-[(3'-chloro-2-fluoro[biphenyl]-3-yl)methyl]-5-fluoro-4-[(methylsulfonyl)amino]-2-azabicyclo[3.1.1]heptane-2-carboxylate

A mixture of the compound synthesized in Example 23E (115 mg), 3-chlorophenylboronic acid (54.5 mg), tetrakis(triphenylphosphine)palladium(0) (20.1 mg), potassium carbonate (96.3 mg), 1,4-dioxane (3.2 mL), and water (0.8 mL) was stirred at 100°C for 4 hours. After adding water, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 90/10 to 40/60 (V/V)] to afford 137 mg (yield: quantitative) of the marked compound as a single diastereomer (racemate).

### (61B) N-{(3S*,4R*)-3-[(3'-Chloro-2-fluoro[biphenyl]-3-yl)methyl]-5-fluoro-2-azabicyclo[3.1.1]heptan-4-yl}methanesulfonamide hydrochloride

Using the compound synthesized in Example 61A (137 mg), 104 mg (yield: 97%) of the crude marked compound was obtained as a single diastereomer (racemate) by the same method as in Example 8H.

### (61C) 1-{(3S*,4R*)-3-[(3'-Chloro-2-fluoro[biphenyl]-3-yl)methyl]-5-fluoro-4-[(methylsulfonyl)amino]-2-azabicyclo[3.1.1]heptan-2-yl}-2-methyl-1-oxopropan-2-yl acetate

To a mixture of the compound synthesized in Example 61B (54.0 mg), triethylamine (0.0808 mL), and tetrahydrofuran (2 mL), 2-acetoxyisobutyryl chloride (0.0338 mL) was added at room temperature and the mixture was stirred overnight. The mixture was diluted with ethyl acetate, sequentially washed with water and a saturated saline solution, and dried over sodium sulfate. After filtration, the filtrate was concentrated under reduced pressure, and the residue was purified by silica gel column chromatography [eluting solvent: n-hexane/ethyl acetate = 60/40 to 20/80 (V/V)] to afford 65.1 mg (yield: quantitative) of the marked compound as a single diastereomer (racemate).

### (61D) N-[(3S*,4R*)-3-[(3'-Chloro-2-fluoro[biphenyl]-3-yl)methyl]-5-fluoro-2-(2-hydroxy-2-methylpropanoyl)-2-azabicyclo[3.1.1]heptan-4-yl]methanesulfonamide

Using the compound synthesized in Example 61C (65.1 mg), 56.8 mg (yield: 95%) of the marked compound was obtained as a single diastereomer (racemate) by the same method as in Example 12H.

### (61E) N-[(3S,4R)-3-[(3'-Chloro-2-fluoro[biphenyl]-3-yl)methyl]-5-fluoro-2-(2-hydroxy-2-methylpropanoyl)-2-azabicyclo[3.1.1]heptan-4-yl]methanesulfonamide or N-[(3R,4S)-3-[(3'-chloro-2-fluoro[biphenyl]-3-yl)methyl]-5-fluoro-2-(2-hydroxy-2-methylpropanoyl)-2-azabicyclo[3.1.1]heptan-4-yl]methanesulfonamide

The compound synthesized in Example 61D (56.8 mg) was purified by chiral HPLC [column: CHIRALPAK (R) IA (20 mm I.D. × 250 mm), mobile phase: n-hexane/ethanol = 60/40 (V/V), temperature: 40°C] to afford 26.9 mg (yield: 47%) of the marked compound, which was eluted first, as a single diastereomer and as a single enantiomer.

### Example 62

### N-{(3S,4S)-3-[(2-Fluoro[biphenyl]-3-yl)methyl]-2-[(1-hydroxycyclobutyl)carbonyl]-2-azabicyclo[3.1.1]heptan-4-yl}methanesulfonamide

### (62A) N-{(3S,4S)-3-[(2-Fluoro[biphenyl]-3-yl)methyl]-2-[(1-hydroxycyclobutyl)carbonyl]-2-azabicyclo[3.1.1]heptan-4-yl}methanesulfonamide

Using the compound synthesized in Example 25B (50 mg) and 1-hydroxycyclobutanecarboxylic acid (17 mg), 18 mg (yield: 31%) of the marked compound was obtained as a single diastereomer and as a single enantiomer by the same method as in Example 31C.

### Example 63

### N-{(3S,4R)-3-[(2,5-Difluoro[biphenyl]-3-yl)methyl]-5-fluoro-2-[(2R)-2-hydroxypropanoyl]-2-azabicyclo[3.1.1]heptan-4-yl}methanesulfonamide

### (63A) 2-tert-Butyl 3-methyl 3-(3-bromo-2,5-difluorobenzyl)-5-fluoro-4-oxo-2-azabicyclo[3.1.1]heptane-2,3-dicarboxylate

To a mixture of the compound synthesized in Example 12C (500 mg), 1-bromo-3-(bromomethyl)-2,5-difluorobenzene (CAS Registration No.: 1805523-99-2, 597 mg), and N,N-dimethylformamide (8.7 mL), cesium carbonate (850 mg) was added at 0°C, and the mixture was then stirred at room temperature for 3.5 hours. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: n-hexane/ethyl acetate = 95/5 to 70/30 (V/V)] to afford 399 mg (yield: 47%) of the marked compound.

### (63B) tert-Butyl 3-(3-bromo-2,5-difluorobenzyl)-5-fluoro-4-oxo-2-azabicyclo[3.1.1]heptane-2-carboxylate

To a mixture of the compound synthesized in Example 63A (275 mg) and dimethyl sulfoxide (7.0 mL), lithium chloride (80 mg) was added at room temperature, and the mixture was then stirred at 130°C for 2 hours. The mixture was diluted with ethyl acetate and washed with water and a saturated saline solution. The organic layer was dried over sodium sulfate and filtered, and the solvent was then distilled off under reduced pressure. The residue was purified by silica gel column chromatography [eluting solvent: n-hexane/ethyl acetate = 95/5 to 70/30 (V/V)] to afford 160 mg (yield: 66%) of the marked compound.

### (63C) tert-Butyl 3-(3-bromo-2,5-difluorobenzyl)-5-fluoro-4-(hydroxyimino)-2-azabicyclo[3.1.1]heptane-2-carboxylate

To a mixture of the compound synthesized in Example 63B (189 mg), hydroxylamine hydrochloride (152 mg), and ethanol (3.6 mL), sodium acetate (180 mg) was added at room temperature, and the mixture was then stirred at 90°C for 3 hours. About 2 mL of the solvent was distilled off under reduced pressure, followed by dilution with ethyl acetate. The organic layer was washed with water and a saturated saline solution, and dried over sodium sulfate. After filtration, the solvent was distilled off under reduced pressure, and the residue was purified by silica gel column chromatography [eluting solvent: n-hexane/ethyl acetate = 90/10 to 50/50 (V/V)] to afford 123 mg (yield: 63%) of the marked compound.

### (63D) tert-Butyl 4-amino-3-(3-bromo-2,5-difluorobenzyl)-5-fluoro-2-azabicyclo[3.1.1]heptane-2-carboxylate

To a mixture of the compound synthesized in Example 63C (1.23 g), molybdenum(VI) oxide (1.18 g), and methanol (45 mL), a solution of lithium borohydride in tetrahydrofuran (2.0 M, 16 mL) was added at room temperature over 30 minutes, and the mixture was then stirred for 1 hour. A saturated aqueous sodium bicarbonate solution was added to the reaction solution, the mixture was stirred for 30 minutes, and the insoluble material was then removed by filtration, followed by washing with ethyl acetate. The organic layer was washed with a saturated saline solution, and dried over sodium sulfate. After filtration, the solvent was distilled off under reduced pressure to afford 1.19 g of the crude marked compound.

### (63E) tert-Butyl (3S*,4R*)-3-(3-bromo-2,5-difluorobenzyl)-5-fluoro-4-[(methylsulfonyl)amino]-2-azabicyclo[3.1.1]heptane-2-carboxylate

To a mixture of the compound synthesized in Example 63D (1.19 g), triethylamine (1.90 mL), and tetrahydrofuran (35 mL), methanesulfonyl chloride (1.1 g) was added at 0°C, and the mixture was then stirred at room temperature for 3 hours. Water was added, the mixture was extracted with ethyl acetate, and the organic layer was washed with a saturated saline solution and then dried over sodium sulfate. After filtration, the solvent was distilled off under reduced pressure, and the residue was purified by silica gel column chromatography [eluting solvent: n-hexane/ethyl acetate = 100/0 to 30/70 (V/V)] to afford 400 mg (yield: 28%) of the marked compound as a single diastereomer (racemate).

### (63F) tert-Butyl (3S*,4R*)-3-[(2,5-difluoro[biphenyl]-3-yl)methyl]-5-fluoro-4-[(methylsulfonyl)amino]-2-azabicyclo[3.1.1]heptane-2-carboxylate

A mixture of the compound synthesized in Example 63E (84 mg), phenylboronic acid (30 mg), tripotassium phosphate (104 mg), chloro(2-dicyclohexylphosphino-2',4',6'-triisopropyl-1,1'-biphenyl)[2-(2'-amino-1,1'-biphenyl)]palladium(II) (9.6 mg), tetrahydrofuran (1.6 mL), and water (0.5 mL) was stirred at 70°C for 2 hours in a nitrogen atmosphere. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: n-hexane/ethyl acetate = 70/30 to 50/50 (V/V)] to afford 77 mg (yield: 93%) of the marked compound as a single diastereomer (racemate).

### (63G) N-{(3S*,4R*)-3-[(2,5-Difluoro[biphenyl]-3-yl)methyl]-5-fluoro-2-azabicyclo[3.1.1]heptan-4-yl}methanesulfonamide hydrochloride

To the compound synthesized in Example 63F (77 mg), a solution of hydrogen chloride in 1,4-dioxane (4 M, 2.0 mL) was added and the mixture was stirred at room temperature for 3 hours. The solvent was distilled off under reduced pressure to afford 68 mg of the crude marked compound as a single diastereomer (racemate).

### (63H) (2R)-1-{(3S*,4R*)-3-[(2,5-Difluoro[biphenyl]-3-yl)methyl]-5-fluoro-4-[(methylsulfonyl)amino]-2-azabicyclo[3.1.1]heptan-2-yl}-1-oxopropan-2-yl acetate

To a mixture of the compound synthesized in Example 63G (68 mg), (R)-2-acetoxypropanoic acid (0.027 mL), and N,N-dimethylformamide (1.5 mL), N,N-diisopropylethylamine (0.085 mL) and O-(7-azabenzotriazol-1-yl)-N,N,N',N'-tetramethyluronium hexafluorophosphate (93 mg) were added at room temperature and the mixture was stirred at room temperature for 2 hours. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: n-hexane/ethyl acetate = 60/40 to 40/60 (V/V)] to afford 97 mg of the marked compound.

### (63I) N-{(3S*,4R*)-3-[(2,5-Difluoro[biphenyl]-3-yl)methyl]-5-fluoro-2-[(2R)-2-hydroxypropanoyl]-2-azabicyclo[3.1.1]heptan-4-yl}methanesulfonamide

To a mixture of the compound synthesized in Example 63H (97 mg), tetrahydrofuran (2.6 mL), methanol (1.0 mL), and water (1.0 mL), lithium hydroxide monohydrate (23 mg) was added at room temperature and the mixture was stirred at room temperature for 2 hours. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: n-hexane/ethyl acetate = 50/50 to 30/70 (V/V)] to afford 78 mg (yield: 87%) of the marked compound.

### (63J) N-{(3S,4R)-3-[(2,5-Difluoro[biphenyl]-3-yl)methyl]-5-fluoro-2-[(2R)-2-hydroxypropanoyl]-2-azabicyclo[3.1.1]heptan-4-yl}methanesulfonamide

The compound synthesized in Example 63I (78 mg) was subjected to chiral HPLC [column: CHIRAL ART Amylose-SA (4.6 mm I.D. × 250 mm), mobile phase: n-hexane/ethanol = 70/30 (V/V), temperature: 40°C] to afford 31 mg (yield: 40%) of the marked compound, which was eluted first, as a single diastereomer and as a single enantiomer.

### Example 64

### N-{(3S,4S)-2-[(2R)-2-Cyclopropyl-2-hydroxyacetyl]-3-[(2-fluoro[biphenyl]-3-yl)methyl]-2-azabicyclo[3.1.1]heptan-4-yl}methanesulfonamide

### (64A) N-{(3S,4S)-2-[(2R)-2-Cyclopropyl-2-hydroxyacetyl]-3-[(2-fluoro[biphenyl]-3-yl)methyl]-2-azabicyclo[3.1.1]heptan-4-yl}methanesulfonamide

Using the compound synthesized in Example 25B (50 mg) and (R)-2-hydroxy-2-cyclopropylacetic acid (14 mg), 22 mg (yield: 38%) of the marked compound was obtained as a single diastereomer and as a single enantiomer by the same method as in Example 31C.

### Example 65

### Ethyl (3S,4S)-3-[(2-fluoro[biphenyl]-3-yl)methyl]-4-[(methylsulfonyl)amino]-2-azabicyclo[3.1.1]heptane-2-carboxylate

### (65A) Ethyl (3S,4S)-3-[(2-fluoro[biphenyl]-3-yl)methyl]-4-[(methylsulfonyl)amino]-2-azabicyclo[3.1.1]heptane-2-carboxylate

To a mixture of the compound synthesized in Example 25B (30 mg), N,N-diisopropylethylamine (50 mg), and tetrahydrofuran (1.0 mL), ethyl chloroformate (14 mg) was added at room temperature and the mixture was stirred for 2 hours. The reaction solution was poured into water and extracted with ethyl acetate. The organic layer was washed with water and a saturated saline solution, and dried over sodium sulfate. After filtration, the solvent was distilled off under reduced pressure, and the residue was purified by reverse phase silica gel column chromatography [eluting solvent: 0.225% aqueous formic acid solution/acetonitrile = 50/50 to 30/70 (V/V)] to afford 16 mg (yield: 46%) of the marked compound as a single diastereomer and as a single enantiomer.

### Example 66

### N-[(3S,4R)-5-Fluoro-3-[(2-fluoro[biphenyl]-3-yl)methyl]-2-{[(1S,2S)-2-fluorocyclopropyl]carbonyl}-2-azabicyclo[3.1.1]heptan-4-yl]methanesulfonamide

### (66A) tert-Butyl (3S*,4R*)-5-fluoro-3-[(2-fluoro[biphenyl]-3-yl)methyl]-4-[(methylsulfonyl)amino]-2-azabicyclo[3.1.1]heptane-2-carboxylate

A mixture of the compound synthesized in Example 23E (1.56 g), phenylboronic acid (576 mg), (2-dicyclohexylphosphino-2',4',6'-triisopropyl-1,1'-biphenyl)[2-(2'-amino-1,1'-biphenyl)]palladium(II) methanesulfonate (200 mg), potassium carbonate (2.01 g), tetrahydrofuran (30 mL), and water (10 mL) was stirred at 65°C for 4 hours. After adding water, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 80/20 to 40/60 (V/V)] to afford 1.59 g (yield: quantitative) of the marked compound as a single diastereomer (racemate).

### (66B) tert-Butyl (3S,4R)-5-fluoro-3-[(2-fluoro[biphenyl]-3-yl)methyl]-4-[(methylsulfonyl)amino]-2-azabicyclo[3.1.1]heptane-2-carboxylate

The compound synthesized in Example 66A (1.59 g) was subjected to chiral HPLC [column: CHIRALPAK (R) IC (20 mm I.D. × 250 mm), mobile phase: n-hexane/2-propanol = 55/45 (V/V), temperature: 40°C] to afford 679 mg (yield: 43%) of the marked compound, which was eluted later, as a single diastereomer and as a single enantiomer.

### (66C) N-{(3S,4R)-5-Fluoro-3-[(2-fluoro[biphenyl]-3-yl)methyl]-2-azabicyclo[3.1.1] heptan-4-yl}methanesulfonamide hydrochloride

To a mixture of the compound synthesized in Example 66B (626 mg) and 1,4-dioxane (5 mL), a solution of hydrogen chloride in 1,4-dioxane (4 M, 30 mL) was added at room temperature and the mixture was stirred for 3 hours. After diluting the reaction mixture with toluene, the solution was concentrated under reduced pressure to afford 518 mg of the crude marked compound as a single diastereomer and as a single enantiomer.

### (66D) N-[(3S,4R)-5-Fluoro-3-[(2-fluoro[biphenyl]-3-yl)methyl]-2-{[(1S,2S)-2-fluorocyclopropyl]carbonyl}-2-azabicyclo[3.1.1]heptan-4-yl]methanesulfonamide

To a mixture of the compound synthesized in Example 66C (30.0 mg), (1S,2S)-2-fluorocyclopropane-1-carboxylic acid (10.9 mg), 1-hydroxy-7-azabenzotriazole (9.5 mg), triethylamine (0.0388 mL), and N,N-dimethylformamide (1 mL), 1-(3-dimethylaminopropyl)-3-ethylcarbodiimide hydrochloride (20.1 mg) was added at room temperature, and the mixture was then stirred at 35°C for 2 hours. The mixture was diluted with ethyl acetate, sequentially washed with water, hydrochloric acid (0.5 M), a saturated aqueous sodium bicarbonate solution, and a saturated saline solution, and dried over sodium sulfate. After filtration, the filtrate was concentrated under reduced pressure, and the residue was purified by silica gel column chromatography [eluting solvent: n-hexane/ethyl acetate = 70/30 to 10/90 (V/V)] to afford 32.0 mg (yield: 96%) of the marked compound as a single diastereomer and as a single enantiomer.

### Example 67

### N-[(3S,4S)-3-[(3'-Chloro-2,5-difluoro[biphenyl]-3-yl)methyl]-2-(2-hydroxy-2-methylpropanoyl)-2-azabicyclo[3.1.1]heptan-4-yl]methanesulfonamide or N-[(3R,4R)-3-[(3'-chloro-2,5-difluoro[biphenyl]-3-yl)methyl]-2-(2-hydroxy-2-methylpropanoyl)-2-azabicyclo[3.1.1]heptan-4-yl]methanesulfonamide

### (67A) tert-Butyl (3S*,4S*)-3-[(3'-chloro-2,5'-difluoro[biphenyl]-3-yl)methyl]-4-[(methylsulfonyl)amino]-2-azabicyclo[3.1.1]heptane-2-carboxylate

To a mixture of the compound synthesized in Example 58F (250 mg) and 1,4-dioxane (4.0 mL), 3-chlorophenylboronic acid (120 mg), [1,1'-bis(diphenylphosphino)ferrocene]palladium(II) dichloride (20 mg), cesium carbonate (340 mg), and water (1.0 mL) were added, and the mixture was degassed and then stirred at 100°C for 2 hours in a nitrogen atmosphere. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: petroleum ether/ethyl acetate = 100/0 to 70/30 (V/V)] to afford 200 mg (yield: 75%) of the marked compound as a single diastereomer (racemate).

### (67B) N-{(3S*,4S*)-3-[(3'-Chloro-2,5-difluoro[biphenyl]-3-yl)methyl]-2-azabicyclo[3.1.1] heptan-4-yl}methanesulfonamide hydrochloride

To a mixture of the compound synthesized in Example 67A (200 mg) and ethyl acetate (1.0 mL), a solution of hydrogen chloride in ethyl acetate (4 M, 4.0 mL) was added and the mixture was stirred at room temperature for 30 minutes. The solvent was distilled off under reduced pressure to afford 160 mg of the crude marked compound as a single diastereomer (racemate).

### (67C) 1-{(3S*,4S*)-3-[(3'-Chloro-2,5-difluoro[biphenyl]-3-yl)methyl]-4-[(methylsulfonyl)amino]-2-azabicyclo[3.1.1]heptan-2-yl}-2-methyl-1-oxopropan-2-yl acetate

To a mixture of the compound synthesized in Example 67B (80 mg), 2-acetoxyisobutyryl chloride (44 mg), and tetrahydrofuran (5.0 mL), N,N-diisopropylethylamine (208 mg) was added at room temperature, and the mixture was then stirred for 2 hours. The mixture was diluted with ethyl acetate, then washed with water and a saturated saline solution, and dried over sodium sulfate. After filtration, the solvent was distilled off under reduced pressure to afford 100 mg of the crude marked compound as a single diastereomer (racemate).

### (67D) N-[(3S*,4S*)-3-[(3'-Chloro-2,5-difluoro[biphenyl]-3-yl)methyl]-2-(2-hydroxy-2-methylpropanoyl)-2-azabicyclo[3.1.1]heptan-4-yl]methanesulfonamide

To a mixture of the compound synthesized in Example 67C (100 mg), water (0.6 mL), and tetrahydrofuran (2.0 mL), lithium hydroxide (22 mg) was added and the mixture was stirred at 45°C for 12 hours. The mixture was diluted with ethyl acetate and washed with water and a saturated saline solution. The mixture was dried over sodium sulfate and filtered. The solvent was distilled off under reduced pressure, and the residue was purified by reverse phase silica gel column chromatography [eluting solvent: 0.225% aqueous formic acid solution/acetonitrile = 55/45 to 25/75 (V/V)] to afford the marked compound as a single diastereomer (racemate).

### (67E) N-[(3S,4S)-3-[(3'-Chloro-2,5-difluoro[biphenyl]-3-yl)methyl]-2-(2-hydroxy-2-methylpropanoyl)-2-azabicyclo[3.1.1]heptan-4-yl]methanesulfonamide or N-[(3R,4R)-3-[(3'-chloro-2,5-difluoro[biphenyl]-3-yl)methyl]-2-(2-hydroxy-2-methylpropanoyl)-2-azabicyclo[3.1.1]heptan-4-yl]methanesulfonamide

The entire amount of the compound synthesized in Example 67D was subjected to chiral SFC [column: CHIRALCEL OJ (30 mm I.D. × 250 mm), mobile phase: carbon dioxide/methanol = 40/60 (V/V)] to afford 32 mg (yield: 35%) of the marked compound, which was eluted later, as a single diastereomer and as a single enantiomer.

### Example 68

### N-{(3S,4S)-3-[3-(5-Chlorothiophen-3-yl)-2-fluorobenzyl]-2-[(2R)-2-hydroxypropanoyl]-2-azabicyclo[3.1.1]heptan-4-yl}methanesulfonamide

### (68A) tert-Butyl (3S,4S)-3-[3-(5-chlorothiophen-3-yl)-2-fluorobenzyl]-4-[(methylsulfonyl)amino]-2-azabicyclo[3.1.1]heptane-2-carboxylate

A mixture of the compound synthesized in Example 21A (100 mg), cesium carbonate (155 mg), 4-bromo-2-chlorothiophene (40 mg), (2-dicyclohexylphosphino-2',4',6'-triisopropyl-1,1'-biphenyl)[2-(2'-amino-1,1'-biphenyl)]palladium(II) methanesulfonate (16 mg), water (1.0 mL), and 1,4-dioxane (6.0 mL) was stirred at 80°C for 10 hours in a nitrogen atmosphere. The mixture was diluted with ethyl acetate and washed with water and a saturated saline solution. The organic layer was dried over sodium sulfate and filtered, and the solvent was then distilled off under reduced pressure. The residue was purified by preparative thin layer silica gel chromatography [eluting solvent: petroleum ether/ethyl acetate = 1/1 (V/V)] to afford 80 mg (yield: 81%) of the marked compound as a single diastereomer and as a single enantiomer.

### (68B) N-{(3S,4S)-3-[3-(5-Chlorothiophen-3-yl)-2-fluorobenzyl]-2-azabicyclo[3.1.1] heptan-4-yl}methanesulfonamide hydrochloride

Using the compound synthesized in Example 68A (80 mg), 80 mg of the crude marked compound was obtained as a single diastereomer and as a single enantiomer by the same method as in Example 5G.

### (68C) (2R)-1-{(3S,4S)-3-[3-(5-Chlorothiophen-3-yl)-2-fluorobenzyl]-4-[(methylsulfonyl)amino]-2-azabicyclo[3.1.1]heptan-2-yl}-1-oxopropan-2-yl acetate

Using the compound synthesized in Example 68B (80 mg) and (R)-2-acetoxypropionic acid chloride (40 mg), 100 mg of the crude marked compound was obtained as a single diastereomer and as a single enantiomer by the same method as in Example 34A.

### (68D) N-{(3S,4S)-3-[3-(5-Chlorothiophen-3-yl)-2-fluorobenzyl]-2-[(2R)-2-hydroxypropanoyl]-2-azabicyclo[3.1.1]heptan-4-yl}methanesulfonamide

To a mixture of the compound synthesized in Example 68C (100 mg), water (1.0 mL), and tetrahydrofuran (5.0 mL), lithium hydroxide monohydrate (24 mg) was added at room temperature and the mixture was stirred for 10 hours. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 60/40 to 30/70 (V/V)] to afford 16 mg (yield: 17%) of the marked compound as a single diastereomer and as a single enantiomer.

### Example 69

### N-{(3S,4R)-5-Fluoro-3-[(2-fluoro[biphenyl]-3-yl)methyl]-2-[(2S)-3,3,3-trifluoro-2-hydroxypropanoyl]-2-azabicyclo[3.1.1]heptan-4-yl}methanesulfonamide

### (69A) N-{(3S,4R)-5-Fluoro-3-[(2-fluoro[biphenyl]-3-yl)methyl]-2-[(2S)-3,3,3-trifluoro-2-hydroxypropanoyl]-2-azabicyclo[3.1.1]heptan-4-yl}methanesulfonamide

To a mixture of (S)-3,3,3-trifluoro-2-hydroxypropionic acid (53.7 mg) and dichloromethane (1 mL), 1-chloro-N,N,2-trimethyl-1-propenylamine (0.0617 mL) was added at 0°C, and the mixture was then stirred at room temperature for 1 hour with ultrasonication. This reaction mixture was added to a mixture of the separately prepared compound synthesized in Example 66C (40.0 mg), N,N-diisopropylethylamine (0.111 mL), and dichloromethane (1 mL) at 0°C, and the mixture was then stirred at room temperature for 2 hours. The mixture was diluted with ethyl acetate, sequentially washed with water, hydrochloric acid (1 M), a saturated aqueous sodium bicarbonate solution, and a saturated saline solution, and dried over sodium sulfate. After filtration, the filtrate was concentrated under reduced pressure, and the residue was sequentially purified by silica gel column chromatography [eluting solvent: n-hexane/ethyl acetate = 70/30 to 20/80 (V/V)] and preparative thin layer silica gel chromatography [eluting solvent: n-hexane/ethyl acetate = 40/60 (V/V)] to afford 15.5 mg (yield: 32%) of the marked compound as a single diastereomer and as a single enantiomer.

### Example 70

### N-{(3S,4R)-5-Fluoro-3-[(2-fluoro[biphenyl]-3-yl)methyl]-2-[(2R)-oxetan-2-ylcarbonyl]-2-azabicyclo[3.1.1]heptan-4-yl}methanesulfonamide

### (70A) N-{(3S,4R)-5-Fluoro-3-[(2-fluoro[biphenyl]-3-yl)methyl]-2-[(2R)-oxetan-2-ylcarbonyl]-2-azabicyclo[3.1.1]heptan-4-yl}methanesulfonamide

To a mixture of the compound synthesized in Example 66C (30.0 mg), (2R)-oxetane-2-carboxylic acid (10.7 mg), 1-hydroxy-7-azabenzotriazole (9.5 mg), triethylamine (0.0388 mL), and N,N-dimethylformamide (1 mL), 1-(3-dimethylaminopropyl)-3-ethylcarbodiimide hydrochloride (20.1 mg) was added at room temperature, and the mixture was then stirred at 30°C for 3 hours. The mixture was diluted with ethyl acetate, sequentially washed with water, hydrochloric acid (0.5 M), a saturated aqueous sodium bicarbonate solution, and a saturated saline solution, and dried over sodium sulfate. After filtration, the filtrate was concentrated under reduced pressure, and the residue was purified by silica gel column chromatography [eluting solvent: n-hexane/ethyl acetate = 50/50 to 0/100 (V/V)] to afford 33.4 mg (yield: quantitative) of the marked compound as a single diastereomer and as a single enantiomer.

### Example 71

### N-{(3S,4R)-5-Fluoro-3-[(2-fluoro[biphenyl]-3-yl)methyl]-2-[(1-hydroxycyclopropyl)carbonyl]-2-azabicyclo[3.1.1]heptan-4-yl}methanesulfonamide

### (71A) N-{(3S,4R)-5-Fluoro-3-[(2-fluoro[biphenyl]-3-yl)methyl]-2-[(1-hydroxycyclopropyl)carbonyl]-2-azabicyclo[3.1.1]heptan-4-yl}methanesulfonamide

To a mixture of the compound synthesized in Example 66C (30.0 mg), 1-hydroxy-1-cyclopropanecarboxylic acid (10.7 mg), 1-hydroxy-7-azabenzotriazole (9.5 mg), triethylamine (0.0388 mL), and N,N-dimethylformamide (1 mL), 1-(3-dimethylaminopropyl)-3-ethylcarbodiimide hydrochloride (20.1 mg) was added at room temperature, and the mixture was then stirred at 35°C for 2.5 hours. The mixture was diluted with ethyl acetate, sequentially washed with water, hydrochloric acid (0.5 M), a saturated aqueous sodium bicarbonate solution, and a saturated saline solution, and dried over sodium sulfate. After filtration, the filtrate was concentrated under reduced pressure, and the residue was purified by silica gel column chromatography [eluting solvent: n-hexane/ethyl acetate = 60/40 to 20/80 (V/V)] to afford 27.7 mg (yield: 83%) of the marked compound as a single diastereomer and as a single enantiomer.

### Example 72

### N-{(3S,4S)-2-(3,3-Difluoro-2-hydroxypropanoyl)-3-[(2-fluoro[biphenyl]-3-yl)methyl]-2-azabicyclo[3.1.1]heptan-4-yl}methanesulfonamide

### (72A) N-{(3S,4S)-2-(3,3-Difluoro-2-hydroxypropanoyl)-3-[(2-fluoro[biphenyl]-3-yl)methyl]-2-azabicyclo[3.1.1]heptan-4-yl}methanesulfonamide

Using the compound synthesized in Example 25B (100 mg) and 3,3-difluorolactic acid (36 mg), 40 mg (yield: 34%) of the marked compound was obtained by the same method as in Example 5H.

### (72B) N-{(3S,4S)-2-(3,3-Difluoro-2-hydroxypropanoyl)-3-[(2-fluoro[biphenyl]-3-yl)methyl]-2-azabicyclo[3.1.1]heptan-4-yl}methanesulfonamide

The compound synthesized in Example 72A (40 mg) was subjected to chiral SFC [column: CHIRALCEL (R) OJ (30 mm I.D. × 250 mm), mobile phase: carbon dioxide/2-propanol = 65/35 (V/V)] to afford 19 mg (yield: 48%) of the marked compound, which was eluted first, as a single diastereomer and as a single enantiomer.

### Example 73

### N-[(3S,4S)-3-[(2-Fluoro[biphenyl]-3-yl)methyl]-2-{[1-(hydroxymethyl)cyclopropyl]carbonyl}-2-azabicyclo[3.1.1]heptan-4-yl]methanesulfonamide

### (73A) N-[(3S,4S)-3-[(2-Fluoro[biphenyl]-3-yl)methyl]-2-{[1-(hydroxymethyl)cyclopropyl]carbonyl}-2-azabicyclo[3.1.1]heptan-4-yl]methanesulfonamide

To a mixture of 1-(hydroxymethyl)cyclopropanecarboxylic acid (CAS Registration No.: 49640-66-6, 8 mg) and N,N-dimethylformamide (1.0 mL), O-(7-azabenzotriazol-1-yl)-N,N,N',N'-tetramethyluronium hexafluorophosphate (41 mg) was added and the mixture was stirred at 40°C for 1 hour. The compound synthesized in Example 25B (30 mg) and triethylamine (73 mg) were added and the mixture was stirred at room temperature for 1 hour. The mixture was diluted with ethyl acetate, washed with water and a saturated saline solution, and then dried over sodium sulfate. After filtration, the solvent was distilled off under reduced pressure, and purification by reverse phase silica gel column chromatography [eluting solvent: 0.225% aqueous formic acid solution/acetonitrile = 70/30 to 40/60 (V/V)] was performed to afford 28 mg (yield: 81%) of the marked compound as a single diastereomer and as a single enantiomer.

### Example 74

### N-[(3S,4R)-3-[(2,5-Difluoro[biphenyl]-3-yl)methyl]-5-fluoro-2-(2-hydroxy-2-methylpropanoyl)-2-azabicyclo[3.1.1]heptan-4-yl]methanesulfonamide

### (74A) tert-Butyl (3S,4R)-3-(3-bromo-2,5-difluorobenzyl)-5-fluoro-4-[(methylsulfonyl)amino]-2-azabicyclo[3.1.1]heptane-2-carboxylate

The compound synthesized in Example 63E (990 mg) was subjected to chiral HPLC [column: CHIRAL ART Amylose-SA (20 mm I.D. × 250 mm), mobile phase: n-hexane/2-propanol = 85/15 (V/V), temperature: 40°C] to afford 371 mg (yield: 37%) of the marked compound, which was eluted first, as a single diastereomer and as a single enantiomer.

### (74B) tert-Butyl (3S,4R)-3-[(2,5-difluoro[biphenyl]-3-yl)methyl]-5-fluoro-4-[(methylsulfonyl)amino]-2-azabicyclo[3.1.1]heptane-2-carboxylate

A mixture of the compound synthesized in Example 74A (178 mg), phenylboronic acid (64 mg), tripotassium phosphate (211 mg), chloro(2-dicyclohexylphosphino-2',4',6'-triisopropyl-1,1'-biphenyl)[2-(2'-amino-1,1'-biphenyl)]palladium(II) (21 mg), tetrahydrofuran (3.5 mL), and water (1.2 mL) was stirred at 70°C for 3 hours in a nitrogen atmosphere. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: n-hexane/ethyl acetate = 70/30 to 50/50 (V/V)] to afford 173 mg (yield: 97%) of the marked compound as a single diastereomer and as a single enantiomer.

### (74C) N-{(3S,4R)-3-[(2,5-Difluoro[biphenyl]-3-yl)methyl]-5-fluoro-2-azabicyclo[3.1.1]heptan-4-yl}methanesulfonamide hydrochloride

To the compound synthesized in Example 74B (173 mg), a solution of hydrogen chloride in 1,4-dioxane (4 M, 4.0 mL) was added and the mixture was stirred at room temperature for 1 hour. The solvent was distilled off under reduced pressure to afford 151 mg of the crude marked compound as a single diastereomer and as a single enantiomer.

### (74D) 1-{(3S,4R)-3-[(2,5-Difluoro[biphenyl]-3-yl)methyl]-5-fluoro-4-[(methylsulfonyl)amino]-2-azabicyclo[3.1.1]heptan-2-yl}-2-methyl-1-oxopropan-2-yl acetate

To a mixture of the compound synthesized in Example 74C (35 mg) and tetrahydrofuran (1.0 mL), triethylamine (0.054 mL) and 2-acetoxyisobutyryl chloride (0.023 mL) were added at room temperature and the mixture was stirred at room temperature for 1 hour. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: n-hexane/ethyl acetate = 50/50 to 10/90 (V/V)] to afford 36 mg (yield: 84%) of the marked compound as a single diastereomer and as a single enantiomer.

### (74E) N-[(3S,4R)-3-[(2,5-Difluoro[biphenyl]-3-yl)methyl]-5-fluoro-2-(2-hydroxy-2-methylpropanoyl)-2-azabicyclo[3.1.1]heptan-4-yl]methanesulfonamide

To a mixture of the compound synthesized in Example 74D (36 mg), tetrahydrofuran (1.0 mL), methanol (0.5 mL), and water (0.5 mL), lithium hydroxide monohydrate (42 mg) was added at room temperature, and the mixture was then stirred at 40°C for 2 hours. After adding hydrochloric acid (2 M), the mixture was diluted with ethyl acetate and washed with water and a saturated saline solution. The organic layer was dried over sodium sulfate and filtered, and the solvent was then distilled off under reduced pressure. The residue was purified by silica gel column chromatography [eluting solvent: n-hexane/ethyl acetate = 70/30 to 50/50 (V/V)] to afford 32 mg (yield: 97%) of the marked compound as a single diastereomer and as a single enantiomer.

### Example 75

### N-{(3S,4S)-3-[(2-Fluoro[biphenyl]-3-yl)methyl]-2-[(2S)-3-fluoro-2-hydroxypropanoyl]-2-azabicyclo[3.1.1]heptan-4-yl}methanesulfonamide

### (75A) N-{(3S,4S)-3-[(2-Fluoro[biphenyl]-3-yl)methyl]-2-[(2S)-3-fluoro-2-hydroxypropanoyl]-2-azabicyclo[3.1.1]heptan-4-yl}methanesulfonamide

Using the compound synthesized in Example 25B (18.0 mg) and (2S)-3-fluoro-2-hydroxypropanoic acid (CAS Registration No.: 97643-52-2, 6.15 mg), 12.2 mg (yield: 60%) of the marked compound was obtained as a single diastereomer and as a single enantiomer by the same method as in Example 5H.

### Example 76

### N-{(3S,4S)-3-[(2-Fluoro[biphenyl]-3-yl)methyl]-2-[(2R)-2-hydroxy(²H₄)propanoyl]-2-azabicyclo[3.1.1]heptan-4-yl}methanesulfonamide

### (76A) N-[(3S,4S)-3-[(2-Fluoro[biphenyl]-3-yl)methyl]-2-(2-oxopropanoyl)-2-azabicyclo[3.1.1] heptan-4-yl]methanesulfonamide

To a mixture of the compound synthesized in Example 25B (107 mg), pyruvic acid (0.0234 mL), and N,N-dimethylformamide (2.6 mL), N,N-diisopropylethylamine (0.134 mL) and O-(7-azabenzotriazol-1-yl)-N,N,N',N'-tetramethyluronium hexafluorophosphate (134 mg) were added at room temperature and the mixture was stirred for 1.5 hours. The mixture was diluted with ethyl acetate, sequentially washed with water and a saturated saline solution, and dried over sodium sulfate. After filtration, the solvent was distilled off under reduced pressure, and the residue was purified by silica gel column chromatography [eluting solvent: n-hexane/ethyl acetate = 2/1 to 1/2 (V/V)] to afford 108 mg (yield: 93%) of the marked compound as a single diastereomer and as a single enantiomer.

### (76B) N-{(3S,4S)-3-[(2-Fluoro[biphenyl]-3-yl)methyl]-2-[(2R)-2-hydroxy(²H₄)propanoyl]-2-azabicyclo[3.1.1]heptan-4-yl}methanesulfonamide

To a mixture of the compound synthesized in Example 76A (20.0 mg), tetrahydrofuran (0.45 mL), and methanol-d4 (0.45 mL), potassium carbonate (9.3 mg) was added at room temperature and the mixture was stirred for 1.5 hours. After adding a solution of acetic acid (0.0077 mL) in methanol-d4 (0.15 mL), sodium borodeuteride (3.8 mg) was added and the mixture was stirred at room temperature for 1 hour. The mixture was diluted with ethyl acetate, to which an aqueous ammonium chloride solution was added. The organic layer was washed with a saturated saline solution, and then dried over sodium sulfate. After filtration, the solvent was distilled off under reduced pressure, and the residue was purified by preparative thin layer silica gel chromatography [eluting solvent: ethyl acetate] to afford 10.3 mg (yield: 51%) of the marked compound as a single diastereomer and as a single enantiomer.

### Example 77

### N-[(3S,4R)-3-[(3'-Chloro-2,5-difluoro[biphenyl]-3-yl)methyl]-5-fluoro-2-(2-hydroxy-2-methylpropanoyl)-2-azabicyclo[3.1.1]heptan-4-yl]methanesulfonamide

### (77A) tert-Butyl (3S,4R)-3-[(3'-chloro-2,5-difluoro[biphenyl]-3-yl)methyl]-5-fluoro-4-[(methylsulfonyl)amino]-2-azabicyclo[3.1.1]heptane-2-carboxylate

A mixture of the compound synthesized in Example 74A (192 mg), 3-chlorophenylboronic acid (88 mg), potassium carbonate (155 mg), tetrakis(triphenylphosphine)palladium(0) (33 mg), 1,4-dioxane (6.2 mL), and water (1.5 mL) was stirred at 100°C for 2.5 hours. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: n-hexane/ethyl acetate = 90/10 to 40/60 (V/V)] and then purified by reverse phase silica gel column chromatography [eluting solvent: 0.1% aqueous formic acid solution/acetonitrile = 100/0 to 0/100 (V/V)] to afford 124 mg (yield: 61%) of the marked compound as a single diastereomer and as a single enantiomer.

### (77B) N-{(3S,4R)-3-[(3'-Chloro-2,5-difluoro[biphenyl]-3-yl)methyl]-5-fluoro-2-azabicyclo[3.1.1]heptan-4-yl}methanesulfonamide hydrochloride

Using the compound synthesized in Example 77A (124 mg), 110 mg of the crude marked compound was obtained as a single diastereomer and as a single enantiomer by the same method as in Example 8H.

### (77C) 1-{(3S,4R)-3-[(3'-Chloro-2,5-difluoro[biphenyl]-3-yl)methyl]-5-fluoro-4-[(methylsulfonyl)amino]-2-azabicyclo[3.1.1]heptan-2-yl}-2-methyl-1-oxopropan-2-yl acetate

To a mixture of the compound synthesized in Example 77B (26 mg) and tetrahydrofuran (1.1 mL), triethylamine (0.038 mL) and 2-acetoxyisobutyryl chloride (0.016 mL) were added at room temperature and the mixture was stirred at room temperature for 1.5 hours. The mixture was diluted with ethyl acetate, washed with water and a saturated saline solution, and then dried over sodium sulfate. After filtration, the solvent was distilled off under reduced pressure, and the residue was purified by silica gel column chromatography [eluting solvent: n-hexane/ethyl acetate = 60/40 to 40/60 (V/V)] to afford 26 mg (yield: 83%) of the marked compound as a single diastereomer and as a single enantiomer.

### (77D) N-[(3S,4R)-3-[(3'-Chloro-2,5-difluoro[biphenyl]-3-yl)methyl]-5-fluoro-2-(2-hydroxy-2-methylpropanoyl)-2-azabicyclo[3.1.1]heptan-4-yl]methanesulfonamide

Using the compound synthesized in Example 77C (26 mg), 26 mg (yield: quantitative) of the marked compound was obtained as a single diastereomer and as a single enantiomer by the same method as in Example 12H.

### Example 78

### N-{(3S,4R)-2-{[(1S)-2,2-Difluorocyclopropyl]carbonyl}-5-fluoro-3-[(2-fluoro[biphenyl]-3-yl)methyl]-2-azabicyclo[3.1.1]heptan-4-yl}methanesulfonamide

### (78A) N-{(3S,4R)-2-{[(1S)-2,2-difluorocyclopropyl]carbonyl}-5-fluoro-3-[(2-fluoro[biphenyl]-3-yl)methyl]-2-azabicyclo[3.1.1]heptan-4-yl}methanesulfonamide

To a mixture of the compound synthesized in Example 66C (30.0 mg), (1S)-2,2-difluorocyclopropane-1-carboxylic acid (12.8 mg), 1-hydroxy-7-azabenzotriazole (9.5 mg), triethylamine (0.0388 mL), and N,N-dimethylformamide (1 mL), 1-(3-dimethylaminopropyl)-3-ethylcarbodiimide hydrochloride (20.1 mg) was added at room temperature and the mixture was stirred at 30°C for 3 hours. The mixture was diluted with ethyl acetate, sequentially washed with water, hydrochloric acid (0.5 M), a saturated aqueous sodium bicarbonate solution, and a saturated saline solution, and dried over sodium sulfate. After filtration, the filtrate was concentrated under reduced pressure, and the residue was purified by silica gel column chromatography [eluting solvent: n-hexane/ethyl acetate = 70/30 to 20/80 (V/V)] to afford 33.4 mg (yield: 96%) of the marked compound as a single diastereomer and as a single enantiomer.

### Example 79

### N-{(3S,4R)-3-[(2,5-Difluoro[biphenyl]-3-yl)methyl]-5-fluoro-2-[(2S)-3,3,3-trifluoro-2-hydroxypropanoyl]-2-azabicyclo[3.1.1]heptan-4-yl}methanesulfonamide

### (79A) N-{(3S,4R)-3-[(2,5-Difluoro[biphenyl]-3-yl)methyl]-5-fluoro-2-[(2S)-3,3,3-trifluoro-2-hydroxypropanoyl]-2-azabicyclo[3.1.1]heptan-4-yl}methanesulfonamide

To a mixture of (S)-3,3,3-trifluoro-2-hydroxypropionic acid (242 mg) and dichloromethane (3.4 mL), 1-chloro-N,N,2-trimethyl-1-propenylamine (0.23 mL) was added at 0°C, and the mixture was then stirred at room temperature for 45 minutes. This reaction mixture and N,N-diisopropylethylamine (0.33 mL) were added to a mixture of the separately prepared compound synthesized in Example 74C (50 mg) and dichloromethane (3.0 mL) at room temperature over 30 minutes, and the mixture was then stirred for 15 minutes. A 10% aqueous ammonia (3.0 mL) was added, and after stirring for 5 minutes, an aqueous ammonium chloride solution was added. The mixture was extracted with ethyl acetate, and the organic layer was washed with a saturated saline solution and then dried over sodium sulfate. After filtration, the filtrate was concentrated under reduced pressure, and the residue was purified by silica gel column chromatography [eluting solvent: n-hexane/ethyl acetate = 50/50 to 0/100 (V/V)] to afford 15 mg (yield: 25%) of the marked compound as a single diastereomer and as a single enantiomer.

### Example 80

### N-{(3S,4S)-3-[(2-Fluoro[biphenyl]-3-yl)methyl]-2-[(2R)-2-hydroxypropanoyl]-2-azabicyclo[3.1.1]heptan-4-yl}ethanesulfonamide

### (80A) tert-Butyl (3S,4S)-3-(3-bromo-2-fluorobenzyl)-4-[(ethylsulfonyl)amino]-2-azabicyclo[3.1.1]heptane-2-carboxylate

To a mixture of the compound synthesized in Example 8F (300 mg), triethylamine (0.105 mL), and tetrahydrofuran (3.0 mL), ethanesulfonyl chloride (204 mg) was added, and the mixture was then stirred at room temperature for 12 hours. The mixture was diluted with ethyl acetate, washed with water and a saturated saline solution, and dried over sodium sulfate. After filtration, the solvent was distilled off under reduced pressure, and purification by reverse phase silica gel column chromatography [eluting solvent: 0.225% aqueous formic acid solution/acetonitrile = 52/48 to 22/78 (V/V)] was performed to afford 120 mg (yield: 33%) of the marked compound as a single diastereomer and as a single enantiomer.

### (80B) tert-Butyl (3S,4S)-4-[(ethylsulfonyl)amino]-3-[(2-fluoro[biphenyl]-3-yl)methyl]-2-azabicyclo[3.1.1]heptane-2-carboxylate

To a mixture of the compound synthesized in Example 80A (270 mg), phenylboronic acid (134 mg), tripotassium phosphate (236 mg), and tetrahydrofuran (3.0 mL), (2-dicyclohexylphosphino-2',4',6'-triisopropyl-1,1'-biphenyl)[2-(2'-amino-1,1'-biphenyl)]palladium(II) methanesulfonate (24 mg) was added at room temperature and the mixture was stirred at 80°C for 12 hours in a nitrogen atmosphere. The mixture was diluted with ethyl acetate, sequentially washed with water and a saturated saline solution, and dried over sodium sulfate. After filtration, the solvent was distilled off under reduced pressure, and the residue was purified by silica gel column chromatography [eluting solvent: petroleum ether/ethyl acetate = 1/0 to 1/1 (V/V)] to afford 250 mg (yield: 93%) of the marked compound as a single diastereomer and as a single enantiomer.

### (80C) N-{(3S,4S)-3-[(2-Fluoro[biphenyl]-3-yl)methyl]-2-azabicyclo[3.1.1]heptan-4-yl}ethanesulfonamide hydrochloride

To a mixture of the compound synthesized in Example 80B (250 mg) and ethyl acetate (2.0 mL), a solution of hydrogen chloride in ethyl acetate (4 M, 5.0 mL) was added at room temperature and the mixture was stirred for 2 hours. The solvent was distilled off under reduced pressure to afford 200 mg of the crude marked compound as a single diastereomer and as a single enantiomer.

### (80D) (2R)-1-{(3S,4S)-4-[(Ethylsulfonyl)amino]-3-[(2-fluoro[biphenyl]-3-yl)methyl]-2-azabicyclo[3.1.1]heptan-2-yl}-1-oxopropan-2-yl acetate

To a mixture of the compound synthesized in Example 80C (100 mg), N,N-diisopropylethylamine (0.40 mL), and tetrahydrofuran (5.0 mL), (R)-2-acetoxypropionic acid chloride (60 mg) was added at room temperature and the mixture was stirred for 3 hours. The mixture was diluted with ethyl acetate, washed with water and a saturated saline solution, and dried over sodium sulfate. After filtration, the solvent was distilled off under reduced pressure to afford 130 mg of the crude marked compound as a single diastereomer and as a single enantiomer.

### (80E) N-{(3S,4S)-3-[(2-Fluoro[biphenyl]-3-yl)methyl]-2-[(2R)-2-hydroxypropanoyl]-2-azabicyclo[3.1.1]heptan-4-yl}ethanesulfonamide

To a mixture of the compound synthesized in Example 80D (130 mg), tetrahydrofuran (2.0 mL), and water (2.0 mL), lithium hydroxide monohydrate (106 mg) was added at room temperature and the mixture was stirred for 12 hours. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 65/35 to 35/65 (V/V)] to afford 42 mg (yield: 35%) of the marked compound as a single diastereomer and as a single enantiomer.

### Example 81

### N-{3-[(2,5-Difluoro[biphenyl]-3-yl)methyl]-2-[(2R)-2-hydroxypropanoyl]-2-azabicyclo[3.1.1]heptan-4-yl}ethanesulfonamide

### (81A) tert-Butyl 3-(3-bromo-2,5-difluorobenzyl)-4-[(ethylsulfonyl)amino]-2-azabicyclo[3.1.1]heptane-2-carboxylate

To a mixture of the compound synthesized in Example 58E (600 mg), triethylamine (437 mg), and tetrahydrofuran (4.0 mL), ethanesulfonyl chloride (277 mg) was added, and the mixture was then stirred at room temperature for 1 hour. The mixture was diluted with ethyl acetate, washed with water and a saturated saline solution, and dried over sodium sulfate. After filtration, the solvent was distilled off under reduced pressure, and purification by silica gel column chromatography [eluting solvent: petroleum ether/ethyl acetate = 1/0 to 1/1 (V/V)] was performed to afford 570 mg (yield: 78%) of the marked compound as a single diastereomer (racemate).

### (81B) tert-Butyl 3-[(2,5-difluoro[biphenyl]-3-yl)methyl]-4-[(ethylsulfonyl)amino]-2-azabicyclo[3.1.1]heptane-2-carboxylate

To a mixture of the compound synthesized in Example 81A (570 mg), phenylboronic acid (205 mg), tripotassium phosphate (475 mg), and tetrahydrofuran (6.0 mL), (2-dicyclohexylphosphino-2',4',6'-triisopropyl-1,1'-biphenyl)[2-(2'-amino-1,1'-biphenyl)]palladium(II) methanesulfonate (95 mg) was added at room temperature and the mixture was stirred at 70°C for 12 hours in a nitrogen atmosphere. The solvent was distilled off under reduced pressure, and the residue was purified by silica gel column chromatography [eluting solvent: petroleum ether/ethyl acetate = 1/0 to 1/1 (V/V)] to afford 500 mg (yield: 88%) of the marked compound as a single diastereomer (racemate).

### (81C) N-{3-[(2,5-Difluoro[biphenyl]-3-yl)methyl]-2-azabicyclo[3.1.1]heptan-4-yl}ethanesulfonamide hydrochloride

To a mixture of the compound synthesized in Example 81B (500 mg) and ethyl acetate (5.0 mL), a solution of hydrogen chloride in ethyl acetate (4 M, 6.0 mL) was added at room temperature and the mixture was stirred for 2 hours. The solvent was distilled off under reduced pressure to afford 450 mg of the crude marked compound as a single diastereomer (racemate).

### (81D) (2R)-1-{3-[(2,5-Difluoro[biphenyl]-3-yl)methyl]-4-[(ethylsulfonyl)amino]-2-azabicyclo[3.1.1]heptan-2-yl}-1-oxopropan-2-yl acetate

To a mixture of the compound synthesized in Example 81C (150 mg), N,N-diisopropylethylamine (437 mg), and dichloromethane (1.0 mL), (R)-2-acetoxypropionic acid chloride (76 mg) was added at room temperature and the mixture was stirred for 1 hour. The mixture was diluted with ethyl acetate, washed with water and a saturated saline solution, and dried over sodium sulfate. After filtration, the solvent was distilled off under reduced pressure to afford 150 mg of the crude marked compound.

### (81E) N-{3-[(2,5-Difluoro[biphenyl]-3-yl)methyl]-2-[(2R)-2-hydroxypropanoyl]-2-azabicyclo[3.1.1]heptan-4-yl}ethanesulfonamide

To a mixture of the compound synthesized in Example 81D (150 mg), tetrahydrofuran (2.0 mL), and water (0.5 mL), lithium hydroxide (34 mg) was added at room temperature and the mixture was stirred for 12 hours. The mixture was diluted with ethyl acetate, washed with water and a saturated saline solution, and then dried over sodium sulfate. After filtration, the solvent was distilled off under reduced pressure, and the residue was purified by reverse phase silica gel column chromatography [eluting solvent: 0.225% aqueous formic acid solution/acetonitrile = 58/42 to 38/62 (V/V)] to afford 80 mg (yield: 58%) of the marked compound.

### (81F) N-{3-[(2,5-Difluoro[biphenyl]-3-yl)methyl]-2-[(2R)-2-hydroxypropanoyl]-2-azabicyclo[3.1.1]heptan-4-yl}ethanesulfonamide

The compound synthesized in Example 81E (60 mg) was subjected to chiral SFC [column: Phenomenex-Cellulose-2 (30 mm I.D. × 250 mm), mobile phase: carbon dioxide/ethanol = 65/35 (V/V)] to afford 28 mg (yield: 46%) of the marked compound, which was eluted first, as a single diastereomer and as a single enantiomer.

### Example 82

### N-{(3S,4R)-3-[(3'-Chloro-2,5-difluoro[biphenyl]-3-yl)methyl]-5-fluoro-2-[(2S)-3,3,3-trifluoro-2-hydroxypropanoyl]-2-azabicyclo[3.1.1]heptan-4-yl}methanesulfonamide

### (82A) N-{(3S,4R)-3-[(3'-Chloro-2,5-difluoro[biphenyl]-3-yl)methyl]-5-fluoro-2-[(2S)-3,3,3-trifluoro-2-hydroxypropanoyl]-2-azabicyclo[3.1.1]heptan-4-yl}methanesulfonamide

To a mixture of (S)-3,3,3-trifluoro-2-hydroxypropionic acid (242 mg) and dichloromethane (3.4 mL), 1-chloro-N,N,2-trimethyl-1-propenylamine (0.23 mL) was added at 0°C, and the mixture was then stirred at room temperature for 45 minutes. This reaction mixture and N,N-diisopropylethylamine (0.33 mL) were added to a mixture of the separately prepared compound synthesized in Example 77B (57 mg) and dichloromethane (3.0 mL) at room temperature over 30 minutes, and the mixture was then stirred for 15 minutes. A 10% aqueous ammonia (3.0 mL) was added, and after stirring for 5 minutes, hydrochloric acid (2 M, 6 mL) and a saturated aqueous sodium bicarbonate solution (3 mL) were sequentially added. The mixture was extracted with ethyl acetate, and the organic layer was washed with a saturated saline solution and then dried over sodium sulfate. After filtration, the filtrate was concentrated under reduced pressure, and the residue was sequentially purified by silica gel column chromatography [eluting solvent: n-hexane/ethyl acetate = 80/20 to 50/50 (V/V)] and NH silica gel column chromatography [eluting solvent: n-hexane/ethyl acetate = 50/50 to 0/100 (V/V)] to afford 25 mg (yield: 38%) of the marked compound as a single diastereomer and as a single enantiomer.

### Example 83

### N-{(3S,4R)-5-Fluoro-3-[(2-fluoro[biphenyl]-3-yl)methyl]-2-[(2R)-2-hydroxypropanoyl]-2-azabicyclo[3.1.1]heptan-4-yl}ethanesulfonamide

### (83A) tert-Butyl (3S*,4R*)-4-amino-5-fluoro-3-[(2-fluoro[biphenyl]-3-yl)methyl]-2-azabicyclo[3.1.1]heptane-2-carboxylate

A mixture of the compound synthesized in Example 23D (15.0 mg), phenylboronic acid (8.8 mg), (2-dicyclohexylphosphino-2',4',6'-triisopropyl-1,1'-biphenyl)[2-(2'-amino-1,1'-biphenyl)]palladium(II) methanesulfonate (3.0 mg), tripotassium phosphate (22.9 mg), tetrahydrofuran (0.6 mL), and water (0.2 mL) was stirred at 60°C for 1.5 hours. A mixture of the compound synthesized in Example 23D (255 mg), phenylboronic acid (149 mg), (2-dicyclohexylphosphino-2',4',6'-triisopropyl-1,1'-biphenyl)[2-(2'-amino-1,1'-biphenyl)]palladium(II) methanesulfonate (51.7 mg), tripotassium phosphate (389 mg), tetrahydrofuran (6 mL), and water (2 mL) was stirred at 60°C for 2 hours. After combining these reaction mixtures and adding a saturated saline solution, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 60/40 to 20/80 (V/V)] to afford 252 mg (yield: 94%) of the marked compound as a single diastereomer (racemate).

### (83B) tert-Butyl (3S*,4R*)-4-[(ethenylsulfonyl)amino]-5-fluoro-3-[(2-fluoro[biphenyl]-3-yl)methyl]-2-azabicyclo[3.1.1]heptane-2-carboxylate

To a mixture of the compound synthesized in Example 83A (252 mg), triethylamine (0.253 mL), and tetrahydrofuran (5 mL), a solution of ethenesulfonyl chloride (154 mg) in tetrahydrofuran (1 mL) was added at 0°C, and the mixture was then stirred at room temperature for 2 hours. After adding water, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 75/25 to 50/50 (V/V)] to afford 294 mg (yield: 96%) of the marked compound as a single diastereomer (racemate).

### (83C) tert-Butyl (3S*,4R*)-4-[(ethylsulfonyl)amino]-5-fluoro-3-[(2-fluoro[biphenyl]-3-yl)methyl]-2-azabicyclo[3.1.1]heptane-2-carboxylate

A mixture of the compound synthesized in Example 83B (10.0 mg), 10% palladium carbon (5 mg), and ethyl acetate (1 mL) was stirred at room temperature for 2 hours in a hydrogen atmosphere. A mixture of the compound synthesized in Example 83B (284 mg), 10% palladium carbon (120 mg), and ethyl acetate (8 mL) was stirred at room temperature for 2 hours in a hydrogen atmosphere. After combining and filtering these reaction mixtures, the filtrate was concentrated under reduced pressure, and the residue was purified by silica gel column chromatography [eluting solvent: n-hexane/ethyl acetate = 75/25 to 40/60 (V/V)] to afford 264 mg (yield: 89%) of the marked compound as a single diastereomer (racemate).

### (83D) tert-Butyl (3S,4R)-4-[(ethylsulfonyl)amino]-5-fluoro-3-[(2-fluoro[biphenyl]-3-yl)methyl]-2-azabicyclo[3.1.1]heptane-2-carboxylate

The compound synthesized in Example 83C (264 mg) was purified by chiral HPLC [column: CHIRALPAK (R) IC (20 mm I.D. × 250 mm), mobile phase: n-hexane/2-propanol = 55/45 (V/V), temperature: 40°C] to afford 112 mg (yield: 42%) of the marked compound, which was eluted later, as a single diastereomer and as a single enantiomer.

### (83E) N-{(3S,4R)-5-Fluoro-3-[(2-fluoro[biphenyl]-3-yl)methyl]-2-azabicyclo[3.1.1] heptan-4-yl}ethanesulfonamide hydrochloride

To a mixture of the compound synthesized in Example 83D (112 mg) and 1,4-dioxane (2.5 mL), a solution of hydrogen chloride in 1,4-dioxane (4 M, 5 mL) was added at room temperature and the mixture was stirred for 4 hours. After diluting the reaction mixture with toluene, the solution was concentrated under reduced pressure to afford 91.7 mg of the crude marked compound.

### (83F) (2R)-1-{(3S,4R)-4-[(Ethylsulfonyl)amino]-5-fluoro-3-[(2-fluoro[biphenyl]-3-yl)methyl]-2-azabicyclo[3.1.1]heptan-2-yl}-1-oxopropan-2-yl acetate

To a mixture of the compound synthesized in Example 83E (25.0 mg), (R)-2-acetoxypropionic acid (14.9 mg), triethylamine (0.0313 mL), and N,N-dimethylformamide (1.5 mL), O-(7-azabenzotriazol-1-yl)-N,N,N',N'-tetramethyluronium hexafluorophosphate (42.9 mg) was added at room temperature and the mixture was stirred overnight. The mixture was diluted with ethyl acetate, sequentially washed with water, hydrochloric acid (0.5 M), a saturated aqueous sodium bicarbonate solution, and a saturated saline solution, and dried over sodium sulfate. After filtration, the filtrate was concentrated under reduced pressure, and the residue was purified by silica gel column chromatography [eluting solvent: n-hexane/ethyl acetate = 60/40 to 20/80 (V/V)] to afford 28.9 mg (yield: 98%) of the marked compound as a single diastereomer and as a single enantiomer.

### (83G) N-{(3S,4R)-5-Fluoro-3-[(2-fluoro[biphenyl]-3-yl)methyl]-2-[(2R)-2-hydroxypropanoyl]-2-azabicyclo[3.1.1]heptan-4-yl}ethanesulfonamide

To a mixture of the compound synthesized in Example 83F (28.9 mg), tetrahydrofuran (1.2 mL), methanol (0.4 mL), and water (0.4 mL), lithium hydroxide monohydrate (7.0 mg) was added at room temperature and the mixture was stirred overnight. The mixture was diluted with ethyl acetate, sequentially washed with water and a saturated saline solution, and dried over sodium sulfate. After filtration, the filtrate was concentrated under reduced pressure, and the residue was purified by silica gel column chromatography [eluting solvent: n-hexane/ethyl acetate = 70/30 to 0/100 (V/V)] to afford 23.7 mg (yield: 89%) of the marked compound as a single diastereomer and as a single enantiomer.

### Example 84

### N-[(3S,4R)-5-Fluoro-3-[(2-fluoro[biphenyl]-3-yl)methyl]-2-(1,2-oxazol-3-ylcarbonyl)-2-azabicyclo[3.1.1]heptan-4-yl]methanesulfonamide

### (84A) N-[(3S,4R)-5-Fluoro-3-[(2-fluoro[biphenyl]-3-yl)methyl]-2-(1,2-oxazol-3-ylcarbonyl)-2-azabicyclo[3.1.1]heptan-4-yl]methanesulfonamide

To a mixture of the compound synthesized in Example 66C (20.0 mg), isoxazole-3-carboxylic acid (7.9 mg), 1-hydroxy-7-azabenzotriazole (6.4 mg), triethylamine (0.0259 mL), and N,N-dimethylformamide (1 mL), 1-(3-dimethylaminopropyl)-3-ethylcarbodiimide hydrochloride (13.4 mg) was added at room temperature, and the mixture was then stirred at 30°C for 2 hours. The mixture was diluted with ethyl acetate, sequentially washed with water, hydrochloric acid (0.5 M), a saturated aqueous sodium bicarbonate solution, and a saturated saline solution, and dried over sodium sulfate. After filtration, the filtrate was concentrated under reduced pressure, and the residue was purified by silica gel column chromatography [eluting solvent: n-hexane/ethyl acetate = 70/30 to 30/70 (V/V)] to afford 9.2 mg (yield: 43%) of the marked compound as a single diastereomer and as a single enantiomer.

### Example 85

### N-[(3S,4R)-5-Fluoro-3-[(2-fluoro[biphenyl]-3-yl)methyl]-2-(2-fluoro-2-methylpropanoyl)-2-azabicyclo[3.1.1]heptan-4-yl]methanesulfonamide

### (85A) N-[(3S,4R)-5-Fluoro-3-[(2-fluoro[biphenyl]-3-yl)methyl]-2-(2-fluoro-2-methylpropanoyl)-2-azabicyclo[3.1.1]heptan-4-yl]methanesulfonamide

Using the compound synthesized in Example 66C (20.0 mg) and 2-fluoroisobutyric acid (7.4 mg), 18.6 mg (yield: 83%) of the marked compound was obtained as a single diastereomer and as a single enantiomer by the same method as in Example 84A.

### Example 86

### N-{(3S,4S)-3-[(2-Fluoro[biphenyl]-3-yl)methyl]-2-[(3S)-3-hydroxybutanoyl]-2-azabicyclo[3.1.1]heptan-4-yl}methanesulfonamide

### (86A) N-{(3S,4S)-3-[(2-Fluoro[biphenyl]-3-yl)methyl]-2-[(3S)-3-hydroxybutanoyl]-2-azabicyclo[3.1.1]heptan-4-yl}methanesulfonamide

Using the compound synthesized in Example 25B (100 mg) and 3-hydroxybutanoic acid (30 mg), 72 mg (yield: 65%) of the marked compound was obtained as a single diastereomer and as a single enantiomer by the same method as in Example 31C.

### Example 87

### N-{(3S,4R)-3-[(2,5-Difluoro[biphenyl]-3-yl)methyl]-5-fluoro-2-[(2R)-2-hydroxy(²H₄)propanoyl]-2-azabicyclo[3.1.1]heptan-4-yl}methanesulfonamide

### (87A) N-[(3S,4R)-3-[(2,5-Difluoro[biphenyl]-3-yl)methyl]-5-fluoro-2-(2-oxopropanoyl)-2-azabicyclo[3.1.1]heptan-4-yl]methanesulfonamide

To a mixture of the compound synthesized in Example 74C (45.0 mg), pyruvic acid (0.0216 mL), and N,N-dimethylformamide (1.01 mL), N,N-diisopropylethylamine (0.0696 mL) and O-(7-azabenzotriazol-1-yl)-N,N,N',N'-tetramethyluronium hexafluorophosphate (115 mg) were added at room temperature and the mixture was stirred for 1 hour. The mixture was diluted with ethyl acetate, sequentially washed with water and a saturated saline solution, and dried over sodium sulfate. After filtration, the solvent was distilled off under reduced pressure, and the residue was purified by silica gel column chromatography [eluting solvent: n-hexane/ethyl acetate = 50/50 to 0/100 (V/V)] to afford 50.7 mg (yield: quantitative) of the marked compound as a single diastereomer and as a single enantiomer.

### (87B) N-{(3S,4R)-3-[(2,5-Difluoro[biphenyl]-3-yl)methyl]-5-fluoro-2-[2-hydroxy(²H₄)propanoyl]-2-azabicyclo[3.1.1]heptan-4-yl}methanesulfonamide

To a mixture of the compound synthesized in Example 87A (50.7 mg), tetrahydrofuran (1.06 mL), and methanol-d4 (1.06 mL), potassium carbonate (21.9 mg) was added at room temperature and the mixture was stirred for 3 hours. After adding a solution of acetic acid (0.0181 mL) in methanol-d4 (0.181 mL), sodium borodeuteride (8.83 mg) was added under ice cooling and the mixture was stirred for 1 hour. The mixture was diluted with ethyl acetate, to which an aqueous ammonium chloride solution was added. The organic layer was washed with a saturated saline solution, and then dried over sodium sulfate. After filtration, the solvent was distilled off under reduced pressure and the residue was purified by preparative thin layer silica gel chromatography [eluting solvent: ethyl acetate] to afford 43.6 mg (yield: 85%) of the marked compound.

### (87C) N-{(3S,4R)-3-[(2,5-Difluoro[biphenyl]-3-yl)methyl]-5-fluoro-2-[(2R)-2-hydroxy(²H₄)propanoyl]-2-azabicyclo[3.1.1]heptan-4-yl}methanesulfonamide

The compound synthesized in Example 87B (43.6 mg) was subjected to chiral HPLC [column: CHIRALPAK (R) IG (20 mm I.D. × 250 mm), mobile phase: n-hexane/ethanol = 70/30 (V/V), temperature: 40°C] to afford 19.2 mg (yield: 44%) of the marked compound, which was eluted later, as a single diastereomer and as a single enantiomer.

### Example 88

### N-{(3S,4S)-3-[(2,5-Difluoro[biphenyl]-3-yl)methyl]-2-[(2R)-2-hydroxy(²H₄)propanoyl]-2-azabicyclo[3.1.1]heptan-4-yl}methanesulfonamide

### (88A) 2,5-Difluoro-3-methyl[biphenyl]

To a mixture of 3-bromo-2,5-difluorotoluene (CAS Registration No.: 1416354-32-9, 550 mg), phenylboronic acid (648 mg), tripotassium phosphate (1.69 g), and tetrahydrofuran (26.6 mL), (2-dicyclohexylphosphino- 2',4',6'-triisopropyl-1,1'-biphenyl)[2-(2'-amino-1,1'-biphenyl)]palladium(II) methanesulfonate (169 mg) and water (8.8 mL) were added at room temperature and the mixture was stirred at 60°C for 3 hours in a nitrogen atmosphere. After cooling to room temperature, the mixture was diluted with n-hexane, washed with a saturated saline solution, and dried over sodium sulfate. After filtration, the solvent was distilled off under reduced pressure and the residue was purified by silica gel column chromatography [eluting solvent: n-hexane] to afford 508 mg (yield: 94%) of the marked compound.

### (88B) 3-(Bromomethyl)-2,5-difluoro[biphenyl]

To a mixture of the compound synthesized in Example 88A (528 mg) and carbon tetrachloride (9.0 mL), N-bromosuccinimide (552 mg) and benzoyl peroxide (125 mg) were added at room temperature and the mixture was heated under reflux for 3 hours. After cooling to room temperature, a saturated aqueous sodium thiosulfate solution and a saturated aqueous sodium bicarbonate solution were added, and the mixture was diluted with n-hexane, sequentially washed with water and a saturated saline solution, and dried over sodium sulfate. After filtration, the solvent was distilled off under reduced pressure and the residue was purified by silica gel column chromatography [eluting solvent: n-hexane] to afford 679 mg (yield: 93%) of the marked compound.

### (88C) 2-tert-Butyl 3-methyl 3-[(2,5-difluoro[biphenyl]-3-yl)methyl]-4-oxo-2-azabicyclo[3.1.1]heptane-2,3-dicarboxylate

To a mixture of the compound synthesized in Example 88B (19.2 g) and N,N-dimethylformamide (230 mL), O4-tert-butyl O3-methyl 2-oxo-4-azabicyclo[3.1.1]heptane-3,4-dicarboxylate (CAS Registration No.: 2241048-56-4, 18.2 g) and cesium carbonate (99.2 g) were added and the mixture was stirred at room temperature for 3 hours. Water was added to the reaction mixture, which was extracted with ethyl acetate. The organic layer was washed with water and a saturated saline solution, and dried over anhydrous sodium sulfate. After filtration, the residue obtained by distilling off the solvent under reduced pressure was purified by silica gel column chromatography [eluting solvent: n-hexane/ethyl acetate = 90/10 to 70/30 (V/V)] to afford 28.7 g (yield: 90%) of the marked compound.

### (88D) tert-Butyl 3-[(2,5-difluoro[biphenyl]-3-yl)methyl]-4-oxo-2-azabicyclo[3.1.1]heptane-2-carboxylate

To a mixture of the compound synthesized in Example 88C (35.6 g) and dimethyl sulfoxide (250 mL), lithium chloride (12.8 g) was added, and the mixture was stirred at 130°C for 4 hours and cooled to room temperature. Water was added to the reaction mixture, which was extracted with ethyl acetate. The organic layer was washed with water and a saturated saline solution, and dried over anhydrous sodium sulfate. The residue obtained by distilling off the solvent under reduced pressure was purified by silica gel column chromatography [eluting solvent: n-hexane/ethyl acetate = 100/0 to 80/20 (V/V)] to afford a mixture of the marked compound and 2-tert-butyl 3-methyl 3-[(2,5-difluoro[biphenyl]-3-yl)methyl]-4-oxo-2-azabicyclo[3.1.1]heptane-2,3-dicarboxylate. Methanol (600 mL) and an aqueous sodium hydroxide solution (1 M, 151 mL) were added and the mixture was stirred at room temperature for 1 hour. The solvent was distilled off under reduced pressure, and the mixture was extracted with ethyl acetate. The organic layer was washed with a saturated saline solution, and dried over anhydrous sodium sulfate. After filtration, the solvent was distilled off under reduced pressure to afford 24.7 g (yield: 79%) of the marked compound.

### (88E) tert-Butyl 3-[(2,5-difluoro[biphenyl]-3-yl)methyl]-4-(hydroxyimino)-2-azabicyclo[3.1.1]heptane-2-carboxylate

To a mixture of the compound synthesized in Example 88D (27.0 g) and ethanol (320 mL), hydroxylamine hydrochloride (13.6 g) and sodium acetate (21.5 g) were added, and the mixture was stirred at 80°C for 2 hours and cooled to room temperature. The solvent was distilled off under reduced pressure, water was added, and the mixture was extracted with ethyl acetate. The organic layer was washed with a saturated saline solution, and dried over anhydrous sodium sulfate. After filtration, the solvent was distilled off under reduced pressure to afford 28.7 g of the crude marked compound.

### (88F) tert-Butyl 4-amino-3-[(2,5-difluoro[biphenyl]-3-yl)methyl]-2-azabicyclo[3.1.1]heptane-2-carboxylate

After adding molybdenum(VI) oxide (7.27 g) to a mixture of the compound synthesized in Example 88E (4.33 g) and methanol (100 mL) at 0°C, sodium borohydride (7.65 g) was added little by little and the mixture was stirred at 0°C for 1 hour. Molybdenum(VI) oxide (1.45 g) was added at 0°C, sodium borohydride (1.53 g) was added little by little, and the mixture was stirred at 0°C for 1 hour. A saturated aqueous sodium bicarbonate solution was added to the reaction mixture. In a separate container, a mixture of the compound synthesized in Example 88E (11.3 g) and methanol (260 mL) was prepared, molybdenum(VI) oxide (22.8 g) was added at 0°C, sodium borohydride (23.9 g) was added little by little, and the mixture was stirred at 0°C for 1 hour. A saturated aqueous sodium bicarbonate solution was added to the reaction mixture. In a separate container, a mixture of the compound synthesized in Example 88E (12.7 g) and methanol (300 mL) was prepared, molybdenum(VI) oxide (25.7 g) was added at 0°C, sodium borohydride (27.0 g) was added little by little, and the mixture was stirred at 0°C for 1 hour. A saturated aqueous sodium bicarbonate solution was added to the reaction mixture. These were combined, ethyl acetate was added, and Celite filtration was carried out twice, followed by washing with ethyl acetate. The filtrate was concentrated under reduced pressure and extracted with ethyl acetate. The organic layer was washed with a saturated saline solution, and dried over anhydrous sodium sulfate. After filtration, the solvent was distilled off under reduced pressure to afford 23.5 g of the crude marked compound.

### (88G) tert-Butyl (3S*,4S*)-3-[(2,5-difluoro[biphenyl]-3-yl)methyl]-4-[(methylsulfonyl)amino]-2-azabicyclo[3.1.1]heptane-2-carboxylate

To a mixture of the compound synthesized in Example 88F (23.8 g) and tetrahydrofuran (290 mL), N,N-diisopropylethylamine (29.5 mL) was added at 0°C and methanesulfonyl chloride (13.3 g) was slowly added dropwise. After stirring at room temperature for 2 hours, water was added and the mixture was extracted with ethyl acetate. The organic layer was washed with a saturated saline solution, and dried over anhydrous sodium sulfate. After filtration, the residue obtained by distilling off the solvent under reduced pressure was purified by silica gel column chromatography [eluting solvent: n-hexane/ethyl acetate = 90/10 to 55/45 (V/V)] to afford 16.4 g (yield: 51% in 3 steps) of the marked compound as a single diastereomer (racemate).

### (88H) tert-Butyl (3S,4S)-3-[(2,5-difluoro[biphenyl]-3-yl)methyl]-4-[(methylsulfonyl)amino]-2-azabicyclo[3.1.1]heptane-2-carboxylate

The compound synthesized in Example 88G (10 g) was subjected to chiral HPLC [column: CHIRALPAK (R) IC (20 mm I.D. × 250 mm), mobile phase: n-hexane/ethanol = 70/30 (V/V), temperature: 40°C] to afford 4.6 g (yield: 46%) of the marked compound, which was eluted later, as a single diastereomer and as a single enantiomer.

### (88I) N-{(3S,4S)-3-[(2,5-Difluoro[biphenyl]-3-yl)methyl]-2-azabicyclo[3.1.1]heptan-4-yl}methanesulfonamide hydrochloride

To a mixture of the compound synthesized in Example 88H (55 mg) and methanol (4 mL), a solution of hydrogen chloride in ethyl acetate (4 M, 2 mL) was added and the mixture was stirred at room temperature for 15 hours. The solvent was distilled off under reduced pressure to afford 51 mg of the crude marked compound as a single diastereomer and as a single enantiomer.

### (88J) N-[(3S,4S)-3-[(2,5-Difluoro[biphenyl]-3-yl)methyl]-2-(2-oxopropanoyl)-2-azabicyclo[3.1.1]heptan-4-yl]methanesulfonamide

To a mixture of the compound synthesized in Example 88I (51.0 mg) and N,N-dimethylformamide (2 mL), pyruvic acid (0.010 mL), O-(7-azabenzotriazol-1-yl)-N,N,N',N'-tetramethyluronium hexafluorophosphate (59 mg), and N,N-diisopropylethylamine (0.061 mL) were added at room temperature and the mixture was stirred at room temperature for 2 hours. Water was added to the reaction mixture, which was extracted with ethyl acetate. The organic layer was washed with a saturated saline solution, and dried over anhydrous sodium sulfate. After filtration, the residue obtained by distilling off the solvent under reduced pressure was purified by silica gel column chromatography [eluting solvent: n-hexane/ethyl acetate = 60/40 to 35/65 (V/V)]. Furthermore, purification by NH silica gel column chromatography [eluting solvent: n-hexane/ethyl acetate = 20/80 to 10/90 (V/V)] was performed to afford 39 mg (yield: 71%) of the marked compound as a single diastereomer and as a single enantiomer.

### (88K) N-{(3S,4S)-3-[(2,5-Difluoro[biphenyl]-3-yl)methyl]-2-[(2R)-2-hydroxy(²H₄)propanoyl]-2-azabicyclo[3.1.1]heptan-4-yl}methanesulfonamide

To a mixture of the compound synthesized in Example 88J (39.0 mg), tetrahydrofuran (0.7 mL), and methanol-d4 (0.7 mL), potassium carbonate (18 mg) was added and the mixture was stirred at room temperature for 1 hour. A mixture of methanol-d4 (0.7 mL) and acetic acid (0.015 mL) was added for neutralization, sodium borodeuteride (8 mg) was added, and the mixture was stirred at room temperature for 1 hour. A saturated aqueous ammonium chloride solution was added and the mixture was extracted with ethyl acetate. The organic layer was washed with a saturated saline solution, and dried over anhydrous sodium sulfate. After filtration, the residue obtained by distilling off the solvent under reduced pressure was purified by silica gel column chromatography [eluting solvent: n-hexane/ethyl acetate = 55/45 to 20/80 (V/V)]. Through the addition of diethyl ether, ultrasonication, and separation by filtration, 27 mg (yield: 68%) of the marked compound was obtained as a single diastereomer and as a single enantiomer.

### Example 89

### N-{(3S,4R)-5-Fluoro-3-[(2-fluoro[biphenyl]-3-yl)methyl]-2-[(2R)-2-hydroxy(²H₄)propanoyl]-2-azabicyclo[3.1.1]heptan-4-yl}methanesulfonamide

### (89A) N-[(3S,4R)-5-Fluoro-3-[(2-fluoro[biphenyl]-3-yl)methyl]-2-(2-oxopropanoyl)-2-azabicyclo[3.1.1]heptan-4-yl]methanesulfonamide

To a mixture of the compound synthesized in Example 66C (40.9 mg), pyruvic acid (0.0100 mL), triethylamine (0.0397 mL), and N,N-dimethylformamide (3 mL), O-(7-azabenzotriazol-1-yl)-N,N,N',N'-tetramethyluronium hexafluorophosphate (54.4 mg) was added at room temperature and the mixture was stirred for 3 hours. The mixture was diluted with ethyl acetate, sequentially washed with water and a saturated saline solution, and dried over sodium sulfate. After filtration, the filtrate was concentrated under reduced pressure, and the residue was purified by silica gel column chromatography [eluting solvent: n-hexane/ethyl acetate = 60/40 to 20/80 (V/V)] to afford 45.1 mg (yield: quantitative) of the marked compound as a single diastereomer and as a single enantiomer.

### (89B) N-{(3S,4R)-5-Fluoro-3-[(2-fluoro[biphenyl]-3-yl)methyl]-2-[2-hydroxy(²H₄)propanoyl]-2-azabicyclo[3.1.1]heptan-4-yl}methanesulfonamide

To a mixture of the compound synthesized in Example 89A (45.1 mg), tetrahydrofuran (0.954 mL), and methanol-d4 (0.954 mL), potassium carbonate (19.8 mg) was added at room temperature and the mixture was stirred for 1 hour. To this reaction mixture, a solution of acetic acid (0.0164 mL) in methanol-d4 (0.328 mL) and sodium borodeuteride (8.0 mg) were added at room temperature and the mixture was stirred for 1 hour. After adding an aqueous ammonium chloride solution, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 50/50 to 0/100 (V/V)] to afford 46.4 mg (yield: quantitative) of the marked compound.

### (89C) N-{(3S,4R)-5-Fluoro-3-[(2-fluoro[biphenyl]-3-yl)methyl]-2-[(2R)-2-hydroxy(²H₄)propanoyl]-2-azabicyclo[3.1.1]heptan-4-yl}methanesulfonamide

The compound synthesized in Example 89B (46.4 mg) was subjected to chiral HPLC [column: CHIRALPAK (R) IG (20 mm I.D. × 250 mm), mobile phase: n-hexane/ethanol = 50/50 (V/V), temperature: 40°C] to afford 28.4 mg (yield: 61%) of the marked compound, which was eluted later, as a single diastereomer and as a single enantiomer.

### Example 90

### N-{(3S,4R)-5-Fluoro-3-[(2-fluoro[biphenyl]-3-yl)methyl]-2-[(2R)-2-hydroxy(²H₄)propanoyl]-2-azabicyclo[3.1.1]heptan-4-yl}ethanesulfonamide

### (90A) N-[(3S,4R)-5-Fluoro-3-[(2-fluoro[biphenyl]-3-yl)methyl]-2-(2-oxopropanoyl)-2-azabicyclo[3.1.1]heptan-4-yl]ethanesulfonamide

To a mixture of the compound synthesized in Example 83E (43.8 mg), pyruvic acid (0.0105 mL), triethylamine (0.0411 mL), and N,N-dimethylformamide (3 mL), O-(7-azabenzotriazol-1-yl)-N,N,N',N'-tetramethyluronium hexafluorophosphate (56.4 mg) was added at room temperature and the mixture was stirred for 2 hours. The mixture was diluted with ethyl acetate, sequentially washed with water and a saturated saline solution, and dried over sodium sulfate. After filtration, the filtrate was concentrated under reduced pressure, and the residue was purified by silica gel column chromatography [eluting solvent: n-hexane/ethyl acetate = 60/40 to 20/80 (V/V)] to afford 47.2 mg (yield: quantitative) of the marked compound as a single diastereomer and as a single enantiomer.

### (90B) N-{(3S,4R)-5-Fluoro-3-[(2-fluoro[biphenyl]-3-yl)methyl]-2-[2-hydroxy(²H₄)propanoyl]-2-azabicyclo[3.1.1]heptan-4-yl}ethanesulfonamide

To a mixture of the compound synthesized in Example 90A (47.2 mg), tetrahydrofuran (0.989 mL), and methanol-d4 (0.989 mL), potassium carbonate (20.5 mg) was added at room temperature and the mixture was stirred for 3.5 hours. To this reaction mixture, a solution of acetic acid (0.0170 mL) in methanol-d4 (0.340 mL) was added at room temperature. Furthermore, sodium borodeuteride (8.3 mg) was added at 0°C, and the mixture was then stirred at room temperature for 2 hours. After adding an aqueous ammonium chloride solution, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 50/50 to 0/100 (V/V)] to afford 48.5 mg (yield: quantitative) of the marked compound.

### (90C) N-{(3S,4R)-5-Fluoro-3-[(2-fluoro[biphenyl]-3-yl)methyl]-2-[(2R)-2-hydroxy(²H₄)propanoyl]-2-azabicyclo[3.1.1]heptan-4-yl}ethanesulfonamide

The compound synthesized in Example 90B (48.5 mg) was subjected to chiral HPLC [column: CHIRALPAK (R) IG (20 mm I.D. × 250 mm), mobile phase: n-hexane/ethanol = 50/50 (V/V), temperature: 40°C] to afford 27.7 mg (yield: 57%) of the marked compound, which was eluted later, as a single diastereomer and as a single enantiomer.

### Example 91

### N-{3-[(2,5-Difluoro[biphenyl]-3-yl)methyl]-5-fluoro-2-[(2R)-2-hydroxypropanoyl]-2-azabicyclo[3.1.1]heptan-4-yl}ethanesulfonamide

### (91A) tert-Butyl 4-amino-3-(3-bromo-2,5-difluorobenzyl)-5-fluoro-2-azabicyclo[3.1.1]heptane-2-carboxylate

To a mixture of the compound synthesized in Example 63C (2.57 g), methanol (95 mL), and molybdenum(VI) oxide (2.47 g), lithium borohydride (about 2 M tetrahydrofuran solution) (34.3 mL) was slowly added dropwise at room temperature over 30 minutes and the mixture was stirred for 30 minutes. After adding a saturated aqueous sodium bicarbonate solution and ethyl acetate to the reaction mixture, Celite filtration was carried out. The solvent in the filtrate was concentrated under reduced pressure and extracted with ethyl acetate. The organic layer was washed with a saturated saline solution, and dried over sodium sulfate. After filtration, the solvent was distilled off under reduced pressure to afford 2.40 g of the crude marked compound.

### (91B) tert-Butyl 4-amino-3-[(2,5-difluoro[biphenyl]-3-yl)methyl]-5-fluoro-2-azabicyclo[3.1.1]heptane-2-carboxylate

To a mixture of the compound synthesized in Example 91A (400 mg), phenylboronic acid (224 mg), tripotassium phosphate (585 mg), and tetrahydrofuran (9.2 mL), (2-dicyclohexylphosphino-2',4',6'-triisopropyl-1,1'-biphenyl)[2-(2'-amino-1,1'-biphenyl)]palladium(II) methanesulfonate (77.8 mg) and water (3.1 mL) were added at room temperature and the mixture was stirred at 60°C for 3 hours in a nitrogen atmosphere. After cooling to room temperature, the mixture was diluted with ethyl acetate, sequentially washed with water and a saturated saline solution, and dried over sodium sulfate. After filtration, the solvent was distilled off under reduced pressure, and the residue was purified by silica gel column chromatography [eluting solvent: n-hexane/ethyl acetate = 60/40 to 20/80 (V/V)] to afford 251 mg (yield: 63%) of the marked compound as a single diastereomer (racemate).

### (91C) tert-Butyl 3-[(2,5-difluoro[biphenyl]-3-yl)methyl]-4-[(ethenylsulfonyl)amino]-5-fluoro-2-azabicyclo[3.1.1]heptane-2-carboxylate

To a mixture of the compound synthesized in Example 91B (250 mg) and tetrahydrofuran (0.50 mL), triethylamine (0.401 mL) and ethenesulfonyl chloride (0.161 mL) were added under ice cooling, and the mixture was then stirred at room temperature for 3 hours. The reaction mixture was diluted with ethyl acetate, washed with a saturated aqueous ammonium chloride solution, and dried over anhydrous sodium sulfate. After filtration, the solvent was distilled off under reduced pressure, and the residue was purified by silica gel column chromatography [eluting solvent: n-hexane/ethyl acetate = 100/0 to 20/80 (V/V)] to afford 185 mg (yield: 61%) of the marked compound as a single diastereomer (racemate).

### (91D) tert-Butyl 3-[(2,5-difluoro[biphenyl]-3-yl)methyl]-4-[(ethylsulfonyl)amino]-2-azabicyclo[3.1.1]heptane-2-carboxylate

To a mixture of the compound synthesized in Example 91C (161 mg) and ethyl acetate (16.3 mL), 10% palladium carbon (wetted with water) (16.3 mg) was added at room temperature and the mixture was stirred for 6 hours in a hydrogen atmosphere. After purging with nitrogen, Celite filtration was carried out. The solvent in the filtrate was distilled off under reduced pressure to afford 161 mg of the crude marked compound as a single diastereomer (racemate).

### (91E) N-{3-[(2,5-Difluoro[biphenyl]-3-yl)methyl]-5-fluoro-2-azabicyclo[3.1.1]heptan-4-yl}ethanesulfonamide hydrochloride

To the compound synthesized in Example 91D (161 mg), a solution of hydrogen chloride in 1,4-dioxane (4 M, 4.0 mL) was added at room temperature and the mixture was stirred for 3 hours. The solvent was distilled off under reduced pressure to afford 136 mg of the crude marked compound as a single diastereomer (racemate).

### (91F) (2R)-1-{3-[(2,5-Difluoro[biphenyl]-3-yl)methyl]-4-[(ethylsulfonyl)amino]-5-fluoro-2-azabicyclo[3.1.1]heptan-2-yl}-1-oxopropan-2-yl acetate

To a mixture of the compound synthesized in Example 91E (42.0 mg), (R)-2-acetoxypropanoic acid (0.0153 mL), and N,N-dimethylformamide (0.84 mL), triethylamine (0.0505 mL) and O-(7-azabenzotriazol-1-yl)-N,N,N',N'-tetramethyluronium hexafluorophosphate (52.0 mg) were added at room temperature and the mixture was stirred for 2 hours. After adding a saturated aqueous sodium bicarbonate solution, the mixture was extracted with ethyl acetate, and the organic layer was sequentially washed with water and a saturated saline solution and dried over sodium sulfate. After filtration, the solvent was distilled off under reduced pressure, and the residue was purified by preparative thin layer silica gel chromatography [eluting solvent: n-hexane/ethyl acetate = 50/50 (V/V)] to afford 20.0 mg (yield: 41%) of the marked compound as a single diastereomer and as a single enantiomer.

### (91G) N-{3-[(2,5-Difluoro[biphenyl]-3-yl)methyl]-5-fluoro-2-[(2R)-2-hydroxypropanoyl]-2-azabicyclo[3.1.1]heptan-4-yl}ethanesulfonamide

To a mixture of the compound synthesized in Example 91F (20.0 mg), tetrahydrofuran (1.00 mL), methanol (0.50 mL), and water (0.50 mL), lithium hydroxide monohydrate (31.2 mg) was added at room temperature and the mixture was stirred at room temperature for 3 hours. After adding a saturated saline solution, the mixture was extracted with ethyl acetate, and the organic layer was sequentially washed with water and a saturated saline solution and dried over sodium sulfate. After filtration, the solvent was distilled off under reduced pressure, and the residue was purified by silica gel column chromatography [eluting solvent: n-hexane/ethyl acetate = 50/50 to 0/100 (V/V)] to afford 17.1 mg (yield: 93%) of the marked compound as a single diastereomer and as a single enantiomer.

### Example 92

### N-{3-[(2,5-Difluoro[biphenyl]-3-yl)methyl]-2-[2-hydroxy(²H₄)propanoyl]-2-azabicyclo[3.1.1]heptan-4-yl}ethanesulfonamide

### (92A) tert-Butyl 3-(3-bromo-2,5-difluorobenzyl)-4-[(ethylsulfonyl)amino]-2-azabicyclo[3.1.1]heptane-2-carboxylate

To a mixture of the compound synthesized in Example 58E (400 mg) and tetrahydrofuran (10 mL), N,N-diisopropylethylamine (0.492 mL) and ethanesulfonyl chloride (0.181 mL) were added and the mixture was stirred at room temperature for 3 hours. N,N-diisopropylethylamine (0.985 mL) and ethanesulfonyl chloride (0.363 mL) were added and the mixture was stirred at room temperature for 1 hour. Water was added to the reaction mixture, which was extracted with ethyl acetate. The organic layer was washed with a saturated saline solution, and dried over anhydrous sodium sulfate. After filtration, the residue obtained by distilling off the solvent under reduced pressure was purified by silica gel column chromatography [eluting solvent: n-hexane/ethyl acetate = 70/30 to 50/50 (V/V)] to afford 344 mg (yield: 70%) of the marked compound as a single diastereomer (racemate).

### (92B) tert-Butyl 3-[(2,5-difluoro[biphenyl]-3-yl)methyl]-4-[(ethylsulfonyl)amino]-2-azabicyclo[3.1.1]heptane-2-carboxylate

To a mixture of the compound synthesized in Example 92A (344 mg), 1,2-dimethoxyethane (6 mL), and water (3 mL), phenylboronic acid (124 mg), tetrakis(triphenylphosphine)palladium(0) (39 mg), and sodium carbonate (537 mg) were added, and the mixture was stirred at 120°C for 3 hours and cooled at room temperature. Water was added to the reaction mixture, which was extracted with ethyl acetate. The organic layer was washed with a saturated saline solution, and dried over anhydrous sodium sulfate. After filtration, the residue obtained by distilling off the solvent under reduced pressure was purified by silica gel column chromatography [eluting solvent: n-hexane/ethyl acetate = 75/25 to 55/45 (V/V)] to afford 227 mg (yield: 66%) of the marked compound as a single diastereomer (racemate).

### (92C) N-{3-[(2,5-Difluoro[biphenyl]-3-yl)methyl]-2-azabicyclo[3.1.1]heptan-4-yl}ethanesulfonamide hydrochloride

To a mixture of the compound synthesized in Example 92B (104 mg) and methanol (4 mL), a solution of hydrogen chloride in 1,4-dioxane (4 M, 2 mL) was added and the mixture was stirred at room temperature for 15 hours. The solvent was distilled off under reduced pressure to afford 87 mg of the crude marked compound as a single diastereomer (racemate).

### (92D) N-{3-[(2,5-Difluoro[biphenyl]-3-yl)methyl]-2-(2-oxopropanoyl)-2-azabicyclo[3.1.1]heptan-4-yl}ethanesulfonamide

To a mixture of the compound synthesized in Example 92C (87.0 mg) and N,N-dimethylformamide (2 mL), pyruvic acid (0.017 mL), O-(7-azabenzotriazol-1-yl)-N,N,N',N'-tetramethyluronium hexafluorophosphate (97 mg), and N,N-diisopropylethylamine (0.101 mL) were added and the mixture was stirred at room temperature for 2 hours. Water was added to the reaction mixture, which was extracted with ethyl acetate. The organic layer was washed with a saturated saline solution, and dried over anhydrous sodium sulfate. After filtration, the residue obtained by distilling off the solvent under reduced pressure was purified by silica gel column chromatography [eluting solvent: n-hexane/ethyl acetate = 65/35 to 45/55 (V/V)]. Furthermore, purification by NH silica gel column chromatography [eluting solvent: n-hexane/ethyl acetate = 40/60 to 20/80 (V/V)] was performed to afford 72 mg (yield: 77%) of the marked compound as a single diastereomer (racemate).

### (92E) N-{3-[(2,5-Difluoro[biphenyl]-3-yl)methyl]-2-[2-hydroxy(²H₄)propanoyl]-2-azabicyclo[3.1.1]heptan-4-yl}ethanesulfonamide

To a mixture of the compound synthesized in Example 92D (72.0 mg), tetrahydrofuran (0.7 mL), and methanol-d4 (0.7 mL), potassium carbonate (31.3 mg) was added and the mixture was stirred at room temperature for 1 hour. A mixed solution of methanol-d4 (0.7 mL) and acetic acid (0.026 mL) was added for neutralization, sodium borodeuteride (13 mg) was added, and the mixture was stirred at room temperature for 1 hour. A saturated aqueous ammonium chloride solution was added to the reaction mixture, which was extracted with ethyl acetate. The organic layer was washed with a saturated saline solution, and dried over anhydrous sodium sulfate. After filtration, the residue obtained by distilling off the solvent under reduced pressure was purified by silica gel column chromatography [eluting solvent: n-hexane/ethyl acetate = 40/60 to 10/90 (V/V)]. Through the addition of diethyl ether, solidification by ultrasonication, and separation by filtration, 55 mg (yield: 75%) of the marked compound was obtained as a single diastereomer (racemate).

### (92F) N-{3-[(2,5-Difluoro[biphenyl]-3-yl)methyl]-2-[2-hydroxy(²H₄)propanoyl]-2-azabicyclo[3.1.1]heptan-4-yl}ethanesulfonamide

The compound synthesized in Example 92E (55 mg) was subjected to chiral HPLC [column: CHIRALPAK (R) IG (20 mm I.D. × 250 mm), mobile phase: n-hexane/ethanol = 15/85 (V/V), temperature: 40°C] to afford 25 mg (yield: 45%) of the marked compound, which was eluted first, as a single diastereomer and as a single enantiomer.

### Example 93

### N-{(3S,4S)-3-[(2-Fluoro[biphenyl]-3-yl)methyl]-2-[2-(1H-pyrazol-1-yl)propanoyl]-2-azabicyclo[3.1.1]heptan-4-yl}methanesulfonamide

### (93A) N-{(3S,4S)-3-[(2-Fluoro[biphenyl]-3-yl)methyl]-2-[2-(1H-pyrazol-1-yl)propanoyl]-2-azabicyclo[3.1.1]heptan-4-yl}methanesulfonamide

Using the compound synthesized in Example 25B (50 mg) and 2-(pyrazol-1-yl)propanoic acid (25 mg), 9.7 mg (yield: 16%) of the marked compound was obtained by the same method as in Example 31C.

### Example 94

### N-{(3S,4S)-3-[(2-Fluoro[biphenyl]-3-yl)methyl]-2-[(2R)-2-hydroxy(²H₄)propanoyl]-2-azabicyclo[3.1.1]heptan-4-yl}ethanesulfonamide

### (94A) N-[(3S,4S)-3-[(2-Fluoro[biphenyl]-3-yl)methyl]-2-(2-oxopropanoyl)-2-azabicyclo[3.1.1]heptan-4-yl]ethanesulfonamide

To a mixture of the compound synthesized in Example 80C (60.0 mg), pyruvic acid (0.0147 mL), triethylamine (0.0587 mL), and N,N-dimethylformamide (2 mL), O-(7-azabenzotriazol-1-yl)-N,N,N',N'-tetramethyluronium hexafluorophosphate (80.5 mg) was added at room temperature and the mixture was stirred for 3 hours. The mixture was diluted with ethyl acetate, sequentially washed with water and a saturated saline solution, and dried over sodium sulfate. After filtration, the filtrate was concentrated under reduced pressure, and the residue was purified by silica gel column chromatography [eluting solvent: n-hexane/ethyl acetate = 55/45 to 10/90 (V/V)] to afford 78.9 mg (yield: quantitative) of the marked compound as a single diastereomer and as a single enantiomer.

### (94B) N-{(3S,4S)-3-[(2-Fluoro[biphenyl]-3-yl)methyl]-2-[2-hydroxy(²H₄)propanoyl]-2-azabicyclo[3.1.1]heptan-4-yl}ethanesulfonamide

To a mixture of the compound synthesized in Example 94A (78.9 mg), tetrahydrofuran (1.41 mL), and methanol-d4 (1.41 mL), potassium carbonate (29.2 mg) was added at room temperature and the mixture was stirred for 1.5 hours. To this reaction mixture, a solution of acetic acid (0.0242 mL) in methanol-d4 (0.484 mL) was added at room temperature. Furthermore, sodium borodeuteride (11.8 mg) was added at 0°C, and the mixture was then stirred at room temperature for 2 hours. After adding an aqueous ammonium chloride solution, the mixture was extracted with ethyl acetate, and the organic layer was washed with a saturated saline solution and dried over sodium sulfate. After filtration, the filtrate was concentrated under reduced pressure, and the residue was purified by silica gel column chromatography [eluting solvent: n-hexane/ethyl acetate = 50/50 to 0/100 (V/V)] to afford 61.8 mg (yield: 94%) of the marked compound.

### (94C) N-{(3S,4S)-3-[(2-Fluoro[biphenyl]-3-yl)methyl]-2-[(2R)-2-hydroxy(²H₄)propanoyl]-2-azabicyclo[3.1.1]heptan-4-yl}ethanesulfonamide

The compound synthesized in Example 94B (61.8 mg) was subjected to chiral HPLC [column: CHIRALPAK (R) IG (20 mm I.D. × 250 mm), mobile phase: n-hexane/ethanol = 45/55 (V/V), temperature: 40°C] to afford 41.8 mg (yield: 68%) of the marked compound, which was eluted later, as a single diastereomer and as a single enantiomer.

### Example 95

### N-{3-[(2,5-Difluoro[biphenyl]-3-yl)methyl]-5-fluoro-2-[2-hydroxy(²H₄)propanoyl]-2-azabicyclo[3.1.1]heptan-4-yl}ethanesulfonamide

### (95A) N-{3-[(2,5-Difluoro[biphenyl]-3-yl)methyl]-5-fluoro-2-(2-oxopropanoyl)-2-azabicyclo[3.1.1] heptan-4-yl}ethanesulfonamide

To a mixture of the compound synthesized in Example 91E (88.8 mg), pyruvic acid (0.0412 mL), and N,N-dimethylformamide (1.93 mL), N,N-diisopropylethylamine (0.133 mL) and O-(7-azabenzotriazol-1-yl)-N,N,N',N'-tetramethyluronium hexafluorophosphate (220 mg) were added at room temperature and the mixture was stirred for 1 hour. The mixture was diluted with ethyl acetate, sequentially washed with water and a saturated saline solution, and dried over sodium sulfate. After filtration, the solvent was distilled off under reduced pressure, and the residue was purified by silica gel column chromatography [eluting solvent: n-hexane/ethyl acetate = 100/0 to 0/100 (V/V)] to afford 80.0 mg (yield: 84%) of the marked compound as a single diastereomer (racemate).

### (95B) N-{3-[(2,5-Difluoro[biphenyl]-3-yl)methyl]-5-fluoro-2-[2-hydroxy(²H₄)propanoyl]-2-azabicyclo[3.1.1]heptan-4-yl}ethanesulfonamide

To a mixture of the compound synthesized in Example 95A (80.0 mg), tetrahydrofuran (1.62 mL), and methanol-d4 (1.62 mL), potassium carbonate (33.5 mg) was added at room temperature and the mixture was stirred for 4 hours. After adding a solution of acetic acid (0.0305 mL) in methanol-d4 (0.305 mL), sodium borodeuteride (20.3 mg) was added at room temperature and the mixture was stirred for 1 hour. The mixture was diluted with ethyl acetate, to which an aqueous ammonium chloride solution was added. The organic layer was washed with a saturated saline solution, and then dried over sodium sulfate. After filtration, the solvent was distilled off under reduced pressure, and the residue was purified by silica gel column chromatography [eluting solvent: n-hexane/ethyl acetate = 100/0 to 0/100 (V/V)] to afford 75.3 mg (yield: 93%) of the marked compound.

### (95C) N-{3-[(2,5-Difluoro[biphenyl]-3-yl)methyl]-5-fluoro-2-[2-hydroxy(²H₄)propanoyl]-2-azabicyclo[3.1.1]heptan-4-yl}ethanesulfonamide

The compound synthesized in Example 95B (73.6 mg) was subjected to chiral HPLC [column: CHIRALPAK (R) IH (20 mm I.D. × 250 mm), mobile phase: n-hexane/2-propanol = 80/20 to 50/50 (V/V), temperature: 40°C] to elute four compounds, among which the second eluting marked compound, 21.5 mg (yield: 29%), was obtained as a single diastereomer and as a single enantiomer.

### Example 96

### N-{3-[(2,5-Difluoro[biphenyl]-3-yl)methyl]-5-fluoro-2-[(2R)-2-hydroxypropanoyl]-2-azabicyclo[3.1.1]heptan-4-yl}ethenesulfonamide

### (96A) N-{3-[(2,5-Difluoro[biphenyl]-3-yl)methyl]-5-fluoro-2-azabicyclo[3.1.1]heptan-4-yl}ethenesulfonamide hydrochloride Using the compound synthesized in Example 91C (16.9 mg), 14.9 mg of the crude marked compound was obtained as a single diastereomer (racemate) by the same method as in Example 8H.

### (96B) (2R)-1-{3-[(2,5-Difluoro[biphenyl]-3-yl)methyl]-4-[(ethenylsulfonyl)amino]-5-fluoro-2-azabicyclo[3.1.1]heptan-2-yl}-1-oxopropan-2-yl acetate

Using the compound synthesized in Example 96A (42.0 mg) and (R)-2-acetoxypropanoic acid (0.0154 mL), 22.3 mg (yield: 45%) of the marked compound was obtained by the same method as in Example 5H.

### (96C) N-{3-[(2,5-Difluoro[biphenyl]-3-yl)methyl]-5-fluoro-2-[(2R)-2-hydroxypropanoyl]-2-azabicyclo[3.1.1]heptan-4-yl}ethenesulfonamide

To a mixture of the compound synthesized in Example 96B (14.8 mg), tetrahydrofuran (1.00 mL), methanol (0.50 mL), and water (0.50 mL), lithium hydroxide monohydrate (23.1 mg) was added at room temperature and the mixture was stirred at room temperature for 3 hours. After adding a saturated saline solution, the mixture was extracted with ethyl acetate, and the organic layer was dried over sodium sulfate. After filtration, the solvent was distilled off under reduced pressure, and the residue was purified by silica gel column chromatography [eluting solvent: n-hexane/ethyl acetate = 100/0 to 0/100 (V/V)]. The obtained compound was subjected to chiral HPLC [column: CHIRALPAK (R) IG (20 mm I.D. × 250 mm), mobile phase: n-hexane/ethanol = 70/30 (V/V), temperature: 40°C] to afford 4.52 mg (yield: 33%) of the marked compound, which was eluted later, as a single diastereomer and as a single enantiomer.

### Example 97

### N-{(3S,4S)-3-[(2-Fluoro[biphenyl]-3-yl)methyl]-2-[(2R)-2-hydroxy(2-²H)propanoyl]-2-azabicyclo[3.1.1]heptan-4-yl}methanesulfonamide

### (97A) N-{(3S,4S)-3-[(2-Fluoro[biphenyl]-3-yl)methyl]-2-[(2R)-2-hydroxy(2-²H)propanoyl]-2-azabicyclo[3.1.1]heptan-4-yl}methanesulfonamide

To a mixture of the compound synthesized in Example 76A (64.0 mg) and tetrahydrofuran (2.1 mL), sodium borodeuteride (12.1 mg) was added at room temperature and the mixture was stirred at room temperature for 1 hour. The mixture was diluted with ethyl acetate, to which an aqueous ammonium chloride solution was added. The organic layer was washed with a saturated saline solution, and then dried over sodium sulfate. After filtration, the solvent was distilled off under reduced pressure, and the residue was purified by silica gel column chromatography [eluting solvent: n-hexane/ethyl acetate = 2/1 to 0/1 (V/V)] to afford 33.9 mg (yield: 53%) of the marked compound as a single diastereomer and as a single enantiomer.

### Example 98

### N-{(3S,4S)-3-[(2,5-Difluoro[biphenyl]-3-yl)methyl]-2-[(2R)-2-hydroxy(2-²H)propanoyl]-2-azabicyclo[3.1.1]heptan-4-yl}methanesulfonamide

### (98A) N-{(3S,4S)-3-[(2,5-Difluoro[biphenyl]-3-yl)methyl]-2-[(2R)-2-hydroxy(2-²H)propanoyl]-2-azabicyclo[3.1.1]heptan-4-yl}methanesulfonamide

To a mixture of the compound synthesized in Example 88J (57.1 mg) and tetrahydrofuran (1.23 mL), sodium borodeuteride (10.3 mg) was added at 0°C and the mixture was stirred at 0°C for 3 hours. An aqueous ammonium chloride solution was added and the mixture was diluted with ethyl acetate. The organic layer was washed with a saturated saline solution, and then dried over sodium sulfate. After filtration, the solvent was distilled off under reduced pressure, and the residue was purified by preparative thin layer silica gel chromatography [eluting solvent: ethyl acetate] to afford 33.1 mg (yield: 58%) of the marked compound as a single diastereomer and as a single enantiomer.

### Example 99

### N-{(3S,4R)-5-Fluoro-3-[(2-fluoro[biphenyl]-3-yl)methyl]-2-[(2R)-2-hydroxy(2-²H)propanoyl]-2-azabicyclo[3.1.1]heptan-4-yl}methanesulfonamide

### (99A) N-{(3S,4R)-5-Fluoro-3-[(2-fluoro[biphenyl]-3-yl)methyl]-2-[2-hydroxy(2-²H)propanoyl]-2-azabicyclo[3.1.1]heptan-4-yl}methanesulfonamide

To a mixture of the compound synthesized in Example 89A (437 mg) and tetrahydrofuran (10.0 mL), sodium borodeuteride (79.1 mg) was added under ice cooling and the mixture was stirred for 3 hours. An aqueous ammonium chloride solution was added and the mixture was diluted with ethyl acetate. The organic layer was washed with a saturated saline solution, and then dried over sodium sulfate. After filtration, the solvent was distilled off under reduced pressure, and the residue was purified by silica gel column chromatography [eluting solvent: n-hexane/ethyl acetate = 100/0 to 0/100 (V/V)] to afford 422 mg (yield: 96%) of the marked compound.

### (99B) N-{(3S,4R)-5-Fluoro-3-[(2-fluoro[biphenyl]-3-yl)methyl]-2-[(2R)-2-hydroxy(2-²H)propanoyl]-2-azabicyclo[3.1.1]heptan-4-yl}methanesulfonamide

The compound synthesized in Example 99A (422 mg) was subjected to chiral HPLC [column: CHIRALPAK (R) IG (20 mm I.D. × 250 mm), mobile phase: n-hexane/ethanol = 50/50 (V/V), temperature: 40°C] to afford 190 mg (yield: 45%) of the marked compound, which was eluted later, as a single diastereomer and as a single enantiomer.

### Example 100

### N-{(3S,4R)-3-[(2,5-Difluoro[biphenyl]-3-yl)methyl]-5-fluoro-2-[(2R)-2-hydroxy(2-²H)propanoyl]-2-azabicyclo[3.1.1]heptan-4-yl}methanesulfonamide

### (100A) N-{(3S,4R)-3-[(2,5-difluoro[biphenyl]-3-yl)methyl]-5-fluoro-2-[2-hydroxy(2-²H)propanoyl]-2-azabicyclo[3.1.1]heptan-4-yl}methanesulfonamide

To a mixture of the compound synthesized in Example 87A (100 mg) and tetrahydrofuran (2.08 mL), sodium borodeuteride (17.4 mg) was added under ice cooling and the mixture was stirred for 2 hours. A saturated aqueous ammonium chloride solution was added and the mixture was diluted with ethyl acetate. The organic layer was washed with a saturated saline solution, and then dried over sodium sulfate. After filtration, the solvent was distilled off under reduced pressure, and the residue was purified by silica gel column chromatography [eluting solvent: n-hexane/ethyl acetate = 50/50 to 0/100 (V/V)] to afford 38.0 mg (yield: 38%) of the marked compound.

### (100B) N-{(3S,4R)-3-[(2,5-Difluoro[biphenyl]-3-yl)methyl]-5-fluoro-2-[(2R)-2-hydroxy(2-²H)propanoyl]-2-azabicyclo[3.1.1]heptan-4-yl}methanesulfonamide

The compound synthesized in Example 100A (38.0 mg) was subjected to chiral HPLC [column: CHIRALPAK (R) IG (20 mm I.D. × 250 mm), mobile phase: n-hexane/ethanol = 70/30 (V/V), temperature: 40°C] to afford 23.3 mg (yield: 61%) of the marked compound, which was eluted later, as a single diastereomer and as a single enantiomer.

### Test Example 1

### Evaluation of OX2R agonist activity

Chinese Hamster Ovary K1 (CHO-K1) cells (GenScript) with forced expression of human OX2R were seeded onto a 384 well microplate (Corning) at 7500 cells/well and cultured for 24 hours. 20 µL of the loading buffer (25 mM HEPES containing 1.25 mM of Probenecid and 2.5 µg/mL of Fluo 4-AM at final concentration, Hank's balanced salt solution, 0.06% Pluronic F-127, Quenching buffer) in the Calcium Kit II (DOJINDO LABORATORIES) was added, and the microplate was allowed to stand for 30 minutes. A reference compound (Example435 of WO 2019/027058, N-[(2S,3S)-2-[[3-(3,5-difluorophenyl)-2-fluoro-phenyl]methyl]-1-(2-hydroxy-2-methylpropanoyl)pyrrolidin-3-yl]ethanesulfonamide) or the test compound was added in 10 µL, and changes in intracellular calcium ion concentration were measured using FDSS7000EX (Hamamatsu Photonics K.K.) as a fluorescence intensity ratio obtained upon dual wavelength excitation at 480 nm and 540 nm. Note that the reference compound or test compound was dissolved in DMSO (final concentration of DMSO was 0.5%) and diluted with an assay buffer (20 mM HEPES, Hank's balanced salt solution, 0.1% BSA). The difference between the maximum and minimum fluorescence values between 5 and 7 seconds after the addition of the test compound or reference compound was used as the indicator. The value of wells to which the assay buffer containing no test compound was added was defined as 0%, and the value of wells to which 5 µM of the reference compound was added was defined as 100%. The 50% effective concentration (EC50 value) was determined from the values obtained when various concentrations of the test compound were added. Table 1 shows the EC50 value for each compound as agonist activity.

**[Table 1]**

| Ex | hOX2R EC50 (nM) | Ex | hOX2R EC50 (nM) | Ex | hOX2R EC50 (nM) |
|---|---|---|---|---|---|
| 1 | 67 | 35 | 75 | 69 | 35 |
| 2 | 89 | 36 | 62 | 70 | 26 |
| 3 | 92 | 37 | 18 | 71 | 39 |
| 4 | 225 | 38 | 68 | 72 | 112 |
| 5 | 239 | 39 | 94 | 73 | 50 |
| 6 | 69 | 40 | 49 | 74 | 72 |
| 7 | 213 | 41 | 31 | 75 | 78 |
| 8 | 83 | 42 | 28 | 76 | 18 |
| 9 | 89 | 43 | 49 | 77 | 46 |
| 10 | 297 | 44 | 22 | 78 | 69 |
| 11 | 60 | 45 | 115 | 79 | 40 |
| 12 | 27 | 46 | 106 | 80 | 16 |
| 13 | 1145 | 47 | 117 | 81 | 43 |
| 14 | 298 | 48 | 33 | 82 | 79 |
| 15 | 303 | 49 | 20 | 83 | 19 |
| 16 | 11 | 50 | 41 | 84 | 231 |
| 17 | 108 | 51 | 33 | 85 | 275 |
| 18 | 784 | 52 | 34 | 86 | 112 |
| 19 | 380 | 53 | 19 | 87 | 30 |
| 20 | 32 | 54 | 22 | 88 | 43 |
| 21 | 217 | 55 | 591 | 89 | 18 |
| 22 | 80 | 56 | 244 | 90 | 21 |
| 23 | 34 | 57 | 38 | 91 | 39 |
| 24 | 15 | 58 | 38 | 92 | 20 |
| 25 | 24 | 59 | 88 | 93 | 23 |
| 26 | 35 | 60 | 75 | 94 | 22 |
| 27 | 61 | 61 | 40 | 95 | 19 |
| 28 | 247 | 62 | 14 | 96 | 9.9 |
| 29 | 253 | 63 | 22 | 97 | 21 |
| 30 | 148 | 64 | 41 | 98 | 36 |
| 31 | 189 | 65 | 393 | 99 | 27 |
| 32 | 535 | 66 | 57 | 100 | 43 |
| 33 | 326 | 67 | 70 | | |
| 34 | 289 | 68 | 37 | | |

### Test Example 2

### Acceleration of locomotor activity

Acceleration of locomotor activity in rodents can be an indicator of arousal. An experiment was carried out on the arousal effect of the test compound in mice, using the acceleration of locomotor activity as the indicator. For the experiment, male C57BL/6J mice (6 weeks old, THE JACKSON LABORATORY JAPAN, INC., 6 to 8 animals per group) were used. Mice were allowed to acclimate for 1 hour or longer in the rearing cage in the room where the measurement was to be performed, until the experiment. After the acclimation, the mice were placed in a locomotor activity measuring apparatus (the behavior analyzer SCANET-40 series, Melquest Ltd.) equipped with an infrared sensor to start the measurement. After 2 hours, either the solvent or the test compound (10 mg/kg of body weight) was orally administered, and the mice were immediately returned to the measuring apparatus and the measurement was further performed for 2 hours. The test compound was suspended in the 0.5 w/v% methylcellulose 400 solution (Wako Pure Chemical Industries, Ltd., Japan). The locomotor activity was counted every 5 minutes, and the cumulative counts for one hour after administration were calculated for each treatment group. The proportion of the locomotor activity of the test compound administration group to the solvent administration group whose locomotor activity was defined as 100% was calculated and shown in Table 2.

**[Table 2]**

| Test compound | Locomotor activity (%) |
|---|---|
| Solvent | 100 |
| Example 97 | 601 |
| Example 98 | 281 |
| Example 87 | 221 |
| Example 89 | 412 |

### Test Example 3

### Evaluation of ERM production from OX2R agonist

The test compound (final concentration was 500 µM, dissolved in DMSO/acetonitrile = 1/9), pooled human liver microsomes (final concentration was 2 mg/mL), L-glutathione reduced (GSH, final concentration was 0.5 mM), potassium phosphate buffer (pH 7.4, final concentration was 100 mM), and stable isotope labeled GSH (SI-GSH, final concentration was 0.5 mM) were admixed and preincubated at 37°C for 3 minutes. Thereafter, a mixed solution of glucose-6-phosphate (final concentration was 25 mM), β-nicotinamide adenine dinucleotide phosphate (final concentration was 0.25 mM), glucose-6-phosphate dehydrogenase (final concentration was 2 U/mL), and magnesium chloride (final concentration was 10 mM) was added, and incubation was performed at 37°C for 60 minutes. After the incubation, the reaction was stopped by adding a 2-fold volume of acetonitrile. A portion of the supernatant after centrifugation (about 1200 × g, 4°C, 15 minutes) was injected into a liquid chromatograph quadrupole time-of-flight mass spectrometer (Q-Tof) for analysis. The mass number of metabolites, GSH adduct and SI-GSH adduct, predicted from the chemical structural formula of the test compound was calculated, and the presence of the corresponding peaks in the measurement sample was checked. Peaks with the same retention time and approximately equal peak area of the detected peaks of the metabolites, GSH adduct and SI-GSH adduct, were extracted as pair peaks. The presence of a pair peak determined to be a metabolite GSH adduct was considered positive for ERM production potential, whereas the absence of such a peak was considered negative for ERM production potential.

The compounds described in Examples 10, 32, 58, 59, 62, 67, 74, 76, 77, 88, 92, 94, 97, and 98 were confirmed to be negative.

### Test Example 4

### Evaluation of metabolic stability of OX2R agonist

The test compound (final concentration was 1 µM, dissolved in DMSO/acetonitrile = 1/99), pooled human liver microsomes (final concentration was 0.5 mg/mL), and sodium phosphate buffer (pH 7.4, final concentration was 100 mM) were admixed and preincubated at 37°C for 15 minutes. Thereafter, a mixed solution of glucose-6-phosphate (final concentration was 10 mM), glucose-6-phosphate dehydrogenase (final concentration was 1 U/mL), and magnesium chloride (final concentration was 3.3 mM) was added and a certain amount of the mixture was collected. Then, an acetonitrile/methanol mixed solution (75/25, v/v) containing a 4.5-fold volume of internal standard substances (final concentrations: phenacetin: 1 ng/mL, niflumic acid: 15 ng/mL, furosemide: 20 ng/mL) was added to stop the reaction (0 min sample). Subsequently, β-nicotinamide adenine dinucleotide phosphate (final concentration was 1 mM) was added, and after 30 minutes of incubation at 37°C, a certain amount of the mixture was collected, to which an acetonitrile/methanol mixed solution (75/25, v/v) containing a 4.5-fold volume of the internal standard substances was added to stop the reaction (30 min sample). For both samples, a portion of the supernatant after centrifugation (about 1200 × g, 4°C, 15 minutes) was injected into a liquid chromatograph tandem quadrupole mass spectrometer (LC-MS/MS) for analysis. The metabolic stability was evaluated by comparing the test compound/internal standard substance ratio of the 30 min sample with the 0 min sample and calculating the residual ratio of the test compound.
As shown in Table 3, improved metabolic stability was observed in the compounds described in Examples 76, 87, 88, 89, 95, 97, 98, 99, and 100, which were deuterated at specific positions, compared to the corresponding compounds that were not deuterated.

**[Table 3]**

| Non-deuterated compound | Human metabolic stability | Deuterated compound | Human metabolic stability |
|---|---|---|---|
| Example | Residual ratio (%) | Example | Residual ratio (%) |
| 25 | 34 | 76 | 59 |
| | | 97 | 57 |
| 53 | 49 | 89 | 75 |
| | | 99 | 78 |
| 58 | 34 | 88 | 59 |
| | | 98 | 58 |
| 63 | 38 | 87 | 70 |
| | | 100 | 70 |
| 91 | 25 | 95 | 52 |

Table 4 below summarizes the physicochemical data for the compounds described in Examples.

**[Table 4]**

| Ex | Data |
|---|---|
| 1A | 1H-NMR (CDCl3) δ: 4.58 (2H, s), 6.82-6.88 (1H, m), 7.05-7.14 (2H, m), 7.20-7.27 (1H, m), 7.35-7.49 (2H, m). |
| 1B | 1H-NMR (CDCl3) δ: 0.29-0.38 (1H, m), 1.44-1.52 (9H, m), 2.06-2.16 (1H, m), 2.39-2.48 (1.6H, m), 2.51-2.58 (0.4H, m), 2.88-2.98 (1H, m), 3.81 (3H, s), 3.83-4.00 (1.6H, m), 4.09-4.17 (0.4H, m), 4.66-4.73 (0.4H, m), 4.84-4.91 (0.6H, m), 6.76-6.85 (1H, m), 6.95-7.04 (2H, m), 7.07-7.18 (2H, m), 7.23-7.33 (1H, m). |
| 1C | 1H-NMR (CDCl3) δ: 1.46-1.63 (7H, m), 1.69-1.78 (1H, m), 2.04 (3H, s), 2.41-2.51 (1H, m), 2.57-2.67 (1H, m), 2.97-3.06 (1H, m), 3.43-3.52 (2H, m), 4.87-4.99 (2H, m), 6.72-6.84 (1H, m), 6.98-7.08 (2H, m), 7.10-7.19 (1H, m), 7.21-7.31 (1H, m), 7.34-7.43 (1H, m). |
| 1D | MS(ESI/APCI) m/z: 461 [M + H]+ |
| 1E | MS(ESI/APCI) m/z: 537 [M - H]- |
| 1G | 1H-NMR (CDCl3) δ: 1.37-1.42 (6H, m), 1.43-1.50 (1H, m), 2.13-2.23 (1H, m), 2.31-2.45 (4H, m), 2.48-2.59 (1H, m), 2.64-2.73 (1H, m), 2.84-2.93 (1H, m), 3.30-3.41 (1H, m), 4.15-4.26 (2H, m), 4.83-4.93 (1H, m), 4.73-4.81 (1H, m), 5.16-5.26 (1H, m), 6.75-6.87 (1H, m), 7.05-7.16 (2H, m), 7.20-7.32 (2H, m), 7.73-7.84 (1H, m). |
| | MS(ESI/APCI) m/z: 497 [M + H]+ |
| 2A | MS(ESI) m/z: 475 [M + H]+ |
| 2B | MS(ESI) m/z: 461 [M + H]+ |
| 2C | MS(ESI) m/z: 461 [M + H]+ |
| 2D | MS(ESI) m/z: 568 [M + H]+ |
| 2E | 1H-NMR(CD3OD) δ: 1.23 (3H, s), 1.27 (3H, s), 1.37-1.44 (1H, m), 2.36 (2H, d, J = 4.0 Hz), 2.52-2.57 (1H, m), 2.63 (1H, s), 2.69 (6H, s), 2.85-2.90 (1H, m), 3.55-3.60 (1H, m), 4.10 (1H, d, J = 8.8 Hz), 5.22-5.28 (2H, m), 6.92-6.97 (1H, m), 7.15-7.30 (4H, m), 7.62-7.65 (1H, m). |
| | MS(ESI/APCI) m/z: 526 [M + H]+ |
| 3A | MS(ESI/APCI) m/z: 551 [M - H]- |
| 3B | 1H-NMR (CDCl3) δ: 1.02-1.11 (3H, m), 1.34-1.40 (6H, m), 1.41-1.49 (1H, m), 2.16-2.25 (1H, m), 2.31-2.40 (1H, m), 2.48-2.58 (1H, m), 2.61-2.75 (3H, m), 2.84-2.94 (1H, m), 3.34-3.45 (1H, m), 4.16-4.23 (2H, m), 4.69-4.79 (2H, m), 5.19-5.29 (1H, m), 6.76-6.86 (1H, m), 7.04-7.13 (2H, m), 7.18-7.30 (2H, m), 7.72-7.79 (1H, m). |
| | MS(ESI/APCI) m/z: 511 [M + H]+ |
| 4A | MS(ESI) m/z: 567 [M + H]+ |
| 4B | 1H-NMR (CDCl3) δ: 1.13-1.20 (6H, m), 1.33-1.37 (6H, m), 1.42-1.47 (1H, m), 2.24-2.26 (1H, m), 2.32-2.36 (1H, m), 2.52-2.54 (1H, m), 2.74-2.75 (1H, m), 2.86-2.91 (2H, m), 3.44-3.47 (1H, m), 4.18-4.23 (2H, m), 4.56-4.59 (1H, m), 4.73-4.75 (1H, m), 5.30-5.33 (1H, m), 6.80-6.82 (1H, m), 7.07-7.09 (2H, m), 7.19-7.26 (2H, m), 7.70-7.74 (1H, m). |
| | MS(ESI/APCI) m/z: 525 [M + H]+ |
| 5A | MS(ESI) m/z: 332 [M + H]+ |
| 5B | MS(ESI) m/z: 376 [M - tert-butyl + H]+ |
| 5C | MS(ESI) m/z: 447 [M + H]+ |
| 5D | MS(ESI) m/z: 377 [M - tert-butyl + H]+ |
| 5F | MS(ESI) m/z: 469 [M - tert-butyl + H]+ |
| 5G | MS(ESI/APCI) m/z: 425 [M + H]+ |
| 5H | 1H-NMR (CDCl3) δ: 0.95-0.99 (3H, m), 1.39-1.43 (1H, m), 2.08-2.16 (7H, m), 2.47-2.48 (1H, m), 2.53-2.56 (4H, m), 2.70-2.77 (1H, m), 2.91-2.95 (1H, m), 3.29-3.33 (1H, m), 4.10-4.13 (1H, m), 4.68-4.72 (1H, m), 4.77-4.80 (1H, m), 5.07-5.09 (1H, m), 6.81-6.84 (1H, m), 7.08-7.10 (2H, m), 7.20-7.24 (2H, m), 7.87-7.89 (1H, m). |
| | MS(ESI/APCI) m/z: 519 [M + H]+ |
| 6A | MS(ESI) m/z: 551 [M + Na]+ |
| 6B | MS(ESI/APCI) m/z: 429 [M + H]+ |
| 6C | MS(ESI/APCI) m/z: 557 [M + H]+ |
| 6D | 1H-NMR (CD3OD) δ: 1.27 (3H, s), 1.30 (3H, s), 1.47 (1H, t, J = 8.8 Hz), 2.28-2.46 (2H, m), 2.49-2.66 (2H, m), 2.86-2.89 (1H, m), 3.52-3.55 (1H, m), 4.25 (1H, d, J = 8.8 Hz), 5.03-5.12 (1H, m), 5.14-5.26 (2H, m), 5.32 (1H, m), 6.93-6.95 (1H, m), 7.15-7.31 (4H, m), 7.63-7.70 (1H, m). |
| | MS(ESI) m/z: 515 [M + H]+ |
| 7A | 1H-NMR (CD3OD) δ: 1.29 (3H, t, J = 7.2 Hz), 1.73-1.78 (1H, m), 2.09-2.14 (1H, m), 2.20-2.31 (2H, m), 2.65-2.68 (1H, m), 2.71 (6H, s), 2.92-2.96 (2H, m), 2.99-3.02 (1H, m), 3.20-3.25 (1H, m), 3.91-3.95 (1H, m), 4.19-4.22 (1H, m), 4.91-4.95 (1H, m), 6.94-6.99 (1H, m), 7.16-7.21 (3H, m), 7.29-7.33 (1H, m), 7.40-7.44 (1H, m). |
| | MS(ESI/APCI) m/z: 496 [M + H]+ |
| 8A | 1H-NMR (CDCl3) δ: 0.22-0.45 (1H, m), 1.44-1.52 (9H, m), 2.07-2.18 (1H, m), 2.36-2.54 (2H, m), 2.87-2.94 (1H, m), 3.76-4.09 (5H, m), 4.62-4.89 (1H, m), 6.85-6.93 (1H, m), 6.97-7.09 (1H, m), 7.37-7.46 (1H, m). |
| 8B | MS(ESI/APCI) m/z: 298 [M + H]+ |
| 8C | 1H-NMR (CDCl3) δ: 0.99-1.45 (1H, m), 1.48 (9H, s), 1.32-1.70 (1H, m), 2.37 (1H, brs), 2.52-2.64 (1H, m), 2.92-3.02 (1H, m), 3.29-3.39 (1H, m), 3.39-3.79 (1H, m), 4.55-4.91 (2H, m), 6.90-6.96 (1H, m), 7.06-7.14 (1H, m), 7.40-7.44 (1H, m). |
| | MS(ESI/APCI) m/z: 342 [M - tert-butyl + H]+ |
| 8D | MS(ESI/APCI) m/z: 313 [M - Boc + H]+ |
| 8F | 1H-NMR (CDCl3) δ: 1.22 (9H, s), 1.29-1.33 (1H, m), 2.13-2.24 (2H, m), 2.30-2.39 (2H, m), 2.85-2.95 (1H, m), 3.38-3.48 (1H, m), 3.74 (1H, d, J = 8.8 Hz), 4.46-4.56 (1H, m), 4.62-4.76 (1H, m), 6.94 (1H, t, J = 7.6 Hz), 7.30 (1H, s), 7.35-7.41 (1H, m). |
| | MS(ESI/APCI) m/z: 399 [M + H]+ |
| 8G | 1H-NMR (CDCl3) δ: 1.25-1.32 (9H, m), 1.35-1.43 (1H, m), 2.07-2.15 (1H, m), 2.24-2.34 (1H, m), 2.37-2.47 (1H, m), 2.55-2.63 (1H, m), 2.77 (3H, s), 2.85-2.94 (1H, m), 3.34 (1H, dd, J = 13.9, 7.2 Hz), 4.18-4.28 (1H, m), 4.51-4.60 (1H, m), 4.72-4.82 (1H, m), 4.90 (1H, d, J = 9.8 Hz), 6.94-7.01 (1H, m), 7.29-7.43 (2H, m). |
| | MS(ESI/APCI) m/z: 421 [M - tert-butyl + H]+ |
| 8I | MS(ESI/APCI) m/z: 505 [M + H]+ |
| 8J | MS(ESI/APCI) m/z: 539 [M + H]+ |
| 8K | 1H-NMR (CDCl3) δ: 1.39 (3H, s), 1.41 (3H, s), 1.44-1.49 (1H, m), 2.11-2.21 (1H, m), 2.30-2.40 (1H, m), 2.44 (3H, s), 2.49-2.58 (1H, m), 2.63-2.73 (1H, m), 2.89-2.99 (1H, m), 3.32-3.42 (1H, m), 4.14-4.32 (2H, m), 4.72-4.82 (1H, m), 4.87 (1H, d, J = 10.4 Hz), 5.26-5.29 (1H, m), 7.10-7.27 (5H, m), 7.75-7.86 (1 H, m) |
| | MS(ESI/APCI) m/z: 497 [M + H]+ |
| 9A | 1H-NMR (CDCl3) δ: 1.12 (3H, t, J = 7.6 Hz), 1.56 (1H, t, J = 9.6 Hz), 2.02-2.09 (1H, m), 2.12-2.18 (2H, m), 2.20-2.28 (1H, m), 2.39 (1H, dd, J = 10.4, 6.0 Hz), 2.68-2.75 (3H, m), 3.13 (1H, dd, J = 14.2, 5.6 Hz), 3.29 (1H, dd, J = 14.4, 8.4 Hz), 3.82-3.92 (4H, m), 4.10 (1H, d, J = 5.2 Hz), 4.16 (1H, t, J = 9.2 Hz), 4.87-4.93 (1H, m), 4.98 (1H, d, J = 10.4 Hz), 6.79-6.89 (1H, m), 7.07-7.16 (2H, m), 7.20-7.28 (2H, m), 7.70-7.77 (1H, m). |
| | MS(ESI/APCI) m/z: 508 [M + H]+ |
| 10A | MS(ESI/APCI) m/z: 503 [M + H]+ |
| 10B | 1H-NMR (CDCl3) δ: 1.43-1.48 (6H, m), 1.54-1.57 (1H, m), 2.17-2.22 (1H, m), 2.36-2.38 (4H, m), 2.48-2.58 (1H, m), 2.70-2.72 (1H, m), 2.86-2.97 (1H, m), 3.35-3.41 (1H, m), 4.16-4.30 (2H, m), 4.78-4.79 (1H, m), 5.02-5.05 (1H, m), 5.23-5.27 (1H, m), 7.20-7.26 (1H, m), 7.28-7.33 (1H, m), 7.35-7.40 (1H, m), 7.45-7.48 (2H, m), 7.57-7.59 (2H, m), 7.77-7.78 (1H, m). |
| | MS(ESI/APCI) m/z: 461 [M + H]+ |
| 11A | MS(ESI/APCI) m/z: 517 [M + H]+ |
| 11B | 1H-NMR (CDCl3): δ 1.40-1.51 (/H, m), 2.13- 2.22 (1H, m), 2.29-2.39 (4H, m), 2.42 (3H, s), 2.49-2.58 (1H, m), 2.65-2.75 (1H, m), 2.85-2.96 (1H, m), 3.30-3.42 (1H, m), 4.15-4.30 (2H, m), 4.73-4.83 (1H, m), 4.92-5.03(1H, m), 5.16-5.29 (1H, m), 7.17-7.26 (2H, m), 7.28-7.41 (4H, m), 7.72-7.80 (1H, m). |
| | MS(ESI/APCI) m/z: 475 [M + H]+ |
| 12B | 1H-NMR (CDCl3) δ: 1.40-1.42 (9H, m), 2.30-2.46 (1H, m), 2.52-2.67 (1H, m), 2.74-2.92 (1H, m), 3.05-3.18 (1H, m), 3.78-3.88 (3H, m), 4.42-4.43 (1H, m), 4.78-5.25 (1H, m). |
| 12C | 1H-NMR (CDCl3) δ: 1.40-1.46 (9H, m), 2.24-2.38 (1H, m), 2.68-2.87 (3H, m), 3.79-3.86 (3H, m), 4.71-5.14 (2H, m). |
| 12D | MS(ESI/APCI) m/z: 408 [M - tert-butyl + H]+ |
| 12E | MS(ESI/APCI) m/z: 474 [M - H]- |
| 12F | MS(ESI/APCI) m/z: 479 [M + H]+ |
| 12G | MS(ESI/APCI) m/z: 555 [M - H]- |
| 12H | MS(ESI/APCI) m/z: 515 [M + H]+ |
| 12I | 1H-NMR (CDCl3) δ: 1.29-1.38 (6H, m), 1.87-1.97 (1H, m), 2.37-2.47 (1H, m), 2.60-2.75 (2H, m), 2.92-2.97 (4H, m), 3.35-3.54 (2H, m), 4.33-4.43 (1H, m), 4.82-4.94 (1H, m), 5.20-5.39 (2H, m), 6.73-6.83 (1H, m), 7.03-7.25 (4H, m), 7.51-7.62 (1H, m). |
| | MS(ESI/APCI) m/z: 515 [M + H]+ |
| 13A | MS(ESI/APCI) m/z: 382, 384 [M - tert-butyl + H]+ |
| 13B | MS(ESI/APCI) m/z: 408, 410 [M + H]+ |
| 13C | MS(ESI/APCI) m/z: 408, 410 [M + H]+ |
| 13D | MS(ESI/APCI) m/z: 485 [M - H]- |
| 13E | MS(ESI/APCI) m/z: 521 [M + H]+ |
| 13F | 1H-NMR (CDCl3) δ: 1.41-1.50 (7H, m), 2.02-2.10 (1H, m), 2.26 (3H, s), 2.31-2.41 (1H, m), 2.46-2.56 (1H, m), 2.64-2.73 (1H, m), 3.04-3.22 (2H, m), 4.18-4.29 (2H, m), 4.68-4.80 (2H, m), 5.20-5.29 (1H, m), 6.74-6.84 (1H, m), 7.09-7.18 (2H, m), 7.36-7.46 (2H, m), 7.55-7.61 (1H, m), 7.65-7.69 (1H, m). |
| | MS(ESI/APCI) m/z: 479 [M + H]+ |
| 14A | MS(ESI/APCI) m/z: 533 [M + H]+ |
| 14B | 1H-NMR (CDCl3) δ: 1.43 (6H, s), 1.48-1.49 (1H, m), 2.14-2.21 (1H, m), 2.31 (3H, s), 2.34-2.42 (1H, m), 2.54-2.55 (1H, m), 2.66-2.74 (1H, m), 2.91-2.93 (1H, m), 3.36-3.39 (1H, m), 3.86 (3H, s), 4.21-4.24 (1H, m), 4.76-4.79 (1H, m), 5.00-5.03 (1H, m), 5.21-5.23 (1H, m), 6.91-6.96 (1H, m), 7.10-7.18 (2H, m), 7.20-7.25 (1H, m), 7.28-7.33 (1H, m), 7.34-7.38 (1H, m),7.78-7.81 (1H, m). |
| | MS(ESI/APCI) m/z: 491 [M + H]+ |
| 15A | 1H-NMR (CDCl3) δ: 1.31 (9H, s), 2.10 (1H, t, J = 10.0 Hz), 2.27-2.32 (1H, m), 2.42 (1H, dt, J = 11.9, 4.2 Hz), 2.58-2.64 (4H, m), 2.91-2.96 (1H, m), 3.36 (1H, dd, J = 14.1, 8.2 Hz), 4.21 (1H, t, J = 9.8 Hz), 4.57 (1H, d, J = 4.7 Hz), 4.79-4.87 (2H, m), 6.81 (1H, tt, J = 9.0, 2.3 Hz), 7.07 (1H, s), 7.09 (1H, d, J = 1.2 Hz), 7.19 (1H, d, J = 7.4 Hz), 7.24 (1H, dd, J = 7.2, 1.4 Hz), 7.49 (1H, br s). |
| | MS(ESI/APCI) m/z: 411 [M - Boc + H]+ |
| 15B | 1H-NMR (CD3OD) δ: 2.00 (1H, dd, J = 10.6, 9.0 Hz), 2.16 (1H, = 12.1, 9.4 Hz), 2.41-2.49 (2H, m), 2.87-2.94 (1H, m), 3.01 (3H, s), 3.03-3.15 (1H, m), 3.47-3.48 (1H, m), 3.85 (1H, q, J = 5.0 Hz), 4.30-4.35 (1H, m), 4.46 (1H, t, J = 6.1 Hz), 7.02 (1H, tt, J = 9.0, 2.4 Hz), 7.22 (2H, d, J = 8.6 Hz), 7.32 (1H, t, J = 7.6 Hz), 7.44-7.53 (2H, m). |
| | MS(ESI/APCI) m/z: 411 [M + H]+ |
| 15C | 1H-NMR (CDCl3) δ: 1.29 (3H, = 6.4 Hz), 1.45-1.50 (1H, m), 2.13-2.21 (1H, m), 2.37 (3H, s), 2.41-2.48 (1H, m), 2.55-2.65 (1H, m), 2.68-2.78 (1H, m), 3.00 (1H, d, J = 14.4 Hz), 3.27-3.42 (1H, m), 3.75-3.94 (1H, m), 4.20-4.24 (1H, m), 4.28-4.49 (2H, m), 5.00 (1H, d, J = 11.2 Hz), 5.10-5.21 (1H, m), 6.77-6.90 (1H, m), 7.14 (2H, d, J = 7.6 Hz), 7.22-7.28 (2H, m), 7.77-7.92 (1H, m). |
| | MS(ESI/APCI) m/z: 483 [M + H]+ |
| 16A | 1H-NMR (CDCl3) δ: 1.13-1.44 (3H, m), 1.46-1.52 (1H, m), 2.19-2.26 (1H, m), 2.38-2.44 (1H, m), 2.51-2.56 (1H, m), 2.60 (3H, s), 2.70-2.71 (1H, m), 2.90-2.97 (1H, m), 3.33-3.39 (1H, m), 4.20-4.31 (3H, m), 4.92-4.93 (1H, m), 5.25-5.36 (1H, m), 6.80-6.84 (1H, m), 7.08-7.10 (2H, m), 7.18-7.27 (2H, m), 7.61-7.65 (1H, m). |
| | MS(ESI/APCI) m/z: 483 [M + H]+ |
| 17A | 1H-NMR (CDCl3) δ: 1.50-1.53 (1H, m), 1.82-1.96 (2H, m), 1.99-2.08 (1H, m), 2.10-2.25 (2H, m), 2.32-2.39 (1H, m), 2.39-2.45 (3H, m), 2.48-2.59 (1H, m), 2.66-2.69 (1H, m), 2.92-2.95 (1H, m), 3.33 (1H, dd, J = 14.1, 9.8 Hz), 3.78-3.95 (2H, m), 4.17-4.20 (1H, m), 4.48-4.64 (2H, m), 4.97-5.00 (1H, m), 5.16-5.20 (1H, m), 6.81-6.85 (1H, m), 7.10-7.15 (2H, m), 7.20-7.28 (2H, m), 7.77-7.91 (1H, m). |
| | MS(ESI/APCI) m/z: 509 [M + H]+ |
| 18A | MS(ESI/APCI) m/z: 571 [M + H]+ |
| 18B | 1H-NMR(CDCl3) δ: 1.42 (3H, s), 1.43 (3H, s), 1.46-1.49 (1H, m), 2.16-2.21 (1H, m), 2.34-2.41 (4H, m), 2.51-2.57 (1H, m), 2.67-2.71 (1H, m), 2.89-2.94 (1H, m), 3.35-3.41 (1H, m), 4.18-4.24 (2H, m), 4.76-4.80 (1H, m), 4.97 (1H, d, J = 11.2 Hz), 5.21-5.27 (1H, m), 7.23-7.31 (2H, m), 7.54-7.61 (1H, m), 7.66-7.69 (1H, m), 7.76-7.82 (3 H, m). |
| | MS(ESI/APCI) m/z: 529 [M + H]+ |
| 19A | 1H-NMR (CDCl3) δ: 1.07-1.20 (2H, m), 1.23-1.35 (2H, m), 1.51-1.56 (1H, m), 2.19-2.21 (1H, m), 2.37-2.45 (4H, m), 2.55-2.56 (1H, m), 2.68-2.70 (1H, m), 3.01-3.02 (1H, m), 3.37-3.38 (1H, m), 4.23-4.25 (1H, m), 4.87-4.89 (1H, m), 4.98 (1H, d, J = 8.0 Hz), 5.10-5.11 (1H, m), 6.81-6.82 (1H, m), 7.07-7.15 (2H, m), 7.19-7.27 (2H, m), 7.70-7.82 (1H, m). |
| | MS(ESI/APCI) m/z: 497 [M + H]+ |
| 20A | 1H-NMR (CDCl3): δ 0.72-0.88 (3H, m), 0.97-1.08 (1H, m), 1.58-1.69 (2H, m), 2.14-2.24 (1H, m), 2.28-2.45 (4H, m), 2.49-2.56 (1H, m), 2.65-2.73 (1H, m), 2.90-3.01 (1H, m), 3.26-3.36 (1H, m), 4.12-4.22 (1H, m), 4.76-4.94 (2H, m), 5.12-5.21 (1H, m), 6.78-6.84 (1H, m), 7.07-7.12 (2H, m), 7.18-7.24 (2H, m), 7.77-7.86 (1H, m). |
| | MS(ESI/APCI) m/z: 479 [M + H]+ |
| 21A | MS(ESI/APCI) m/z: 425 [M - Boc + H]+ |
| 21B | MS(ESI/APCI) m/z: 510 [M + H]+ |
| 21C | MS(ESI/APCI) m/z: 410 [M + H]+ |
| 21D | MS(ESI/APCI) m/z: 538 [M + H]+ |
| 21E | 1H-NMR (CDCl3) δ: 1.36-1.39 (6H, m), 1.43-1.48 (1H, m), 2.17-2.22 (1H, m), 2.35-2.37 (1H, m), 2.46-2.58 (4H, m), 2.68-2.69 (1H, m), 2.95-2.97 (1H, m), 3.32-3.36 (1H, m), 4.20-4.25 (1H, m), 4.29-4.40 (1H, m), 4.92-4.95 (1H, m), 5.01-5.02 (1H, m), 5.23-5.26 (1H, m), 7.27-7.34 (2H, m), 7.66-7.83 (4H, m). |
| | MS(ESI/APCI) m/z: 496 [M + H]+ |
| 22A | 1H-NMR (CDCl3) δ: 2.3/-2.41 (3H, m), 6.84-6.91 (1H, m), 7.15-7.20 (1H, m), 7.52-7.60 (2H, m), 8.33-8.39 (1H, m). |
| 22B | 1H-NMR (CDCl3) δ: 4.50-4.53 (2H, m), 6.86-6.95 (1H, m), 7.36-7.41 (1H, m), 7.54-7.63 (2H, m), 8.47-8.54 (1H, m). |
| 22C | MS(ESI/APCI) m/z: 491 [M + H]+ |
| 22D | MS(ESI/APCI) m/z: 433 [M + H]+ |
| 22E | MS(ESI/APCI) m/z: 365 [M + Na]+ |
| 22F | MS(ESI/APCI) m/z: 401 [M + H]+ |
| 22G | MS(ESI/APCI) m/z: 416 [M + H]+ |
| 22H | MS(ESI/APCI) m/z: 402 [M + H]+ |
| 22I | MS(ESI/APCI) m/z: 480 [M + H]+ |
| 22J | 1H-NMR (CDCl3) δ: 0.66-0.84 (3H, m), 0.92-1.07 (1H, m), 1.48-1.52 (1H, m), 2.13-2.24 (1H, m), 2.40-2.74 (6H, m), 2.86-3.11 (1H, m), 3.27-3.47 (1H, m), 4.13-4.32 (1H, m), 4.71-4.92 (2H, m), 5.20 (1H, brs), 6.78-6.95 (1H, m), 7.48-7.58 (2H, m), 7.69 (1H, brs), 8.42 (1H, d, J = 4.8 Hz). |
| | MS(ESI/APCI) m/z: 480 [M + H]+ |
| 23A | MS(ESI/APCI) m/z: 376, 374 [M - Boc + H]+ |
| 23B | 1H-NMR (CDCl3) δ: 1.49 (9H, s), 2.16-2.50 (3H, m), 2.61-2.71 (1H, m), 3.29-3.38 (1H, m), 3.51-3.80 (1H, m), 4.63-4.89 (2H, m), 6.91-6.97 (1H, m), 7.02-7.12 (1H, m), 7.39-7.50 (1H, m). |
| 23C | MS(ESI/APCI) m/z: 433, 431 [M + H]+ |
| 23D | MS(ESI/APCI) m/z: 319, 317 [M - Boc +H]+ |
| 23E | 1H-NMR (CDCl3) δ: 1.23 (9H, s), 1.84 (1H, t, J = 9.5 Hz), 2.34-2.41 (1H, m), 2.52-2.63 (2H, m), 2.85-2.93 (1H, m), 2.96 (3H, s), 3.39 (1H, dd, J = 14.1, 6.7 Hz), 4.33-4.38 (1H, m), 4.51-4.59 (1H, m), 4.78-4.83 (2H, m), 6.97 (1H, t, J = 8.3 Hz), 7.24-7.26 (1H, m), 7.38-7.42 (1H, m). |
| | MS(ESI/APCI) m/z: 397, 395 [M - Boc + H]+ |
| 23F | 1H-NMR (CDCl3) δ: 1.25 (9H, s), 1.84 (1H, t, J = 9.2 Hz), 2.32-2.38 (1H, m), 2.50-2.60 (2H, m), 2.92 (3H, s), 2.96 (1H, dd, J = 14.1, 8.6 Hz), 3.41 (1H, dd, J = 14.1, 7.4 Hz), 4.36 (1H, t, J = 9.2 Hz), 4.55 (1H, td, J = 12.4, 6.5 Hz), 4.73 (1H, d, J = 8.6 Hz), 4.84-4.90 (1H, m), 6.78 (1H, t, J = 8.9 Hz), 7.05-7.07 (2H, m), 7.13-7.18 (1H, m), 7.21-7.23 (1H, m), 7.36-7.40 (1H, m). |
| | MS(ESI/APCI) m/z: 429 [M - Boc + H]+ |
| 23G | MS(ESI/APCI) m/z: 429 [M + H]+ |
| 23H | MS(ESI/APCI) m/z: 543 [M + H]+ |
| 23I | MS(ESI/APCI) m/z: 501 [M + H]+ |
| 23J | 1H-NMR (CDCl3) δ: 1.05 (3H, d, J = 6.7 Hz), 1.96 (1H, t, J = 9.8 Hz), 2.46-2.52 (1H, m), 2.67-2.74 (2H, m), 2.93 (3H, s), 2.93-3.00 (1H, m), 3.38-3.43 (2H, m), 4.17-4.31 (2H, m), 4.37-4.42 (1H, m), 4.90 (1H, d, J = 8.6 Hz), 5.37-5.39 (1H, m), 6.77-6.83 (1H, m), 7.06-7.08 (2H, m), 7.17-7.23 (2H, m), 7.49-7.53 (1H, m). |
| | MS(ESI/APCI) m/z: 501 [M + H]+ |
| 24A | MS(ESI/APCI) m/z: 547 [M + Na]+ |
| 24B | MS(ESI/APCI) m/z: 425 [M + H]+ |
| 24C | 1H-NMR (CDCl3) δ: 1.15 (3H, = 6.8 Hz), 1.45-1.49 (1H, m), 2.17-2.24 (1H, m), 2.36-2.41 (1H, m), 2.48-2.52 (1H, m), 2.53 (3H, s), 2.68-2.71 (1H, m), 2.89-2.96 (1H, m), 3.32-3.39 (1H, m), 3.59 (1H, d, J = 7.2 Hz), 4.19-4.30 (3H, m), 4.87-4.95 (1H, m), 5.24-5.34 (1H, m), 6.55 (1H, t, J = 16.4 Hz), 7.21-7.25 (1H, m), 7.27-7.31 (1H, m), 7.51-7.55 (2H, m), 7.61-7.69 (3H, m). |
| | MS(ESI/APCI) m/z: 497 [M + H]+ |
| 25A | 1H-NMR (CDCl3) δ: 1.34 (9H, s), 1.36-1.43 (1H, m), 2.06-2.13 (1H, m), 2.25-2.32 (1H, m), 2.37-2.45 (1H, m), 2.53 (3H, s), 2.57-2.64 (1H, m), 2.95 (1H, dd, J = 14.1, 6.1 Hz), 3.36 (1H, dd, J = 14.1, 8.6 Hz), 4.18-4.25 (1H, m), 4.54-4.90 (3H, m), 7.15-7.20 (1H, m), 7.26-7.31 (1H, m), 7.33-7.40 (1H, m), 7.41-7.57 (5H, m). |
| | MS(ESI/APCI) m/z: 375 [M - Boc + H]+ |
| 25B | 1H-NMR (DMSO-D6) δ: 2.01-2.27 (4H, m), 2.66-2.77 (1H, m), 2.95 (3H, s), 3.12 (1H, dd, J = 14.7, 9.8 Hz), 3.20-3.29 (1H, m), 3.72-3.80 (1H, m), 4.19-4.32 (2H, m), 7.25-7.32 (1H, m), 7.39-7.53 (5H, m), 7.55-7.61 (3H, m), 8.89-9.00 9.15 |
| | (1H, m), (1H, s). MS(ESI/APCI) m/z: 375 [M + H]+ |
| 25C | 1H-NMR (CDCl3) δ: 1.15-1.17 (3H, m), 1.48-1.51 (1H, m), 2.20-2.25 (1H, m), 2.36-2.40 (1H, m), 2.49 (3H, s), 2.50-2.54 (1H, m), 2.68-2.72 (1H, m), 2.90-2.96 (1H, m), 3.30-3.36 (1H, m), 3.58-3.65 (1H, m), 4.22-4.31 (3H, m), 4.90-4.96 (1H, m), 5.26-5.32 (1H, m), 7.20-7.23 (1H, m), 7.27-7.31 (1H, m), 7.36-7.39 (1H, m), 7.39-7.46 (2H, m), 7.50-7.55 (2H, m), 7.59-7.63 (1H, m) |
| | MS(ESI/APCI) m/z: 447 [M + H]+ |
| 26A | 1H-NMR (CDCl3) δ: 0.89 (3H, t, J = 7.2 Hz), 1.21-1.27 (1H, m), 1.47-1.56 (2H, m), 2.16-2.26 (1H, m), 2.38-2.43 (1H, m), 2.49-2.54 (1H, m), 2.56 (3H, s), 2.67-2.74 (1H, m), 2.88-2.98 (1H, m), 3.29-3.39 (1H, m), 3.42-3.52 (1H, m), 4.08-4.16 (1H, m), 4.20-4.25 (1H, m), 4.25-4.33 (1H, m), 4.95 (1H, d, J = 11.2 Hz), 5.25-5.37 (1H, m), 6.76-6.88 (1H, m), 7.07-7.13 (2H, m), 7.19-7.27 (2H, m), 7.62-7.69 (1H, m). |
| | MS(ESI/APCI) m/z: 497 [M + H]+ |
| 27A | 1H-NMR (CDCl3): δ 1.44-1.48 (1H, m), 2.11-2.31 (1H, m), 2.31-2.38 (1H, m), 2.38-2.52 (1H, m), 2.55 (3H, s), 2.61-2.65 (1H, m), 2.67 (3H, d, J = 4.4 Hz), 3.01-3.11 (1H, m), 3.34-3.44 (1H, m), 4.09 (1H, d, J = 4.4 Hz), 4.14-4.20 (1H, m), 4.20-4.32 (1H, m), 4.80-4.94 (2H, m), 6.76-6.88 (1H, m), 7.07-7.13 (2H, m), 7.19-7.27 (2H, m), 7.62-7.74 (1H, m). |
| | MS(ESI/APCI) m/z: 468 [M + H]+ |
| 28A | 1H-NMR (CDCl3) δ: 1.42-1.47 (1H, m), 2.10-2.16 (1H, m), 2.34-2.37 (1H, m), 2.39 (3H, s), 2.49-2.54 (1H, m), 2.65-2.69 (1H, m), 2.92-2.98 (1H, m), 3.28-3.34 (1H, m), 3.35 (3H, s), 4.00-4.06 (2H, m), 4.13-4.19 (1H, m), 4.36-4.39 (1H, m, ), 4.98 (1H, d, J = 10.8 Hz), 5.15-5.21 (1H, m), 6.78-6.83 (1H, m), 7.09-7.13 (2H, m), 7.19-7.25 (2H, m), 7.79-7.83 (1H, m). |
| | MS(ESI/APCI) m/z: 483 [M + H]+ |
| 29A | 1H-NMR (CDCl3) δ: 2.0/-2.23 (2H, m), 2.31-2.48 (5H, m), 2.57 (1H, br s), 2.70 (1H, s), 2.89 (1H, d, J = 13.7 Hz), 3.30 (1H, t, J = 11.5 Hz), 4.17 (1H, t, J = 10.2 Hz), 4.72 (2H, s), 4.89 (1H, br s), 4.96 (1H, d, J = 10.6 Hz), 5.17 (1H, br s), 6.81 (1H, tt, J = 9.0, 2.3 Hz), 7.09 (2H, d, J = 5.1 Hz), 7.17-7.25 (2H, m), 7.71 (1H, br s). |
| | MS(ESI/APCI) m/z: 497 [M + H]+ |
| 30A | 1H-NMR (CDCl3) δ: 2.08-2.25 (2H, m), 2.32-2.42 (4H, m), 2.44 (1H, br s), 2.56 (1H, s), 2.70 (1H, s), 2.92 (1H, d, J = 16.0 Hz), 3.33 (1H, t, J = 11.9 Hz), 4.16 (1H, dd, J = 13.5, 8.0 Hz), 4.74 (2H, br s), 4.91 (2H, d, J = 10.2 Hz), 5.13 (1H, br s), 6.82 (1H, t, J = 9.0 Hz), 7.11 (2H, d, J = 7.0 Hz), 7.18-7.27 (2H, m), 7.73 (1H, br s). |
| | MS(ESI/APCI) m/z: 497 [M + H]+ |
| 31A | MS(ESI/APCI) m/z: 418 [M - Boc + H]+ |
| 31B | MS(ESI/APCI) m/z: 418 [M + H]+ |
| 31C | MS(ESI/APCI) m/z: 532 [M + H]+ |
| 31D | 1H-NMR (CDCl3) δ: 1.12 (3H, d, J = 6.1 Hz), 1.49 (1H, t, J = 9.8 Hz), 2.18-2.26 (1H, m), 2.38-2.46 (1H, m), 2.50-2.58 (1H, m), 2.64-2.73 (1H, m), 2.65 (3H, s), 2.91 (1H, dd, J = 13.5, 6.1 Hz), 3.38 (1H, dd, J = 14.1, 8.6 Hz), 3.55 (1H, d, J = 8.0 Hz), 4.18-4.32 (3H, m), 4.89 (1H, d, J = 10.4 Hz), 5.26-5.35 (1H, m), 7.20-7.29 (2H, m), 7.33-7.39 (1H, m), 7.53-7.59 (1H, m), 7.62-7.69 (2H, m). |
| | MS(ESI/APCI) m/z: 490 [M + H]+ |
| 32A | MS(ESI/APCI) m/z: 479 [M + H]+ |
| 32B | MS(ESI/APCI) m/z: 379 [M + H]+ |
| 32C | MS(ESI/APCI) m/z: 493 [M + H]+ |
| 32D | 1H-NMR (CDCl3) δ: 1.20 (3H, d, J = 6.7 Hz), 1.48 (1H, t, J = 9.5 Hz), 2.18 (1H, t, J = 10.1 Hz), 2.35-2.46 (1H, m), 2.43 (3H, s), 2.47-2.54 (1H, m), 2.71 (1H, q, J = 5.7 Hz), 2.86-2.97 (1H, m), 3.32 (1H, dd, J = 14.7, 9.2 Hz), 3.65 (1H, d, J = 7.4 Hz), 3.96 (3H, s), 4.23 (1H, t, J = 9.8 Hz), 4.27-4.35 (2H, m), 4.95 (1H, d, J = 11.0 Hz), 5.18-5.27 (1H, m), 6.64-6.71 (1H, m), 7.19 (1H, t, J = 7.7 Hz), 7.37-7.43 (1H, m), 7.59 (1H, t, J = 7.1 Hz), 7.82 (1H, t, J = 7.7 Hz). |
| | MS(ESI/APCI) m/z: 451 [M + H]+ |
| 33A | 1H-NMR (CDCl3) δ: 1.27-1.35 (2H, m), 1.42-1.55 (2H, m), 1.60-1.69 (1H, m), 2.18-2.25 (1H, m), 2.44-2.52 (1H, m), 2.50 (3H, s), 2.64-2.76 (2H, m), 2.91 (1H, dd, J = 14.1, 4.3 Hz), 3.37 (1H, dd, J = 14.4, 9.5 Hz), 4.23 (1H, t, J = 9.8 Hz), 4.84-4.91 (1H, m), 4.96 (1H, q, J = 4.9 Hz), 5.04-5.13 (1H, m), 6.78-6.86 (1H, m), 7.07-7.14 (2H, m), 7.18-7.25 (2H, m), 7.63-7.70 (1H, m). |
| | MS(ESI/APCI) m/z: 504 [M + H]+ |
| 34A | MS(ESI/APCI) m/z: 511 [M + H]+ |
| 34B | 1H-NMR (CDCl3) δ: 1.43-1.49 (1H, m), 2.15-2.20 (1H, m), 2.38-2.44 (1H, m), 2.51 (3H, s), 2.53-2.58 (1H, m), 2.68-2.73 (1H, m), 2.95-3.02 (1H, m), 3.32-3.42 (1H, m), 3.51 (1H, brs), 3.98-4.15 (3H, m), 4.18-4.24 (1H, m), 4.97 (1H, d, J = 10.0 Hz), 5.16-5.26 (1H, m), 6.78-6.88 (1H, m), 7.09-7.12 (2H, m), 7.20-7.27 (2H, m), 7.66-7.76 (1H, m). |
| | MS(ESI/APCI) m/z: 469 [M + H]+ |
| 35A | 1H-NMR (CDCl3) δ: 0.91 (3H, d, J = 6.0 Hz), 0.99 (3H, d, J = 6.0 Hz), 1.45-1.49 (1H, m), 2.14-2.22 (1H, m), 2.29-2.37 (1H, m), 2.41-2.49 (1H, m), 2.59-2.65 (1H, m), 2.67 (3H, s), 3.00-3.12 (1H, m), 3.37-3.47 (1H, m), 3.72-3.83 (2H, m), 4.16-4.21 (1H, m), 4.26-4.34 (1H, m), 4.68-4.81 (1H, m), 4.89 (1H, d, J = 10.4 Hz), 6.77-6.87 (1H, m), 7.02-7.12 (2H, m), 7.18-7.25 (1H, m), 7.27-7.33 (1H, m),7.57-7.64 (1H, m). |
| | MS(ESI/APCI) m/z: 496 [M + H]+ |
| 36A | MS(ESI/APCI) m/z: 497 [M + H]+ |
| 36B | 1H-NMR (CDCl3) δ: 0.63-0.91 (4H, m), 1.24-1.28 (1H, m), 1.88-1.96 (1H, m), 2.45-2.51 (1H, m), 2.65-2.73 (2H, m), 2.82-3.08 (4H, m), 3.38-3.49 (1H, m), 4.35-4.39 (1H, m), 4.79-4.89 (2H, m), 5.10-5.20 (1H, m), 6.77-6.83 (1H, m), 7.06-7.08 (2H, m), 7.16-7.25 (2H, m), 7.48-7.58 (1H, m). |
| | MS(ESI/APCI) m/z: 497 [M + H]+ |
| 37A | 1H-NMR (CDCl3) δ: 0.93 (3H, t, J = 7.2 Hz), 1.45-1.49 (1H, m), 2.14-2.23 (1H, m), 2.27-2.37 (1H, m), 2.40-2.52 (1H, m), 2.60-2.64 (4H, m), 2.97-3.13 (3H, m), 3.38-3.48 (1H, m), 3.93 (1H, brs), 4.16-4.24 (1H, m), 4.28-4.39 (1H, m), 4.67-4.80 (1H, m), 4.92-5.03 (1H, m), 7.16-7.24 (1H, m), 7.28-7.40 (2H, m), 7.42-7.46 (2H, m), 7.48-7.62 (3H, m). |
| | MS(ESI/APCI) m/z: 446 [M + H]+ |
| 38A | MS(ESI/APCI) m/z: 555 [M + H]+ |
| 38B | MS(ESI/APCI) m/z: 455 [M + H]+ |
| 38C | 1H-NMR (CDCl3) δ: 1.13 (3H, d, J = 6.8 Hz), 1.45-1.47 (1H, m), 2.18-2.25 (1H, m), 2.34-2.37 (1H, m), 2.49-2.53 (1H, m), 2.57 (3H, s), 2.67-2.71 (1H, m), 2.93-2.99 (1H, m), 3.33-3.38 (1H, m), 3.53-3.64 (1H, m), 4.23-4.27 (3H, m), 4.82-4.88 (1H, m), 5.23-5.36 (1H, m), 7.07-7.11 (1H, m), 7.12-7.16 (1H, m), 7.17-7.25 (2H, m), 7.31-7.36 (1H, m), 7.63-7.72 (1H, m). |
| | MS(ESI/APCI) m/z: 527 [M + H]+ |
| 39A | 1H-NMR (CDCl3) δ: 0.04-0.13 (2H, m), 0.36-0.45 (2H, m), 0.75-0.87 (1H, m), 1.49-1.53 (1H, m), 2.17-2.22 (1H, m), 2.32-2.39 (1H, m), 2.44-2.50 (1H, m), 2.61 (3H, s), 2.61-2.67 (1H, m), 2.87-3.01 (2H, m), 3.04-3.12 (1H, m), 3.35-3.45 (1H, m), 4.13-4.27 (2H, m), 4.29-4.35 (1H, m), 4.84 (1H, d, J = 7.6 Hz), 4.87-4.95 (1H, m), 6.78-6.86 (1H, m), 7.05-7.13 (2H, m), 7.20-7.25 (1H, m), 7.26 (1H, d, J = 2.0 Hz),7.62-7.68 (1H, m). |
| | MS(ESI/APCI) m/z: 508 [M + H]+ |
| 40A | MS(ESI/APCI) m/z: 393 [M - Boc + H]+ |
| 40B | MS(ESI/APCI) m/z: 393 [M + H]+ |
| 40C | MS(ESI/APCI) m/z: 507 [M + H]+ |
| 40D | 1H-NMR (CDCl3) δ: 1.15 (3H, d, J = 6.8 Hz), 1.46-1.50 (1H, m), 2.18-2.25 (1H, m), 2.37-2.43 (1H, m), 2.49-2.57 (4H, m), 2.66-2.72 (1H, m), 2.89-2.97 (1H, m), 3.27-3.42 (1H, m), 3.62 (1H, d, J = 7.2 Hz), 4.19-4.31 (3H, m), 4.93 (1H, d, J = 10.4 Hz), 5.25-5.35 (1H, m), 7.03-7.11 (1H, m), 7.19-7.24 (1H, m), 7.28-7.35 (3H, m), 7.36-7.42 (1H, m), 7.60-7.67 (1H, m). |
| | MS(ESI/APCI) m/z: 465 [M + H]+ |
| 41A | MS(ESI/APCI) m/z: 415 [M - Boc + H]+ |
| 41B | MS(ESI/APCI) m/z: 415 [M + H]+ |
| 41C | MS(ESI/APCI) m/z: 529 [M + H]+ |
| 41D | 1H-NMR (CDCl3) δ: 0.73-0.78 (2H, m), 0.96-1.02 (2H, m), 1.17-1.19 (3H, m), 1.48-1.51 (1H, m), 1.90-1.98 (1H, m), 2.17-2.24 (1H, m), 2.37-2.39 (1H, m), 2.47 (3H, s), 2.49-2.54 (1H, m), 2.71-2.72 (1H, m), 2.93-2.95 (1H, m), 3.33-3.37 (1H, m), 3.63-3.65 (1H, m), 4.19-4.32 (3H, m), 5.02-5.05 (1H, m), 5.27-5.31 (1H, m), 7.07-7.09 (1H, m), 7.17-7.22 (1H, m), 7.23-7.27 (1H, m), 7.27-7.37 (3H, m),7.62-7.63 (1H, m). |
| | MS(ESI/APCI) m/z: 487 [M + H]+ |
| 42A | MS(ESI/APCI) m/z: 459 [M - Boc + H]+ |
| 42B | MS(ESI/APCI) m/z: 459 [M + H]+ |
| 42C | 1H-NMR (DMSO) δ: 0.84-0.89 (3H, m), 1.28-1.37 (1H, m), 2.22-2.29 (2H, m), 2.37-2.45 (2H, m), 2.82-2.89 (4H, m), 3.98-4.22 (3H, m), 4.34-4.39 (1H, m), 4.46-4.58 (1H, m), 5.07-5.15 (1H, m), 7.12-7.18 (1H, m), 7.28-7.34 (1H, m), 7.37-7.42 (1H, m), 7.43-7.48 (1H, m), 7.50-7.53 (1H, m), 7.55-7.64 (2H, m). |
| | MS(ESI/APCI) m/z: 531 [M + H]+ |
| 43A | MS(ESI/APCI) m/z: 516 [M + H]+ |
| 43B | MS(ESI/APCI) m/z: 416 [M + H]+ |
| 43C | MS(ESI/APCI) m/z: 530 [M + H]+ |
| 43D | 1H-NMR (CDCl3) δ: 1.00-1.02 (2H, m), 1.05-1.11 (2H, m), 1.18-1.20 (3H, m), 1.46-1.51 (1H, m), 2.06-2.13 (1H, m), 2.16-2.24 (1H, m), 2.39-2.41 (1H, m), 2.47-2.54 (4H, m), 2.71-2.72 (1H, m), 2.92-2.94 (1H, m), 3.34-3.36 (1H, m), 3.58-3.76 (1H, m), 4.20-4.33 (3H, m), 5.02-5.04 (1H, m), 5.25-5.29 (1H, m), 7.08-7.09 (1H, m), 7.22-7.26 (1H, m), 7.50-7.59 (3H, m), 7.76-7.78 (1H, m). |
| | MS(ESI/APCI) m/z: 488 [M + H]+ |
| 44B | MS(ESI/APCI) m/z: 401 [M - Boc + H]+ |
| 44C | MS(ESI/APCI) m/z: 401 [M + H]+ |
| 44D | MS(ESI/APCI) m/z: 515 [M + H]+ |
| 44E | 1H-NMR (CDCl3) δ: 0.73-0.86 (2H, m), 0.99-1.09 (2H, m), 1.12 (3H, d, J = 6.1 Hz), 1.42-1.50 (1H, m), 1.80-1.90 (1H, m), 2.19-2.27 (1H, m), 2.33-2.43 (1H, m), 2.45-2.54 (1H, m), 2.69-2.77 (1H, m), 2.87-2.98 (1H, m), 3.41 (1H, dd, J = 14.1, 8.6 Hz), 3.58 (1H, d, J = 7.4 Hz), 4.18-4.32 (3H, m), 4.83 (1H, d, J = 10.4 Hz), 5.27-5.38 (1H, m), 7.18 (1H, t, J = 7.7 Hz), 7.27-7.31 (1H, m), 7.33-7.40 (1H, m), 7.41-7.47 (2H, m), 7.51-7.60 (3H, m). |
| | MS(ESI/APCI) m/z: 473 [M + H]+ |
| 45A | 1H-NMR (CDCl3) δ: 0.54-0.66 (2H, m), 0.83-1.10 (3H, m), 1.42-1.50 (2H, m), 1.77-1.93 (2H, m), 2.11-2.19 (1H, m), 2.36-2.44 (1H, m), 2.48-2.56 (1H, m), 2.69-2.76 (1H, m), 2.96 (1H, d, J = 14.1 Hz), 3.32-3.44 (1H, m), 4.12-4.19 (1H, m), 4.63-4.85 (2H, m), 4.88-4.96 (1H, m), 5.25-5.36 (1H, m), 7.15-7.21 (1H, m), 7.22-7.26 (1H, m), 7.33-7.40 (1H, m), 7.42-7.48 (2H, m), 7.56 (2H, d, J = 8.0 Hz), 7.76-7.83 (1H, m). |
| | MS(ESI/APCI) m/z: 487 [M + H]+ |
| 46B | MS(ESI/APCI) m/z: 417 [M + H]+ |
| 46C | MS(ESI/APCI) m/z: 519 [M + Na]+ |
| 46D | MS(ESI/APCI) m/z: 427 [M - Boc + H]+ |
| 46E | MS(ESI/APCI) m/z: 427 [M + H]+ |
| 46F | 1H-NMR (CDCl3) δ: 1.45-1.55 (1H, m), 1.85-1.94 (1H, m), 1.97-2.09 (2H, m), 2.41-2.48 (1H, m), 2.61-2.71 (2H, m), 2.78-2.80 (2H, m), 2.89-3.13 (2H, m), 3.26-3.46 (1H, m), 4.21-4.44 (2H, m), 4.97-5.34 (2H, m), 7.17-7.21 (1H, m), 7.25-7.35 (3H, m), 7.38-7.69 (3H, m). |
| | MS(ESI/APCI) m/z: 469 [M + H]+ |
| 47A | MS(ESI/APCI) m/z: 526 [M + H]+ |
| 47B | MS(ESI/APCI) m/z: 426 [M + H]+ |
| 47C | MS(ESI/APCI) m/z: 540 [M + H]+ |
| 47D | 1H-NMR (CDCl3) δ: 1.18 (3H, d, J = 6.8 Hz), 1.46-1.52 (1H, m), 2.16-2.23 (1H, m), 2.40-2.42 (1H, m), 2.49-2.56 (4H, m), 2.70-2.73 (1H, m), 2.87-2.95 (1H, m), 3.36-3.39 (1H, m), 3.56-3.76 (1H, m), 4.20-4.26 (1H, m), 4.27-4.36 (2H, m), 4.93 (1H, d, J = 10.8 Hz), 5.27-5.30 (1H, m), 6.68 (1H, t, J = 15.2 Hz), 7.26-7.29 (1H, m), 7.59-7.63 (1H, m), 7.68-7.72 (1H, m), 7.78-7.82 (1H, m), 7.89-7.94 (2H, m). |
| | MS(ESI/APCI) m/z: 498 [M + H]+ |
| 48A | 1H-NMR (CDCl3) δ: 1.32 (3H, d, J = 6.8 Hz), 1.48 (1H, t, J = 9.6 Hz), 2.08-2.21 (1H, m), 2.35-2.44 (4H, m), 2.49-2.60 (1H, m), 2.71 (1H, q, J = 5.2 Hz), 2.90-3.00 (1H, m), 3.27-3.42 (4H, m), 4.09 (1H, q, J = 6.8 Hz), 4.20 (1H, t, J = 10.0 Hz), 4.73 (1H, q, J = 4.8 Hz), 4.97 (1H, d, J = 10.0 Hz), 5.23 (1H, td, J = 9.6, 4.0 Hz), 6.83 (1H, tt, J = 8.8, 2.4 Hz), 7.13 (2H, d, J = 7.6 Hz), 7.20-7.28 (2H, m), 7.80-7.90 (1H, m). |
| | MS(ESI/APCI) m/z: 497 [M + H]+ |
| 49A | MS(ESI/APCI) m/z: 443 [M - Boc + H]+ |
| 49B | MS(ESI/APCI) m/z: 443 [M + H]+ |
| 49C | MS(ESI/APCI) m/z: 571 [M + H]+ |
| 49D | 1H-NMR (CDCl3) δ: 1.35-1.37 (6H, m), 1.91-1.96 (1H, m), 2.39-2.47 (1H, m), 2.61-2.73 (2H, m), 2.83 (3H, s), 2.94-2.97 (1H, m), 3.37-3.48 (2H, m), 4.39-4.43 (1H, m), 4.84-4.97 (2H, m), 5.29-5.41 (1H, m), 6.52-6.65 (1H, m), 7.18-7.23 (1H, m), 7.27-7.32 (1H, m), 7.49-7.55 (2H, m), 7.61-7.63 (1H, m),7.65-7.72 (2H, m). |
| | MS(ESI/APCI) m/z: 529 [M + H]+ |
| 50A | MS(ESI/APCI) m/z: 515 [M + Na]+ |
| 50B | MS(ESI/APCI) m/z: 393 [M + H]+ |
| 50C | MS(ESI/APCI) m/z: 521 [M + H]+ |
| 50D | 1H-NMR (CDCl3) δ: 1.33-1.36 (6H, m), 1.90-1.95 (1H, m), 2.37-2.46 (1H, m), 2.61-2.73 (2H, m), 2.79 (3H, s), 2.96-2.99 (1H, m), 3.35-3.47 (2H, m), 4.41-4.45 (1H, m), 4.82-4.98 (2H, m), 5.34-5.38 (1H, m), 7.15-7.21 (1H, m), 7.30-7.31 (1H, m), 7.33-7.39 (1H, m), 7.43-7.45 (2H, m), 7.51-7.59 (3H, m). |
| | MS(ESI/APCI) m/z: 479 [M + H]+ |
| 51A | MS(ESI/APCI) m/z: 443 [M - Boc + H]+ |
| 51B | MS(ESI/APCI) m/z: 443 [M + H]+ |
| 51C | MS(ESI/APCI) m/z: 571 [M + H]+ |
| 51D | 1H-NMR (CDCl3) δ: 1.41 (3H, s), 1.42 (3H, s), 1.45-1.50 (1H, m), 2.15-2.22 (1H, m), 2.35-2.41 (1H, m), 2.43 (3H, s), 2.51-2.60 (1H, m), 2.67-2.74 (1H, m), 2.86-2.96 (1H, m), 3.33-3.44 (1H, m), 4.12-4.27 (2H, m), 4.75-4.83 (1H, m), 4.90-4.98 (1H, m), 5.18-5.28 (1H, m), 6.51-6.87 (1H, m), 7.22-7.27 (2H, m), 7.28-7.32 (1H, m), 7.43 (1H, d, J = 9.6 Hz), 7.51 (1H, s), 7.77-7.84 (1H, m). |
| | MS(ESI/APCI) m/z: 529 [M + H]+ |
| 52A | MS(ESI/APCI) m/z: 427 [M - Boc + H]+ |
| 52B | MS(ESI/APCI) m/z: 427 [M + H]+ |
| 52C | MS(ESI/APCI) m/z: 555 [M + H]+ |
| 52D | 1H-NMR (CDCl3) δ: 1.39 (3H, s), 1.41 (3H, s), 1.43-1.51 (1H, m), 2.14-2.22 (1H, m), 2.33-2.41 (1H, m), 2.45 (3H, s), 2.51-2.59 (1H, m), 2.65-2.72 (1H, m), 2.85-2.96 (1H, m), 3.32-3.43 (1H, m), 4.08-4.30 (2H, m), 4.75-4.82 (1H, m), 4.93-5.02 (1H, m), 5.18-5.30 (1H, m), 7.08-7.12 (1H, m), 7.19-7.27 (3H, m), 7.35-7.38 (1H, m), 7.75-7.81 (1H, m). |
| | MS(ESI/APCI) m/z: 513 [M + H]+ |
| 53A | MS(ESI/APCI) m/z: 507 [M + H]+ |
| 53B | 1H-NMR (CDCl3) δ: 1.03-1.04 (3H, m), 1.87-2.00 (1 H, m), 2.41-2.50 (1H, m), 2.64-2.76 (2H, m), 2.89-2.90 (3H, m), 2.99-3.07 (1H, m), 3.41-3.44 (2H, m), 4.08-4.32 (2H, m), 4.40-4.41 (1H, m), 4.90-5.07 (1H, m), 5.33-5.46 (1H, m), 7.14-7.21 (1H, m), 7.27-7.31 (1H, m), 7.33-7.38 (1H, m), 7.43-7.45 (3H, m), 7.47-7.54 (2H, m). |
| | MS(ESI/APCI) m/z: 465 [M + H]+ |
| 54A | MS(ESI/APCI) m/z: 380 [M - Boc + H]+ |
| 54C | MS(ESI/APCI) m/z: 494 [M + H]+ |
| 54D | 1H-NMR (CDCl3) δ: 1.17 (3H, = 6.7 Hz), 1.45-1.51 (1H, m), 2.20 (1H, dd, J = 11.0, 9.2 Hz), 2.35-2.43 (1H, m), 2.45-2.55 (4H, m), 2.67-2.75 (1H, m), 2.93 (1H, dd, J = 14.4, 5.8 Hz), 3.34 (1H, dd, J = 14.1, 9.2 Hz), 3.61 (1H, d, J = 7.4 Hz), 4.20-4.34 (3H, m), 4.91 (1H, d, J = 9.2 Hz), 5.24-5.32 (1H, m), 7.19-7.24 (1H, m), 7.27-7.33 (1H, m), 7.59-7.66 (1H, m). |
| | MS(ESI/APCI) m/z: 452 [M + H]+ |
| 55A | 1H-NMR (CDCl3) δ: 2.00-2.05 (1H, m), 2.43-2.50 (1H, m), 2.64-2.73 (2H, m), 2.79-2.88 (3H, m), 3.11-3.14 (1H, m), 3.46-3.49 (1H, m), 4.48-4.50 (1H, m), 4.94-4.96 (1H, m), 5.37-5.83 (2H, m), 7.05-7.16 (1H, m), 7.22-7.26 (1H, m), 7.33-7.38 (1H, m), 7.39-7.56 (5H, m), 7.78 (1H, s), 7.87-8.11 (1H, m). |
| | MS(ESI/APCI) m/z: 488 [M + H]+ |
| 56A | 1H-NMR (CDCl3) δ: 1.15 (3H, = 7.2 Hz), 1.40-1.47 (1H, m), 2.10-2.16 (1H, m), 2.24 (3H, s), 2.27-2.41 (3H, m), 2.46-2.54 (1H, m), 2.70 (1H, d, J = 5.2 Hz), 2.95 (1H, d, J = 14.4 Hz), 3.24-3.36 (1H, m), 4.13-4.18 (1H, m), 4.42 (1H, d, J = 4.4 Hz), 5.00 (1H, d, J = 10.0 Hz), 5.15-5.28 (1H, m), 7.20-7.25 (1H, m), 7.27-7.32 (1H, m), 7.34-7.40 (1H, m), 7.40-7.49 (2H, m), 7.54-7.60 (2H, m),7.82-7.87 (1H, m). |
| | MS(ESI/APCI) m/z: 431 [M + H]+ |
| 57A | 1H-NMR (CDCl3) δ: 1.11 (6H, d, J = 6.0 Hz), 1.41-1.45 (1H, m), 2.07-2.17 (1H, m), 2.24 (3H, s), 2.34-2.42 (1H, m), 2.49-2.57 (1H, m), 2.63-2.78 (2H, m), 2.91 (1H, d, J = 14.4 Hz), 3.23-3.36 (1H, m), 4.14-4.20 (1H, m), 4.52 (1H, d, J = 3.6 Hz), 5.01 (1H, d, J = 9.2 Hz), 5.13-5.28 (1H, m), 7.19-7.24 (1H, m), 7.26-7.30 (1H, m), 7.34-7.40 (1H, m), 7.43-7.46 (2H, m), 7.56-7.57 (2H, m),7.87-7.89 (1H, m). |
| | MS(ESI/APCI) m/z: 445 [M + H]+ |
| 58A | MS(ESI/APCI) m/z: 374 [M - Boc + H]+ |
| 58B | MS(ESI/APCI) m/z: 316 [M + H]+ |
| 58C | MS(ESI/APCI) m/z: 316 [M - Boc + H]+ |
| 58D | MS(ESI/APCI) m/z: 375 [M + H]+ |
| 58E | MS(ESI/APCI) m/z: 361 [M + H]+ |
| 58F | MS(ESI/APCI) m/z: 395, 397 [M - Boc + H]+ |
| 58G | MS(ESI/APCI) m/z: 393 [M - Boc + H]+ |
| 58H | MS(ESI/APCI) m/z: 393 [M + H]+ |
| 58I | MS(ESI/APCI) m/z: 507 [M + H]+ |
| 58J | 1H-NMR (CDCl3) δ: 1.17 (3H, = 6.8 Hz), 1.44-1.52 (1H, m), 2.11-2.22 (1H, m), 2.34-2.45 (1H, m), 2.48-2.56 (1H, m), 2.61 (3H, s), 2.67-2.76 (1H, m), 2.82-2.93 (1H, m), 3.28-3.39 (1H, m), 3.55-3.63 (1H, m), 4.19-4.34 (3H, m), 4.82-4.93 (1H, m), 5.19-5.30 (1H, m), 6.93-7.06 (1H, m), 7.29-7.59 (6H, m). |
| | MS(ESI/APCI) m/z: 465 [M + H]+ |
| 59A | MS(ESI/APCI) m/z: 521 [M + H]+ |
| 59B | MS(ESI/APCI) m/z: 479 [M + H]+ |
| 59C | 1H-NMR (CDCl3) δ: 1.40-1.44 (6H, m), 1.44-1.51 (1H, m), 2.09-2.17 (1H, m), 2.32-2.41 (1H, m), 2.46 (3H, s), 2.50-2.58 (1H, m), 2.66-2.73 (1H, m), 2.81-2.90 (1H, m), 3.29-3.39 (1H, m), 4.16-4.27 (2H, m), 4.74-4.80 (1H, m), 4.87-4.93 (1H, m), 5.11-5.20 (1H, m), 6.97-7.04 (1H, m), 7.36-7.57 (6H, m). |
| | MS(ESI/APCI) m/z: 479 [M + H]+ |
| 60A | 1H-NMR (CDCl3) δ: 0.45-0.52 (0.5H, m), 0.72-0.79 (0.5H, m), 1.46 (4.5H, s), 1.49 (4.5H, s), 2.24-2.36 (1H, m), 2.42-2.56 (2H, m), 2.96-3.04 (1H, m), 3.76-3.80 (2H, m), 3.81 (1.5H, s), 3.88 (1H, d, J = 2.7 Hz), 4.04 (0.5H, d, J = 13.7 Hz), 4.67-4.75 (0.5H, m), 4.87-4.94 (0.5H, m), 7.06 (0.5H, t, J = 4.9 Hz), 7.16 (0.5H, t, J = 4.9 Hz), 8.08 (1H, t, J = 4.9 Hz). |
| | MS(ESI/APCI) m/z: 457, 459 [M + H]+ |
| 60B | 1H-NMR (CDCl3) δ: 1.43-1.48 (10H, m), 1.78 (1H, dd, J = 11.0, 9.0 Hz), 2.50 (1H, br s), 2.57-2.66 (1H, m), 3.03 (1H, q, J = 5.6 Hz), 3.35 (1H, dd, J = 13.1, 4.5 Hz), 3.51 (1H, br s), 4.69 (2H, br s), 7.14 (1H, br s), 8.09 (1H, d, J = 5.1 Hz). |
| | MS(ESI/APCI) m/z: 399, 401 [M + H]+ |
| 60C | 1H-NMR (CDCl3) δ: 1.20-1.36 (4.5H, m), 1.44 (4.5H, s), 1.48-1.56 (1H, m), 2.53 (2.5H, br s), 3.00-3.37 (2.5H, m), 3.50-3.65 (0.5H, m), 4.04 (0.5H, q, J = 5.7 Hz), 4.51-4.97 (1.5H, m), 5.29-5.59 (0.5H, m), 7.10-7.37 (1H, m), 8.10 (1H, d, J = 4.7 Hz). |
| | MS(ESI/APCI) m/z: 414, 416 [M + H]+ |
| 60D | 1H-NMR (CDCl3) δ: 1.26 (9H, s), 2.09 (1H, = 9.0 Hz), 2.16-2.26 (1H, m), 2.29-2.36 (1H, m), 2.40-2.60 (1H, m), 2.90 (1H, dd, J = 13.3, 8.6 Hz), 3.20-3.33 (1H, m), 3.37-3.48 (1H, m), 3.72 (1H, d, J = 8.6 Hz), 4.52 (1H, d, J = 4.3 Hz), 4.67 (1H, d, J = 7.0 Hz), 7.31 (1H, br s), 8.07 (1H, d, J = 4.7 Hz). |
| | MS(ESI/APCI) m/z: 400, 402 [M + H]+ |
| 60E | 1H-NMR (CDCl3) δ: 1.26 (9H, s), 1.38 (1H, t, J = 9.6 Hz), 2.03-2.12 (1H, m), 2.26-2.35 (1H, m), 2.39-2.47 (1H, m), 2.57 (1H, q, J = 5.7 Hz), 2.84-2.98 (4H, m), 3.37 (1H, d, J = 7.8 Hz), 4.22 (1H, t, J = 9.2 Hz), 4.53 (1H, br s), 4.80 (1H, br s), 5.00 (1H, d, J = 9.4 Hz), 7.32 (1H, br s), 8.10 (1H, d, J = 4.7 Hz). |
| | MS(ESI/APCI) m/z: 478, 480 [M + H]+ |
| 60F | 1H-NMR (CDCl3) δ: 1.24 (9H, s), 1.40 (1H, t, J = 10.0 Hz), 2.10 (1H, t, J = 10.4 Hz), 2.28-2.36 (1H, m), 2.40-2.47 (1H, m), 2.59 (1H, q, J = 5.5 Hz), 2.80 (3H, s), 2.95 (1H, dd, J = 13.5, 8.0 Hz), 3.42 (1H, dd, J = 13.1, 6.8 Hz), 4.23 (1H, t, J = 9.4 Hz), 4.56 (1H, s), 4.76-4.91 (2H, m), 7.34-7.41 (3H, m), 7.78-7.84 (1H, m), 7.93 (1H, br s), 8.41 (1H, d, J = 4.7 Hz). |
| 60H | 1H-NMR (CD3OD) δ: 2.03-2.13 (1H, m), 2.21-2.30 (1H, m), 2.43-2.54 (2H, m), 2.87-2.94 (1H, m), 2.97 (3H, s), 3.66 (2H, s), 3.68-3.77 (1H, m), 3.98 (1H, br s), 4.40 (1H, t, J = 6.1 Hz), 4.68 (1H, br s), 7.60-7.74 (2H, m), 7.82-7.86 (1H, m), 7.98 (1H, s), 8.14 (1H, br s), 8.70 (1H, br s). |
| | MS(ESI/APCI) m/z: 410, 412 [M + H]+ |
| 60I | 1H-NMR (CDCl3) δ: 0.69-0.83 (3H, m), 1.00 (1H, br s), 1.48-1.57 (1H, m), 2.16 (1H, t, J = 10.0 Hz), 2.38-2.59 (5H, m), 2.68 (1H, br s), 2.94 (1H, br s), 3.33 (1H, br s), 4.20 (1H, t, J = 9.2 Hz), 4.78 (2H, d, J = 9.8 Hz), 5.19 (1H, br s), 7.37-7.41 (2H, m), 7.68 (1H, br s), 7.82 (1H, br s), 7.94 (1H, br s), 8.43 (1H, d, J = 5.1 Hz). |
| | MS(ESI/APCI) m/z: 478 [M + H]+ |
| 61A | 1H-NMR (CDCl3) δ: 1.24 (9H, s), 1.85 (1H, t, J = 9.5 Hz), 2.34-2.40 (1H, m), 2.54-2.63 (2H, m), 2.93 (3H, s), 2.96 (1H, dd, J = 13.8, 8.6 Hz), 3.41 (1H, dd, J = 13.8, 7.1 Hz), 4.35-4.39 (1H, m), 4.53-4.61 (1H, m), 4.80-4.87 (2H, m), 7.16 (1H, t, J = 7.7 Hz), 7.23-7.26 (1H, m), 7.31-7.42 (4H, m), 7.51-7.52 (1H, m). |
| | MS(ESI/APCI) m/z: 427 [M + H -Boc]+ |
| 61B | MS(ESI/APCI) m/z: 427 [M + H]+ |
| 61C | MS(ESI/APCI) m/z: 555 [M + H]+ |
| 61D | MS(ESI/APCI) m/z: 513 [M + H]+ |
| 61E | 1H-NMR (CDCl3) 1.34 (3H, s), 1.37 (3H, s), 1.92 (1H, t, J = 9.5 Hz), 2.40-2.46 (1H, m), 2.63 (1H, dd, J = 10.4, 9.5 Hz), 2.70 (1H, td, J = 10.4, 6.1 Hz), 2.82 (3H, s), 2.94 (1H, dd, J = 14.0, 6.1 Hz), 3.41 (1H, dd, J = 14.0, 8.5 Hz), 3.42 (1H, s), 4.40 (1H, t, J = 8.9 Hz), 4.85-4.92 (2H, m), 5.31-5.37 (1H, m), 7.19 (1H, t, J = 7.3 Hz), 7.23-7.26 (1H, m), 7.32-7.38 (2H, m), 7.42-7.44 (1H, m), 7.52-7.55 (1H, m), 7.58-7.62 (1H, m). |
| | MS(ESI/APCI) m/z: 513 [M + H]+ |
| 62A | 1H-NMR (CDCl3) δ: 1.44-1.49 (1H, m), 1.59-1.69 (1H, m), 1.94-2.02 (2H, m), 2.04-2.18 (3H, m), 2.31-2.34 (4H, m), 2.43-2.53 (1H, m), 2.57-2.74 (3H, m), 2.90-3.02 (1H, m), 3.36-3.42 (1H, m), 4.20-4,25 (1H, m), 4.62-4.75 (1H, m), 5.00 (1H, d, J = 10.4 Hz), 5.10-5.19 (1H, m), 7.20-7.27 (1H, m), 7.28-7.34 (1H, m), 7.36-7.42 (1H, m), 7.43-7.49 (2H, m), 7.54-7.61 (2H, m), 7.80-7.84 (1H, m). |
| | MS(ESI/APCI) m/z: 473 [M + H]+ |
| 63A | MS(ESI/APCI) m/z: 392 [M - Boc + H]+ |
| 63B | 1H-NMR (CDCl3) δ: 1.45-1.53 (9H, m), 2.18-2.27 (1H, m), 2.45-2.74 (2H, m), 3.27-3.36 (1H, m), 3.43-3.67 (1H, m), 3.74-3.85 (1H, m), 4.64-4.88 (2H, m), 6.80-6.89 (1H, m), 7.16-7.23 (1H, m). |
| 63C | MS(ESI/APCI) m/z: 449 [M + H]+ |
| 63D | MS(ESI/APCI) m/z: 335 [M - Boc + H]+ |
| 63E | MS(ESI/APCI) m/z: 415 [M - Boc + H]+ |
| 63F | MS(ESI/APCI) m/z: 411 [M - Boc + H]+ |
| 63G | MS(ESI/APCI) m/z: 411 [M + H]+ |
| 63H | MS(ESI/APCI) m/z: 525 [M + H]+ |
| 63I | MS(ESI/APCI) m/z: 483 [M + H]+ |
| 63J | 1H-NMR (CDCI3) δ: 1.07 (3H, d, J = 6.7 Hz), 1.91-2.01 (1H, m), 2.42-2.53 (1H, m), 2.61-2.76 (2H, m), 2.87-3.00 (1H, m), 2.93 (3H, s), 3.34-3.47 (2H, m), 4.14-4.45 (3H, m), 4.87-4.98 (1H, m), 5.27-5.40 (1H, m), 6.93-7.03 (1H, m), 7.10-7.20 (1H, m), 7.33-7.54 (5H, m). |
| | MS(ESI/APCI) m/z: 483 [M + H]+ |
| 64A | 1H-NMR (CDCl3) δ: 0.31-0.47 (4H, m), 0.84-0.94 (1H, m), 1.50-1.55 (1H, m), 2.14-2.22 (1H, m), 2.37-2.44 (4H, m), 2.48-2.56 (1H, m), 2.69-2.75 (1H, m), 2.91-2.98 (1H, m), 3.33-3.39 (1H, m), 3.47 (1H, d, J = 7.2 Hz), 4.19-4.28 (2H, m), 4.44-4.56 (1H, m), 4.98 (1H, d, J = 10.8 Hz), 5.20-5.33 (1H, m), 7.18-7.24 (1H, m), 7.28-7.33 (1H, m), 7.34-7.40 (1H, m), 7.42-7.48 (2H, m), 7.52-7.58 (2H, m), 7.66-7.71 (1H, m). |
| | MS(ESI/APCI) m/z: 473 [M + H]+ |
| 65A | 1H-NMR (CDCl3) δ: 1.16-1.19 (3H, m), 1.41-1.47 (1H, m), 2.07-2.13 (1H, m), 2.30-2.35 (1H, m), 2.42-2.46 (1H, m), 2.48-2.59 (3H, m), 2.62-2.66 (1 H, m), 2.96-3.01 (1H, m), 3.38-3.44 (1H, m), 3.94-4.12 (2H, m), 4.22-4.28 (1H, m), 4.64-4.67 (1H, m), 4.85-4.91 (2H, m), 7.18-7.22 (1H, m), 7.28-7.34 (2H, m), 7.37-7.41 (1H, m), 7.44-7.48 (2H, m), 7.55-7.57 (2H, m). |
| | MS(ESI/APCI) m/z: 447 [M + H]+ |
| 66A | 1H-NMR (CDCl3) δ: 1.22 (9H, s), 1.85 (1H, t, J = 9.5 Hz), 2.32-2.39 (1H, m), 2.55-2.62 (2H, m), 2.91 (3H, s), 2.97 (1H, dd, J = 14.1, 9.5 Hz), 3.41 (1H, dd, J = 14.1, 6.7 Hz), 4.36-4.41 (1H, m), 4.53-4.61 (1H, m), 4.82-4.87 (2H, m), 7.15 (1H, t, J = 7.7 Hz), 7.28-7.38 (3H, m), 7.41-7.45 (2H, m), 7.50-7.52 (2H, m). |
| | MS(ESI/APCI) m/z: 393 [M - Boc + H]+ |
| 66B | MS(ESI/APCI) m/z: 393 [M - Boc + H]+ |
| 66C | MS(ESI/APCI) m/z: 393 [M + H]+ |
| 66D | 1H-NMR (CDCl3) δ: 0.81-1.16 (1H, m), 1.61-2.01 (3H, m), 2.41-2.50 (1H, m), 2.55-2.80 (4H, m), 2.95-3.67 (4H, m), 4.31-4.42 (1H, m), 4.52-4.95 (3H, m), 7.13-7.19 (1H, m), 7.26-7.30 (2H, m), 7.34-7.38 (1H, m), 7.41-7.55 (4H, m). |
| | MS(ESI/APCI) m/z: 479 [M + H]+ |
| 67A | MS(ESI/APCI) m/z: 427 [M - Boc + H]+ |
| 67B | MS(ESI/APCI) m/z: 427 [M + H]+ |
| 67C | MS(ESI/APCI) m/z: 555 [M + H]+ |
| 67E | 1H-NMR (CDCl3) δ: 1.41-1.42 (6H, m), 1.45-1.50 (1H, m), 2.11-2.16 (1H, m), 2.35-2.41 (1H, m), 2.53-2.58 (4H, m), 2.69-2.71 (1H, m), 2.84-2.88 (1H, m), 3.33-3.38 (1H, m), 4.14 (1H, s), 4.21-4.26 (1H, m), 4.77-4.81 (1H, m), 4.87-4.90 (1H, m), 5.15-5.21 (1H, m), 6.96-7.01 (1H, m), 7.37-7.45 (3H, m), 7.51-7.56 (2H, m). |
| | MS(ESI/APCI) m/z: 513 [M + H]+ |
| 68A | MS(ESI/APCI) m/z: 415 [M - Boc + H]+ |
| 68B | MS(ESI/APCI) m/z: 415 [M + H]+ |
| 68C | MS(ESI/APCI) m/z: 529 [M + H]+ |
| 68D | 1H-NMR(COCI3) δ: 1.15 (3H, = 6.4 Hz), 1.46-1.51 (1H, m), 2.21-2.26 (1H, m), 2.39-2.44 (1H, m), 2.50-2.56 (4H, m), 2.69-2.73 (1H, m), 2.90-2.95 (1H, m), 3.31-3.37 (1H, m), 3.56-3.66 (1H, m), 4.20-4.30 (3H, m), 5.08 (1H, d, J = 10.4 Hz), 5.24-5.30 (1H, m), 7.15-7.18 (1H, m), 7.28 (1H, s), 7.31-7.35 (2H, m), 7.53-7.58 (1H, m). |
| | MS(ESI/APCI) m/z: 487 [M + H]+ |
| 69A | 1H-NMR (CDCl3) 1.80 (0.3H, t, J = 9.8 Hz), 1.99 (0.7H, t, J = 9.8 Hz), 2.50-2.56 (1H, m), 2.67-2.76 (2H, m), 2.82 (2.1H, s), 2.85-2.98 (1H, m), 3.09 (0.9H, s), 3.19-3.25 (0.3H, m), 3.44 (0.7H, dd, J = 14.4, 8.3 Hz), 3.51-3.54 (0.7H, m), 3.79-3.85 (0.3H, m), 4.28-4.48 (2H, m), 4.55-4.62 (0.3H, m), 4.64-4.71 (0.7H, m), 4.95-4.97 (1H, m), 5.14-5.21 (0.3H, m), 5.38-5.44 (0.7H, m), 7.17 (1H, t, J = 7.7 Hz), 7.28-7.38 (2H, m), 7.41-7.54 (5H, m). |
| | MS(ESI/APCI) m/z: 519 [M + H]+ |
| 70A | 1H-NMR (CDCl3) δ: 1.96 (1H, t, J = 9.5 Hz), 2.36-2.42 (1H, m), 2.51-2.56 (1H, m), 2.65 (1H, td, J = 10.4, 5.5 Hz), 2.72 (3H, s), 2.73-2.79 (1H, m), 2.96-3.00 (2H, m), 3.38 (1H, dd, J = 14.4, 9.5 Hz), 4.11-4.18 (1H, m), 4.41 (1H, t, J = 9.5 Hz), 4.49 (1H, dt, J = 8.6, 6.1 Hz), 4.59-4.64 (1H, m), 4.90 (1H, d, J = 9.8 Hz), 5.21-5.25 (2H, m), 7.21 (1H, t, J = 7.7 Hz), 7.28-7.29 (1H, m), 7.34-7.38 (1H, m), 7.41-7.45 (2H, m), 7.53-7.55 (2H, m), 7.65-7.69 (1H, m). |
| | MS(ESI/APCI) m/z: 477 [M + H]+ |
| 71A | 1H-NMR (CDCl3) δ: 0.8/-0.90 (2H, m), 0.92-0.96 (2H, m), 1.95 (1H, t, J = 9.4 Hz), 2.38-2.44 (1H, m), 2.56 (1H, dd, J = 10.6, 8.8 Hz), 2.67 (1H, td, J = 10.6, 6.1 Hz), 2.81 (3H, s), 2.98 (1H, s), 3.00 (1H, dd, J = 14.1, 6.1 Hz), 3.39 (1H, dd, J = 14.1, 8.8 Hz), 4.38 (1H, t, J = 9.4 Hz), 5.01 (1H, d, J = 10.6 Hz), 5.04 (1H, dt, J = 17.2, 6.1 Hz), 5.16-5.21 (1H, m), 7.15 (1H, t, J = 7.7 Hz), 7.24-7.28 (1H, m), 7.33-7.37 (1H, m), 7.41-7.44 (2H, m), 7.48-7.54 (3H, m). |
| | MS(ESI/APCI) m/z: 477 [M + H]+ |
| 72A | MS(ESI/APCI) m/z: 483 [M + H]+ |
| 72B | 1H-NMR (CDCl3) δ: 1.47-1.51 (1H, m), 2.15-2.19 (1H, m), 2.40-2.45 (4H, m), 2.52-2.54 (1H, m), 2.70-2.76 (1H, m), 2.90-2.94 (1H, m), 3.35-3.41 (1H, m), 3.96 (1H, d, J = 8.0 Hz), 4.22-4.27 (1H, m), 4.50-4.58 (2H, m), 4.92 (1H, d, J = 11.6 Hz), 5.28-5.30 (1H, m), 5.41 (1H, t, J = 15.6 Hz), 7.19-7.23 (1H, m), 7.29-7.33 (1H, m), 7.35-7.39 (1H, m), 7.42-7.46 (2H, m), 7.52-7.57 (2H, m), 7.61-7.66 (1H, m). |
| | MS(ESI/APCI) m/z: 483 [M + H]+ |
| 73A | 1H-NMR (CDCl3) δ: 0.73-0.76 (2H, m), 0.86-0.89 (2H, m), 1.44-1.49 (1H, m), 2.13 (1H, t, J = 9.6 Hz), 2.33-2.39 (4H, m), 2.48-2.56 (1H, m), 2.65-2.69 (1H, m), 2.91-2.98 (1H, m), 3.29-3.31 (1H, m), 3.46-3.50 (1H, m), 3.60-3.65 (1H, m), 4.18-4.23 (1H, m), 4.88-4.97 (2H, m), 5.14-5.22 (1H, m), 7.17-7.22 (1H, m), 7.25-7.30 (1H, m), 7.34-7.39 (1H, m), 7.41-7.48 (2H, m), 7.53-7.58 (2H, m), 7.72-7.75 (1H, m). |
| | MS(ESI/APCI) m/z: 473 [M + H]+ |
| 74A | MS(ESI/APCI) m/z: 413 [M - Boc + H]+ |
| 74B | MS(ESI/APCI) m/z: 411 [M - Boc + H]+ |
| 74C | MS(ESI/APCI) m/z: 411 [M + H]+ |
| 74D | MS(ESI/APCI) m/z: 539 [M + H]+ |
| 74E | 1H-NMR (CDCl3) δ: 1.36 (3H, s), 1.39 (3H, s), 1.90-1.96 (1H, m), 2.38-2.47 (1H, m), 2.55-2.62 (1H, m), 2.66-2.75 (1H, m), 2.83-2.84 (3H, m), 2.89 (1H, dd, J = 14.7, 5.5 Hz), 3.33-3.45 (2H, m), 4.35-4.45 (1H, m), 4.83-4.95 (2H, m), 5.23-5.33 (1H, m), 6.95-7.01 (1H, m), 7.28-7.47 (4H, m), 7.50-7.56 (2H, m). |
| | MS(ESI/APCI) m/z: 497 [M + H]+ |
| 75A | 1H-NMR (CDCl3) δ: 1.45-1.54 (1H, m), 2.14-2.25 (1H, m), 2.34-2.45 (1H, m), 2.46-2.56 (4H, m), 2.66-2.74 (1H, m), 2.87-3.05 (2H, m), 3.32-3.43 (1H, m), 3.70-3.77 (1H, m), 4.17-4.29 (2H, m), 4.31-4.47 (1H, m), 4.48-4.59 (1H, m), 4.96-5.05 (1H, m), 5.24-5.36 (1H, m), 7.15-7.63 (8H, m) |
| | MS(ESI/APCI) m/z: 465 [M + H]+ |
| 76A | 1H-NMR (CDCl3) δ: 1.50-1.55 (1H, m), 2.12-2.22 (1H, m), 2.30-2.45 (6H, m), 2.47-2.60 (1H, m), 2.65-2.75 (1H, m), 2.94-3.07 (2H, m), 3.33-3.45 (1H, m), 4.20-4.30 (1H, m), 4.32-4.42 (1H, m), 4.90-5.05 (1H, m), 5.09-5.35 (1H, m), 7.15-7.25 (1H, m), 7.28-7.60 (6H, m), 7.64-7.72 (1H, m). |
| | MS(ESI/APCI) m/z: 445 [M + H]+ |
| 76B | 1H-NMR (CDCl3) δ: 1.45-1.52 (1H, m), 2.16-2.25 (1H, m), 2.35-2.43 (1H, m), 2.46-2.56 (4H, m), 2.68-2.75 (1H, m), 2.93 (1H, dd, J = 14.7, 4.9 Hz), 3.34 (1H, dd, J = 14.4, 9.5 Hz), 3.58 (1H, s), 4.19-4.30 (2H, m), 4.90 (1H, d, J = 10.4 Hz), 5.25-5.33 (1H, m), 7.19-7.24 (1H, m), 7.27-7.32 (1H, m), 7.35-7.40 (1H, m), 7.41-7.48 (2H, m), 7.51-7.57 (2H, m), 7.60-7.66 (1H, m). |
| | MS(ESI/APCI) m/z: 451 [M + H]+ |
| 77A | MS(ESI/APCI) m/z: 445 [M - Boc + H]+ |
| 77B | MS(ESI/APCI) m/z: 445 [M + H]+ |
| 77C | MS(ESI/APCI) m/z: 573 [M + H]+ |
| 77D | 1H-NMR (CDCl3) δ: 1.38 (3H, s), 1.40 (3H, s), 1.91-1.98 (1H, m), 2.40-2.49 (1H, m), 2.57 (1H, dd, J = 10.7, 8.9 Hz), 2.67-2.77 (1H, m), 2.82-2.91 (1H, m), 2.87 (3H, s), 3.33-3.43 (2H, m), 4.35-4.43 (1H, m), 4.81-4.97 (2H, m), 5.19-5.32 (1H, m), 6.91-7.01 (1H, m), 7.32-7.46 (4H, m), 7.53-7.58 (1H, m). |
| | MS(ESI/APCI) m/z: 531 [M + H]+ |
| 78A | 1H-NMR (CDCl3) δ: 1.38-1.48 (2H, m), 1.88-2.02 (2H, m), 2.41-2.47 (1H, m), 2.65-2.71 (2H, m), 2.79-2.93 (3H, m), 3.00-3.09 (1H, m), 3.46-3.55 (1H, m), 4.37-4.41 (1H, m), 4.61-4.76 (1H, m), 4.81-4.83 (1H, m), 4.92-5.09 (1H, m), 7.16 (1H, t, J = 7.7 Hz), 7.26-7.35 (2H, m), 7.39-7.42 (3H, m), 7.47-7.49 (2H, m). |
| | MS(ESI/APCI) m/z: 497 [M + H]+ |
| 79A | 1H-NMR (CDCl3) δ: 1.92-2.02 (1H, m), 2.46-2.56 (1H, m), 2.59-2.76 (2H, m), 2.82-3.14 (4H, m), 3.38-3.54 (1H, m), 4.02-4.13 (1H, m), 4.33-4.73 (3H, m), 4.85 (1H, d, J = 8.6 Hz), 5.28-5.42 (1H, m), 6.93-7.18 (2H, m), 7.33-7.55 (5H, m). |
| | MS(ESI/APCI) m/z: 537 [M + H]+ |
| 80A | MS (ESI) m/z: 435 [M - tert-butyl + H]+ |
| 80B | MS(ESI) m/z: 389 [M - Boc + H]+ |
| 80C | MS(ESI/APCI) m/z: 389 [M + H]+ |
| 80D | MS(ESI) m/z: 503 [M + H]+ |
| 80E | 1H-NMR (CDCl3) δ: 1.08 (3H, d, J = 6.4 Hz), 1.15 (3H, t, J = 7.2 Hz), 1.44-1.46 (1H, m), 2.22-2.41 (2H, m), 2.43-2.54 (1H, m), 2.66-2.83 (3H, m), 2.82-3.01 (1H, m), 3.25-3.45 (1H, m), 3.54-3.66 (1H, m), 4.13-4.31 (3H, m), 5.05-5.17 (1H, m), 5.29-5.35 (1H, m), 7.13-7.21 (1H, m), 7.24-7.30 (1H, m), 7.33-7.39 (1H, m), 7.39-7.46 (2H, m), 7.48- 7.56 (3H, m). |
| | MS(ESI/APCI) m/z: 461 [M + H]+ |
| 81A | MS(ESI/APCI) m/z: 411 [M - Boc + H]+ |
| 81B | MS(ESI) m/z: 407 [M - Boc + H]+ |
| 81C | MS(ESI/APCI) m/z: 407 [M + H]+ |
| 81D | MS(ESI) m/z: 521 [M + H]+ |
| 81E | MS(ESI/APCI) m/z: 479 [M + H]+ |
| 81F | 1H-NMR (CDCl3) δ: 1.12-1.19 (6H, m), 1.42-1.48 (1H, m), 2.15-2.22 (1H, m), 2.35-2.41 (1H, m), 2.48-2.53 (1H, m), 2.70-2.74 (1H, m), 2.76-2.90 (3H, m), 3.34-3.40 (1H, m), 3.56 (1H, d, J = 7.6 Hz), 4.17-4.28 (3H, m), 4.71 (1H, d, J = 10.8 Hz), 5.23-5.29 (1H, m), 6.97-7.01 (1H, m), 7.27-7.31 (1H, m), 7.36-7.41 (1H, m), 7.43-7.46 (2H, m), 7.48-7.52 (2H, m). MS(ESI/APCI) m/z: 479 [M + H]+ |
| 82A | 1H-NMR (CDCl3) δ: 1.92-2.00 (1H, m), 2.46-2.56 (1H, m), 2.59-2.76 (2H, m), 2.80-3.04 (4H, m), 3.36-3.53 (1H, m), 3.97-4.07 (1H, m), 4.32-4.52 (2H, m), 4.57-4.73 (1H, m), 4.80 (1H, d, J = 8.5 Hz), 5.26-5.45 (1H, m), 6.89-7.03 (1H, m), 7.09-7.21 (1H, m), 7.29-7.43 (3H, m), 7.47-7.56 (1H, m). |
| | MS(ESI/APCI) m/z: 571 [M + H]+ |
| 83A | MS(ESI/APCI) m/z: 415 [M + H]+ |
| 83B | 1H-NMR (CDCl3) δ: 1.20 (8H, s), 1.22 (1H, s), 1.80 (1H, t, J = 9.3 Hz), 2.30-2.36 (1H, m), 2.52-2.63 (2H, m), 2.99 (1H, dd, J = 13.8, 9.3 Hz), 3.46 (1H, dd, J = 13.8, 6.4 Hz), 4.17-4.22 (1H, m), 4.51-4.59 (1H, m), 4.78-4.86 (2H, m), 5.91 (1H, d, J = 9.5 Hz), 6.27 (1H, d, J = 16.6 Hz), 6.39 (1H, dd, J = 16.6, 9.5 Hz), 7.16 (1H, t, J = 7.7 Hz), 7.28-7.37 (3H, m), 7.41-7.45 (2H, m), 7.51-7.53 (2H, m). |
| | MS(ESI/APCI) m/z: 405 [M - Boc + H]+ |
| 83C | 1H-NMR (COCI3) δ: 1.19 (8H, s), 1.22 (1H, s), 1.35 (3H, t, J = 7.4 Hz), 1.83 (1H, t, J = 9.2 Hz), 2.32-2.38 (1H, m), 2.54-2.63 (2H, m), 2.98 (1H, dd, J = 14.1, 9.8 Hz), 3.01-3.10 (2H, m), 3.44 (1H, dd, J = 14.1, 6.1 Hz), 4.33-4.37 (1H, m), 4.53-4.60 (1H, m), 4.72 (1H, d, J = 9.2 Hz), 4.82-4.88 (1H, m), 7.15 (1H, t, J = 7.7 Hz), 7.28-7.38 (3H, m), 7.41-7.45 (2H, m), 7.50-7.52 (2H, m). |
| | MS(ESI/APCI) m/z: 407 [M - Boc + H]+ |
| 83E | MS(ESI/APCI) m/z: 407 [M + H]+ |
| 83F | MS(ESI/APCI) m/z: 521 [M + H]+ |
| 83G | 1H-NMR (CDCl3) δ: 0.98 (3H, d, J = 6.1 Hz), 1.33 (3H, t, J = 7.1 Hz), 1.92 (1H, t, J = 9.2 Hz), 2.41-2.47 (1H, m), 2.67 (1H, td, J = 10.3, 5.9 Hz), 2.74-2.79 (1H, m), 2.98-3.07 (3H, m), 3.38-3.46 (2H, m), 4.14-4.24 (2H, m), 4.35-4.39 (1H, m), 4.80 (1H, d, J = 8.6 Hz), 5.37-5.45 (1H, m), 7.17 (1H, t, J = 7.7 Hz), 7.26-7.29 (1H, m), 7.33-7.37 (1H, m), 7.40-7.44 (3H, m), 7.48-7.50 (2H, m). |
| | MS(ESI/APCI) m/z: 479 [M + H]+ |
| 84A | 1H-NMR (CDCl3) δ: 2.04 (1H, t, J = 9.2 Hz), 2.40-2.46 (1H, m), 2.62-2.74 (2H, m), 2.85 (3H, s), 3.10 (1H, dd, J = 14.6, 7.4 Hz), 3.50 (1H, dd, J = 14.6, 7.7 Hz), 4.48-4.52 (1H, m), 4.79-4.82 (1H, m), 5.11-5.18 (1H, m), 5.54 (1H, br s), 6.40 (1H, br s), 7.10-7.14 (1H, m), 7.23-7.26 (1H, m), 7.31-7.35 (1H, m), 7.39-7.50 (5H, m), 8.31 (1H, br s). |
| | MS(ESI/APCI) m/z: 488 [M + H]+ |
| 85A | 1H-NMR (CDCl3) δ: 1.42 (3H, d, J = 22.1 Hz), 1.52 (3H, d, J = 21.5 Hz), 1.94 (1H, t, J = 9.2 Hz), 2.38-2.43 (1H, m), 2.62-2.73 (2H, m), 2.77 (3H, s), 2.97 (1H, dd, J = 14.3, 5.8 Hz), 3.40 (1H, dd, J = 14.3, 8.6 Hz), 4.38-4.43 (1H, m), 4.87 (1H, d, J = 9.8 Hz), 4.94 (1H, dt, J = 16.6, 6.7 Hz), 5.27-5.33 (1H, m), 7.18 (1H, t, J = 7.7 Hz), 7.25-7.29 (1H, m), 7.34-7.37 (1H, m), 7.41-7.45 (2H, m), 7.53-7.59 (3H, m). |
| | MS(ESI/APCI) m/z: 481 [M + H]+ |
| 86A | 1H-NMR (CDCl3) δ: 1.21 (3H, d, J = 6.2 Hz), 1.42-1.48 (1H, m), 2.12-2.25 (2H, m), 2.34-2.41 (4H, m), 2.43-2.55 (2H, m), 2.66-2.74 (1H, m), 2.92-2.98 (1H, m), 3.27-3.37 (1H, m), 3.96 (1H, s), 4.13-4.25 (2H, m), 4.32-4.42 (1H, m), 4.98 (1H, d, J = 10.0 Hz), 5.23-5.30 (1H, m), 7.20-7.25 (1H, m), 7.27-7.33 (1H, m), 7.35-7.40 (1H, m), 7.42-7.49 (2H, m), 7.53-7.59 (2H, m), 7.68-7.72 (1H, m). |
| | MS(ESI/APCI) m/z: 461 [M + H]+ |
| 87A | MS(ESI/APCI) m/z: 481 M + H]+ |
| 87B | MS(ESI/APCI) m/z: 487 [M + H]+ |
| 87C | 1H-NMR (CDCI3) δ: 1.88-2.02 (1H, m), 2.39-2.51 (1H, m), 2.61-2.74 (2H, m), 2.85-3.01 (4H, m), 3.30-3.48 (2H, m), 4.07-4.30 (1H, m), 4.31-4.45 (1H, m), 4.99-5.14 (1H, m), 5.26-5.43 (1H, m), 6.91-7.02 (1H, m), 7.07-7.18 (1H, m), 7.32-7.52 (5H, m). |
| | MS(ESI/APCI) m/z: 487 [M + H]+ |
| 88A | 1H-NMR (CDCl3) δ: 2.33 (3H, d, J = 2.7 Hz), 6.84-6.92 (1H, m), 6.92-6.99 (1H, m), 7.32-7.48 (3H, m), 7.50-7.63 (2H, m). |
| 88B | 1H-NMR (CDCl3) δ: 4.54 (2H, = 1.2 Hz), 7.06-7.16 (2H, m), 7.39-7.50 (3H, m), 7.51-7.56 (2H, m). |
| 88C | 1H-NMR (COCI3) δ: 0.49-0.61 (1H, m), 1.48 (9H, s), 2.13-2.25 (1H, m), 2.41-2.56 (2H, m), 2.97 (1H, t, J = 5.3 Hz), 3.77-4.09 (5H, m), 4.66-4.73 (0.4H, m), 4.88-4.94 (0.6H, m), 6.76-6.86 (1H, m), 6.99-7.07 (1H, m), 7.35-7.48 (5H, m). |
| 88D | 1H-NMR (CDCl3) δ: 1.28-1.35 (1H, m), 1.47 (9H, s), 1.77 (1H, dd, J = 10.6, 9.0 Hz), 2.39 (1H, br s), 2.59 (1H, br s), 3.00 (1H, d, J = 5.1 Hz), 3.30-3.41 (1H, m), 3.67 (1H, br s), 4.68 (2H, br s), 6.83-6.90 (1H, m), 6.98-7.04 (1H, m), 7.34-7.51 (5H, m). |
| 88E | 1H-NMR (CDCl3) δ: 1.22-1.36 (4.5H, m), 1.44 (4.5H, s), 1.50 (1H, t, J = 9.6 Hz), 1.74-1.85 (0.5H, m), 1.92-2.00 (0.5H, m), 2.24-2.64 (2H, m), 3.02-3.36 (2H, m), 3.46-3.70 (0.5H, m), 3.98-4.06 (0.5H, m), 4.51-4.97 (1.5H, m), 5.36-5.60 (0.5H, m), 6.88-7.09 (2H, m), 7.34-7.46 (3H, m), 7.47-7.54 (2H, m). |
| | MS(ESI/APCI) m/z: 373 [M - tert-butyl + H]+ |
| 88F | 1H-NMR (CDCl3) δ: 1.25-1.31 (10H, m), 2.10 (1H, dd, J = 10.8, 9.2 Hz), 2.17-2.23 (1H, m), 2.31-2.36 (2H, m), 2.88 (1H, dd, J = 14.1, 7.8 Hz), 3.46 (1H, dd, J = 14.1, 6.7 Hz), 3.73 (1H, d, J = 9.0 Hz), 4.51-4.59 (1H, m), 4.63-4.72 (1H, m), 6.94-7.00 (1H, m), 7.06-7.12 (1H, m), 7.35-7.46 (3H, m), 7.48-7.53 (2H, m). |
| 88G | 1H-NMR (COCI3) δ: 1.34 (9H, s), 1.39 (1H, t, J = 9.4 Hz), (1H, m), 2.24-2.32 (1H, m), 2.38-2.45 (1H, m), 2.60 (1H, q, J = 5.6 Hz), 2.67 (3H, s), 2.91 (1H, dd, J = 14.1, 7.0 Hz), 3.34 (1H, dd, J = 13.9, 8.0 Hz), 4.22 (1H, t, J = 9.6 Hz), 4.57 (1H, br s), 4.71-4.85 (2H, m), 6.95-7.02 (1H, m), 7.17 (1H, br s), 7.35-7.41 (1H, m), 7.41-7.47 (2H, m), 7.49-7.54 (2H, m). |
| | MS(ESI/APCI) m/z: 437 [M - tert-butyl + H]+ |
| 88I | 1H-NMR (CD3OD) δ: 1.97-2.06 (1H, m), 2.16 (1H, dd, J = 12.1, 9.4 Hz), 2.41-2.49 (2H, m), 2.87-2.94 (1H, m), 3.01 (3H, s), 3.03-3.09 (1H, m), 3.48 (1H, d, J = 13.7 Hz), 3.87 (1H, q, J = 4.8 Hz), 4.29-4.37 (1H, m), 4.46 (1H, t, J = 5.9 Hz), 7.18-7.27 (2H, m), 7.38-7.50 (3H, m), 7.55-7.60 (2H, m). |
| | MS(ESI/APCI) m/z: 393 [M + H]+ |
| 88J | 1H-NMR (CDCl3) δ: 1.54 (1H, = 10.0 Hz), 2.14 (1H, = 11.3, 9.4 Hz), 2.31 (3H, s), 2.32-2.39 (1H, m), 2.47-2.55 (1H, m), 2.58 (3H, s), 2.64-2.71 (1H, m), 2.92-2.99 (1H, m), 3.36 (1H, dd, J = 14.1, 9.0 Hz), 4.25 (1H, t, J = 9.8 Hz), 4.35 (1H, q, J = 5.0 Hz), 5.02 (1H, d, J = 10.6 Hz), 5.09 (1H, td, J = 9.0, 5.5 Hz), 6.97-7.03 (1H, m), 7.33-7.48 (4H, m), 7.50-7.55 (2H, m). |
| | MS(ESI/APCI) m/z: 463 [M + H]+ |
| 88K | 1H-NMR (CDCl3) δ: 1.48 (1H, t, J = 9.8 Hz), 2.18 (1H, dd, J = 11.3, 9.4 Hz), 2.34-2.42 (1H, m), 2.47-2.54 (1H, m), 2.63 (3H, s), 2.69 (1H, q, J = 5.5 Hz), 2.87 (1H, dd, J = 14.5, 5.9 Hz), 3.33 (1H, dd, J = 14.1, 8.2 Hz), 3.55 (1H, s), 4.19-4.29 (2H, m), 5.00-5.09 (1H, m), 5.25 (1H, dd, J = 15.5, 8.8 Hz), 6.95-7.01 (1H, m), 7.27-7.32 (1H, m), 7.35-7.40 (1H, m), 7.41-7.46 (2H, m), 7.48-7.53 (2H, m). |
| | MS(ESI/APCI) m/z: 469 [M + H]+ |
| 89A | MS(ESI/APCI) m/z: 463 [M + H]+ |
| 89B | MS(ESI/APCI) m/z: 469 [M + H]+ |
| 89C | 1H-NMR (COCI3) δ: 1.94 (1H, t, J = 9.2 Hz), 2.42-2.48 (1H, m), 2.65-2.75 (2H, m), 2.89 (3H, s), 2.99-3.04 (1H, m), 3.37-3.42 (2H, m), 4.14-4.21 (1H, m), 4.38-4.43 (1H, m), 4.91 (1H, d, J = 9.2 Hz), 5.37-5.43 (1H, m), 7.17 (1H, t, J = 7.7 Hz), 7.26-7.30 (1H, m), 7.33-7.37 (1H, m), 7.40-7.44 (3H, m), 7.49-7.50 (2H, m). |
| | MS(ESI/APCI) m/z: 469 [M + H]+ |
| 90A | MS(ESI/APCI) m/z: 477 [M + H]+ |
| 90B | MS(ESI/APCI) m/z: 483 [M + H]+ |
| 90C | 1H-NMR (CDCl3) δ: 1.33 (3H, t, J = 7.3 Hz), 1.92 (1H, t, J = 9.1 Hz), 2.41-2.47 (1H, m), 2.67 (1H, td, J = 10.5, 5.7 Hz), 2.74-2.79 (1H, m), 3.00-3.08 (3H, m), 3.36-3.45 (2H, m), 4.13-4.20 (1H, m), 4.35-4.39 (1H, m), 4.82 (1H, d, J = 9.1 Hz), 5.38-5.45 (1H, m), 7.17 (1H, t, J = 7.3 Hz), 7.26-7.30 (1H, m), 7.33-7.37 (1H, m), 7.40-7.44 (3H, m), 7.48-7.50 (2H, m). |
| | MS(ESI/APCI) m/z: 483 [M + H]+ |
| 91A | MS(ESI/APCI) m/z: 379, 381 [M - tert-butyl + H]+ |
| 91B | MS(ESI/APCI) m/z: 377 [M - tert-butyl + H]+ |
| 91C | MS(ESI/APCI) m/z: 423 [M - Boc + H]+ |
| 91D | MS(ESI/APCI) m/z: 425 [M - Boc + H]+ |
| 91E | MS(ESI/APCI) m/z: 425 [M + H]+ |
| 91F | MS(ESI/APCI) m/z: 539 [M + H]+ |
| 91G | 1H-NMR (CDCl3) δ: 0.9/-1.08 (3H, m), 1.29-1.39 (3H, m), 1.86-1.98 (1H, m), 2.39-2.50 (1H, m), 2.61-2.76 (2H, m), 2.89-3.14 (3H, m), 3.35-3.47 (2H, m), 4.10-4.29 (2H, m), 4.30-4.41 (1H, m), 4.89-4.99 (1H, m), 5.30-5.44 (1H, m), 6.91-7.00 (1H, m), 7.07-7.15 (1H, m), 7.32-7.50 (5H, m). MS(ESI/APCI) m/z: 497 [M + H]+ |
| 92A | 1H-NMR (CDCl3) δ: 1.22-1.31 (12H, m), 1.36 (1H, t, J = 9.6 Hz), 2.08 (1H, t, J = 10.4 Hz), 2.23-2.32 (1H, m), 2.37-2.45 (1H, m), 2.58 (1H, q, J = 5.5 Hz), 2.80-3.05 (3H, m), 3.35 (1H, dd, J = 13.7, 5.9 Hz), 4.17 (1H, t, J = 10.2 Hz), 4.53 (1H, br s), 4.68-4.80 (2H, m), 7.00-7.16 (2H, m). |
| | MS(ESI/APCI) m/z: 453, 455 [M - tert-butyl + H]+ |
| 92B | 1H-NMR (CDCl3) δ: 1.18-1.33 (12H, m), 1.37 (1H, t, J = 9.6 Hz), 2.11 (1H, t, J = 10.2 Hz), 2.23-2.31 (1H, m), 2.36-2.45 (1H, m), 2.60 (1H, q, J = 5.5 Hz), 2.78-2.96 (3H, m), 3.37 (1H, dd, J = 13.7, 7.0 Hz), 4.18 (1H, t, J = 9.8 Hz), 4.56 (1H, br s), 4.79 (2H, d, J = 10.6 Hz), 6.94-7.01 (1H, m), 7.12 (1H, br s), 7.35-7.40 (1H, m), 7.41-7.46 (2H, m), 7.47-7.54 (2H, m). |
| | MS(ESI/APCI) m/z: 451 [M - tert-butyl + H]+ |
| 92C | 1H-NMR (CD3OD) δ: 1.34 (3H, t, J = 7.4 Hz), 1.99-2.06 (1H, m), 2.10-2.17 (1H, m), 2.40-2.50 (2H, m), 2.84-2.91 (1H, m), 2.99-3.18 (3H, m), 3.45-3.53 (1H, m), 3.86 (1H, q, J = 5.0 Hz), 4.29-4.37 (1H, m), 4.44 (1H, t, J = 6.1 Hz), 7.19-7.27 (2H, m), 7.38-7.50 (3H, m), 7.55-7.60 (2H, m). |
| | MS(ESI/APCI) m/z: 407 [M + H]+ |
| 92D | 1H-NMR (CDCl3) δ: 1.15 (3H, t, J = 7.4 Hz), 1.52 (1H, t, J = 9.8 Hz), 2.18 (1H, dd, J = 11.3, 9.0 Hz), 2.27 (3H, s), 2.29-2.36 (1H, m), 2.45-2.52 (1H, m), 2.64-2.71 (1H, m), 2.72-2.84 (2H, m), 2.95 (1H, dd, J = 14.9, 5.9 Hz), 3.40 (1H, dd, J = 14.1, 8.2 Hz), 4.20 (1H, t, J = 9.8 Hz), 4.30 (1H, q, J = 5.0 Hz), 5.00-5.14 (2H, m), 6.96-7.02 (1H, m), 7.29-7.35 (1H, m), 7.35-7.55 (5H, m). |
| | MS(ESI/APCI) m/z: 477 [M + H]+ |
| 92E | MS(ESI/APCI) m/z: 483 [M + H]+ |
| 92F | 1H-NMR (CDCl3) δ: 1.18 (3H, t, J = 7.8 Hz), 1.44-1.51 (1H, m), 2.17-2.25 (1H, m), 2.34-2.43 (1H, m), 2.52 (1H, dd, J = 10.4, 5.3 Hz), 2.68-2.93 (4H, m), 3.38 (1H, dd, J = 14.1, 7.8 Hz), 3.55 (1H, s), 4.16-4.29 (2H, m), 4.79 (1H, d, J = 10.6 Hz), 5.22-5.32 (1H, m), 6.96-7.03 (1H, m), 7.28-7.33 (1H, m), 7.36-7.55 (5H, m). |
| 93A | 1H-NMR (CDCl3) δ: 1.40-1.52 (1H, m), 1.59-1.69 (3H, m), 2.00-2.24 (2H, m), 2.26-3.38 (4H, m), 2.54-2.69 (1H, m), 2.83-3.05 (1H, m), 3.23-3.40 (1H, m), 4.04-4.25 (1H, m), 4.55-4.70 (1H, m), 4.86-4.99 (1H, m), 5.13-5.25 (1H, m), 5.32-5.44 (1H, m), 6.22-6.32 (1H, m), 7.17-7.24 (1H, m), 7.27-7.32 (1H, m), 7.35-7.40 (1H, m), 7.42-7.51 (4H, m), 7.53-7.58 (2H, m), 7.72-7.82 (1H, m). |
| | MS(ESI/APCI) m/z: 497 [M + H]+ |
| 94A | MS(ESI/APCI) m/z: 459 [M + H]+ |
| 94B | MS(ESI/APCI) m/z: 465 [M + H]+ |
| 94C | 1H-NMR (CDCl3) δ: 1.11 (3H, t, J = 7.4 Hz), 1.46 (1H, t, J = 9.5 Hz), 2.23 (1H, dd, J = 11.7, 9.5 Hz), 2.35-2.40 (1H, m), 2.47-2.52 (1H, m), 2.68-2.76 (3H, m), 2.93 (1H, dd, J = 14.3, 5.8 Hz), 3.36 (1H, dd, J = 14.3, 8.6 Hz), 3.55 (1H, s), 4.17-4.26 (2H, m), 4.72 (1H, d, J = 10.4 Hz), 5.27-5.32 (1H, m), 7.20 (1H, t, J = 7.7 Hz), 7.27-7.31 (1H, m), 7.35-7.38 (1H, m), 7.42-7.46 (2H, m), 7.51-7.53 (2H, m), 7.57-7.61 (1H, m). |
| | MS(ESI/APCI) m/z: 465 [M + H]+ |
| 95A | MS(ESI/APCI) m/z: 495 [M + H]+ |
| 95B | MS(ESI/APCI) m/z: 501 [M + H]+ |
| 95C | 1H-NMR (CDCI3) δ: 1.30-1.40 (3H, m), 1.87-1.98 (1H, m), 2.40-2.51 (1H, m), 2.63-2.76 (2H, m), 2.91-3.13 (3H, m), 3.31-3.49 (2H, m), 4.10-4.23 (1H, m), 4.29-4.42 (1H, m), 4.89-4.99 (1H, m), 5.30-5.44 (1H, m), 6.92-7.02 (1H, m), 7.07-7.16 (1H, m), 7.33-7.52 (5H, m) |
| | MS(ESI/APCI) m/z: 501 [M + H]+ |
| 96A | MS(ESI/APCI) m/z: 423 [M + H]+ |
| 96B | MS(ESI/APCI) m/z: 537 [M + H]+ |
| 96C | 1H-NMR (CDCl3) δ: 0.98-1.08 (3H, m), 1.87-1.96 (1H, m), 2.40-49 (1H, m), 2.61-2.80 (2H, m), 2.92-3.05 (1H, m), 3.36-3.54 (2H, m), 4.11-4.31 (3H, m), 4.92-5.03 (1H, m), 5.30-5.43 (1H, m), 5.92-6.01 (1H, m), 6.24-6.34 (1H, m), 6.35-6.45 (1H, m), 6.94-7.03 (1H, m), 7.09-7.18 (1H, m), 7.34-7.56 (5H, m) |
| | MS(ESI/APCI) m/z: 495 [M + H]+ |
| 97A | 1H-NMR (CDCl3) δ: 1.16 (3H, s), 1.45-1.52 (1H, m), 2.15-2.27 (1H, m), 2.36-2.43 (1H, m), 2.45-2.56 (4H, m), 2.67-2.75 (1H, m), 2.93 (1H, dd, J = 13.8, 5.2 Hz), 3.34 (1H, dd, J = 14.1, 9.2 Hz), 3.58 (1H, s), 4.19-4.31 (2H, m), 4.90 (1H, d, J = 11.7 Hz), 5.24-5.33 (1H, m), 7.17-7.25 (1H, m), 7.28-7.40 (2H, m), 7.41-7.48 (2H, m), 7.51-7.57 (2H, m), 7.59-7.65 (1H, |
| | MS(ESI/APCI) m/z: 448 [M + H]+ |
| 98A | 1H-NMR (CDCl3) δ: 1.16 (3H, s), 1.45-1.53 (1H, m), 2.14-2.24 (1H, m), 2.34-2.44 (1H, m), 2.48-2.57 (1H, m), 2.64 (3H, s), 2.66-2.75 (1H, m), 2.84-2.93 (1H, m), 3.29-3.40 (1H, m), 3.56 (1H, s), 4.19-4.32 (2H, m), 5.00-5.10 (1H, m), 5.21-5.32 (1H, m), 6.96-7.03 (1H, m), 7.27-7.35 (1H, m), 7.36-7.57 (5H, m). |
| | MS(ESI/APCI) m/z: 466 [M + H]+ |
| 99A | MS(ESI/APCI) m/z: M + H]+ |
| 99B | 1H-NMR (CDCl3) δ: 1.01 (3H, s), 1.94 (1H, t, J = 9.2 Hz), 2.42-2.48 (1H, m), 2.65-2.75 (2H, m), 2.89 (3H, s), 2.99-3.04 (1H, m), 3.37-3.42 (2H, m), 4.14-4.21 (1H, m), 4.38-4.43 (1H, m), 4.91 (1H, d, J = 9.2 Hz), 5.32-5.46 (1H, m), 7.17 (1H, t, J = 7.7 Hz), 7.26-7.30 (1H, m), 7.31-7.38 (1H, m), 7.40-7.44 (3H, m), 7.49-7.53 (2H, m). |
| | MS(ESI/APCI) m/z: 466 [M + H]+ |
| 100A | MS(ESI/APCI) m/z: 484 [M + H]+ |
| 100B | 1H-NMR (CDCI3) δ: 1.19-1.35 (3H, m), 1.87-2.01 (1H, m), 2.41-2.64 (1H, m), 2.69-2.85 (2H, m), 2.90-2.99 (4H, m), 3.30-3.44 (2H, m), 4.11-4.24 (1H, m), 4.32-4.45 (1H, m), 5.08-5.19 (1H, m), 5.28-5.42 (1H, m), 6.92-7.01 (1H, m), 7.10-7.17 (1H, m), 7.32-7.53 (5H, m). |
| | MS(ESI/APCI) m/z: 484 [M + H]+ |

Table 5 below shows the structural formula of the compound obtained in each Example.

**[Table 5]**

| Ex | Structure | Ex | Structure | Ex | Structure |
|---|---|---|---|---|---|
| 1A | 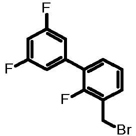 | 1B | 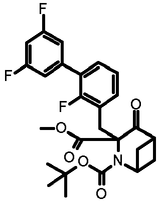 | 1C | 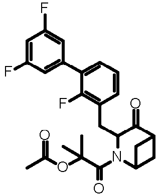 |
| 1D | 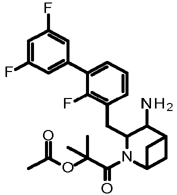 | 1E | 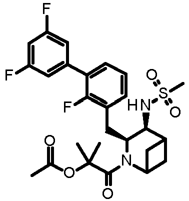 racemate | 1G | 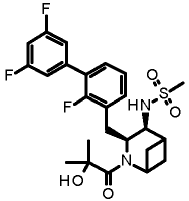 |
| 2A | 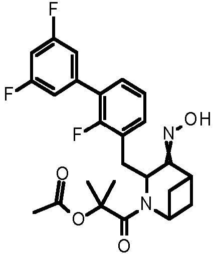 | 2B | 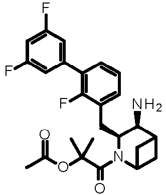 racemate | 2C | 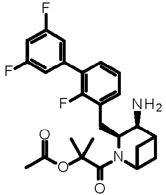 |
| 2D | 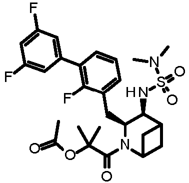 | 2E | 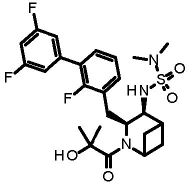 | 3A | 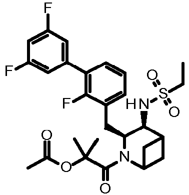 |
| 3B | 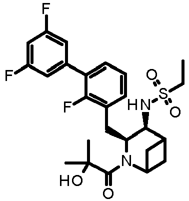 | 4A | 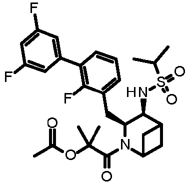 | 4B | 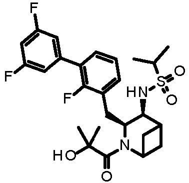 |
| 5A | 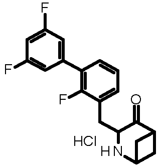 | 5B | 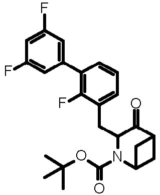 | 5C | 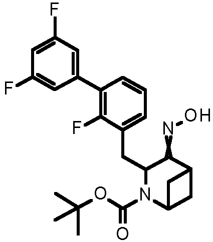 |
| 5D | 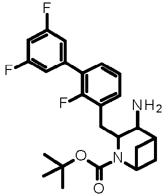 | 5F | 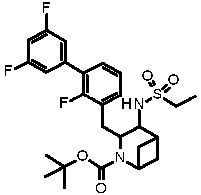 | 5G | 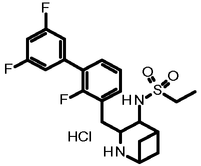 |
| 5H | 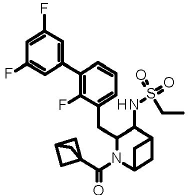 | 6A | 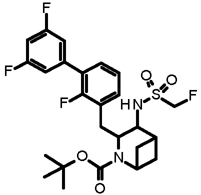 | 6B | 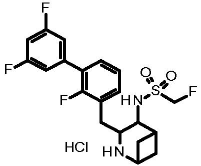 |
| 6C | 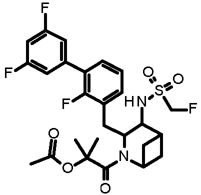 | 6D | 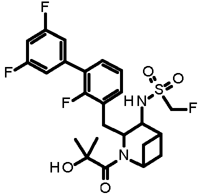 | 7A | 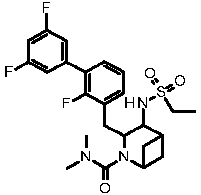 |
| 8A | 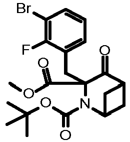 | 8B | 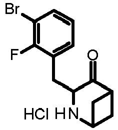 | 8C | 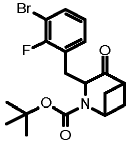 |
| 8D | 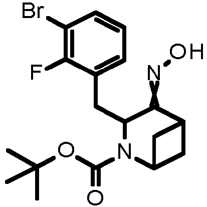 | 8F | 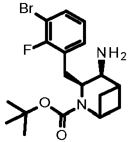 | 8G | 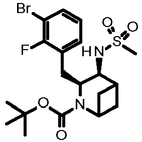 |
| 8I | 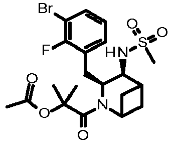 | 8J | 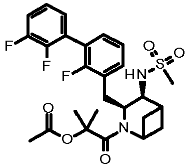 | 8K | 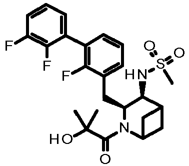 |
| 9A | 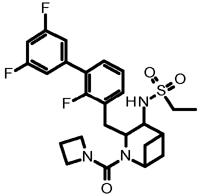 | 10A | 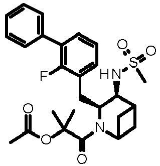 | 10B | 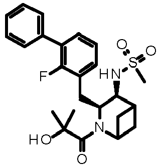 |
| 11A | 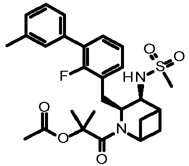 | 11B | 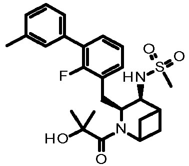 | 12B | 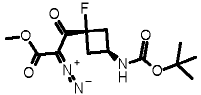 |
| 12C | 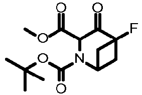 | 12D | 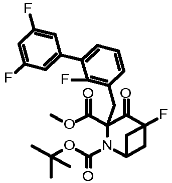 | 12E | 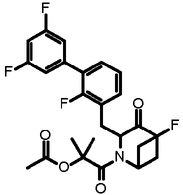 |
| 12F | 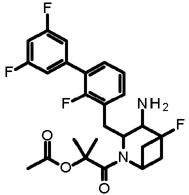 | 12G | 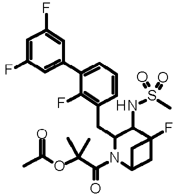 | 12H | 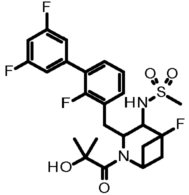 |
| 121 | 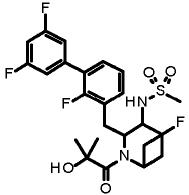 | 13A | 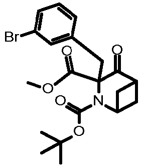 | 13B | 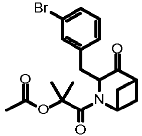 |
| 13C | 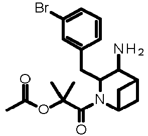 | 13D | 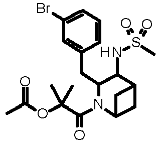 | 13E | 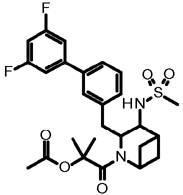 |
| 13F | 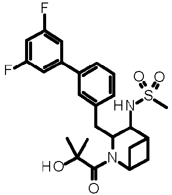 | 14A | 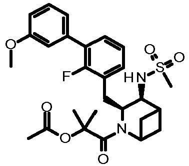 | 14B | 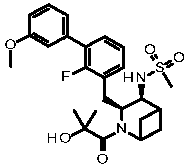 |
| 15A | 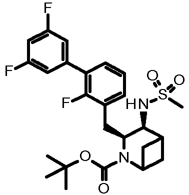 | 15B | 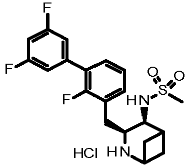 | 15C | 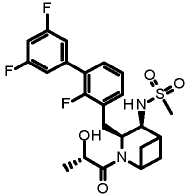 |
| 16A | 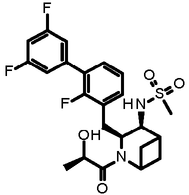 | 17A | 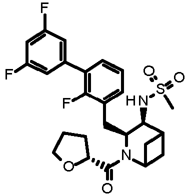 | 18A | 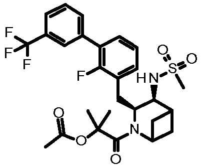 |
| 18B | 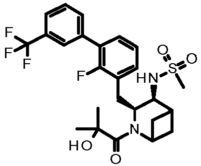 | 19A | 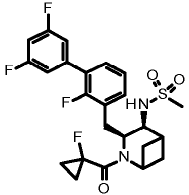 | 20A | 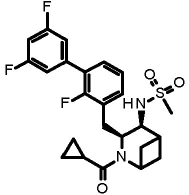 |
| 21A | 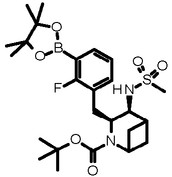 | 21B | 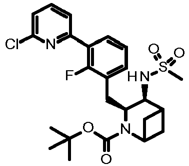 | 21C | 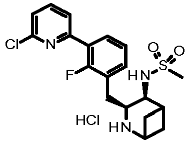 |
| 21D | 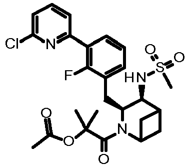 | 21E | 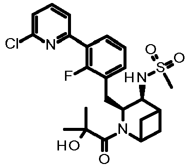 | 22A | 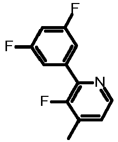 |
| 22B | 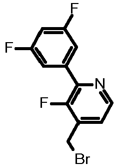 | 22C | 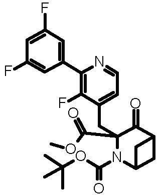 | 22D | 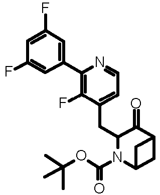 |
| 22E | 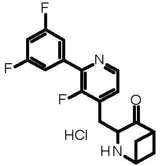 | 22F | 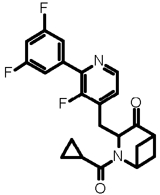 | 22G | 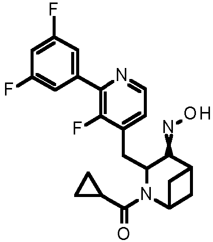 |
| 22H | 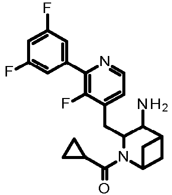 | 22I | 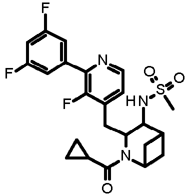 | 22J | 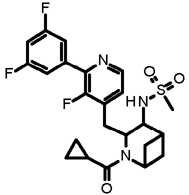 |
| 23A | 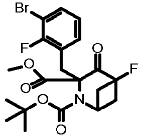 | 23B | 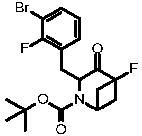 | 23C | 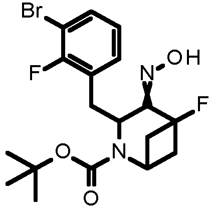 |
| 23D | 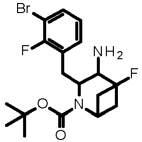 | 23E | 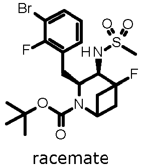 | 23F | 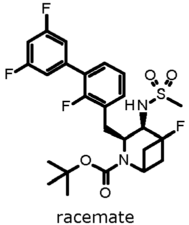 |
| 23G | 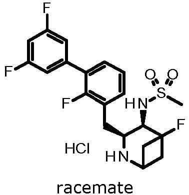 | 23H | 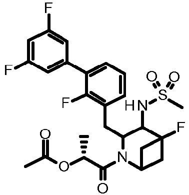 | 23I | 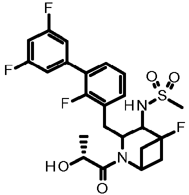 |
| 23J | 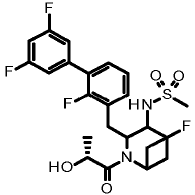 | 24A | 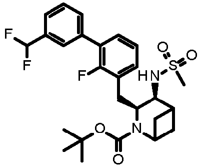 | 24B | 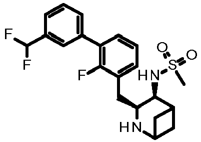 |
| 24C | 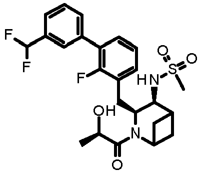 | 25A | 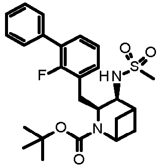 | 25B | 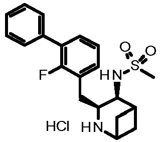 |
| 25C | 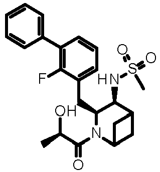 | 26A | 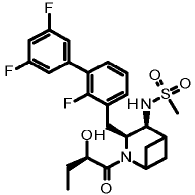 | 27A | 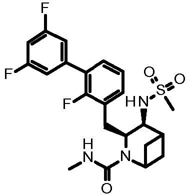 |
| 28A | 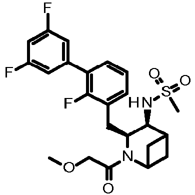 | 29A | 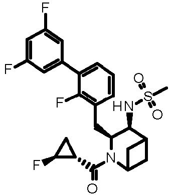 | 30A | 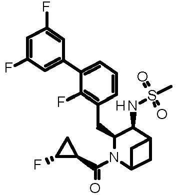 |
| 31A | 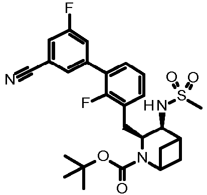 | 31B | 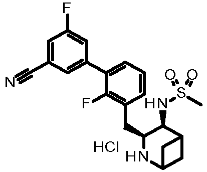 | 31C | 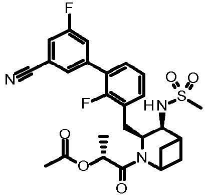 |
| 31D | 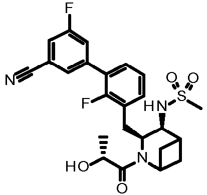 | 32A | 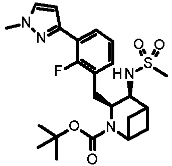 | 32B | 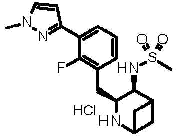 |
| 32C | 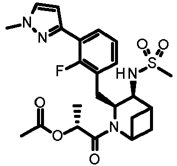 | 32D | 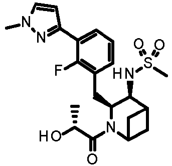 | 33A | 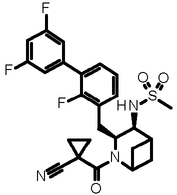 |
| 34A | 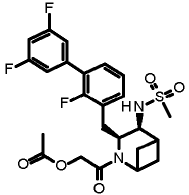 | 34B | 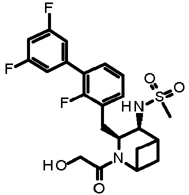 | 35A | 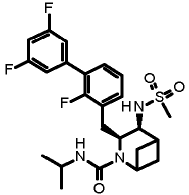 |
| 36A | 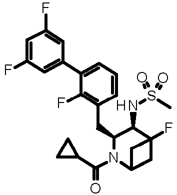 racemate | 36B | 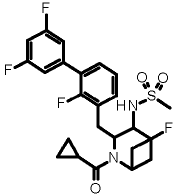 | 37A | 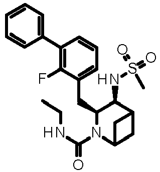 |
| 38A | 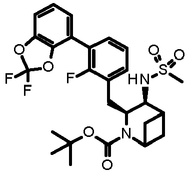 | 38B | 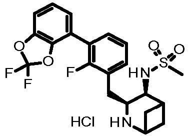 | 38C | 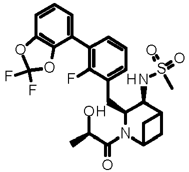 |
| 39A | 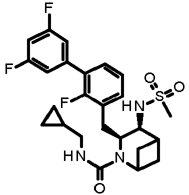 | 40A | 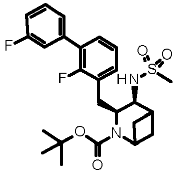 | 40B | 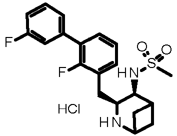 |
| 40C | 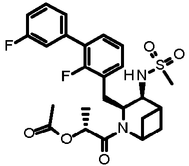 | 40D | 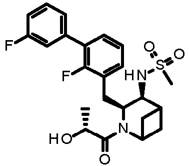 | 41A | 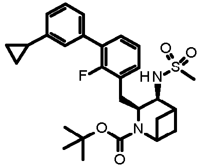 |
| 41B | 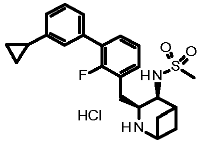 | 41C | 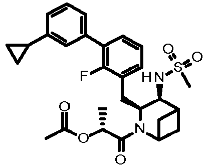 | 41D | 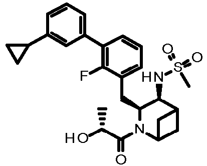 |
| 42A | 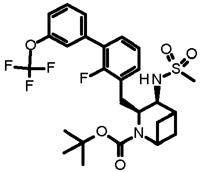 | 42B | 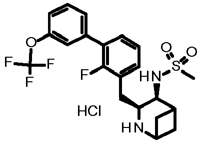 | 42C | 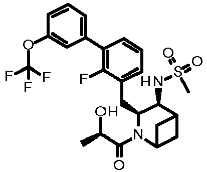 |
| 43A | 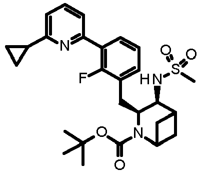 | 43B | 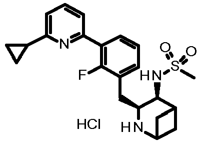 | 43C | 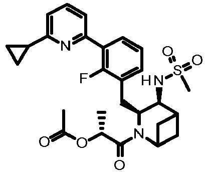 |
| 43D | 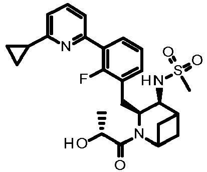 | 44B | 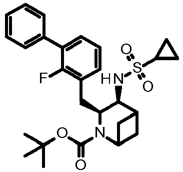 | 44C | 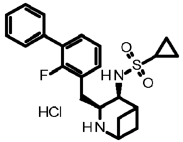 |
| 44D | 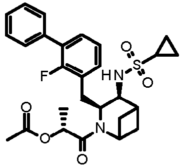 | 44E | 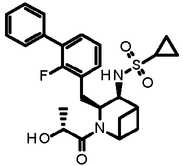 | 45A | 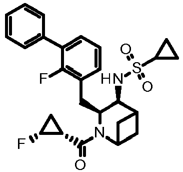 |
| 46B | 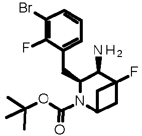 | 46C | 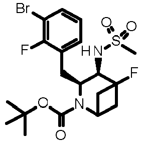 | 46D | 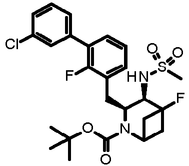 |
| 46E | 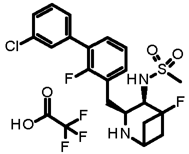 | 46F | 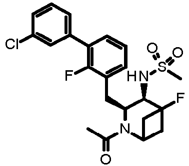 | 47A | 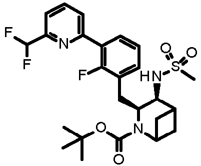 |
| 47B | 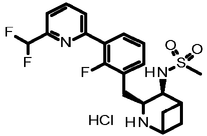 | 47C | 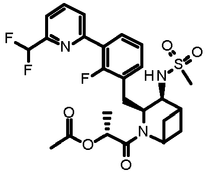 | 47D | 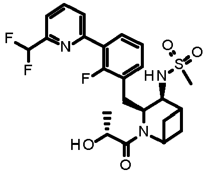 |
| 48A | 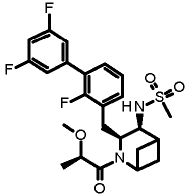 | 49A | 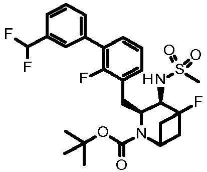 | 49B | 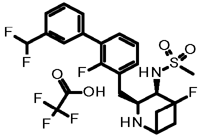 |
| 49C | 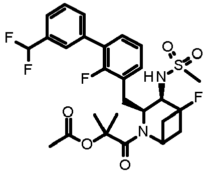 | 49D | 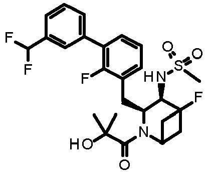 | 50A | 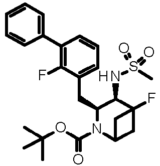 |
| 50B | 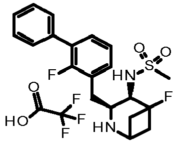 | 50C | 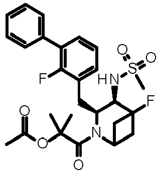 | 50D | 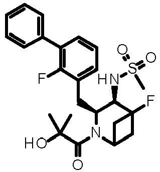 |
| 51A | 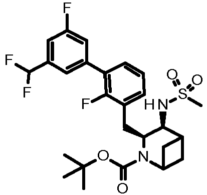 | 51B | 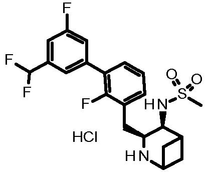 | 51C | 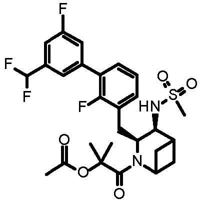 |
| 51D | 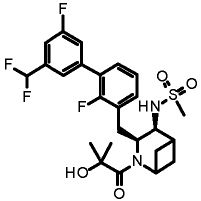 | 52A | 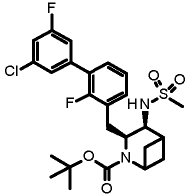 | 52B | 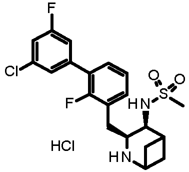 |
| 52C | 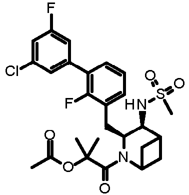 | 52D | 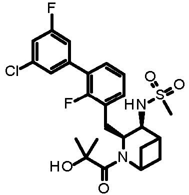 | 53A | 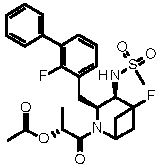 |
| 53B | 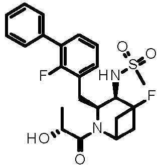 | 54A | 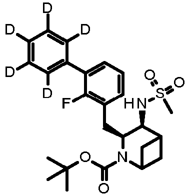 | 54C | 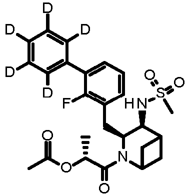 |
| 54D | 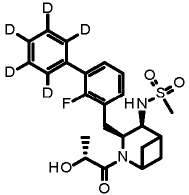 | 55A | 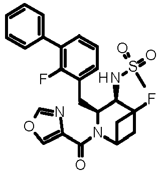 | 56A | 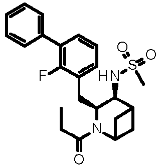 |
| 57A | 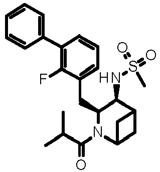 | 58A | 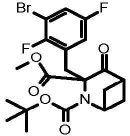 | 58B | 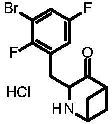 |
| 58C | 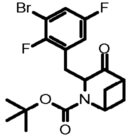 | 58D | 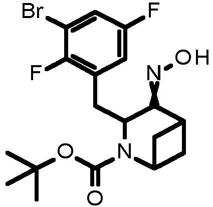 | 58E | 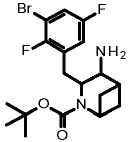 |
| 58F | 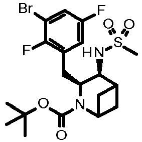 racemate | 58G | 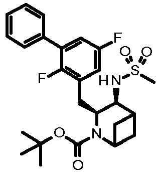 racemate | 58H | 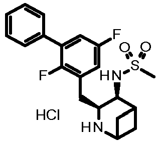 racemate |
| 58I | 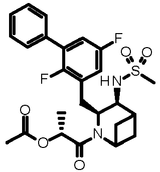 | 58J | 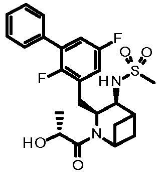 | 59A | 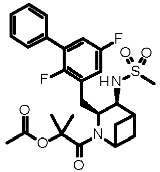 racemate |
| 59B | 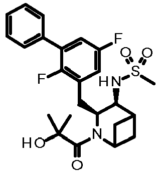 racemate | 59C | 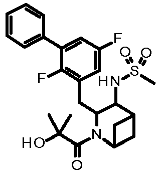 | 60A | 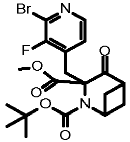 |
| 60B | 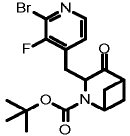 | 60C | 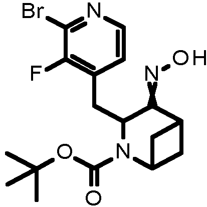 | 60D | 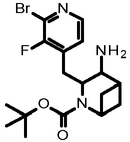 |
| 60E | 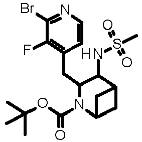 | 60F | 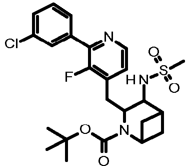 | 60H | 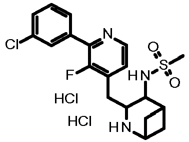 |
| 60I | 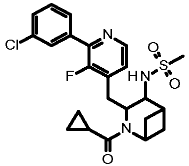 | 61A | 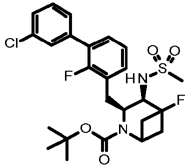 racemate | 61B | 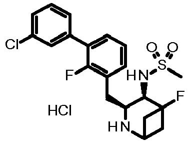 racemate |
| 61C | 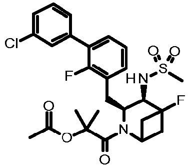 racemate | 61D | 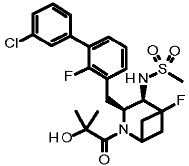 racemate | 61E | 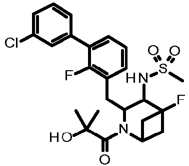 |
| 62A | 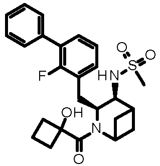 | 63A | 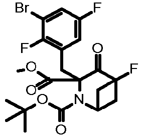 | 63B | 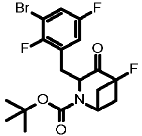 |
| 63C | 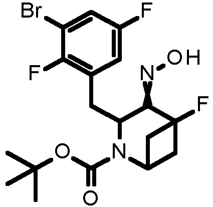 | 63D | 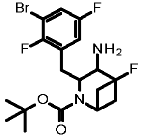 | 63E | 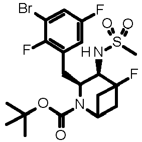 racemate |
| 63F | 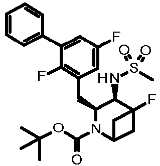 racemate | 63G | 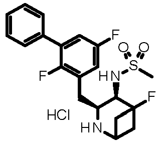 racemate | 63H | 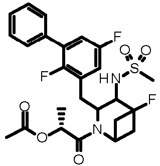 |
| 63I | 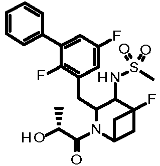 | 63J | 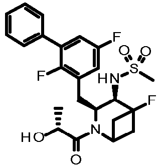 | 64A | 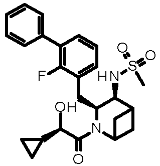 |
| 65A | 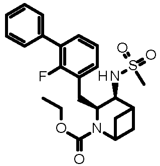 | 66A | 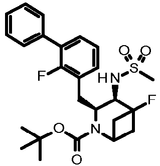 racemate | 66B | 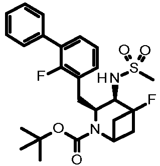 |
| 66C | 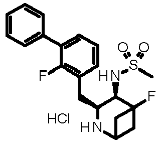 | 66D | 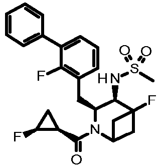 | 67A | 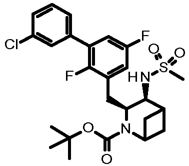 racemate |
| 67B | 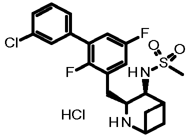 racemate | 67C | 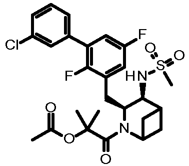 racemate | 67E | 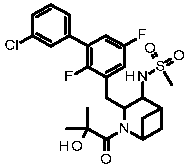 |
| 68A | 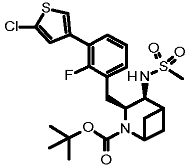 | 68B | 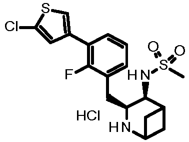 | 68C | 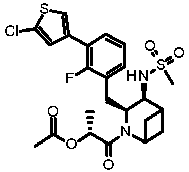 |
| 68D | 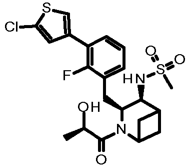 | 69A | 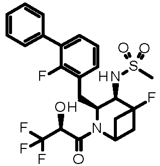 | 70A | 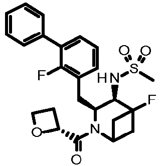 |
| 71A | 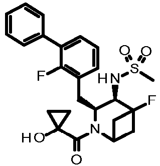 | 72A | 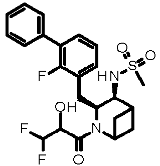 | 72B | 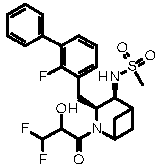 |
| 73A | 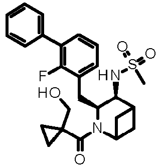 | 74A | 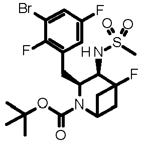 | 74B | 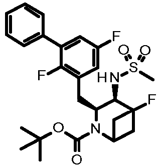 |
| 74C | 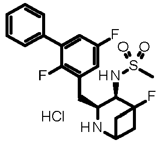 | 74D | 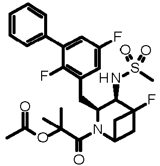 | 74E | 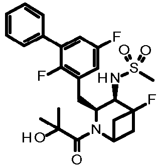 |
| 75A | 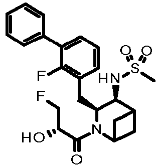 | 76A | 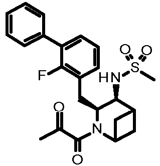 | 76B | 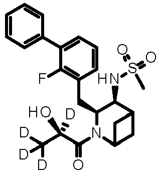 |
| 77A | 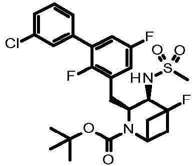 | 77B | 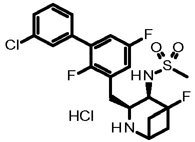 | 77C | 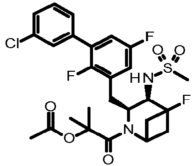 |
| 77D | 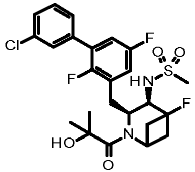 | 78A | 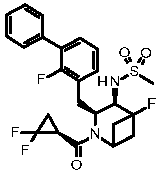 | 79A | 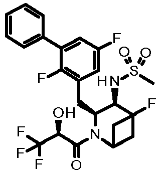 |
| 80A | 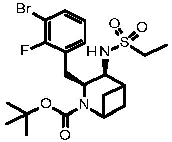 | 80B | 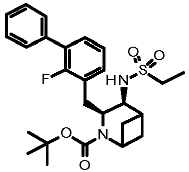 | 80C | 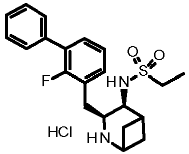 |
| 80D | 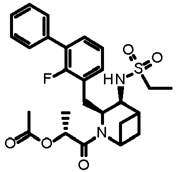 | 80E | 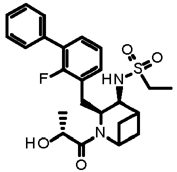 | 81A | 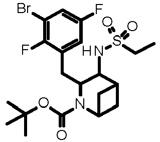 |
| 81B | 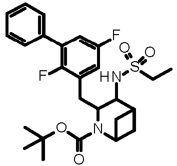 | 81C | 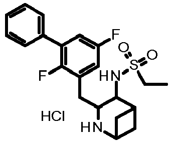 | 81D | 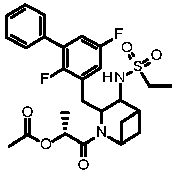 |
| 81E | 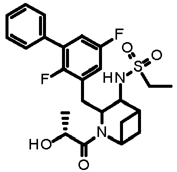 | 81F | 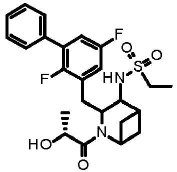 | 82A | 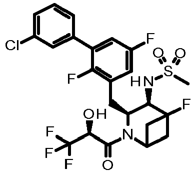 |
| 83A | 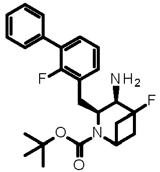 racemate | 83B | 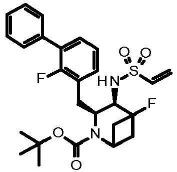 racemate | 83C | 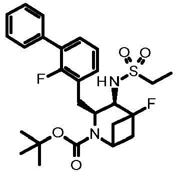 racemate |
| 83E | 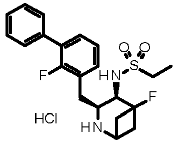 | 83F | 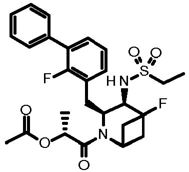 | 83G | 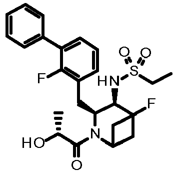 |
| 84A | 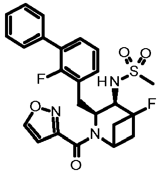 | 85A | 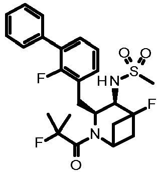 | 86A | 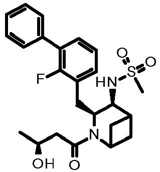 |
| 87A | 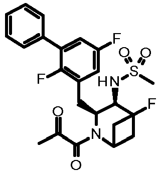 | 87B | 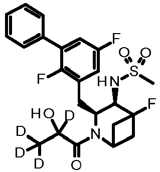 | 87C | 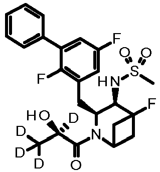 |
| 88A | 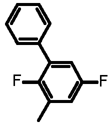 | 88B | 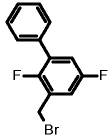 | 88C | 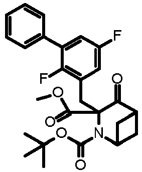 |
| 88D | 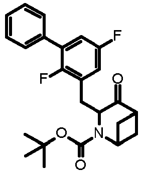 | 88E | 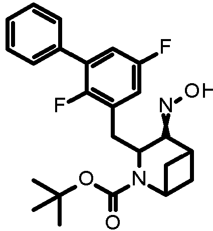 | 88F | 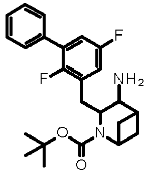 |
| 88G | 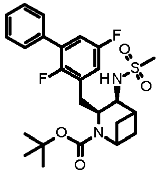 racemate | 88I | 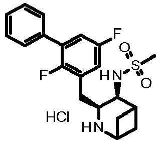 | 88J | 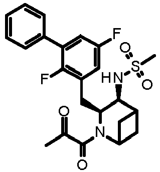 |
| 88K | 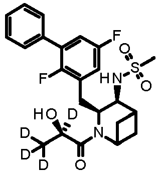 | 89A | 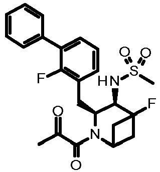 | 89B | 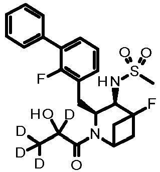 |
| 89C | 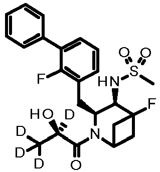 | 90A | 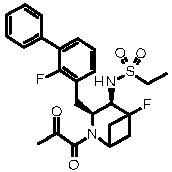 | 90B | 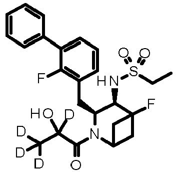 |
| 90C | 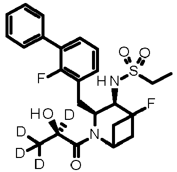 | 91A | 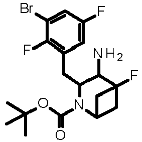 | 91B | 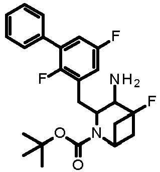 |
| 91C | 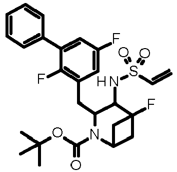 | 91D | 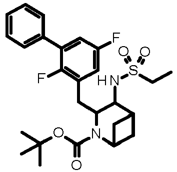 | 91E | 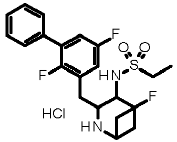 |
| 91F | 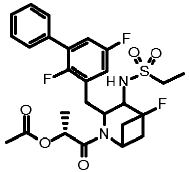 | 91G | 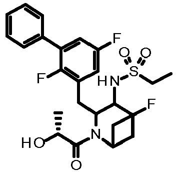 | 92A | 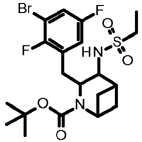 |
| 92B | 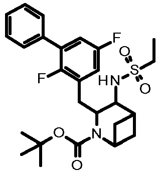 | 92C | 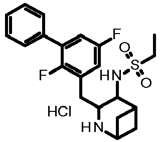 | 92D | 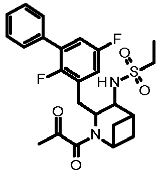 |
| 92E | 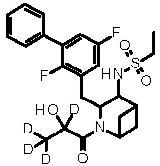 | 92F | 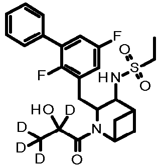 | 93A | 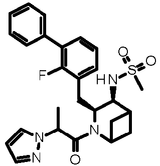 |
| 94A | 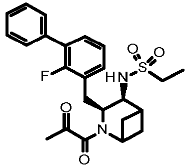 | 94B | 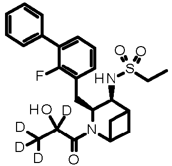 | 94C | 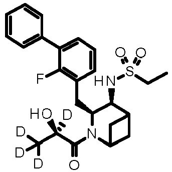 |
| 95A | 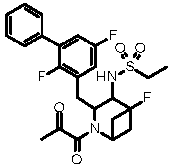 | 95B | 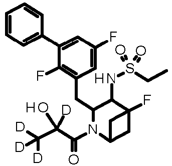 | 95C | 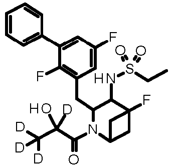 |
| 96A | 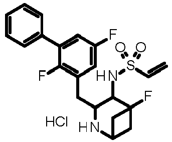 | 96B | 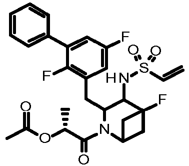 | 96C | 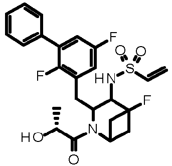 |
| 97A | 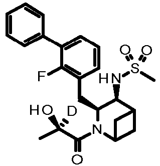 | 98A | 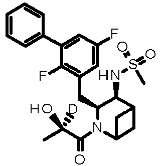 | 99A | 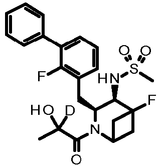 |
| 99B | 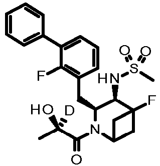 | 100 A | 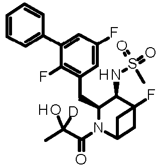 | 100 B | 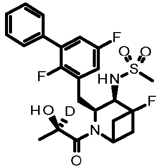 |

## Claims

1. A compound represented by the following general formula (I) or pharmaceutically acceptable salt thereof: 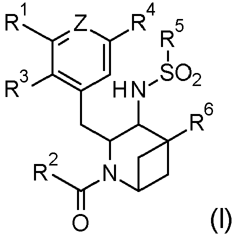 wherein
R¹ represents a phenyl group or pyridyl group optionally having 1 or 2 substituents independently selected from group A below, a phenyl group substituted with 5 deuterium atoms, a 2,2-difluoro-1,3-benzodioxolyl group, or a 5-membered heteroaryl group containing 1 or 2 atoms independently selected from the group consisting of a nitrogen atom and a sulfur atom in a ring;
the heteroaryl group optionally has 1 or 2 substituents independently selected from group B below;
R² represents a C₁-C₆ alkyl group optionally having 1 to 5 substituents independently selected from group C below, a C₃-C₆ cycloalkyl group optionally having 1 or 2 substituents selected from group D below, a 4- or 5-membered saturated heterocyclic group containing 1 atom selected from the group consisting of a nitrogen atom and an oxygen atom in a ring, a 5-membered heteroaryl group containing 1 or 2 atoms independently selected from the group consisting of a nitrogen atom and an oxygen atom in a ring, a C₁-C₆ alkoxy group, a C₁-C₆ alkylamino group, a di-C₁-C₆ alkylamino group, or a bicyclopentyl group;
R³ represents a hydrogen atom or a halogen atom;
R⁴ represents a hydrogen atom or a halogen atom;
R⁵ represents a C₁-C₆ alkyl group optionally substituted with 1 to 3 halogen atoms, a C₃-C₆ cycloalkyl group, a di-C₁-C₆ alkylamino group, or a vinyl group;
R⁶ represents a hydrogen atom or a halogen atom; and
Z represents CH or a nitrogen atom.
Group A: a halogen atom, a C₁-C₆ alkyl group optionally substituted with 1 to 3 halogen atoms, a C₁-C₆ alkoxy group optionally substituted with 1 to 3 halogen atoms, a C₃-C₆ cycloalkyl group, and a cyano group,
Group B: a halogen atom and a C₁-C₆ alkyl group
Group C: a hydroxy group, a halogen atom, a deuterium atom, a C₁-C₆ alkoxy group, a C₃-C₆ cycloalkyl group, and a pyrazolyl group
Group D: a halogen atom, a C₁-C₆ alkyl group optionally substituted with 1 to 3 hydroxy groups, a cyano group, and a hydroxy group

2. The compound or pharmaceutically acceptable salt thereof according to claim 1,
wherein, in the above formula (I),
R¹ represents a phenyl group or pyridyl group optionally having 1 or 2 substituents independently selected from the group consisting of a fluorine atom, a chlorine atom, a difluoromethyl group, a trifluoromethyl group, and a cyclopropyl group.

3. The compound or pharmaceutically acceptable salt thereof according to claim 1 or 2,
wherein, in the above formula (I),
R² represents a C₁-C₆ alkyl group optionally having 1 to 5 substituents independently selected from the group consisting of a hydroxy group, a fluorine atom, and a deuterium atom, a cyclopropyl group optionally substituted with 1 or 2 fluorine atoms, or an isoxazolyl group.

4. The compound or pharmaceutically acceptable salt thereof according to any one of claims 1 to 3,
wherein, in the above formula (I),
R³ represents a fluorine atom;
R⁴ represents a hydrogen atom or a fluorine atom; and
Z represents CH or a nitrogen atom.

5. The compound or pharmaceutically acceptable salt thereof according to any one of claims 1 to 4,
wherein, in the above formula (I),
R⁵ represents a methyl group, an ethyl group, a monofluoromethyl group, or a cyclopropyl group; and
R⁶ represents a hydrogen atom or a fluorine atom.

6. The compound or pharmaceutically acceptable salt thereof according to any one of claims 1 to 5,
wherein, in the above formula (I),
R¹ represents the following formula (II): 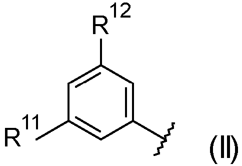 wherein
R¹¹ and R¹² each independently represent a hydrogen atom, a fluorine atom, or a chlorine atom;
R² is any one of the following R^{2a} to R^{2e}: 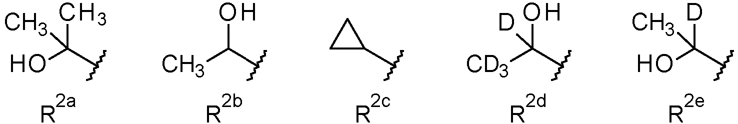
R³, R⁴, and R⁶ each independently represent a hydrogen atom or a fluorine atom;
R⁵ represents a methyl group or an ethyl group; and
Z represents CH or a nitrogen atom.

7. A compound or pharmaceutically acceptable salt thereof, which is any one selected from the following group:
N-{(3S,4S)-3-[(2-fluoro[biphenyl]-3-yl)methyl]-2-[(2R)-2-hydroxy(²H₄)propanoyl]-2-azabicyclo[3.1.1]heptan-4-yl}methanesulfonamide;
N-{(3S,4R)-3-[(2,5-difluoro[biphenyl]-3-yl)methyl]-5-fluoro-2-[(2R)-2-hydroxy(²H₄)propanoyl]-2-azabicyclo[3.1.1]heptan-4-yl}methanesulfonamide;
N-{(3S,4S)-3-[(2,5-difluoro[biphenyl]-3-yl)methyl]-2-[(2R)-2-hydroxy(²H₄)propanoyl]-2-azabicyclo[3.1.1]heptan-4-yl}methanesulfonamide;
N-{(3S,4R)-5-fluoro-3-[(2-fluoro[biphenyl]-3-yl)methyl]-2-[(2R)-2-hydroxy(²H₄)propanoyl]-2-azabicyclo[3.1.1]heptan-4-yl}methanesulfonamide;
N-{(3S,4S)-3-[(2-fluoro[biphenyl]-3-yl)methyl]-2-[(2R)-2-hydroxy(2-²H)propanoyl]-2-azabicyclo[3.1.1]heptan-4-yl}methanesulfonamide;
N-{(3S,4S)-3-[(2,5-difluoro[biphenyl]-3-yl)methyl]-2-[(2R)-2-hydroxy(2-²H)propanoyl]-2-azabicyclo[3.1.1]heptan-4-yl}methanesulfonamide;
N-{(3S,4R)-5-fluoro-3-[(2-fluoro[biphenyl]-3-yl)methyl]-2-[(2R)-2-hydroxy(2-²H)propanoyl]-2-azabicyclo[3.1.1]heptan-4-yl}methanesulfonamide;
and
N-{(3S,4R)-3-[(2,5-difluoro[biphenyl]-3-yl)methyl]-5-fluoro-2-[(2R)-2-hydroxy(2-²H)propanoyl]-2-azabicyclo[3.1.1]heptan-4-yl}methanesulfonamide.

8. A compound or pharmaceutically acceptable salt thereof, which is any one selected from the following group:
N-{(3S,4S)-3-[(2-fluoro[biphenyl]-3-yl)methyl]-2-[(2R)-2-hydroxy(2-²H)propanoyl]-2-azabicyclo[3.1.1]heptan-4-yl}methanesulfonamide;
and
N-{(3S,4S)-3-[(2,5-difluoro[biphenyl]-3-yl)methyl]-2-[(2R)-2-hydroxy(2-²H)propanoyl]-2-azabicyclo[3.1.1]heptan-4-yl}methanesulfonamide.

9. A pharmaceutical composition comprising the compound or pharmaceutically acceptable salt thereof according to any one of claims 1 to 8.

10. An orexin type 2 receptor agonist comprising the compound or pharmaceutically acceptable salt thereof according to any one of claims 1 to 8.

11. A prophylactic or therapeutic agent for narcolepsy, comprising the compound or pharmaceutically acceptable salt thereof according to any one of claims 1 to 8.

12. A method for preventing or treating narcolepsy, comprising administering the compound or pharmaceutically acceptable salt thereof according to any one of claims 1 to 8 in an effective amount.

13. The compound or pharmaceutically acceptable salt thereof according to any one of claims 1 to 8, for use in prevention or treatment of narcolepsy.

14. Use of the compound or pharmaceutically acceptable salt thereof according to any one of claims 1 to 8, in production of a medicament for prevention or treatment of narcolepsy.
